(19) Europäisches Patentamt
European Patent Office
Office européen des brevets

(11) EP 1 098 979 B1

# (12) EUROPEAN PATENT SPECIFICATION

(45) Date of publication and mention of the grant of the patent:
**14.03.2007 Bulletin 2007/11**

(21) Application number: **99923439.6**

(22) Date of filing: **22.04.1999**

(51) Int Cl.:
***C12N 15/13*** *(2006.01)* ***C07K 16/40*** *(2006.01)*
***C07K 16/46*** *(2006.01)* ***C12N 15/62*** *(2006.01)*
***C12N 15/85*** *(2006.01)* ***C12N 5/10*** *(2006.01)*
***C07K 19/00*** *(2006.01)* ***A61K 47/48*** *(2006.01)*
***A61K 51/10*** *(2006.01)* ***A61K 39/395*** *(2006.01)*
***G01N 33/577*** *(2006.01)* ***G01N 33/574*** *(2006.01)*

(86) International application number:
**PCT/EP1999/002711**

(87) International publication number:
**WO 1999/057151 (11.11.1999 Gazette 1999/45)**

## (54) ANTIBODY WITH IMPROVED PRODUCIBILITY

ANTIKÖRPER MIT VERBESSERTER PRODUZIERBARKEIT

ANTICORPS A APTITUDE A LA PRODUCTION AMELIOREE

(84) Designated Contracting States:
**AT BE CH CY DE DK ES FI FR GB GR IE IT LI LU MC NL PT SE**
Designated Extension States:
**LT LV RO SI**

(30) Priority: **30.04.1998 EP 98107925**

(43) Date of publication of application:
**16.05.2001 Bulletin 2001/20**

(73) Proprietor: **Boehringer Ingelheim International GmbH**
**55216 Ingelheim (DE)**

(72) Inventors:
- **PARK, John, Edward**
**D-88400 Biberach (DE)**
- **GARIN-CHESA, Pilar**
**D-88400 Biberach (DE)**
- **BAMBERGER, Uwe**
**D-88416 Ochsenhausen (DE)**
- **RETTIG, Wolfgang, J.**
**D-88400 Biberach (DE)**
- **LEGER, Olivier**
**F-74100 Annemasse (FR)**
- **SALDANHA, Jose**
**Middlesex EN1 1TE (GB)**

(74) Representative: **Kläs, Heinz-Gerd et al**
**Boehringer Ingelheim GmbH**
**Binger Strasse 173**
**55216 Ingelheim/Rhein (DE)**

(56) References cited:
**WO-A-93/05804** **WO-A-94/05690**
**WO-A-97/08320** **WO-A-97/41244**
**US-A- 5 693 761**

- **WELT ET AL.: "Antibody targeting in metastatic colon cancer: a phase I study of monoclonal antibody F19 against a cell-surface protein of reactive tumor stromal fibroblasts" JOURNAL OF CLINICAL ONCOLOGY, vol. 12, no. 6, June 1994 (1994-06), pages 1193-1203, XP002088696**
- **STUDNICKA G M ET AL: "Human-engineered monoclonal antibodies retain full specific binding activity by preserving non- CDR complementarity-modulating residues." PROTEIN ENGINEERING, (1994 JUN) 7 (6) 805-14. JOURNAL CODE: PR1. ISSN: 0269-2139., XP000447301 ENGLAND: United Kingdom**
- **WRIGHT A ET AL: "Genetically engineered antibodies: progress and prospects." CRITICAL REVIEWS IN IMMUNOLOGY, (1992) 12 (3-4) 125-68. REF: 252 JOURNAL CODE: AF1. ISSN: 1040-8401., XP000616488 United States**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 098 979 B1

SEQUENCE LISTING

(1) GENERAL INFORMATION:

(i) APPLICANT:

(A) NAME: Boehringer Ingelheim International GmbH
(B) STREET: Rheinstrasse
(C) CITY: Ingelheim am Rhein
(E) COUNTRY: Germany
(F) POSTAL CODE (ZIP) : 55216
(G) TELEPHONE: ++49-6132-772770
(H) TELEFAX: ++49-6132-774377

(ii) TITLE OF INVENTION: FAP alpha-specific antibody with improved producibility

(iii) NUMBER OF SEQUENCES: 101

(iv) COMPUTER READABLE FORM:

(A) MEDIUM TYPE: Floppy disk
(B) COMPUTER: IBM PC compatible
(C) OPERATING SYSTEM: PC-DOS/MS-DOS
(D) SOFTWARE: Patent In Release #1.0, Version #1.30 (EPO)

(2) INFORMATION FOR SEQ ID NO: 1:

(i) SEQUENCE CHARACTERISTICS:

(A) LENGTH: 339 base pairs
(B) TYPE: nucleic acid
(C) STRANDEDNESS: double
(D) TOPOLOGY: linear

(ii) MOLECULE TYPE: cDNA

(xi) SEQUENCE DESCRIPTION: SEQ ID NO: 1:

```
GACATTGTGA TGACCCAATC TCCAGACTCT TTGGCTGTGT CTCTAGGGGA GAGGGCCACC    60
ATCAACTGCA AGTCCAGTCA GAGCCTTTTA TATTCTAGAA ATCAAAAGAA CTACTTGGCC   120
TGGTATCAGC AGAAACCAGG ACAGCCACCC AAACTCCTCA TCTTTTGGGC TAGCACTAGG   180
GAATCTGGGG TACCTGATAG GTTCAGTGGC AGTGGGTTTG GGACAGACTT CACCCTCACC   240
ATTAGCAGCC TGCAGGCTGA AGATGTGGCA GTTTATTACT GTCAGCAATA TTTTAGCTAT   300
CCGCTCACGT TCGGACAAGG GACCAAGGTG GAAATAAAA                          339
```

(2) INFORMATION FOR SEQ ID NO: 2:

(i) SEQUENCE CHARACTERISTICS:

(A) LENGTH: 113 amino acids
(B) TYPE: amino acid
(C) STRANDEDNESS: single
(D) TOPOLOGY: linear

(ii) MOLECULE TYPE: peptide
(xi) SEQUENCE DESCRIPTION: SEQ ID NO: 2:

```
Asp Ile Val Met Thr Gln Ser Pro Asp Ser Leu Ala Val Ser Leu Gly
1               5                   10                  15

Glu Arg Ala Thr Ile Asn Cys Lys Ser Ser Gln Ser Leu Leu Tyr Ser
            20                  25                  30

Arg Asn Gln Lys Asn Tyr Leu Ala Trp Tyr Gln Gln Lys Pro Gly Gln
        35                  40                  45

Pro Pro Lys Leu Leu Ile Phe Trp Ala Ser Thr Arg Glu Ser Gly Val
    50                  55                  60

Pro Asp Arg Phe Ser Gly Ser Gly Phe Gly Thr Asp Phe Thr Leu Thr
65                  70                  75                  80

Ile Ser Ser Leu Gln Ala Glu Asp Val Ala Val Tyr Tyr Cys Gln Gln
                85                  90                  95

Tyr Phe Ser Tyr Pro Leu Thr Phe Gly Gln Gly Thr Lys Val Glu Ile
            100                 105                 110

Lys
```

(2) INFORMATION FOR SEQ ID NO: 3:

(i) SEQUENCE CHARACTERISTICS:

(A) LENGTH: 339 base pairs
(B) TYPE: nucleic acid
(C) STRANDEDNESS: double
(D) TOPOLOGY: linear

(ii) MOLECULE TYPE: cDNA
(xi) SEQUENCE DESCRIPTION: SEQ ID NO: 3:

```
GACATTGTGA TGACCCAATC TCCAGACTCT TTGGCTGTGT CTCTAGGGGA GAGGGCCACC    60
ATCAACTGCA AGTCCAGTCA GAGCCTTTTA TATTCTAGAA ATCAAAAGAA CTACTTGGCC   120
TGGTTCCAGC AGAAACCAGG ACAGCCACCC AAACTCCTCA TCTTTTGGGC TAGCACTAGG   180
GAATCTGGGG TACCTGATAG GTTCAGTGGC AGTGGGTTTG GGACAGACTT CACCCTCACC   240
ATTAGCAGCC TGCAGGCTGA AGATGTGGCA GTTTATGACT GTCAACAATA TTTTAGCTAT   300
CCGCTCACGT TCGGACAAGG GACCAAGGTG GAAATAAAA                          339
```

(2) INFORMATION FOR SEQ ID NO: 4:

(i) SEQUENCE CHARACTERISTICS:

(A) LENGTH: 113 amino acids
(B) TYPE: amino acid
(C) STRANDEDNESS: single
(D) TOPOLOGY: linear

(ii) MOLECULE TYPE: peptide
(xi) SEQUENCE DESCRIPTION: SEQ ID NO: 4:

```
Asp Ile Val Met Thr Gln Ser Pro Asp Ser Leu Ala Val Ser Leu Gly
1               5                   10                  15

Glu Arg Ala Thr Ile Asn Cys Lys Ser Ser Gln Ser Leu Leu Tyr Ser
            20                  25                  30

Arg Asn Gln Lys Asn Tyr Leu Ala Trp Phe Gln Gln Lys Pro Gly Gln
        35                  40                  45

Pro Pro Lys Leu Leu Ile Phe Trp Ala Ser Thr Arg Glu Ser Gly Val
    50                  55                  60

Pro Asp Arg Phe Ser Gly Ser Gly Phe Gly Thr Asp Phe Thr Leu Thr
65                  70                  75                  80

Ile Ser Ser Leu Gln Ala Glu Asp Val Ala Val Tyr Asp Cys Gln Gln
                85                  90                  95

Tyr Phe Ser Tyr Pro Leu Thr Phe Gly Gln Gly Thr Lys Val Glu Ile
            100                 105                 110

Lys
```

(2) INFORMATION FOR SEQ ID NO: 5:

(i) SEQUENCE CHARACTERISTICS:

(A) LENGTH: 339 base pairs
(B) TYPE: nucleic acid
(C) STRANDEDNESS: double
(D) TOPOLOGY: linear

(ii) MOLECULE TYPE: cDNA
(xi) SEQUENCE DESCRIPTION: SEQ ID NO: 5:

```
GACATTGTGA TGACCCAATC TCCAGACTCT TTGGCTGTGT CTCTAGGGGA GAGGGCCACC    60
ATCAACTGCA AGTCCAGTCA GAGCCTTTTA TATTCTAGAA ATCAAAAGAA CTACTTGGCC   120
TGGTATCAGC AGAAACCAGG ACAGCCACCC AAACTCCTCA TCTATTGGGC TAGCACTAGG   180
GAATCTGGGG TACCTGATAG GTTCAGTGGC AGTGGGTTTG GGACAGACTT CACCCTCACC   240
ATTAGCAGCC TGCAGGCTGA AGATGTGGCA GTTTATTACT GTCAGCAATA TTTTAGCTAT   300

CCGCTCACGT TCGGACAAGG GACCAAGGTG GAAATAAAA                          339
```

(2) INFORMATION FOR SEQ ID NO: 6:

(i) SEQUENCE CHARACTERISTICS:

(A) LENGTH: 113 amino acids
(B) TYPE: amino acid
(C) STRANDEDNESS: single
(D) TOPOLOGY: linear

(ii) MOLECULE TYPE: peptide
(xi) SEQUENCE DESCRIPTION: SEQ ID NO: 6:

```
Asp Ile Val Met Thr Gln Ser Pro Asp Ser Leu Ala Val Ser Leu Gly
1               5                   10                  15

Glu Arg Ala Thr Ile Asn Cys Lys Ser Ser Gln Ser Leu Leu Tyr Ser
            20                  25                  30

Arg Asn Gln Lys Asn Tyr Leu Ala Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Ser Gly Ser Gly Phe Gly Thr Asp Phe Thr Leu Thr
65                  70                  75                  80

Ile Ser Ser Leu Gln Ala Glu Asp Val Ala Val Tyr Tyr Cys Gln Gln
                85                  90                  95

Tyr Phe Ser Tyr Pro Leu Thr Phe Gly Gln Gly Thr Lys Val Glu Ile
            100                 105                 110

Lys
```

(2) INFORMATION FOR SEQ ID NO: 7:

(i) SEQUENCE CHARACTERISTICS:

(A) LENGTH: 372 base pairs
(B) TYPE: nucleic acid
(C) STRANDEDNESS: double
(D) TOPOLOGY: linear

(ii) MOLECULE TYPE: cDNA
(xi) SEQUENCE DESCRIPTION: SEQ ID NO: 7:

```
CAGGTGCAAC TAGTGCAGTC CGGCGCCGAA GTGAAGAAAC CCGGTGCTTC CGTGAAAGTC    60
AGCTGTAAAA CTAGTAGATA CACCTTCACT GAATACACCA TACACTGGGT TAGACAGGCC   120
CCTGGCCAAA GGCTGGAGTG GATAGGAGGT ATTAATCCTA ACAATGGTAT TCCTAACTAC   180
AACCAGAAGT TCAAGGGCCG GGCCACCTTG ACCGTAGGCA AGTCTGCCAG CACCGCCTAC   240
ATGGAACTGT CCAGCCTGCG CTCCGAGGAC ACTGCAGTCT ACTACTGCGC CAGAAGAAGA   300
ATCGCCTATG GTTACGACGA GGGCCATGCT ATGGACTACT GGGGTCAAGG AACCCTTGTC   360
ACCGTCTCCT CA                                                       372
```

(2) INFORMATION FOR SEQ ID NO: 8:

(i) SEQUENCE CHARACTERISTICS:

(A) LENGTH: 124 amino acids
(B) TYPE: amino acid
(C) STRANDEDNESS: single
(D) TOPOLOGY: linear

(ii) MOLECULE TYPE: peptide
(xi) SEQUENCE DESCRIPTION: SEQ ID NO: 8:

```
Gln Val Gln Leu Val Gln Ser Gly Ala Glu Val Lys Lys Pro Gly Ala
1               5                   10                  15

Ser Val Lys Val Ser Cys Lys Thr Ser Arg Tyr Thr Phe Thr Glu Tyr
            20                  25                  30

Thr Ile His Trp Val Arg Gln Ala Pro Gly Gln Arg Leu Glu Trp Ile
        35                  40                  45

Gly Gly Ile Asn Pro Asn Asn Gly Ile Pro Asn Tyr Asn Gln Lys Phe
    50                  55                  60

Lys Gly Arg Ala Thr Leu Thr Val Gly Lys Ser Ala Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Arg Arg Ile Ala Tyr Gly Tyr Asp Glu Gly His Ala Met Asp
            100                 105                 110

Tyr Trp Gly Gln Gly Thr Leu Val Thr Val Ser Ser
        115                 120
```

(2) INFORMATION FOR SEQ ID NO: 9:

(i) SEQUENCE CHARACTERISTICS:

(A) LENGTH: 372 base pairs
(B) TYPE: nucleic acid
(C) STRANDEDNESS: double
(D) TOPOLOGY: linear

(ii) MOLECULE TYPE: cDNA
(xi) SEQUENCE DESCRIPTION: SEQ ID NO: 9:

```
CAGGTGCAAC TAGTGCAGTC CGGCGCCGAA GTGAAGAAAC CCGGTGCTTC CGTGAAAGTC    60
AGCTGTAAAA CTAGTAGATA CACCTTCACT GAATACACCA TACACTGGGT TAGACAGGCC   120
CCTGGCCAAA GGCTGGAGTG GATAGGAGGT ATTAATCCTA ACAATGGTAT TCCTAACTAC   180
AACCAGAAGT TCAAGGGCCG GGCCACCTTG ACCGTAGGCA AGTCTGCCAG CACCGCCTAC   240
ATGGAACTGT CCAGCCTGCG CTCCGAGGAC ACTGCAGTCT ACTTCTGCGC CAGAAGAAGA   300
ATCGCCTATG GTTACGACGA GGGCCATGCT ATGGACTACT GGGGTCAAGG AACCCTTGTC   360
ACCGTCTCCT CA                                                       372
```

(2) INFORMATION FOR SEQ ID NO: 10:

(i) SEQUENCE CHARACTERISTICS:

(A) LENGTH: 124 amino acids
(B) TYPE: amino acid
(C) STRANDEDNESS: single
(D) TOPOLOGY: linear

(ii) MOLECULE TYPE: peptide
(xi) SEQUENCE DESCRIPTION: SEQ ID NO: 10:

```
Gln Val Gln Leu Val Gln Ser Gly Ala Glu Val Lys Lys Pro Gly Ala
1               5                   10                  15

Ser Val Lys Val Ser Cys Lys Thr Ser Arg Tyr Thr Phe Thr Glu Tyr
            20                  25                  30

Thr Ile His Trp Val Arg Gln Ala Pro Gly Gln Arg Leu Glu Trp Ile
        35                  40                  45

Gly Gly Ile Asn Pro Asn Asn Gly Ile Pro Asn Tyr Asn Gln Lys Phe
    50                  55                  60

Lys Gly Arg Ala Thr Leu Thr Val Gly Lys Ser Ala Ser Thr Ala Tyr
65                  70                  75                  80

Met Glu Leu Ser Ser Leu Arg Ser Glu Asp Thr Ala Val Tyr Phe Cys
                85                  90                  95

Ala Arg Arg Arg Ile Ala Tyr Gly Tyr Asp Glu Gly His Ala Met Asp
            100                 105                 110

Tyr Trp Gly Gln Gly Thr Leu Val Thr Val Ser Ser
        115                 120
```

(2) INFORMATION FOR SEQ ID NO: 11:

(i) SEQUENCE CHARACTERISTICS:

(A) LENGTH: 372 base pairs
(B) TYPE: nucleic acid
(C) STRANDEDNESS: double
(D) TOPOLOGY: linear

(ii) MOLECULE TYPE: cDNA
(xi) SEQUENCE DESCRIPTION: SEQ ID NO: 11:

```
CAGGTGCAAC TAGTGCAGTC CGGCGCCGAA GTGAAGAAAC CCGGTGCTTC CGTGAAAGTC    60
AGCTGTAAAA CTAGTAGATA CACCTTCACT GAATACACCA TACACTGGGT TAGACAGGCC   120
CCTGGCCAAA GGCTGGAGTG GATAGGAGGT ATTAATCCTA ACAATGGTAT TCCTAACTAC   180
AACCAGAAGT TCAAGGGCCG GGTCACCATC ACCGTAGACA CCTCTGCCAG CACCGCCTAC   240
ATGGAACTGT CCAGCCTGCG CTCCGAGGAC ACTGCAGTCT ACTACTGCGC CAGAAGAAGA   300
ATCGCCTATG GTTACGACGA GGGCCATGCT ATGGACTACT GGGGTCAAGG AACCCTTGTC   360
ACCGTCTCCT CA                                                       372
```

(2) INFORMATION FOR SEQ ID NO: 12:

(i) SEQUENCE CHARACTERISTICS:

(A) LENGTH: 124 amino acids
(B) TYPE: amino acid
(C) STRANDEDNESS: single
(D) TOPOLOGY: linear

(ii) MOLECULE TYPE: peptide
(xi) SEQUENCE DESCRIPTION: SEQ ID NO: 12:

```
Gln Val Gln Leu Val Gln Ser Gly Ala Glu Val Lys Lys Pro Gly Ala
1               5                   10                  15

Ser Val Lys Val Ser Cys Lys Thr Ser Arg Tyr Thr Phe Thr Glu Tyr
            20                  25                  30

Thr Ile His Trp Val Arg Gln Ala Pro Gly Gln Arg Leu Glu Trp Ile
        35                  40                  45

Gly Gly Ile Asn Pro Asn Asn Gly Ile Pro Asn Tyr Asn Gln Lys Phe
    50                  55                  60

Lys Gly Arg Val Thr Ile Thr Val Asp Thr Ser Ala Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Arg Arg Ile Ala Tyr Gly Tyr Asp Glu Gly His Ala Met Asp
            100                 105                 110

Tyr Trp Gly Gln Gly Thr Leu Val Thr Val Ser Ser
        115                 120
```

(2) INFORMATION FOR SEQ ID NO: 13:

(i) SEQUENCE CHARACTERISTICS:

(A) LENGTH: 372 base pairs
(B) TYPE: nucleic acid
(C) STRANDEDNESS: double
(D) TOPOLOGY: linear

(ii) MOLECULE TYPE: cDNA
(xi) SEQUENCE DESCRIPTION: SEQ ID NO: 13:

```
CAGGTGCAAC TAGTGCAGTC CGGCGCCGAA GTGAAGAAAC CCGGTGCTTC CGTGAAAGTC    60
AGCTGTAAAA CTAGTAGATA CACCTTCACT GAATACACCA TACACTGGGT TAGACAGGCC   120
CCTGGCCAAA GGCTGGAGTG GATAGGAGGT ATTAATCCTA ACAATGGTAT TCCTAACTAC   180
AACCAGAAGT TCAAGGGCCG GGTCACCATC ACCGTAGACA CCTCTGCCAG CACCGCCTAC   240
ATGGAACTGT CCAGCCTGCG CTCCGAGGAC ACTGCAGTCT ACTTCTGCGC CAGAAGAAGA   300
ATCGCCTATG GTTACGACGA GGGCCATGCT ATGGACTACT GGGGTCAAGG AACCCTTGTC   360
ACCGTCTCCT CA                                                       372
```

(2) INFORMATION FOR SEQ ID NO: 14:

(i) SEQUENCE CHARACTERISTICS:

(A) LENGTH: 124 amino acids
(B) TYPE: amino acid
(C) STRANDEDNESS: single
(D) TOPOLOGY: linear

(ii) MOLECULE TYPE: peptide
(xi) SEQUENCE DESCRIPTION: SEQ ID NO: 14:

```
Gln Val Gln Leu Val Gln Ser Gly Ala Glu Val Lys Lys Pro Gly Ala
1               5                   10                  15

Ser Val Lys Val Ser Cys Lys Thr Ser Arg Tyr Thr Phe Thr Glu Tyr
            20                  25                  30

Thr Ile His Trp Val Arg Gln Ala Pro Gly Gln Arg Leu Glu Trp Ile
        35                  40                  45

Gly Gly Ile Asn Pro Asn Asn Gly Ile Pro Asn Tyr Asn Gln Lys Phe
    50                  55                  60

Lys Gly Arg Val Thr Ile Thr Val Asp Thr Ser Ala Ser Thr Ala Tyr
65                  70                  75                  80

Met Glu Leu Ser Ser Leu Arg Ser Glu Asp Thr Ala Val Tyr Phe Cys
                85                  90                  95

Ala Arg Arg Arg Ile Ala Tyr Gly Tyr Asp Glu Gly His Ala Met Asp
            100                 105                 110

Tyr Trp Gly Gln Gly Thr Leu Val Thr Val Ser Ser
        115                 120
```

(2) INFORMATION FOR SEQ ID NO: 15:

(i) SEQUENCE CHARACTERISTICS:

(A) LENGTH: 372 base pairs
(B) TYPE: nucleic acid
(C) STRANDEDNESS: double
(D) TOPOLOGY: linear

(ii) MOLECULE TYPE: cDNA
(xi) SEQUENCE DESCRIPTION: SEQ ID NO: 15:

```
CAGGTGCAAC TAGTGCAGTC CGGCGCCGAA GTGAAGAAAC CCGGTGCTTC CGTGAAAGTC     60
AGCTGTAAAA CTAGTGGATA CACCTTCACT GAATACACCA TACACTGGGT TAGACAGGCC    120
CCTGGCCAAA GGCTGGAGTG GATAGGAGGT ATTAATCCTA ACAATGGTAT TCCTAACTAC    180
AACCAGAAGT TCAAGGGCCG GGTCACCATC ACCGTAGACA CCTCTGCCAG CACCGCCTAC    240
ATGGAACTGT CCAGCCTGCG CTCCGAGGAC ACTGCAGTCT ACTACTGCGC CAGAAGAAGA    300
ATCGCCTATG GTTACGACGA GGGCCATGCT ATGGACTACT GGGGTCAAGG AACCCTTGTC    360
ACCGTCTCCT CA                                                        372
```

(2) INFORMATION FOR SEQ ID NO: 16:

(i) SEQUENCE CHARACTERISTICS:

(A) LENGTH: 124 amino acids
(B) TYPE: amino acid
(C) STRANDEDNESS: single
(D) TOPOLOGY: linear

(ii) MOLECULE TYPE: peptide
(xi) SEQUENCE DESCRIPTION: SEQ ID NO: 16:

```
Gln Val Gln Leu Val Gln Ser Gly Ala Glu Val Lys Lys Pro Gly Ala
1               5                   10                  15

Ser Val Lys Val Ser Cys Lys Thr Ser Gly Tyr Thr Phe Thr Glu Tyr
            20                  25                  30

Thr Ile His Trp Val Arg Gln Ala Pro Gly Gln Arg Leu Glu Trp Ile
        35                  40                  45

Gly Gly Ile Asn Pro Asn Asn Gly Ile Pro Asn Tyr Asn Gln Lys Phe
    50                  55                  60

Lys Gly Arg Val Thr Ile Thr Val Asp Thr Ser Ala Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Arg Arg Ile Ala Tyr Gly Tyr Asp Glu Gly His Ala Met Asp
            100                 105                 110

Tyr Trp Gly Gln Gly Thr Leu Val Thr Val Ser Ser
        115                 120
```

(2) INFORMATION FOR SEQ ID NO: 17:

(i) SEQUENCE CHARACTERISTICS:

(A) LENGTH: 220 amino acids
(B) TYPE: amino acid
(C) STRANDEDNESS: single
(D) TOPOLOGY: linear

(ii) MOLECULE TYPE: peptide
(xi) SEQUENCE DESCRIPTION: SEQ ID NO: 17:

```
Asp Ile Val Met Ser Gln Ser Pro Ser Ser Leu Ala Val Ser Val Gly
1               5                   10                  15

Glu Lys Val Thr Met Ser Cys Lys Ser Ser Gln Ser Leu Leu Tyr Ser
            20                  25                  30

Arg Asn Gln Lys Asn Tyr Leu Ala Trp Phe Gln Gln Lys Pro Gly Gln
        35                  40                  45

Ser Pro Lys Leu Leu Ile Phe Trp Ala Ser Thr Arg Glu Ser Gly Val
    50                  55                  60

Pro Asp Arg Phe Thr Gly Ser Gly Phe Gly Thr Asp Phe Asn Leu Thr
65                  70                  75                  80

Ile Ser Ser Val Gln Ala Glu Asp Leu Ala Val Tyr Asp Cys Gln Gln
                85                  90                  95

Tyr Phe Ser Tyr Pro Leu Thr Phe Gly Ala Gly Thr Lys Leu Glu Leu
            100                 105                 110

Lys Arg Thr Val Ala Ala Pro Ser Val Phe Ile Phe Pro Pro Ser Asp
        115                 120                 125

Glu Gln Leu Lys Ser Gly Thr Ala Ser Val Val Cys Leu Leu Asn Asn
    130                 135                 140

Phe Tyr Pro Arg Glu Ala Lys Val Gln Trp Lys Val Asp Asn Ala Leu
145                 150                 155                 160

Gln Ser Gly Asn Ser Gln Glu Ser Val Thr Glu Gln Asp Ser Lys Asp

                165                 170                 175

Ser Thr Tyr Ser Leu Ser Ser Thr Leu Thr Leu Ser Lys Ala Asp Tyr
            180                 185                 190

Glu Lys His Lys Val Tyr Ala Cys Glu Val Thr His Gln Gly Leu Ser
        195                 200                 205

Ser Pro Val Thr Lys Ser Phe Asn Arg Gly Glu Cys
    210                 215                 220
```

(2) INFORMATION FOR SEQ ID NO: 18:

(i) SEQUENCE CHARACTERISTICS:

(A) LENGTH: 453 amino acids
(B) TYPE: amino acid
(C) STRANDEDNESS: single
(D) TOPOLOGY: linear

(ii) MOLECULE TYPE: peptide
(xi) SEQUENCE DESCRIPTION: SEQ ID NO: 18:

```
Val Gln Leu Gln Gln Ser Gly Pro Glu Leu Val Lys Pro Gly Ala Ser
1               5                   10                  15

Val Lys Met Ser Cys Lys Thr Ser Arg Tyr Thr Phe Thr Glu Tyr Thr
            20                  25                  30

Ile His Trp Val Arg Gln Ser His Gly Lys Ser Leu Glu Trp Ile Gly
        35                  40                  45

Gly Ile Asn Pro Asn Asn Gly Ile Pro Asn Tyr Asn Gln Lys Phe Lys
    50                  55                  60

Gly Arg Ala Thr Leu Thr Val Gly Lys Ser Ser Ser Thr Ala Tyr Met
65                  70                  75                  80

Glu Leu Arg Ser Leu Thr Ser Glu Asp Ser Ala Val Tyr Phe Cys Ala
                85                  90                  95

Arg Arg Arg Ile Ala Tyr Gly Tyr Asp Glu Gly His Ala Met Asp Tyr
            100                 105                 110

Trp Gly Gln Gly Thr Ser Val Thr Val Ser Ser Ala Ser Thr Lys Gly
        115                 120                 125

Pro Ser Val Phe Pro Leu Ala Pro Ser Ser Lys Ser Thr Ser Gly Gly
    130                 135                 140

Thr Ala Ala Leu Gly Cys Leu Val Lys Asp Tyr Phe Pro Glu Pro Val
145                 150                 155                 160

Thr Val Ser Trp Asn Ser Gly Ala Leu Thr Ser Gly Val His Thr Phe
                165                 170                 175

Pro Ala Val Leu Gln Ser Ser Gly Leu Tyr Ser Leu Ser Ser Val Val
            180                 185                 190
```

```
Thr Val Pro Ser Ser Ser Leu Gly Thr Gln Thr Tyr Ile Cys Asn Val
        195                 200                 205

Asn His Lys Pro Ser Asn Thr Lys Val Asp Lys Lys Val Glu Pro Lys
    210                 215                 220

Ser Cys Asp Lys Thr His Thr Cys Pro Pro Cys Pro Ala Pro Glu Leu
225                 230                 235                 240

Leu Gly Gly Pro Ser Val Phe Leu Phe Pro Pro Lys Pro Lys Asp Thr
                245                 250                 255

Leu Met Ile Ser Arg Thr Pro Glu Val Thr Cys Val Val Val Asp Val
            260                 265                 270

Ser His Glu Asp Pro Glu Val Lys Phe Asn Trp Tyr Val Asp Gly Val
        275                 280                 285

Glu Val His Asn Ala Lys Thr Lys Pro Arg Glu Glu Gln Tyr Asn Ser
    290                 295                 300

Thr Tyr Arg Val Val Ser Val Leu Thr Val Leu His Gln Asp Trp Leu
305                 310                 315                 320

Asn Gly Lys Glu Tyr Lys Cys Lys Val Ser Asn Lys Ala Leu Pro Ala
                325                 330                 335

Pro Ile Glu Lys Thr Ile Ser Lys Ala Lys Gly Gln Pro Arg Glu Pro
            340                 345                 350

Gln Val Tyr Thr Leu Pro Pro Ser Arg Glu Glu Met Thr Lys Asn Gln
        355                 360                 365

Val Ser Leu Thr Cys Leu Val Lys Gly Phe Tyr Pro Ser Asp Ile Ala
    370                 375                 380

Val Glu Trp Glu Ser Asn Gly Gln Pro Glu Asn Asn Tyr Lys Thr Thr
385                 390                 395                 400

Pro Pro Val Leu Asp Ser Asp Gly Ser Phe Phe Leu Tyr Ser Lys Leu
                405                 410                 415

Thr Val Asp Lys Ser Arg Trp Gln Gln Gly Asn Val Phe Ser Cys Ser
            420                 425                 430

Val Met His Glu Ala Leu His Asn His Tyr Thr Gln Lys Ser Leu Ser
        435                 440                 445

Leu Ser Pro Gly Lys
    450
```

(2) INFORMATION FOR SEQ ID NO: 19:

(i) SEQUENCE CHARACTERISTICS:

(A) LENGTH: 321 base pairs
(B) TYPE: nucleic acid
(C) STRANDEDNESS: double
(D) TOPOLOGY: linear

(ii) MOLECULE TYPE: cDNA

(xi) SEQUENCE DESCRIPTION: SEQ ID NO: 19:

```
CGTACTGTGG CTGCACCATC TGTCTTCATC TTCCCGCCAT CTGATGAGCA GTTGAAATCT    60
GGAACTGCCT CTGTTGTGTG CCTGCTGAAT AACTTCTATC CCAGAGAGGC CAAAGTACAG   120
TGGAAGGTGG ATAACGCCCT CCAATCGGGT AACTCCCAGG AGAGTGTCAC AGAGCAGGAC   180
AGCAAGGACA GCACCTACAG CCTCAGCAGC ACCCTGACGC TGAGCAAAGC AGACTACGAG   240
AAACACAAAG TCTACGCCTG CGAAGTCACC CATCAGGGCC TGAGCTCGCC CGTCACAAAG   300
AGCTTCAACA GGGGAGAGTG T                                              321
```

(2) INFORMATION FOR SEQ ID NO: 20:

(i) SEQUENCE CHARACTERISTICS:

(A) LENGTH: 107 amino acids
(B) TYPE: amino acid
(C) STRANDEDNESS: single
(D) TOPOLOGY: linear

(ii) MOLECULE TYPE: peptide
(xi) SEQUENCE DESCRIPTION: SEQ ID NO: 20:

```
Arg Thr Val Ala Ala Pro Ser Val Phe Ile Phe Pro Pro Ser Asp Glu
1               5                   10                  15

Gln Leu Lys Ser Gly Thr Ala Ser Val Val Cys Leu Leu Asn Asn Phe
            20                  25                  30

Tyr Pro Arg Glu Ala Lys Val Gln Trp Lys Val Asp Asn Ala Leu Gln
        35                  40                  45

Ser Gly Asn Ser Gln Glu Ser Val Thr Glu Gln Asp Ser Lys Asp Ser
    50                  55                  60

Thr Tyr Ser Leu Ser Ser Thr Leu Thr Leu Ser Lys Ala Asp Tyr Glu
65                  70                  75                  80

Lys His Lys Val Tyr Ala Cys Glu Val Thr His Gln Gly Leu Ser Ser
                85                  90                  95

Pro Val Thr Lys Ser Phe Asn Arg Gly Glu Cys
            100                 105
```

(2) INFORMATION FOR SEQ ID NO: 21:

(i) SEQUENCE CHARACTERISTICS:

(A) LENGTH: 990 base pairs
(B) TYPE: nucleic acid
(C) STRANDEDNESS: double

(D) TOPOLOGY: linear

(ii) MOLECULE TYPE: cDNA
(xi) SEQUENCE DESCRIPTION: SEQ ID NO: 21:

```
GCCTCCACCA AGGGCCCATC GGTCTTCCCC CTGGCACCCT CCTCCAAGAG CACCTCTGGG      60
GGCACAGCGG CCCTGGGCTG CCTGGTCAAG GACTACTTCC CCGAACCGGT GACGGTGTCG     120
TGGAACTCAG GCGCCCTGAC CAGCGGCGTG CACACCTTCC CGGCTGTCCT ACAGTCCTCA     180
GGACTCTACT CCCTCAGCAG CGTGGTGACC GTGCCCTCCA GCAGCTTGGG CACCCAGACC     240
TACATCTGCA ACGTGAATCA CAAGCCCAGC AACACCAAGG TGGACAAGAA AGTTGAGCCC     300
AAATCTTGTG ACAAAACTCA CACATGCCCA CCGTGCCCAG CACCTGAACT CCTGGGGGGA     360
CCGTCAGTCT TCCTCTTCCC CCCAAAACCC AAGGACACCC TCATGATCTC CCGGACCCCT     420
GAGGTCACAT GCGTGGTGGT GGACGTGAGC CACGAAGACC CTGAGGTCAA GTTCAACTGG     480
TACGTGGACG GCGTGGAGGT GCATAATGCC AAGACAAAGC CGCGGGAGGA GCAGTACAAC     540
AGCACGTACC GGGTGGTCAG CGTCCTCACC GTCCTGCACC AGGACTGGCT GAATGGCAAG     600
GAGTACAAGT GCAAGGTCTC CAACAAAGCC CTCCCAGCCC CCATCGAGAA AACCATCTCC     660
AAAGCCAAAG GGCAGCCCCG AGAACCACAG GTGTACACCC TGCCCCCATC CCGGGAGGAG     720
ATGACCAAGA ACCAGGTCAG CCTGACCTGC CTGGTCAAAG GCTTCTATCC CAGCGACATC     780
GCCGTGGAGT GGGAGAGCAA TGGGCAGCCG GAGAACAACT ACAAGACCAC GCCTCCCGTG     840
CTGGACTCCG ACGGCTCCTT CTTCCTCTAC AGCAAGCTCA CCGTGGACAA GAGCAGGTGG     900
CAGCAGGGGA ACGTCTTCTC ATGCTCCGTG ATGCATGAGG CTCTGCACAA CCACTACACG     960
CAGAAGAGCC TCTCCCTGTC TCCGGGTAAA                                      990
```

(2) INFORMATION FOR SEQ ID NO: 22:

(i) SEQUENCE CHARACTERISTICS:

(A) LENGTH: 330 amino acids
(B) TYPE: amino acid
(C) STRANDEDNESS: single
(D) TOPOLOGY: linear

(ii) MOLECULE TYPE: peptide
(xi) SEQUENCE DESCRIPTION: SEQ ID NO: 22:

```
Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
```

```
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330
```

(2) INFORMATION FOR SEQ ID NO: 23:

(i) SEQUENCE CHARACTERISTICS:

(A) LENGTH: 427 base pairs
(B) TYPE: nucleic acid
(C) STRANDEDNESS: double
(D) TOPOLOGY: linear

(ii) MOLECULE TYPE: DNA (genomic)
(xi) SEQUENCE DESCRIPTION: SEQ ID NO: 23:

```
AAGCTTGCCG CCACCATGGA TTCACAGGCC CAGGTTCTTA TGTTACTGCC GCTATGGGTA     60
TCTGGTACCT GTGGGGACAT TGTGATGTCA CAGTCTCCAT CCTCCCTAGC TGTGTCAGTT    120
GGAGAGAAGG TTACTATGAG CTGCAAGTCC AGTCAGAGCC TTTTATATAG TCGTAATCAA    180
AAGAACTACT TGGCCTGGTT CCAGCAGAAG CCAGGGCAGT CTCCTAAACT GCTGATTTTC    240
TGGGCATCCA CTAGGGAATC TGGGGTCCCT GATCGCTTCA CAGGCAGTGG ATTTGGGACG    300
GATTTCAATC TCACCATCAG CAGTGTGCAG GCTGAGGACC TGGCAGTTTA TGACTGTCAG    360
CAATATTTTA GCTATCCGCT CACGTTCGGT GCTGGGACCA AGCTGGAGCT GAAACGTGAG    420
TGGATCC                                                              427
```

(2) INFORMATION FOR SEQ ID NO: 24:

(i) SEQUENCE CHARACTERISTICS:

(A) LENGTH: 133 amino acids
(B) TYPE: amino acid
(C) STRANDEDNESS: single
(D) TOPOLOGY: linear

(ii) MOLECULE TYPE: peptide
(xi) SEQUENCE DESCRIPTION: SEQ ID NO: 24:

```
Met Asp Ser Gln Ala Gln Val Leu Met Leu Leu Pro Leu Trp Val Ser
1               5                   10                  15

Gly Thr Cys Gly Asp Ile Val Met Ser Gln Ser Pro Ser Ser Leu Ala
            20                  25                  30

Val Ser Val Gly Glu Lys Val Thr Met Ser Cys Lys Ser Ser Gln Ser
        35                  40                  45

Leu Leu Tyr Ser Arg Asn Gln Lys Asn Tyr Leu Ala Trp Phe Gln Gln
    50                  55                  60

Lys Pro Gly Gln Ser Pro Lys Leu Leu Ile Phe Trp Ala Ser Thr Arg
65                  70                  75                      80

Glu Ser Gly Val Pro Asp Arg Phe Thr Gly Ser Gly Phe Gly Thr Asp
                85                  90                  95

Phe Asn Leu Thr Ile Ser Ser Val Gln Ala Glu Asp Leu Ala Val Tyr
            100                 105                 110

Asp Cys Gln Gln Tyr Phe Ser Tyr Pro Leu Thr Phe Gly Ala Gly Thr
        115                 120                 125

Lys Leu Glu Leu Lys
    130
```

(2) INFORMATION FOR SEQ ID NO: 25:

(i) SEQUENCE CHARACTERISTICS:

(A) LENGTH: 457 base pairs
(B) TYPE: nucleic acid
(C) STRANDEDNESS: double
(D) TOPOLOGY: linear

(ii) MOLECULE TYPE: DNA (genomic)
(xi) SEQUENCE DESCRIPTION: SEQ ID NO: 25:

```
AAGCTTGCCG CCACCATGGG ATGGAGCTGG GTCTTTCTCT TTCTCCTGTC AGGAACTGCA    60
GGTGTCCTCT CTGAGGTCCA GCTGCAACAG TCTGGACCTG AGCTGGTGAA GCCTGGGGCT   120
TCAGTAAAGA TGTCCTGCAA GACTTCTAGA TACACATTCA CTGAATACAC CATACACTGG   180
GTGAGACAGA GCCATGGAAA GAGCCTTGAG TGGATTGGAG GTATTAATCC TAACAATGGT   240
ATTCCTAACT ACAACCAGAA GTTCAAGGGC AGGGCCACAT TGACTGTAGG CAAGTCCTCC   300
AGCACCGCCT ACATGGAGCT CCGCAGCCTG ACATCTGAGG ATTCTGCGGT CTATTTCTGT   360
GCAAGAAGAA GAATCGCCTA TGGTTACGAC GAGGGCCATG CTATGGACTA CTGGGGTCAA   420
GGAACCTCAG TCACCGTCTC CTCAGGTGAG TGGATCC                             457
```

(2) INFORMATION FOR SEQ ID NO: 26:

(i) SEQUENCE CHARACTERISTICS:

(A) LENGTH: 143 amino acids
(B) TYPE: amino acid
(C) STRANDEDNESS: single
(D) TOPOLOGY: linear

(ii) MOLECULE TYPE: peptide
(xi) SEQUENCE DESCRIPTION: SEQ ID NO: 26:

```
Met Gly Trp Ser Trp Val Phe Leu Phe Leu Leu Ser Gly Thr Ala Gly
1               5                   10                  15

Val Leu Ser Glu Val Gln Leu Gln Gln Ser Gly Pro Glu Leu Val Lys
            20                  25                  30

Pro Gly Ala Ser Val Lys Met Ser Cys Lys Thr Ser Arg Tyr Thr Phe
        35                  40                  45

Thr Glu Tyr Thr Ile His Trp Val Arg Gln Ser His Gly Lys Ser Leu
    50                  55                  60

Glu Trp Ile Gly Gly Ile Asn Pro Asn Asn Gly Ile Pro Asn Tyr Asn
65                  70                  75                  80

Gln Lys Phe Lys Gly Arg Ala Thr Leu Thr Val Gly Lys Ser Ser Ser
                85                  90                  95

Thr Ala Tyr Met Glu Leu Arg Ser Leu Thr Ser Glu Asp Ser Ala Val
            100                 105                 110

Tyr Phe Cys Ala Arg Arg Arg Ile Ala Tyr Gly Tyr Asp Glu Gly His
        115                 120                 125

Ala Met Asp Tyr Trp Gly Gln Gly Thr Ser Val Thr Val Ser Ser
    130                 135                 140
```

(2) INFORMATION FOR SEQ ID NO: 27:

(i) SEQUENCE CHARACTERISTICS:

(A) LENGTH: 8068 base pairs
(B) TYPE: nucleic acid
(C) STRANDEDNESS: double
(D) TOPOLOGY: linear

(ii) MOLECULE TYPE: DNA (genomic)
(xi) SEQUENCE DESCRIPTION: SEQ ID NO: 27:

GAATTCCAGC ACACTGGCGG CCGTTACTAG TTATTAATAG TAATCAATTA CGGGGTCATT 60

AGTTCATAGC CCATATATGG AGTTCCGCGT TACATAACTT ACGGTAAATG GCCCGCCTGG 120

CTGACCGCCC AACGACCCCC GCCCATTGAC GTCAATAATG ACGTATGTTC CCATAGTAAC 180

GCCAATAGGG ACTTTCCATT GACGTCAATG GGTGGAGTAT TTACGGTAAA CTGCCCACTT 240

GGCAGTACAT CAAGTGTATC ATATGCCAAG TACGCCCCCT ATTGACGTCA ATGACGGTAA 300

ATGGCCCGCC TGGCATTATG CCCAGTACAT GACCTTATGG GACTTTCCTA CTTGGCAGTA 360

CATCTACGTA TTAGTCATCG CTATTACCAT GGTGATGCGG TTTTGGCAGT ACATCAATGG 420

GCGTGGATAG CGGTTTGACT CACGGGGATT TCCAAGTCTC CACCCCATTG ACGTCAATGG 480

GAGTTTGTTT TGGCACCAAA ATCAACGGGA CTTTCCAAAA TGTCGTAACA ACTCCGCCCC 540

ATTGACGCAA ATGGGCGGTA GGCGTGTACG GTGGGAGGTC TATATAAGCA GAGCTCGTTT 600

AGTGAACCGT CAGATCGCCT GGAGACGCCA TCCACGCTGT TTTGACCTCC ATAGAAGACA 660

CCGGGACCGA TCCAGCCTCC GCGGCCGGGA ACGGTGCATT GGAACGCGGA TTCCCCGTGC 720

CAAGAGTGAC GTAAGTACCG CCTATAGAGT CTATAGGCCC ACCCCCTTGG CTTCTTATGC 780

ATGCTATACT GTTTTTGGCT TGGGGTCTAT ACACCCCCGC TTCCTCATGT TATAGGTGAT 840

GGTATAGCTT AGCCTATAGG TGTGGGTTAT TGACCATTAT TGACCACTCC CCTATTGGTG 900

ACGATACTTT CCATTACTAA TCCATAACAT GGCTCTTTGC CACAACTCTC TTTATTGGCT 960

ATATGCCAAT ACACTGTCCT TCAGAGACTG ACACGGACTC TGTATTTTTA CAGGATGGGG 1020

TCTCATTTAT TATTTACAAA TTCACATATA CAACACCACC GTCCCCAGTG CCCGCAGTTT 1080

TTATTAAACA TAACGTGGGA TCTCCACGCG AATCTCGGGT ACGTGTTCCG GACATGGGCT 1140

CTTCTCCGGT AGCGGCGGAG CTTCTACATC CGAGCCCTGC TCCCATGCCT CCAGCGACTC 1200

ATGGTCGCTC GGCAGCTCCT TGCTCCTAAC AGTGGAGGCC AGACTTAGGC ACAGCACGAT 1260

GCCCACCACC ACCAGTGTGC CGCACAAGGC CGTGGCGGTA GGGTATGTGT CTGAAAATGA 1320

GCTCGGGGAG CGGGCTTGCA CCGCTGACGC ATTTGGAAGA CTTAAGGCAG CGGCAGAAGA 1380

AGATGCAGGC AGCTGAGTTG TTGTGTTCTG ATAAGAGTCA GAGGTAACTC CCGTTGCGGT 1440

GCTGTTAACG GTGGAGGGCA GTGTAGTCTG AGCAGTACTC GTTGCTGCCG CGCGCGCCAC 1500

CAGACATAAT AGCTGACAGA CTAACAGACT GTTCCTTTCC ATGGGTCTTT TCTGCAGTCA 1560

CCGTCCTTGA CACGCGTCTC GGGAAGCTTG CCGCCACCAT GGATTCACAG GCCCAGGTTC 1620

TTATGTTACT GCCGCTATGG GTATCTGGTA CCTGTGGGGA CATTGTGATG TCACAGTCTC 1680

CATCCTCCCT AGCTGTGTCA GTTGGAGAGA AGGTTACTAT GAGCTGCAAG TCCAGTCAGA 1740

GCCTTTTATA TTCTAGAAAT CAAAAGAACT ACTTGGCCTG GTTCCAGCAG AAGCCAGGGC 1800

AGTCTCCTAA ACTGCTGATT TTCTGGGCAT CCACTAGGGA ATCTGGGGTC CCTGATCGCT 1860

TCACAGGCAG TGGATTTGGG ACGGATTTCA ATCTCACCAT CAGCAGTGTG CAGGCTGAGG 1920

ACCTGGCAGT TTATGACTGT CAGCAATATT TTAGCTATCC GCTCACGTTC GGTGCTGGGA 1980

CCAAGCTGGA GCTGAAACGT GAGTGGATCC ATCTGGGATA AGCATGCTGT TTTCTGTCTG 2040

TCCCTAACAT GCCCTGTGAT TATGCGCAAA CAACACACCC AAGGGCAGAA CTTTGTTACT 2100

TAAACACCAT CCTGTTTGCT TCTTTCCTCA GGAACTGTGG CTGCACCATC TGTCTTCATC 2160

TTCCCGCCAT CTGATGAGCA GTTGAAATCT GGAACTGCCT CTGTTGTGTG CCTGCTGAAT 2220

AACTTCTATC CCAGAGAGGC CAAAGTACAG TGGAAGGTGG ATAACGCCCT CCAATCGGGT 2280

AACTCCCAGG AGAGTGTCAC AGAGCAGGAC AGCAAGGACA GCACCTACAG CCTCAGCAGC 2340

ACCCTGACGC TGAGCAAAGC AGACTACGAG AAACACAAAG TCTACGCCTG CGAAGTCACC 2400

CATCAGGGCC TGAGCTCGCC CGTCACAAAG AGCTTCAACA GGGGAGAGTG TTAGAGGGAG 2460

AAGTGCCCCC ACCTGCTCCT CAGTTCCAGC CTGACCCCCT CCCATCCTTT GGCCTCTGAC 2520

CCTTTTTCCA CAGGGGACCT ACCCCTATTG CGGTCCTCCA GCTCATCTTT CACCTCACCC 2580

CCCTCCTCCT CCTTGGCTTT AATTATGCTA ATGTTGGAGG AGAATGAATA AATAAAGTGA 2640

ATCTTTGCAC CTGTGGTGGA TCTAATAAAA GATATTTATT TTCATTAGAT ATGTGTGTTG 2700

GTTTTTTGTG TGCAGTGCCT CTATCTGGAG GCCAGGTAGG GCTGGCCTTG GGGGAGGGGG 2760

AGGCCAGAAT GACTCCAAGA GCTACAGGAA GGCAGGTCAG AGACCCCACT GGACAAACAG 2820

TGGCTGGACT CTGCACCATA ACACACAATC AACAGGGGAG TGAGCTGGAA ATTTGCTAGC 2880

GAATTCTTGA AGACGAAAGG GCCTCGTGAT ACGCCTATTT TTATAGGTTA ATGTCATGAT 2940

AATAATGGTT TCTTAGACGT CAGGTGGCAC TTTTCGGGGA AATGTGCGCG GAACCCCTAT 3000

TTGTTTATTT TTCTAAATAC ATTCAAATAT GTATCCGCTC ATGAGACAAT AACCCTGATA 3060

AATGCTTCAA TAATATTGAA AAAGGAAGAG TATGAGTATT CAACATTTCC GTGTCGCCCT 3120

TATTCCCTTT TTTGCGGCAT TTTGCCTTCC TGTTTTTGCT CACCCAGAAA CGCTGGTGAA 3180

AGTAAAAGAT GCTGAAGATC AGTTGGGTGC ACGAGTGGGT TACATCGAAC TGGATCTCAA 3240

CAGCGGTAAG ATCCTTGAGA GTTTTCGCCC CGAAGAACGT TTTCCAATGA TGAGCACTTT 3300

TAAAGTTCTG CTATGTGGCG CGGTATTATC CCGTGTTGAC GCCGGGCAAG AGCAACTCGG 3360

TCGCCGCATA CACTATTCTC AGAATGACTT GGTTGAGTAC TCACCAGTCA CAGAAAAGCA 3420

TCTTACGGAT GGCATGACAG TAAGAGAATT ATGCAGTGCT GCCATAACCA TGAGTGATAA 3480

CACTGCGGCC AACTTACTTC TGACAACGAT CGGAGGACCG AAGGAGCTAA CCGCTTTTTT 3540

GCACAACATG GGGGATCATG TAACTCGCCT TGATCGTTGG GAACCGGAGC TGAATGAAGC 3600

CATACCAAAC GACGAGCGTG ACACCACGAT GCCTGCAGCA ATGGCAACAA CGTTGCGCAA 3660

ACTATTAACT GGCGAACTAC TTACTCTAGC TTCCCGGCAA CAATTAATAG ACTGGATGGA 3720

GGCGGATAAA GTTGCAGGAC CACTTCTGCG CTCGGCCCTT CCGGCTGGCT GGTTTATTGC 3780

TGATAAATCT GGAGCCGGTG AGCGTGGGTC TCGCGGTATC ATTGCAGCAC TGGGGCCAGA 3840

TGGTAAGCCC TCCCGTATCG TAGTTATCTA CACGACGGGG AGTCAGGCAA CTATGGATGA 3900

ACGAAATAGA CAGATCGCTG AGATAGGTGC CTCACTGATT AAGCATTGGT AACTGTCAGA 3960

CCAAGTTTAC TCATATATAC TTTAGATTGA TTTAAAACTT CATTTTTAAT TTAAAAGGAT 4020

CTAGGTGAAG ATCCTTTTTG ATAATCTCAT GACCAAAATC CCTTAACGTG AGTTTTCGTT 4080

CCACTGAGCG TCAGACCCCG TAGAAAAGAT CAAAGGATCT TCTTGAGATC CTTTTTTTCT 4140

GCGCGTAATC TGCTGCTTGC AAACAAAAAA ACCACCGCTA CCAGCGGTGG TTTGTTTGCC 4200

GGATCAAGAG CTACCAACTC TTTTTCCGAA GGTAACTGGC TTCAGCAGAG CGCAGATACC 4260

AAATACTGTC CTTCTAGTGT AGCCGTAGTT AGGCCACCAC TTCAAGAACT CTGTAGCACC 4320

GCCTACATAC CTCGCTCTGC TAATCCTGTT ACCAGTGGCT GCTGCCAGTG GCGATAAGTC 4380

GTGTCTTACC GGGTTGGACT CAAGACGATA GTTACCGGAT AAGGCGCAGC GGTCGGGCTG 4440

AACGGGGGGT TCGTGCACAC AGCCCAGCTT GGAGCGAACG ACCTACACCG AACTGAGATA 4500

CCTACAGCGT GAGCTATGAG AAAGCGCCAC GCTTCCCGAA GGGAGAAAGG CGGACAGGTA 4560

TCCGGTAAGC GGCAGGGTCG GAACAGGAGA GCGCACGAGG GAGCTTCCAG GGGGAAACGC 4620

CTGGTATCTT TATAGTCCTG TCGGGTTTCG CCACCTCTGA CTTGAGCGTC GATTTTTGTG 4680

ATGCTCGTCA GGGGGGCGGA GCCTATGGAA AAACGCCAGC AACGCGGCCT TTTTACGGTT 4740

CCTGGCCTTT TGCTGGCCTT TTGCTCACAT GTTCTTTCCT GCGTTATCCC CTGATTCTGT 4800

GGATAACCGT ATTACCGCCT TTGAGTGAGC TGATACCGCT CGCCGCAGCC GAACGACCGA 4860

GCGCAGCGAG TCAGTGAGCG AGGAAGCGGA AGAGCGCCTG ATGCGGTATT TTCTCCTTAC 4920

GCATCTGTGC GGTATTTCAC ACCGCATATG GTGCACTCTC AGTACAATCT GCTCTGATGC 4980

CGCATAGTTA AGCCAGTATA CACTCCGCTA TCGCTACGTG ACTGGGTCAT GGCTGCGCCC 5040

CGACACCCGC CAACACCCGC TGACGCGCCC TGACGGGCTT GTCTGCTCCC GGCATCCGCT 5100

TACAGACAAG CTGTGACCGT CTCCGGGAGC TGCATGTGTC AGAGGTTTTC ACCGTCATCA 5160

CCGAAACGCG CGAGGCAGCT GTGGAATGTG TGTCAGTTAG GGTGTGGAAA GTCCCCAGGC 5220

TCCCCAGCAG GCAGAAGTAT GCAAAGCATG CATCTCAATT AGTCAGCAAC CAGGCTCCCC 5280

AGCAGGCAGA AGTATGCAAA GCATGCATCT CAATTAGTCA GCAACCATAG TCCCGCCCCT 5340

AACTCCGCCC ATCCCGCCCC TAACTCCGCC CAGTTCCGCC CATTCTCCGC CCCATGGCTG 5400

ACTAATTTTT TTTATTTATG CAGAGGCCGA GGCCGCCTCG GCCTCTGAGC TATTCCAGAA 5460

GTAGTGAGGA GGCTTTTTTG GAGGCCTAGG CTTTTGCAAA AAGCTAGCTT CACGCTGCCG 5520

CAAGCACTCA GGGCGCAAGG GCTGCTAAAG GAAGCGGAAC ACGTAGAAAG CCAGTCCGCA 5580

GAAACGGTGC TGACCCCGGA TGAATGTCAG CTACTGGGCT ATCTGGACAA GGGAAAACGC 5640

AAGCGCAAAG AGAAAGCAGG TAGCTTGCAG TGGGCTTACA TGGCGATAGC TAGACTGGGC 5700

GGTTTTATGG ACAGCAAGCG AACCGGAATT GCCAGCTGGG GCGCCCTCTG GTAAGGTTGG 5760

GAAGCCCTGC AAAGTAAACT GGATGGCTTT CTTGCCGCCA AGGATCTGAT GGCGCAGGGG 5820

ATCAAGATCT GATCAAGAGA CAGGATGAGG ATCGTTTCGC ATGATTGAAC AAGATGGATT 5880

GCACGCAGGT TCTCCGGCCG CTTGGGTGGA GAGGCTATTC GGCTATGACT GGGCACAACA 5940

GACAATCGGC TGCTCTGATG CCGCCGTGTT CCGGCTGTCA GCGCAGGGGC GCCCGGTTCT 6000

TTTTGTCAAG ACCGACCTGT CCGGTGCCCT GAATGAACTG CAGGACGAGG CAGCGCGGCT 6060

ATCGTGGCTG GCCACGACGG GCGTTCCTTG CGCAGCTGTG CTCGACGTTG TCACTGAAGC 6120

GGGAAGGGAC TGGCTGCTAT TGGGCGAAGT GCCGGGGCAG GATCTCCTGT CATCTCACCT 6180

TGCTCCTGCC GAGAAAGTAT CCATCATGGC TGATGCAATG CGGCGGCTGC ATACGCTTGA 6240

TCCGGCTACC TGCCCATTCG ACCACCAAGC GAAACATCGC ATCGAGCGAG CACGTACTCG 6300

GATGGAAGCC GGTCTTGTCG ATCAGGATGA TCTGGACGAA GAGCATCAGG GGCTCGCGCC 6360

AGCCGAACTG TTCGCCAGGC TCAAGGCGCG CATGCCCGAC GGCGAGGATC TCGTCGTGAC 6420

CCATGGCGAT GCCTGCTTGC CGAATATCAT GGTGGAAAAT GGCCGCTTTT CTGGATTCAT 6480

CGACTGTGGC CGGCTGGGTG TGGCGGACCG CTATCAGGAC ATAGCGTTGG CTACCCGTGA 6540

TATTGCTGAA GAGCTTGGCG GCGAATGGGC TGACCGCTTC CTCGTGCTTT ACGGTATCGC 6600

CGCTCCCGAT TCGCAGCGCA TCGCCTTCTA TCGCCTTCTT GACGAGTTCT TCTGAGCGGG 6660

ACTCTGGGGT TCGAAATGAC CGACCAAGCG ACGCCCAACC TGCCATCACG AGATTTCGAT 6720

TCCACCGCCG CCTTCTATGA AAGGTTGGGC TTCGGAATCG TTTTCCGGGA CGCCGGCTGG 6780

ATGATCCTCC AGCGCGGGGA TCTCATGCTG GAGTTCTTCG CCCACCCCGG GCTCGATCCC 6840

CTCGCGAGTT GGTTCAGCTG CTGCCTGAGG CTGGACGACC TCGCGGAGTT CTACCGGCAG 6900

TGCAAATCCG TCGGCATCCA GGAAACCAGC AGCGGCTATC CGCGCATCCA TGCCCCCGAA 6960

CTGCAGGAGT GGGGAGGCAC GATGGCCGCT TTGGTCCCGG ATCTTTGTGA AGGAACCTTA 7020

CTTCTGTGGT GTGACATAAT TGGACAAACT ACCTACAGAG ATTTAAAGCT CTAAGGTAAA 7080

TATAAAATTT TTAAGTGTAT AATGTGTTAA ACTACTGATT CTAATTGTTT GTGTATTTTA 7140

GATTCCAACC TATGGAACTG ATGAATGGGA GCAGTGGTGG AATGCCTTTA ATGAGGAAAA 7200

CCTGTTTTGC TCAGAAGAAA TGCCATCTAG TGATGATGAG GCTACTGCTG ACTCTCAACA 7260

TTCTACTCCT CCAAAAAAGA AGAGAAAGGT AGAAGACCCC AAGGACTTTC CTTCAGAATT 7320

GCTAAGTTTT TTGAGTCATG CTGTGTTTAG TAATAGAACT CTTGCTTGCT TTGCTATTTA 7380

CACCACAAAG GAAAAAGCTG CACTGCTATA CAAGAAAATT ATGGAAAAAT ATTCTGTAAC 7440

CTTTATAAGT AGGCATAACA GTTATAATCA TAACATACTG TTTTTTCTTA CTCCACACAG 7500

GCATAGAGTG TCTGCTATTA ATAACTATGC TCAAAAATTG TGTACCTTTA GCTTTTTAAT 7560

TTGTAAAGGG GTTAATAAGG AATATTTGAT GTATAGTGCC TTGACTAGAG ATCATAATCA 7620

GCCATACCAC ATTTGTAGAG GTTTTACTTG CTTTAAAAAA CCTCCCACAC CTCCCCCTGA 7680

ACCTGAAACA TAAAATGAAT GCAATTGTTG TTGTTAACTT GTTTATTGCA GCTTATAATG 7740

GTTACAAATA AAGCAATAGC ATCACAAATT TCACAAATAA AGCATTTTTT TCACTGCATT 7800

CTAGTTGTGG TTTGTCCAAA CTCATCAATG TATCTTATCA TGTCTGGATC TAATAAAAGA 7860

TATTTATTTT CATTAGATAT GTGTGTTGGT TTTTTGTGTG CAGTGCCTCT ATCTGGAGGC 7920

CAGGTAGGGC TGGCCTTGGG GGAGGGGGAG GCCAGAATGA CTCCAAGAGC TACAGGAAGG 7980

CAGGTCAGAG ACCCCACTGG ACAAACAGTG GCTGGACTCT GCACCATAAC ACACAATCAA 8040

```
CAGGGGAGTG AGCTGGAAAT TTGCTAGC                                               8068
```

(2) INFORMATION FOR SEQ ID NO: 28:

(i) SEQUENCE CHARACTERISTICS:

(A) LENGTH: 239 amino acids
(B) TYPE: amino acid
(C) STRANDEDNESS: single
(D) TOPOLOGY: linear

(ii) MOLECULE TYPE: peptide
(xi) SEQUENCE DESCRIPTION: SEQ ID NO: 28:

```
Asp Ser Gln Ala Gln Val Leu Met Leu Leu Pro Leu Trp Val Ser Gly
1               5                   10                  15

Thr Cys Gly Asp Ile Val Met Ser Gln Ser Pro Ser Ser Leu Ala Val
            20                  25                  30

Ser Val Gly Glu Lys Val Thr Met Ser Cys Lys Ser Ser Gln Ser Leu
        35                  40                  45

Leu Tyr Ser Arg Asn Gln Lys Asn Tyr Leu Ala Trp Phe Gln Gln Lys
    50                  55                  60

Pro Gly Gln Ser Pro Lys Leu Leu Ile Phe Trp Ala Ser Thr Arg Glu
65                  70                  75                  80

Ser Gly Val Pro Asp Arg Phe Thr Gly Ser Gly Phe Gly Thr Asp Phe
                85                  90                  95

Asn Leu Thr Ile Ser Ser Val Gln Ala Glu Asp Leu Ala Val Tyr Asp
            100                 105                 110

Cys Gln Gln Tyr Phe Ser Tyr Pro Leu Thr Phe Gly Ala Gly Thr Lys
        115                 120                 125

Leu Glu Leu Lys Arg Thr Val Ala Ala Pro Ser Val Phe Ile Phe Pro
    130                 135                 140

Pro Ser Asp Glu Gln Leu Lys Ser Gly Thr Ala Ser Val Val Cys Leu
145                 150                 155                 160

Leu Asn Asn Phe Tyr Pro Arg Glu Ala Lys Val Gln Trp Lys Val Asp
                165                 170                 175

Asn Ala Leu Gln Ser Gly Asn Ser Gln Glu Ser Val Thr Glu Gln Asp
            180                 185                 190

Ser Lys Asp Ser Thr Tyr Ser Leu Ser Ser Thr Leu Thr Leu Ser Lys
        195                 200                 205

Ala Asp Tyr Glu Lys His Lys Val Tyr Ala Cys Glu Val Thr His Gln
    210                 215                 220

Gly Leu Ser Ser Pro Val Thr Lys Ser Phe Asn Arg Gly Glu Cys
225                 230                 235
```

(2) INFORMATION FOR SEQ ID NO: 29:

(i) SEQUENCE CHARACTERISTICS:

(A) LENGTH: 7731 base pairs
(B) TYPE: nucleic acid
(C) STRANDEDNESS: double
(D) TOPOLOGY: linear

(ii) MOLECULE TYPE: DNA (genomic)
(xi) SEQUENCE DESCRIPTION: SEQ ID NO: 29:

```
TTGAAGACGA AAGGGCCTCG TGATACGCCT ATTTTTATAG GTTAATGTCA TGATAATAAT     60
GGTTTCTTAG ACGTCAGGTG GCACTTTTCG GGGAAATGTG CGCGGAACCC CTATTTGTTT    120
ATTTTTCTAA ATACATTCAA ATATGTATCC GCTCATGAGA CAATAACCCT GATAAATGCT    180
TCAATAATAT TGAAAAAGGA AGAGTATGAG TATTCAACAT TTCCGTGTCG CCCTTATTCC    240
CTTTTTTGCG GCATTTTGCC TTCCTGTTTT TGCTCACCCA GAAACGCTGG TGAAAGTAAA    300
AGATGCTGAA GATCAGTTGG GTGCACGAGT GGGTTACATC GAACTGGATC TCAACAGCGG    360
TAAGATCCTT GAGAGTTTTC GCCCCGAAGA ACGTTTTCCA ATGATGAGCA CTTTTAAAGT    420
TCTGCTATGT GGCGCGGTAT TATCCCGTGT TGACGCCGGG CAAGAGCAAC TCGGTCGCCG    480
CATACACTAT TCTCAGAATG ACTTGGTTGA GTACTCACCA GTCACAGAAA AGCATCTTAC    540
GGATGGCATG ACAGTAAGAG AATTATGCAG TGCTGCCATA ACCATGAGTG ATAACACTGC    600
GGCCAACTTA CTTCTGACAA CGATCGGAGG ACCGAAGGAG CTAACCGCTT TTTTGCACAA    660
CATGGGGGAT CATGTAACTC GCCTTGATCG TTGGGAACCG GAGCTGAATG AAGCCATACC    720
AAACGACGAG CGTGACACCA CGATGCCTGC AGCAATGGCA ACAACGTTGC GCAAACTATT    780
AACTGGCGAA CTACTTACTC TAGCTTCCCG GCAACAATTA ATAGACTGGA TGGAGGCGGA    840
TAAAGTTGCA GGACCACTTC TGCGCTCGGC CCTTCCGGCT GGCTGGTTTA TTGCTGATAA    900
ATCTGGAGCC GGTGAGCGTG GGTCTCGCGG TATCATTGCA GCACTGGGGC CAGATGGTAA    960
GCCCTCCCGT ATCGTAGTTA TCTACACGAC GGGGAGTCAG GCAACTATGG ATGAACGAAA   1020
TAGACAGATC GCTGAGATAG GTGCCTCACT GATTAAGCAT TGGTAACTGT CAGACCAAGT   1080
TTACTCATAT ATACTTTAGA TTGATTTAAA ACTTCATTTT TAATTTAAAA GGATCTAGGT   1140
GAAGATCCTT TTTGATAATC TCATGACCAA AATCCCTTAA CGTGAGTTTT CGTTCCACTG   1200
AGCGTCAGAC CCCGTAGAAA AGATCAAAGG ATCTTCTTGA GATCCTTTTT TTCTGCGCGT   1260
AATCTGCTGC TTGCAAACAA AAAAACCACC GCTACCAGCG GTGGTTTGTT TGCCGGATCA   1320
AGAGCTACCA ACTCTTTTTC CGAAGGTAAC TGGCTTCAGC AGAGCGCAGA TACCAAATAC   1380
```

TGTCCTTCTA GTGTAGCCGT AGTTAGGCCA CCACTTCAAG AACTCTGTAG CACCGCCTAC 1440
ATACCTCGCT CTGCTAATCC TGTTACCAGT GGCTGCTGCC AGTGGCGATA AGTCGTGTCT 1500
TACCGGGTTG GACTCAAGAC GATAGTTACC GGATAAGGCG CAGCGGTCGG GCTGAACGGG 1560
GGGTTCGTGC ACACAGCCCA GCTTGGAGCG AACGACCTAC ACCGAACTGA GATACCTACA 1620
GCGTGAGCTA TGAGAAAGCG CCACGCTTCC CGAAGGGAGA AAGGCGGACA GGTATCCGGT 1680
AAGCGGCAGG GTCGGAACAG GAGAGCGCAC GAGGGAGCTT CCAGGGGGAA ACGCCTGGTA 1740
TCTTTATAGT CCTGTCGGGT TTCGCCACCT CTGACTTGAG CGTCGATTTT TGTGATGCTC 1800
GTCAGGGGGG CGGAGCCTAT GGAAAAACGC CAGCAACGCG GCCTTTTTAC GGTTCCTGGC 1860
CTTTTGCTGG CCTTTTGCTC ACATGTTCTT TCCTGCGTTA TCCCCTGATT CTGTGGATAA 1920
CCGTATTACC GCCTTTGAGT GAGCTGATAC CGCTCGCCGC AGCCGAACGA CCGAGCGCAG 1980
CGAGTCAGTG AGCGAGGAAG CGGAAGAGCG CCTGATGCGG TATTTTCTCC TTACGCATCT 2040
GTGCGGTATT TCACACCGCA TATGGTGCAC TCTCAGTACA ATCTGCTCTG ATGCCGCATA 2100
GTTAAGCCAG TATACACTCC GCTATCGCTA CGTGACTGGG TCATGGCTGC GCCCCGACAC 2160
CCGCCAACAC CCGCTGACGC GCCCTGACGG GCTTGTCTGC TCCCGGCATC CGCTTACAGA 2220
CAAGCTGTGA CCGTCTCCGG GAGCTGCATG TGTCAGAGGT TTTCACCGTC ATCACCGAAA 2280
CGCGCGAGGC AGCATGCATC TCAATTAGTC AGCAACCATA GTCCCGCCCC TAACTCCGCC 2340
CATCCCGCCC CTAACTCCGC CCAGTTCCGC CCATTCTCCG CCCCATGGCT GACTAATTTT 2400
TTTTATTTAT GCAGAGGCCG AGGCCGCCTC GGCCTCTGAG CTATTCCAGA AGTAGTGAGG 2460
AGGCTTTTTT GGAGGCCTAG GCTTTTGCAA AAAGCTAGCT TACAGCTCAG GGCTGCGATT 2520
TCGCGCCAAA CTTGACGGCA ATCCTAGCGT GAAGGCTGGT AGGATTTTAT CCCCGCTGCC 2580
ATCATGGTTC GACCATTGAA CTGCATCGTC GCCGTGTCCC AAAATATGGG GATTGGCAAG 2640
AACGGAGACC TACCCTGGCC TCCGCTCAGG AACGAGTTCA AGTACTTCCA AAGAATGACC 2700
ACAACCTCTT CAGTGGAAGG TAAACAGAAT CTGGTGATTA TGGGTAGGAA AACCTGGTTC 2760
TCCATTCCTG AGAAGAATCG ACCTTTAAAG GACAGAATTA ATATAGTTCT CAGTAGAGAA 2820
CTCAAAGAAC CACCACGAGG AGCTCATTTT CTTGCCAAAA GTTTGGATGA TGCCTTAAGA 2880
CTTATTGAAC AACCGGAATT GGCAAGTAAA GTAGACATGG TTTGGATAGT CGGAGGCAGT 2940
TCTGTTTACC AGGAAGCCAT GAATCAACCA GGCCACCTCA GACTCTTTGT GACAAGGATC 3000
ATGCAGGAAT TTGAAAGTGA CACGTTTTTC CCAGAAATTG ATTTGGGGAA ATATAAACTT 3060
CTCCCAGAAT ACCCAGGCGT CCTCTCTGAG GTCCAGGAGG AAAAAGGCAT CAAGTATAAG 3120
TTTGAAGTCT ACGAGAAGAA AGACTAACAG GAAGATGCTT TCAAGTTCTC TGCTCCCCTC 3180

CTAAAGCTAT GCATTTTTAT AAGACCATGG GACTTTTGCT GGCTTTAGAT CTTTGTGAAG 3240

GAACCTTACT TCTGTGGTGT GACATAATTG GACAAACTAC CTACAGAGAT TTAAAGCTCT 3300

AAGGTAAATA TAAAATTTTT AAGTGTATAA TGTGTTAAAC TACTGATTCT AATTGTTTGT 3360

GTATTTTAGA TTCCAACCTA TGGAACTGAT GAATGGGAGC AGTGGTGGAA TGCCTTTAAT 3420

GAGGAAAACC TGTTTTGCTC AGAAGAAATG CCATCTAGTG ATGATGAGGC TACTGCTGAC 3480

TCTCAACATT CTACTCCTCC AAAAAAGAAG AGAAAGGTAG AAGACCCCAA GGACTTTCCT 3540

TCAGAATTGC TAAGTTTTTT GAGTCATGCT GTGTTTAGTA ATAGAACTCT TGCTTGCTTT 3600

GCTATTTACA CCACAAAGGA AAAAGCTGCA CTGCTATACA AGAAAATTAT GGAAAAATAT 3660

TCTGTAACCT TTATAAGTAG GCATAACAGT TATAATCATA ACATACTGTT TTTTCTTACT 3720

CCACACAGGC ATAGAGTGTC TGCTATTAAT AACTATGCTC AAAAATTGTG TACCTTTAGC 3780

TTTTTAATTT GTAAAGGGGT TAATAAGGAA TATTTGATGT ATAGTGCCTT GACTAGAGAT 3840

CATAATCAGC CATACCACAT TTGTAGAGGT TTTACTTGCT TTAAAAAACC TCCCACACCT 3900

CCCCCTGAAC CTGAAACATA AAATGAATGC AATTGTTGTT GTTAACTTGT TTATTGCAGC 3960

TTATAATGGT TACAAATAAA GCAATAGCAT CACAAATTTC ACAAATAAAG CATTTTTTTC 4020

ACTGCATTCT AGTTGTGGTT TGTCCAAACT CATCAATGTA TCTTATCATG TCTGGATCTA 4080

ATAAAAGATA TTTATTTTCA TTAGATATGT GTGTTGGTTT TTTGTGTGCA GTGCCTCTAT 4140

CTGGAGGCCA GGTAGGGCTG GCCTTGGGGG AGGGGGAGGC CAGAATGACT CCAAGAGCTA 4200

CAGGAAGGCA GGTCAGAGAC CCCACTGGAC AAACAGTGGC TGGACTCTGC ACCATAACAC 4260

ACAATCAACA GGGGAGTGAG CTGGAAATTT GCTAGCGAAT TCCAGCACAC TGGCGGCCGT 4320

TACTAGTTAT TAATAGTAAT CAATTACGGG GTCATTAGTT CATAGCCCAT ATATGGAGTT 4380

CCGCGTTACA TAACTTACGG TAAATGGCCC GCCTGGCTGA CCGCCCAACG ACCCCCGCCC 4440

ATTGACGTCA ATAATGACGT ATGTTCCCAT AGTAACGCCA ATAGGGACTT TCCATTGACG 4500

TCAATGGGTG GAGTATTTAC GGTAAACTGC CCACTTGGCA GTACATCAAG TGTATCATAT 4560

GCCAAGTACG CCCCCTATTG ACGTCAATGA CGGTAAATGG CCCGCCTGGC ATTATGCCCA 4620

GTACATGACC TTATGGGACT TTCCTACTTG GCAGTACATC TACGTATTAG TCATCGCTAT 4680

TACCATGGTG ATGCGGTTTT GGCAGTACAT CAATGGGCGT GGATAGCGGT TTGACTCACG 4740

GGGATTTCCA AGTCTCCACC CCATTGACGT CAATGGGAGT TTGTTTTGGC ACCAAAATCA 4800

ACGGGACTTT CCAAAATGTC GTAACAACTC CGCCCCATTG ACGCAAATGG GCGGTAGGCG 4860

TGTACGGTGG GAGGTCTATA TAAGCAGAGC TCGTTTAGTG AACCGTCAGA TCGCCTGGAG 4920

ACGCCATCCA CGCTGTTTTG ACCTCCATAG AAGACACCGG GACCGATCCA GCCTCCGCGG 4980

CCGGGAACGG TGCATTGGAA CGCGGATTCC CCGTGCCAAG AGTGACGTAA GTACCGCCTA 5040

TAGAGTCTAT AGGCCCACCC CCTTGGCTTC TTATGCATGC TATACTGTTT TTGGCTTGGG 5100
GTCTATACAC CCCCGCTTCC TCATGTTATA GGTGATGGTA TAGCTTAGCC TATAGGTGTG 5160
GGTTATTGAC CATTATTGAC CACTCCCCTA TTGGTGACGA TACTTTCCAT TACTAATCCA 5220
TAACATGGCT CTTTGCCACA ACTCTCTTTA TTGGCTATAT GCCAATACAC TGTCCTTCAG 5280
AGACTGACAC GGACTCTGTA TTTTTACAGG ATGGGGTCTC ATTTATTATT TACAAATTCA 5340
CATATACAAC ACCACCGTCC CCAGTGCCCG CAGTTTTTAT TAAACATAAC GTGGGATCTC 5400
CACGCGAATC TCGGGTACGT GTTCCGGACA TGGGCTCTTC TCCGGTAGCG GCGGAGCTTC 5460
TACATCCGAG CCCTGCTCCC ATGCCTCCAG CGACTCATGG TCGCTCGGCA GCTCCTTGCT 5520
CCTAACAGTG GAGGCCAGAC TTAGGCACAG CACGATGCCC ACCACCACCA GTGTGCCGCA 5580
CAAGGCCGTG GCGGTAGGGT ATGTGTCTGA AAATGAGCTC GGGGAGCGGG CTTGCACCGC 5640
TGACGCATTT GGAAGACTTA AGGCAGCGGC AGAAGAAGAT GCAGGCAGCT GAGTTGTTGT 5700
GTTCTGATAA GAGTCAGAGG TAACTCCCGT TGCGGTGCTG TTAACGGTGG AGGGCAGTGT 5760
AGTCTGAGCA GTACTCGTTG CTGCCGCGCG CGCCACCAGA CATAATAGCT GACAGACTAA 5820
CAGACTGTTC CTTTCCATGG GTCTTTTCTG CAGTCACCGT CCTTGACACG CGTCTCGGGA 5880
AGCTTGCCGC CACCATGGGA TGGAGCTGGG TCTTTCTCTT TCTCCTGTCA GGAACTGCAG 5940
GTGTCCTCTC TGAGGTCCAG CTGCAACAGT CTGGACCTGA GCTGGTGAAG CCTGGGGCTT 6000
CAGTAAAGAT GTCCTGCAAG ACTTCTAGAT ACACATTCAC TGAATACACC ATACACTGGG 6060
TGAGACAGAG CCATGGAAAG AGCCTTGAGT GGATTGGAGG TATTAATCCT AACAATGGTA 6120
TTCCTAACTA CAACCAGAAG TTCAAGGGCA GGGCCACATT GACTGTAGGC AAGTCCTCCA 6180
GCACCGCCTA CATGGAGCTC CGCAGCCTGA CATCTGAGGA TTCTGCGGTC TATTTCTGTG 6240
CAAGAAGAAG AATCGCCTAT GGTTACGACG AGGGCCATGC TATGGACTAC TGGGGTCAAG 6300
GAACCTCAGT CACCGTCTCC TCAGGTGAGT GGATCCTCTG CGCCTGGGCC CAGCTCTGTC 6360
CCACACCGCG GTCACATGGC ACCACCTCTC TTGCAGCCTC CACCAAGGGC CCATCGGTCT 6420
TCCCCCTGGC ACCCTCCTCC AAGAGCACCT CTGGGGGCAC AGCGGCCCTG GGCTGCCTGG 6480
TCAAGGACTA CTTCCCCGAA CCGGTGACGG TGTCGTGGAA CTCAGGCGCC CTGACCAGCG 6540
GCGTGCACAC CTTCCCGGCT GTCCTACAGT CCTCAGGACT CTACTCCCTC AGCAGCGTGG 6600
TGACCGTGCC CTCCAGCAGC TTGGGCACCC AGACCTACAT CTGCAACGTG AATCACAAGC 6660
CCAGCAACAC CAAGGTGGAC AAGAAAGTTG AGCCCAAATC TTGTGACAAA ACTCACACAT 6720
GCCCACCGTG CCCAGCACCT GAACTCCTGG GGGGACCGTC AGTCTTCCTC TTCCCCCCAA 6780
AACCCAAGGA CACCCTCATG ATCTCCCGGA CCCCTGAGGT CACATGCGTG GTGGTGGACG 6840

TGAGCCACGA AGACCCTGAG GTCAAGTTCA ACTGGTACGT GGACGGCGTG GAGGTGCATA 6900

ATGCCAAGAC AAAGCCGCGG GAGGAGCAGT ACAACAGCAC GTACCGGGTG GTCAGCGTCC 6960

TCACCGTCCT GCACCAGGAC TGGCTGAATG GCAAGGAGTA CAAGTGCAAG GTCTCCAACA 7020

AAGCCCTCCC AGCCCCCATC GAGAAAACCA TCTCCAAAGC CAAAGGGCAG CCCCGAGAAC 7080

CACAGGTGTA CACCCTGCCC CCATCCCGGG AGGAGATGAC CAAGAACCAG GTCAGCCTGA 7140

CCTGCCTGGT CAAAGGCTTC TATCCCAGCG ACATCGCCGT GGAGTGGGAG AGCAATGGGC 7200

AGCCGGAGAA CAACTACAAG ACCACGCCTC CCGTGCTGGA CTCCGACGGC TCCTTCTTCC 7260

TCTACAGCAA GCTCACCGTG GACAAGAGCA GGTGGCAGCA GGGGAACGTC TTCTCATGCT 7320

CCGTGATGCA TGAGGCTCTG CACAACCACT ACACGCAGAA GAGCCTCTCC CTGTCTCCGG 7380

GTAAATGAGT GCGACGGCCG GCAAGCCCCG CTCCCCGGGC TCTCGCGGTC GCACGAGGAT 7440

GCTTGGCACG TACCCCCTGT ACATACTTCC CGGGCGCCCA GCATGGAAAT AAAGCACCGG 7500

ATCTAATAAA AGATATTTAT TTTCATTAGA TATGTGTGTT GGTTTTTTGT GTGCAGTGCC 7560

TCTATCTGGA GGCCAGGTAG GGCTGGCCTT GGGGGAGGGG GAGGCCAGAA TGACTCCAAG 7620

AGCTACAGGA AGGCAGGTCA GAGACCCCAC TGGACAAACA GTGGCTGGAC TCTGCACCAT 7680

AACACACAAT CAACAGGGGA GTGAGCTGGA AATTTGCTAG CGAATTAATT C 7731

(2) INFORMATION FOR SEQ ID NO: 30:

(i) SEQUENCE CHARACTERISTICS:

(A) LENGTH: 472 amino acids
(B) TYPE: amino acid
(C) STRANDEDNESS: single
(D) TOPOLOGY: linear

(ii) MOLECULE TYPE: peptide
(xi) SEQUENCE DESCRIPTION: SEQ ID NO: 30:

```
Met Gly Trp Ser Trp Val Phe Leu Phe Leu Leu Ser Gly Thr Ala Gly
1               5                   10                  15

Val Leu Ser Glu Val Gln Leu Gln Gln Ser Gly Pro Glu Leu Val Lys
            20                  25                  30

Pro Gly Ala Ser Val Lys Met Ser Cys Lys Thr Ser Arg Tyr Thr Phe
        35                  40                  45

Thr Glu Tyr Thr Ile His Trp Val Arg Gln Ser His Gly Lys Ser Leu
    50                  55                  60

Glu Trp Ile Gly Gly Ile Asn Pro Asn Asn Gly Ile Pro Asn Tyr Asn
65                  70                  75                  80

Gln Lys Phe Lys Gly Arg Ala Thr Leu Thr Val Gly Lys Ser Ser Ser
```

85 90 95

Thr Ala Tyr Met Glu Leu Arg Ser Leu Thr Ser Glu Asp Ser Ala Val
100 105 110

Tyr Phe Cys Ala Arg Arg Arg Ile Ala Tyr Gly Tyr Asp Glu Gly His
115 120 125

Ala Met Asp Tyr Trp Gly Gln Gly Thr Ser Val Thr Val Ser Ser Ser
130 135 140

Thr Lys Gly Pro Ser Val Phe Pro Leu Ala Pro Ser Ser Lys Ser Thr
145 150 155 160

Ser Gly Gly Thr Ala Ala Leu Gly Cys Leu Val Lys Asp Tyr Phe Pro
165 170 175

Glu Pro Val Thr Val Ser Trp Asn Ser Gly Ala Leu Thr Ser Gly Val
180 185 190

His Thr Phe Pro Ala Val Leu Gln Ser Ser Gly Leu Tyr Ser Leu Ser
195 200 205

Ser Val Val Thr Val Pro Ser Ser Ser Leu Gly Thr Gln Thr Tyr Ile
210 215 220

Cys Asn Val Asn His Lys Pro Ser Asn Thr Lys Val Asp Lys Lys Val
225 230 235 240

Glu Pro Lys Ser Cys Asp Lys Thr His Thr Cys Pro Pro Cys Pro Ala
245 250 255

Pro Glu Leu Leu Gly Gly Pro Ser Val Phe Leu Phe Pro Pro Lys Pro
260 265 270

Lys Asp Thr Leu Met Ile Ser Arg Thr Pro Glu Val Thr Cys Val Val
275 280 285

Val Asp Val Ser His Glu Asp Pro Glu Val Lys Phe Asn Trp Tyr Val
290 295 300

Asp Gly Val Glu Val His Asn Ala Lys Thr Lys Pro Arg Glu Glu Gln
305 310 315 320

Tyr Asn Ser Thr Tyr Arg Val Val Ser Val Leu Thr Val Leu His Gln
325 330 335

Asp Trp Leu Asn Gly Lys Glu Tyr Lys Cys Lys Val Ser Asn Lys Ala
340 345 350

Leu Pro Ala Pro Ile Glu Lys Thr Ile Ser Lys Ala Lys Gly Gln Pro
355 360 365

Arg Glu Pro Gln Val Tyr Thr Leu Pro Pro Ser Arg Glu Glu Met Thr
370 375 380

Lys Asn Gln Val Ser Leu Thr Cys Leu Val Lys Gly Phe Tyr Pro Ser
385 390 395 400

Asp Ile Ala Val Glu Trp Glu Ser Asn Gly Gln Pro Glu Asn Asn Tyr
405 410 415

```
Lys Thr Thr Pro Pro Val Leu Asp Ser Asp Gly Ser Phe Phe Leu Tyr
            420                 425                 430

Ser Lys Leu Thr Val Asp Lys Ser Arg Trp Gln Gln Gly Asn Val Phe
        435                 440                 445

Ser Cys Ser Val Met His Glu Ala Leu His Asn His Tyr Thr Gln Lys
    450                 455                 460

Ser Leu Ser Leu Ser Pro Gly Lys
465                 470
```

(2) INFORMATION FOR SEQ ID NO: 31:

(i) SEQUENCE CHARACTERISTICS:

(A) LENGTH: 339 base pairs
(B) TYPE: nucleic acid
(C) STRANDEDNESS: double
(D) TOPOLOGY: linear

(ii) MOLECULE TYPE: cDNA
(xi) SEQUENCE DESCRIPTION: SEQ ID NO: 31:

```
GACATTGTGA TGACCCAATC TCCAGACTCT TTGGCTGTGT CTCTAGGGGA GAGGGCCACC     60

ATCAACTGCA AGTCCAGTCA GAGCCTTTTA TATTCTAGAA ATCAAAAGAA CTACTTGGCC    120

TGGTATCAGC AGAAACCAGG ACAGCCACCC AAACTCCTCA TCTTTTGGGC TAGCACTAGG    180

GAATCTGGGG TACCTGATAG GTTCAGTGGC AGTGGGTTTG GGACAGACTT CACCCTCACC    240

ATTAGCAGCC TGCAGGCTGA AGATGTGGCA GTTTATTACT GTCAGCAATA TTTTAGCTAT    300

CCGCTCACGT TCGGACAAGG GACCAAGGTG GAAATAAAA                           339
```

(2) INFORMATION FOR SEQ ID NO: 32:

(i) SEQUENCE CHARACTERISTICS:

(A) LENGTH: 113 amino acids
(B) TYPE: amino acid
(C) STRANDEDNESS: single
(D) TOPOLOGY: linear

(ii) MOLECULE TYPE: peptide
(xi) SEQUENCE DESCRIPTION: SEQ ID NO: 32:

```
Asp Ile Val Met Thr Gln Ser Pro Asp Ser Leu Ala Val Ser Leu Gly
1               5                   10                  15

Glu Arg Ala Thr Ile Asn Cys Lys Ser Ser Gln Ser Leu Leu Tyr Ser
            20                  25                  30
```

```
Arg Asn Gln Lys Asn Tyr Leu Ala Trp Tyr Gln Gln Lys Pro Gly Gln
        35                  40                  45

Pro Pro Lys Leu Leu Ile Phe Trp Ala Ser Thr Arg Glu Ser Gly Val
    50                  55                  60

Pro Asp Arg Phe Ser Gly Ser Gly Phe Gly Thr Asp Phe Thr Leu Thr
65                  70                  75                  80

Ile Ser Ser Leu Gln Ala Glu Asp Val Ala Val Tyr Tyr Cys Gln Gln
                85                  90                  95

Tyr Phe Ser Tyr Pro Leu Thr Phe Gly Gln Gly Thr Lys Val Glu Ile
            100                 105                 110

Lys
```

(2) INFORMATION FOR SEQ ID NO: 33:

(i) SEQUENCE CHARACTERISTICS:

(A) LENGTH: 113 amino acids
(B) TYPE: amino acid
(C) STRANDEDNESS: single
(D) TOPOLOGY: linear

(ii) MOLECULE TYPE: peptide
(xi) SEQUENCE DESCRIPTION: SEQ ID NO: 33:

```
Asp Ile Val Met Thr Gln Ser Pro Asp Ser Leu Ala Val Ser Leu Gly
1               5                   10                  15

Glu Arg Ala Thr Ile Asn Cys Lys Ser Ser Gln Ser Leu Leu Tyr Ser
            20                  25                  30

Arg Asn Gln Lys Asn Tyr Leu Ala Trp Phe Gln Gln Lys Pro Gly Gln
        35                  40                  45

Pro Pro Lys Leu Leu Ile Phe Trp Ala Ser Thr Arg Glu Ser Gly Val
    50                  55                  60

Pro Asp Arg Phe Ser Gly Ser Gly Phe Gly Thr Asp Phe Thr Leu Thr
65                  70                  75                  80

Ile Ser Ser Leu Gln Ala Glu Asp Val Ala Val Tyr Asp Cys Gln Gln
                85                  90                  95

Tyr Phe Ser Tyr Pro Leu Thr Phe Gly Gln Gly Thr Lys Val Glu Ile
            100                 105                 110

Lys
```

(2) INFORMATION FOR SEQ ID NO: 34:

(i) SEQUENCE CHARACTERISTICS:

(A) LENGTH: 113 amino acids
(B) TYPE: amino acid

(C) STRANDEDNESS: single
(D) TOPOLOGY: linear

(ii) MOLECULE TYPE: peptide
(xi) SEQUENCE DESCRIPTION: SEQ ID NO: 34:

```
Asp Ile Val Met Thr Gln Ser Pro Asp Ser Leu Ala Val Ser Leu Gly
1               5                   10                  15

Glu Arg Ala Thr Ile Asn Cys Lys Ser Ser Gln Ser Leu Leu Tyr Ser
            20                  25                  30

Arg Asn Gln Lys Asn Tyr Leu Ala Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Ser Gly Ser Gly Phe Gly Thr Asp Phe Thr Leu Thr
65                  70                  75                  80

Ile Ser Ser Leu Gln Ala Glu Asp Val Ala Val Tyr Tyr Cys Gln Gln
                85                  90                  95

Tyr Phe Ser Tyr Pro Leu Thr Phe Gly Gln Gly Thr Lys Val Glu Ile
            100                 105                 110

Lys
```

(2) INFORMATION FOR SEQ ID NO: 35:

(i) SEQUENCE CHARACTERISTICS:

(A) LENGTH: 8068 base pairs
(B) TYPE: nucleic acid
(C) STRANDEDNESS: double
(D) TOPOLOGY: linear

(ii) MOLECULE TYPE: DNA (genomic)
(xi) SEQUENCE DESCRIPTION: SEQ ID NO: 35:

```
GAATTCCAGC ACACTGGCGG CCGTTACTAG TTATTAATAG TAATCAATTA CGGGGTCATT     60

AGTTCATAGC CCATATATGG AGTTCCGCGT TACATAACTT ACGGTAAATG GCCCGCCTGG    120

CTGACCGCCC AACGACCCCC GCCCATTGAC GTCAATAATG ACGTATGTTC CCATAGTAAC    180

GCCAATAGGG ACTTTCCATT GACGTCAATG GGTGGAGTAT TTACGGTAAA CTGCCCACTT    240

GGCAGTACAT CAAGTGTATC ATATGCCAAG TACGCCCCCT ATTGACGTCA ATGACGGTAA    300

ATGGCCCGCC TGGCATTATG CCCAGTACAT GACCTTATGG GACTTTCCTA CTTGGCAGTA    360
```

CATCTACGTA TTAGTCATCG CTATTACCAT GGTGATGCGG TTTTGGCAGT ACATCAATGG 420
GCGTGGATAG CGGTTTGACT CACGGGGATT TCCAAGTCTC CACCCCATTG ACGTCAATGG 480
GAGTTTGTTT TGGCACCAAA ATCAACGGGA CTTTCCAAAA TGTCGTAACA ACTCCGCCCC 540
ATTGACGCAA ATGGGCGGTA GGCGTGTACG GTGGGAGGTC TATATAAGCA GAGCTCGTTT 600
AGTGAACCGT CAGATCGCCT GGAGACGCCA TCCACGCTGT TTTGACCTCC ATAGAAGACA 660
CCGGGACCGA TCCAGCCTCC GCGGCCGGGA ACGGTGCATT GGAACGCGGA TTCCCCGTGC 720
CAAGAGTGAC GTAAGTACCG CCTATAGAGT CTATAGGCCC ACCCCCTTGG CTTCTTATGC 780
ATGCTATACT GTTTTTGGCT TGGGGTCTAT ACACCCCCGC TTCCTCATGT TATAGGTGAT 840
GGTATAGCTT AGCCTATAGG TGTGGGTTAT TGACCATTAT TGACCACTCC CCTATTGGTG 900
ACGATACTTT CCATTACTAA TCCATAACAT GGCTCTTTGC CACAACTCTC TTTATTGGCT 960
ATATGCCAAT ACACTGTCCT TCAGAGACTG ACACGGACTC TGTATTTTTA CAGGATGGGG 1020
TCTCATTTAT TATTTACAAA TTCACATATA CAACACCACC GTCCCCAGTG CCCGCAGTTT 1080
TTATTAAACA TAACGTGGGA TCTCCACGCG AATCTCGGGT ACGTGTTCCG GACATGGGCT 1140
CTTCTCCGGT AGCGGCGGAG CTTCTACATC CGAGCCCTGC TCCCATGCCT CCAGCGACTC 1200
ATGGTCGCTC GGCAGCTCCT TGCTCCTAAC AGTGGAGGCC AGACTTAGGC ACAGCACGAT 1260
GCCCACCACC ACCAGTGTGC CGCACAAGGC CGTGGCGGTA GGGTATGTGT CTGAAAATGA 1320
GCTCGGGGAG CGGGCTTGCA CCGCTGACGC ATTTGGAAGA CTTAAGGCAG CGGCAGAAGA 1380
AGATGCAGGC AGCTGAGTTG TTGTGTTCTG ATAAGAGTCA GAGGTAACTC CCGTTGCGGT 1440
GCTGTTAACG GTGGAGGGCA GTGTAGTCTG AGCAGTACTC GTTGCTGCCG CGCGCGCCAC 1500
CAGACATAAT AGCTGACAGA CTAACAGACT GTTCCTTTCC ATGGGTCTTT TCTGCAGTCA 1560
CCGTCCTTGA CACGCGTCTC GGGAAGCTTG CCGCCACCAT GGAGACAGAC ACACTCCTGC 1620
TATGGGTGCT GCTGCTCTGG GTTCCAGGTT CCTCCGGAGA CATTGTGATG ACCCAATCTC 1680
CAGACTCTTT GGCTGTGTCT CTAGGGGAGA GGGCCACCAT CAACTGCAAG TCCAGTCAGA 1740
GCCTTTTATA TTCTAGAAAT CAAAAGAACT ACTTGGCCTG GTATCAGCAG AAACCAGGAC 1800
AGCCACCCAA ACTCCTCATC TTTTGGGCTA GCACTAGGGA ATCTGGGGTA CCTGATAGGT 1860
TCAGTGGCAG TGGGTTTGGG ACAGACTTCA CCCTCACCAT TAGCAGCCTG CAGGCTGAAG 1920
ATGTGGCAGT TTATTACTGT CAGCAATATT TTAGCTATCC GCTCACGTTC GGACAAGGGA 1980
CCAAGGTGGA AATAAAACGT GAGTGGATCC ATCTGGGATA AGCATGCTGT TTTCTGTCTG 2040
TCCCTAACAT GCCCTGTGAT TATGCGCAAA CAACACACCC AAGGGCAGAA CTTTGTTACT 2100
TAAACACCAT CCTGTTTGCT TCTTTCCTCA GGAACTGTGG CTGCACCATC TGTCTTCATC 2160

TTCCCGCCAT CTGATGAGCA GTTGAAATCT GGAACTGCCT CTGTTGTGTG CCTGCTGAAT 2220
AACTTCTATC CCAGAGAGGC CAAAGTACAG TGGAAGGTGG ATAACGCCCT CCAATCGGGT 2280
AACTCCCAGG AGAGTGTCAC AGAGCAGGAC AGCAAGGACA GCACCTACAG CCTCAGCAGC 2340
ACCCTGACGC TGAGCAAAGC AGACTACGAG AAACACAAAG TCTACGCCTG CGAAGTCACC 2400
CATCAGGGCC TGAGCTCGCC CGTCACAAAG AGCTTCAACA GGGGAGAGTG TTAGAGGGAG 2460
AAGTGCCCCC ACCTGCTCCT CAGTTCCAGC CTGACCCCCT CCCATCCTTT GGCCTCTGAC 2520
CCTTTTTCCA CAGGGGACCT ACCCCTATTG CGGTCCTCCA GCTCATCTTT CACCTCACCC 2580
CCCTCCTCCT CCTTGGCTTT AATTATGCTA ATGTTGGAGG AGAATGAATA AATAAAGTGA 2640
ATCTTTGCAC CTGTGGTGGA TCTAATAAAA GATATTTATT TTCATTAGAT ATGTGTGTTG 2700
GTTTTTTGTG TGCAGTGCCT CTATCTGGAG GCCAGGTAGG GCTGGCCTTG GGGGAGGGGG 2760
AGGCCAGAAT GACTCCAAGA GCTACAGGAA GGCAGGTCAG AGACCCCACT GGACAAACAG 2820
TGGCTGGACT CTGCACCATA ACACACAATC AACAGGGGAG TGAGCTGGAA ATTTGCTAGC 2880
GAATTCTTGA AGACGAAAGG GCCTCGTGAT ACGCCTATTT TTATAGGTTA ATGTCATGAT 2940
AATAATGGTT TCTTAGACGT CAGGTGGCAC TTTTCGGGGA AATGTGCGCG GAACCCCTAT 3000
TTGTTTATTT TTCTAAATAC ATTCAAATAT GTATCCGCTC ATGAGACAAT AACCCTGATA 3060
AATGCTTCAA TAATATTGAA AAAGGAAGAG TATGAGTATT CAACATTTCC GTGTCGCCCT 3120
TATTCCCTTT TTTGCGGCAT TTTGCCTTCC TGTTTTTGCT CACCCAGAAA CGCTGGTGAA 3180
AGTAAAAGAT GCTGAAGATC AGTTGGGTGC ACGAGTGGGT TACATCGAAC TGGATCTCAA 3240
CAGCGGTAAG ATCCTTGAGA GTTTTCGCCC CGAAGAACGT TTTCCAATGA TGAGCACTTT 3300
TAAAGTTCTG CTATGTGGCG CGGTATTATC CCGTGTTGAC GCCGGGCAAG AGCAACTCGG 3360
TCGCCGCATA CACTATTCTC AGAATGACTT GGTTGAGTAC TCACCAGTCA CAGAAAAGCA 3420
TCTTACGGAT GGCATGACAG TAAGAGAATT ATGCAGTGCT GCCATAACCA TGAGTGATAA 3480
CACTGCGGCC AACTTACTTC TGACAACGAT CGGAGGACCG AAGGAGCTAA CCGCTTTTTT 3540
GCACAACATG GGGGATCATG TAACTCGCCT TGATCGTTGG GAACCGGAGC TGAATGAAGC 3600
CATACCAAAC GACGAGCGTG ACACCACGAT GCCTGCAGCA ATGGCAACAA CGTTGCGCAA 3660
ACTATTAACT GGCGAACTAC TTACTCTAGC TTCCCGGCAA CAATTAATAG ACTGGATGGA 3720
GGCGGATAAA GTTGCAGGAC CACTTCTGCG CTCGGCCCTT CCGGCTGGCT GGTTTATTGC 3780
TGATAAATCT GGAGCCGGTG AGCGTGGGTC TCGCGGTATC ATTGCAGCAC TGGGGCCAGA 3840
TGGTAAGCCC TCCCGTATCG TAGTTATCTA CACGACGGGG AGTCAGGCAA CTATGGATGA 3900
ACGAAATAGA CAGATCGCTG AGATAGGTGC CTCACTGATT AAGCATTGGT AACTGTCAGA 3960
CCAAGTTTAC TCATATATAC TTTAGATTGA TTTAAAACTT CATTTTTAAT TTAAAAGGAT 4020

CTAGGTGAAG ATCCTTTTTG ATAATCTCAT GACCAAAATC CCTTAACGTG AGTTTTCGTT 4080
CCACTGAGCG TCAGACCCCG TAGAAAAGAT CAAAGGATCT TCTTGAGATC CTTTTTTTCT 4140
GCGCGTAATC TGCTGCTTGC AAACAAAAAA ACCACCGCTA CCAGCGGTGG TTTGTTTGCC 4200
GGATCAAGAG CTACCAACTC TTTTTCCGAA GGTAACTGGC TTCAGCAGAG CGCAGATACC 4260
AAATACTGTC CTTCTAGTGT AGCCGTAGTT AGGCCACCAC TTCAAGAACT CTGTAGCACC 4320
GCCTACATAC CTCGCTCTGC TAATCCTGTT ACCAGTGGCT GCTGCCAGTG GCGATAAGTC 4380
GTGTCTTACC GGGTTGGACT CAAGACGATA GTTACCGGAT AAGGCGCAGC GGTCGGGCTG 4440
AACGGGGGGT TCGTGCACAC AGCCCAGCTT GGAGCGAACG ACCTACACCG AACTGAGATA 4500
CCTACAGCGT GAGCTATGAG AAAGCGCCAC GCTTCCCGAA GGGAGAAAGG CGGACAGGTA 4560
TCCGGTAAGC GGCAGGGTCG GAACAGGAGA GCGCACGAGG GAGCTTCCAG GGGGAAACGC 4620
CTGGTATCTT TATAGTCCTG TCGGGTTTCG CCACCTCTGA CTTGAGCGTC GATTTTTGTG 4680
ATGCTCGTCA GGGGGGCGGA GCCTATGGAA AAACGCCAGC AACGCGGCCT TTTTACGGTT 4740
CCTGGCCTTT TGCTGGCCTT TTGCTCACAT GTTCTTTCCT GCGTTATCCC CTGATTCTGT 4800
GGATAACCGT ATTACCGCCT TTGAGTGAGC TGATACCGCT CGCCGCAGCC GAACGACCGA 4860
GCGCAGCGAG TCAGTGAGCG AGGAAGCGGA AGAGCGCCTG ATGCGGTATT TTCTCCTTAC 4920
GCATCTGTGC GGTATTTCAC ACCGCATATG GTGCACTCTC AGTACAATCT GCTCTGATGC 4980
CGCATAGTTA AGCCAGTATA CACTCCGCTA TCGCTACGTG ACTGGGTCAT GGCTGCGCCC 5040
CGACACCCGC CAACACCCGC TGACGCGCCC TGACGGGCTT GTCTGCTCCC GGCATCCGCT 5100
TACAGACAAG CTGTGACCGT CTCCGGGAGC TGCATGTGTC AGAGGTTTTC ACCGTCATCA 5160
CCGAAACGCG CGAGGCAGCT GTGGAATGTG TGTCAGTTAG GGTGTGGAAA GTCCCCAGGC 5220
TCCCCAGCAG GCAGAAGTAT GCAAAGCATG CATCTCAATT AGTCAGCAAC CAGGCTCCCC 5280
AGCAGGCAGA AGTATGCAAA GCATGCATCT CAATTAGTCA GCAACCATAG TCCCGCCCCT 5340
AACTCCGCCC ATCCCGCCCC TAACTCCGCC CAGTTCCGCC CATTCTCCGC CCCATGGCTG 5400
ACTAATTTTT TTTATTTATG CAGAGGCCGA GGCCGCCTCG GCCTCTGAGC TATTCCAGAA 5460
GTAGTGAGGA GGCTTTTTTG GAGGCCTAGG CTTTTGCAAA AAGCTAGCTT CACGCTGCCG 5520
CAAGCACTCA GGGCGCAAGG GCTGCTAAAG GAAGCGGAAC ACGTAGAAAG CCAGTCCGCA 5580
GAAACGGTGC TGACCCCGGA TGAATGTCAG CTACTGGGCT ATCTGGACAA GGGAAAACGC 5640
AAGCGCAAAG AGAAAGCAGG TAGCTTGCAG TGGGCTTACA TGGCGATAGC TAGACTGGGC 5700
GGTTTTATGG ACAGCAAGCG AACCGGAATT GCCAGCTGGG GCGCCCTCTG GTAAGGTTGG 5760
GAAGCCCTGC AAAGTAAACT GGATGGCTTT CTTGCCGCCA AGGATCTGAT GGCGCAGGGG 5820

ATCAAGATCT GATCAAGAGA CAGGATGAGG ATCGTTTCGC ATGATTGAAC AAGATGGATT 5880

GCACGCAGGT TCTCCGGCCG CTTGGGTGGA GAGGCTATTC GGCTATGACT GGGCACAACA 5940

GACAATCGGC TGCTCTGATG CCGCCGTGTT CCGGCTGTCA GCGCAGGGGC GCCCGGTTCT 6000

TTTTGTCAAG ACCGACCTGT CCGGTGCCCT GAATGAACTG CAGGACGAGG CAGCGCGGCT 6060

ATCGTGGCTG GCCACGACGG GCGTTCCTTG CGCAGCTGTG CTCGACGTTG TCACTGAAGC 6120

GGGAAGGGAC TGGCTGCTAT TGGGCGAAGT GCCGGGGCAG GATCTCCTGT CATCTCACCT 6180

TGCTCCTGCC GAGAAAGTAT CCATCATGGC TGATGCAATG CGGCGGCTGC ATACGCTTGA 6240

TCCGGCTACC TGCCCATTCG ACCACCAAGC GAAACATCGC ATCGAGCGAG CACGTACTCG 6300

GATGGAAGCC GGTCTTGTCG ATCAGGATGA TCTGGACGAA GAGCATCAGG GGCTCGCGCC 6360

AGCCGAACTG TTCGCCAGGC TCAAGGCGCG CATGCCCGAC GGCGAGGATC TCGTCGTGAC 6420

CCATGGCGAT GCCTGCTTGC CGAATATCAT GGTGGAAAAT GGCCGCTTTT CTGGATTCAT 6480

CGACTGTGGC CGGCTGGGTG TGGCGGACCG CTATCAGGAC ATAGCGTTGG CTACCCGTGA 6540

TATTGCTGAA GAGCTTGGCG GCGAATGGGC TGACCGCTTC CTCGTGCTTT ACGGTATCGC 6600

CGCTCCCGAT TCGCAGCGCA TCGCCTTCTA TCGCCTTCTT GACGAGTTCT TCTGAGCGGG 6660

ACTCTGGGGT TCGAAATGAC CGACCAAGCG ACGCCCAACC TGCCATCACG AGATTTCGAT 6720

TCCACCGCCG CCTTCTATGA AAGGTTGGGC TTCGGAATCG TTTTCCGGGA CGCCGGCTGG 6780

ATGATCCTCC AGCGCGGGGA TCTCATGCTG GAGTTCTTCG CCCACCCCGG GCTCGATCCC 6840

CTCGCGAGTT GGTTCAGCTG CTGCCTGAGG CTGGACGACC TCGCGGAGTT CTACCGGCAG 6900

TGCAAATCCG TCGGCATCCA GGAAACCAGC AGCGGCTATC CGCGCATCCA TGCCCCCGAA 6960

CTGCAGGAGT GGGGAGGCAC GATGGCCGCT TTGGTCCCGG ATCTTTGTGA AGGAACCTTA 7020

CTTCTGTGGT GTGACATAAT TGGACAAACT ACCTACAGAG ATTTAAAGCT CTAAGGTAAA 7080

TATAAAATTT TTAAGTGTAT AATGTGTTAA ACTACTGATT CTAATTGTTT GTGTATTTTA 7140

GATTCCAACC TATGGAACTG ATGAATGGGA GCAGTGGTGG AATGCCTTTA ATGAGGAAAA 7200

CCTGTTTTGC TCAGAAGAAA TGCCATCTAG TGATGATGAG GCTACTGCTG ACTCTCAACA 7260

TTCTACTCCT CCAAAAAAGA AGAGAAAGGT AGAAGACCCC AAGGACTTTC CTTCAGAATT 7320

GCTAAGTTTT TTGAGTCATG CTGTGTTTAG TAATAGAACT CTTGCTTGCT TTGCTATTTA 7380

CACCACAAAG GAAAAAGCTG CACTGCTATA CAAGAAAATT ATGGAAAAAT ATTCTGTAAC 7440

CTTTATAAGT AGGCATAACA GTTATAATCA TAACATACTG TTTTTTCTTA CTCCACACAG 7500

GCATAGAGTG TCTGCTATTA ATAACTATGC TCAAAAATTG TGTACCTTTA GCTTTTTAAT 7560

TTGTAAAGGG GTTAATAAGG AATATTTGAT GTATAGTGCC TTGACTAGAG ATCATAATCA 7620

GCCATACCAC ATTTGTAGAG GTTTTACTTG CTTTAAAAAA CCTCCCACAC CTCCCCCTGA 7680

```
ACCTGAAACA TAAAATGAAT GCAATTGTTG TTGTTAACTT GTTTATTGCA GCTTATAATG    7740
GTTACAAATA AAGCAATAGC ATCACAAATT TCACAAATAA AGCATTTTTT TCACTGCATT    7800
CTAGTTGTGG TTTGTCCAAA CTCATCAATG TATCTTATCA TGTCTGGATC TAATAAAAGA    7860
TATTTATTTT CATTAGATAT GTGTGTTGGT TTTTTGTGTG CAGTGCCTCT ATCTGGAGGC    7920
CAGGTAGGGC TGGCCTTGGG GGAGGGGGAG GCCAGAATGA CTCCAAGAGC TACAGGAAGG    7980
CAGGTCAGAG ACCCCACTGG ACAAACAGTG GCTGGACTCT GCACCATAAC ACACAATCAA    8040
CAGGGGAGTG AGCTGGAAAT TTGCTAGC                                       8068
```

(2) INFORMATION FOR SEQ ID NO: 36:

(i) SEQUENCE CHARACTERISTICS:

(A) LENGTH: 234 amino acids
(B) TYPE: amino acid
(C) STRANDEDNESS: single
(D) TOPOLOGY: linear

(ii) MOLECULE TYPE: protein
(xi) SEQUENCE DESCRIPTION: SEQ ID NO: 36:

```
Met Glu Thr Asp Thr Leu Leu Leu Trp Val Leu Leu Leu Trp Val Pro
1               5                   10                  15

Gly Ser Ser Gly Asp Ile Val Met Thr Gln Ser Pro Asp Ser Leu Ala
            20                  25                  30

Val Ser Leu Gly Glu Arg Ala Thr Ile Asn Cys Lys Ser Ser Gln Ser
        35                  40                  45

Leu Leu Tyr Ser Arg Asn Gln Lys Asn Tyr Leu Ala Trp Tyr Gln Gln
    50                  55                  60

Lys Pro Gly Gln Pro Pro Lys Leu Leu Ile Phe Trp Ala Ser Thr Arg
65                  70                  75                  80

Glu Ser Gly Val Pro Asp Arg Phe Ser Gly Ser Gly Phe Gly Thr Asp
                85                  90                  95

Phe Thr Leu Thr Ile Ser Ser Leu Gln Ala Glu Asp Val Ala Val Tyr
            100                 105                 110

Tyr Cys Gln Gln Tyr Phe Ser Tyr Pro Leu Thr Phe Gly Gln Gly Thr
        115                 120                 125

Lys Val Glu Ile Lys Arg Val Phe Ile Phe Pro Pro Ser Asp Glu Gln
    130                 135                 140

Leu Lys Ser Gly Thr Ala Ser Val Val Cys Leu Leu Asn Asn Phe Tyr
145                 150                 155                 160

Pro Arg Glu Ala Lys Val Gln Trp Lys Val Asp Asn Ala Leu Gln Ser
```

```
                165                     170                     175

Gly Asn Ser Gln Glu Ser Val Thr Glu Gln Asp Ser Lys Asp Ser Thr
            180                     185                     190

Tyr Ser Leu Ser Ser Thr Leu Thr Leu Ser Lys Ala Asp Tyr Glu Lys
        195                     200                     205

His Lys Val Tyr Ala Cys Glu Val Thr His Gln Gly Leu Ser Ser Pro
    210                     215                     220

Val Thr Lys Ser Phe Asn Arg Gly Glu Cys
225                 230
```

(2) INFORMATION FOR SEQ ID NO: 37:

(i) SEQUENCE CHARACTERISTICS:

(A) LENGTH: 372 base pairs
(B) TYPE: nucleic acid
(C) STRANDEDNESS: double
(D) TOPOLOGY: linear

(ii) MOLECULE TYPE: cDNA
(xi) SEQUENCE DESCRIPTION: SEQ ID NO: 37:

```
CAGGTGCAAC TAGTGCAGTC CGGCGCCGAA GTGAAGAAAC CCGGTGCTTC CGTGAAAGTC      60

AGCTGTAAAA CTAGTAGATA CACCTTCACT GAATACACCA TACACTGGGT TAGACAGGCC     120

CCTGGCCAAA GGCTGGAGTG GATAGGAGGT ATTAATCCTA ACAATGGTAT TCCTAACTAC     180

AACCAGAAGT TCAAGGGCCG GGCCACCTTG ACCGTAGGCA AGTCTGCCAG CACCGCCTAC     240

ATGGAACTGT CCAGCCTGCG CTCCGAGGAC ACTGCAGTCT ACTACTGCGC CAGAAGAAGA     300

ATCGCCTATG GTTACGACGA GGGCCATGCT ATGGACTACT GGGGTCAAGG AACCCTTGTC     360

ACCGTCTCCT CA                                                         372
```

(2) INFORMATION FOR SEQ ID NO: 38:

(i) SEQUENCE CHARACTERISTICS:

(A) LENGTH: 124 amino acids
(B) TYPE: amino acid
(C) STRANDEDNESS: single
(D) TOPOLOGY: linear

(ii) MOLECULE TYPE: peptide
(xi) SEQUENCE DESCRIPTION: SEQ ID NO: 38:

```
Gln Val Gln Leu Val Gln Ser Gly Ala Glu Val Lys Lys Pro Gly Ala
1               5                   10                  15
```

```
Ser Val Lys Val Ser Cys Lys Thr Ser Arg Tyr Thr Phe Thr Glu Tyr
            20                  25                  30

Thr Ile His Trp Val Arg Gln Ala Pro Gly Gln Arg Leu Glu Trp Ile
        35                  40                  45

Gly Gly Ile Asn Pro Asn Asn Gly Ile Pro Asn Tyr Asn Gln Lys Phe
    50                  55                  60

Lys Gly Arg Ala Thr Leu Thr Val Gly Lys Ser Ala Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Arg Arg Ile Ala Tyr Gly Tyr Asp Glu Gly His Ala Met Asp
            100                 105                 110

Tyr Trp Gly Gln Gly Thr Leu Val Thr Val Ser Ser
        115                 120
```

(2) INFORMATION FOR SEQ ID NO: 39:

(i) SEQUENCE CHARACTERISTICS:

(A) LENGTH: 124 amino acids
(B) TYPE: amino acid
(C) STRANDEDNESS: single
(D) TOPOLOGY: linear

(ii) MOLECULE TYPE: peptide
(xi) SEQUENCE DESCRIPTION: SEQ ID NO: 39:

```
Gln Val Gln Leu Val Gln Ser Gly Ala Glu Val Lys Lys Pro Gly Ala
1               5                   10                  15

Ser Val Lys Val Ser Cys Lys Thr Ser Arg Tyr Thr Phe Thr Glu Tyr
            20                  25                  30

Thr Ile His Trp Val Arg Gln Ala Pro Gly Gln Arg Leu Glu Trp Ile
        35                  40                  45

Gly Gly Ile Asn Pro Asn Asn Gly Ile Pro Asn Tyr Asn Gln Lys Phe
    50                  55                  60

Lys Gly Arg Ala Thr Leu Thr Val Gly Lys Ser Ala Ser Thr Ala Tyr
65                  70                  75                  80

Met Glu Leu Ser Ser Leu Arg Ser Glu Asp Thr Ala Val Tyr Phe Cys
                85                  90                  95

Ala Arg Arg Arg Ile Ala Tyr Gly Tyr Asp Glu Gly His Ala Met Asp
            100                 105                 110

Tyr Trp Gly Gln Gly Thr Leu Val Thr Val Ser Ser
        115                 120
```

(2) INFORMATION FOR SEQ ID NO: 40:

(i) SEQUENCE CHARACTERISTICS:

(A) LENGTH: 124 amino acids
(B) TYPE: amino acid
(C) STRANDEDNESS: single
(D) TOPOLOGY: linear

(ii) MOLECULE TYPE: peptide
(xi) SEQUENCE DESCRIPTION: SEQ ID NO: 40:

```
Gln Val Gln Leu Val Gln Ser Gly Ala Glu Val Lys Lys Pro Gly Ala
1               5                   10                  15

Ser Val Lys Val Ser Cys Lys Thr Ser Arg Tyr Thr Phe Thr Glu Tyr
            20                  25                  30

Thr Ile His Trp Val Arg Gln Ala Pro Gly Gln Arg Leu Glu Trp Ile
        35                  40                  45

Gly Gly Ile Asn Pro Asn Asn Gly Ile Pro Asn Tyr Asn Gln Lys Phe
    50                  55                  60

Lys Gly Arg Val Thr Ile Thr Val Asp Thr Ser Ala Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Arg Arg Ile Ala Tyr Gly Tyr Asp Glu Gly His Ala Met Asp
            100                 105                 110

Tyr Trp Gly Gln Gly Thr Leu Val Thr Val Ser Ser
        115                 120
```

(2) INFORMATION FOR SEQ ID NO: 41:

(i) SEQUENCE CHARACTERISTICS:

(A) LENGTH: 124 amino acids
(B) TYPE: amino acid
(C) STRANDEDNESS: single
(D) TOPOLOGY: linear

(ii) MOLECULE TYPE: peptide
(xi) SEQUENCE DESCRIPTION: SEQ ID NO: 41:

```
Gln Val Gln Leu Val Gln Ser Gly Ala Glu Val Lys Lys Pro Gly Ala
1               5                   10                  15

Ser Val Lys Val Ser Cys Lys Thr Ser Gly Tyr Thr Phe Thr Glu Tyr
            20                  25                  30

Thr Ile His Trp Val Arg Gln Ala Pro Gly Gln Arg Leu Glu Trp Ile
        35                  40                  45
```

```
Gly Gly Ile Asn Pro Asn Asn Gly Ile Pro Asn Tyr Asn Gln Lys Phe
    50                  55                  60

Lys Gly Arg Val Thr Ile Thr Val Asp Thr Ser Ala Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Arg Arg Ile Ala Tyr Gly Tyr Asp Glu Gly His Ala Met Asp
            100                 105                 110

Tyr Trp Gly Gln Gly Thr Leu Val Thr Val Ser Ser
        115                 120
```

(2) INFORMATION FOR SEQ ID NO: 42:

(i) SEQUENCE CHARACTERISTICS:

(A) LENGTH: 7731 base pairs
(B) TYPE: nucleic acid
(C) STRANDEDNESS: double
(D) TOPOLOGY: linear

(ii) MOLECULE TYPE: DNA (genomic)
(xi) SEQUENCE DESCRIPTION: SEQ ID NO: 42:

```
TTGAAGACGA AAGGGCCTCG TGATACGCCT ATTTTTATAG GTTAATGTCA TGATAATAAT     60

GGTTTCTTAG ACGTCAGGTG GCACTTTTCG GGGAAATGTG CGCGGAACCC CTATTTGTTT    120

ATTTTTCTAA ATACATTCAA ATATGTATCC GCTCATGAGA CAATAACCCT GATAAATGCT    180

TCAATAATAT TGAAAAAGGA AGAGTATGAG TATTCAACAT TTCCGTGTCG CCCTTATTCC    240

CTTTTTTGCG GCATTTTGCC TTCCTGTTTT TGCTCACCCA GAAACGCTGG TGAAAGTAAA    300

AGATGCTGAA GATCAGTTGG GTGCACGAGT GGGTTACATC GAACTGGATC TCAACAGCGG    360

TAAGATCCTT GAGAGTTTTC GCCCCGAAGA ACGTTTTCCA ATGATGAGCA CTTTTAAAGT    420

TCTGCTATGT GGCGCGGTAT TATCCCGTGT TGACGCCGGG CAAGAGCAAC TCGGTCGCCG    480

CATACACTAT TCTCAGAATG ACTTGGTTGA GTACTCACCA GTCACAGAAA AGCATCTTAC    540

GGATGGCATG ACAGTAAGAG AATTATGCAG TGCTGCCATA ACCATGAGTG ATAACACTGC    600

GGCCAACTTA CTTCTGACAA CGATCGGAGG ACCGAAGGAG CTAACCGCTT TTTTGCACAA    660

CATGGGGGAT CATGTAACTC GCCTTGATCG TTGGGAACCG GAGCTGAATG AAGCCATACC    720

AAACGACGAG CGTGACACCA CGATGCCTGC AGCAATGGCA ACAACGTTGC GCAAACTATT    780

AACTGGCGAA CTACTTACTC TAGCTTCCCG GCAACAATTA ATAGACTGGA TGGAGGCGGA    840

TAAAGTTGCA GGACCACTTC TGCGCTCGGC CCTTCCGGCT GGCTGGTTTA TTGCTGATAA    900
```

ATCTGGAGCC GGTGAGCGTG GGTCTCGCGG TATCATTGCA GCACTGGGGC CAGATGGTAA 960

GCCCTCCCGT ATCGTAGTTA TCTACACGAC GGGGAGTCAG GCAACTATGG ATGAACGAAA 1020

TAGACAGATC GCTGAGATAG GTGCCTCACT GATTAAGCAT TGGTAACTGT CAGACCAAGT 1080

TTACTCATAT ATACTTTAGA TTGATTTAAA ACTTCATTTT TAATTTAAAA GGATCTAGGT 1140

GAAGATCCTT TTTGATAATC TCATGACCAA AATCCCTTAA CGTGAGTTTT CGTTCCACTG 1200

AGCGTCAGAC CCCGTAGAAA AGATCAAAGG ATCTTCTTGA GATCCTTTTT TTCTGCGCGT 1260

AATCTGCTGC TTGCAAACAA AAAAACCACC GCTACCAGCG GTGGTTTGTT TGCCGGATCA 1320

AGAGCTACCA ACTCTTTTTC CGAAGGTAAC TGGCTTCAGC AGAGCGCAGA TACCAAATAC 1380

TGTCCTTCTA GTGTAGCCGT AGTTAGGCCA CCACTTCAAG AACTCTGTAG CACCGCCTAC 1440

ATACCTCGCT CTGCTAATCC TGTTACCAGT GGCTGCTGCC AGTGGCGATA AGTCGTGTCT 1500

TACCGGGTTG GACTCAAGAC GATAGTTACC GGATAAGGCG CAGCGGTCGG GCTGAACGGG 1560

GGGTTCGTGC ACACAGCCCA GCTTGGAGCG AACGACCTAC ACCGAACTGA GATACCTACA 1620

GCGTGAGCTA TGAGAAAGCG CCACGCTTCC CGAAGGGAGA AAGGCGGACA GGTATCCGGT 1680

AAGCGGCAGG GTCGGAACAG GAGAGCGCAC GAGGGAGCTT CCAGGGGGAA ACGCCTGGTA 1740

TCTTTATAGT CCTGTCGGGT TTCGCCACCT CTGACTTGAG CGTCGATTTT TGTGATGCTC 1800

GTCAGGGGGG CGGAGCCTAT GGAAAAACGC CAGCAACGCG GCCTTTTTAC GGTTCCTGGC 1860

CTTTTGCTGG CCTTTTGCTC ACATGTTCTT TCCTGCGTTA TCCCCTGATT CTGTGGATAA 1920

CCGTATTACC GCCTTTGAGT GAGCTGATAC CGCTCGCCGC AGCCGAACGA CCGAGCGCAG 1980

CGAGTCAGTG AGCGAGGAAG CGGAAGAGCG CCTGATGCGG TATTTTCTCC TTACGCATCT 2040

GTGCGGTATT TCACACCGCA TATGGTGCAC TCTCAGTACA ATCTGCTCTG ATGCCGCATA 2100

GTTAAGCCAG TATACACTCC GCTATCGCTA CGTGACTGGG TCATGGCTGC GCCCCGACAC 2160

CCGCCAACAC CCGCTGACGC GCCCTGACGG GCTTGTCTGC TCCCGGCATC CGCTTACAGA 2220

CAAGCTGTGA CCGTCTCCGG GAGCTGCATG TGTCAGAGGT TTTCACCGTC ATCACCGAAA 2280

CGCGCGAGGC AGCATGCATC TCAATTAGTC AGCAACCATA GTCCCGCCCC TAACTCCGCC 2340

CATCCCGCCC CTAACTCCGC CCAGTTCCGC CCATTCTCCG CCCCATGGCT GACTAATTTT 2400

TTTTATTTAT GCAGAGGCCG AGGCCGCCTC GGCCTCTGAG CTATTCCAGA AGTAGTGAGG 2460

AGGCTTTTTT GGAGGCCTAG GCTTTTGCAA AAAGCTAGCT TACAGCTCAG GGCTGCGATT 2520

TCGCGCCAAA CTTGACGGCA ATCCTAGCGT GAAGGCTGGT AGGATTTTAT CCCCGCTGCC 2580

ATCATGGTTC GACCATTGAA CTGCATCGTC GCCGTGTCCC AAAATATGGG GATTGGCAAG 2640

AACGGAGACC TACCCTGGCC TCCGCTCAGG AACGAGTTCA AGTACTTCCA AAGAATGACC 2700

ACAACCTCTT CAGTGGAAGG TAAACAGAAT CTGGTGATTA TGGGTAGGAA AACCTGGTTC 2760

TCCATTCCTG AGAAGAATCG ACCTTTAAAG GACAGAATTA ATATAGTTCT CAGTAGAGAA 2820
CTCAAAGAAC CACCACGAGG AGCTCATTTT CTTGCCAAAA GTTTGGATGA TGCCTTAAGA 2880
CTTATTGAAC AACCGGAATT GGCAAGTAAA GTAGACATGG TTTGGATAGT CGGAGGCAGT 2940
TCTGTTTACC AGGAAGCCAT GAATCAACCA GGCCACCTCA GACTCTTTGT GACAAGGATC 3000
ATGCAGGAAT TTGAAAGTGA CACGTTTTTC CCAGAAATTG ATTTGGGGAA ATATAAACTT 3060
CTCCCAGAAT ACCCAGGCGT CCTCTCTGAG GTCCAGGAGG AAAAAGGCAT CAAGTATAAG 3120
TTTGAAGTCT ACGAGAAGAA AGACTAACAG GAAGATGCTT TCAAGTTCTC TGCTCCCCTC 3180
CTAAAGCTAT GCATTTTTAT AAGACCATGG GACTTTTGCT GGCTTTAGAT CTTTGTGAAG 3240
GAACCTTACT TCTGTGGTGT GACATAATTG GACAAACTAC CTACAGAGAT TTAAAGCTCT 3300
AAGGTAAATA TAAAATTTTT AAGTGTATAA TGTGTTAAAC TACTGATTCT AATTGTTTGT 3360
GTATTTTAGA TTCCAACCTA TGGAACTGAT GAATGGGAGC AGTGGTGGAA TGCCTTTAAT 3420
GAGGAAAACC TGTTTTGCTC AGAAGAAATG CCATCTAGTG ATGATGAGGC TACTGCTGAC 3480
TCTCAACATT CTACTCCTCC AAAAAAGAAG AGAAAGGTAG AAGACCCCAA GGACTTTCCT 3540
TCAGAATTGC TAAGTTTTTT GAGTCATGCT GTGTTTAGTA ATAGAACTCT TGCTTGCTTT 3600
GCTATTTACA CCACAAAGGA AAAAGCTGCA CTGCTATACA AGAAAATTAT GGAAAAATAT 3660
TCTGTAACCT TTATAAGTAG GCATAACAGT TATAATCATA ACATACTGTT TTTTCTTACT 3720
CCACACAGGC ATAGAGTGTC TGCTATTAAT AACTATGCTC AAAAATTGTG TACCTTTAGC 3780
TTTTTAATTT GTAAAGGGGT TAATAAGGAA TATTTGATGT ATAGTGCCTT GACTAGAGAT 3840
CATAATCAGC CATACCACAT TTGTAGAGGT TTTACTTGCT TTAAAAAACC TCCCACACCT 3900
CCCCCTGAAC CTGAAACATA AAATGAATGC AATTGTTGTT GTTAACTTGT TTATTGCAGC 3960
TTATAATGGT TACAAATAAA GCAATAGCAT CACAAATTTC ACAAATAAAG CATTTTTTTC 4020
ACTGCATTCT AGTTGTGGTT TGTCCAAACT CATCAATGTA TCTTATCATG TCTGGATCTA 4080
ATAAAAGATA TTTATTTTCA TTAGATATGT GTGTTGGTTT TTTGTGTGCA GTGCCTCTAT 4140
CTGGAGGCCA GGTAGGGCTG GCCTTGGGGG AGGGGGAGGC CAGAATGACT CCAAGAGCTA 4200
CAGGAAGGCA GGTCAGAGAC CCCACTGGAC AAACAGTGGC TGGACTCTGC ACCATAACAC 4260
ACAATCAACA GGGGAGTGAG CTGGAAATTT GCTAGCGAAT TCCAGCACAC TGGCGGCCGT 4320
TACTAGTTAT TAATAGTAAT CAATTACGGG GTCATTAGTT CATAGCCCAT ATATGGAGTT 4380
CCGCGTTACA TAACTTACGG TAAATGGCCC GCCTGGCTGA CCGCCCAACG ACCCCCGCCC 4440
ATTGACGTCA ATAATGACGT ATGTTCCCAT AGTAACGCCA ATAGGGACTT TCCATTGACG 4500
TCAATGGGTG GAGTATTTAC GGTAAACTGC CCACTTGGCA GTACATCAAG TGTATCATAT 4560

GCCAAGTACG CCCCCTATTG ACGTCAATGA CGGTAAATGG CCCGCCTGGC ATTATGCCCA 4620
GTACATGACC TTATGGGACT TTCCTACTTG GCAGTACATC TACGTATTAG TCATCGCTAT 4680
TACCATGGTG ATGCGGTTTT GGCAGTACAT CAATGGGCGT GGATAGCGGT TTGACTCACG 4740
GGGATTTCCA AGTCTCCACC CCATTGACGT CAATGGGAGT TTGTTTTGGC ACCAAAATCA 4800
ACGGGACTTT CCAAAATGTC GTAACAACTC CGCCCCATTG ACGCAAATGG GCGGTAGGCG 4860
TGTACGGTGG GAGGTCTATA TAAGCAGAGC TCGTTTAGTG AACCGTCAGA TCGCCTGGAG 4920
ACGCCATCCA CGCTGTTTTG ACCTCCATAG AAGACACCGG GACCGATCCA GCCTCCGCGG 4980
CCGGGAACGG TGCATTGGAA CGCGGATTCC CCGTGCCAAG AGTGACGTAA GTACCGCCTA 5040
TAGAGTCTAT AGGCCCACCC CCTTGGCTTC TTATGCATGC TATACTGTTT TTGGCTTGGG 5100
GTCTATACAC CCCCGCTTCC TCATGTTATA GGTGATGGTA TAGCTTAGCC TATAGGTGTG 5160
GGTTATTGAC CATTATTGAC CACTCCCCTA TTGGTGACGA TACTTTCCAT TACTAATCCA 5220
TAACATGGCT CTTTGCCACA ACTCTCTTTA TTGGCTATAT GCCAATACAC TGTCCTTCAG 5280
AGACTGACAC GGACTCTGTA TTTTTACAGG ATGGGGTCTC ATTTATTATT TACAAATTCA 5340
CATATACAAC ACCACCGTCC CCAGTGCCCG CAGTTTTTAT TAAACATAAC GTGGGATCTC 5400
CACGCGAATC TCGGGTACGT GTTCCGGACA TGGGCTCTTC TCCGGTAGCG GCGGAGCTTC 5460
TACATCCGAG CCCTGCTCCC ATGCCTCCAG CGACTCATGG TCGCTCGGCA GCTCCTTGCT 5520
CCTAACAGTG GAGGCCAGAC TTAGGCACAG CACGATGCCC ACCACCACCA GTGTGCCGCA 5580
CAAGGCCGTG GCGGTAGGGT ATGTGTCTGA AAATGAGCTC GGGGAGCGGG CTTGCACCGC 5640
TGACGCATTT GGAAGACTTA AGGCAGCGGC AGAAGAAGAT GCAGGCAGCT GAGTTGTTGT 5700
GTTCTGATAA GAGTCAGAGG TAACTCCCGT TGCGGTGCTG TTAACGGTGG AGGGCAGTGT 5760
AGTCTGAGCA GTACTCGTTG CTGCCGCGCG CGCCACCAGA CATAATAGCT GACAGACTAA 5820
CAGACTGTTC CTTTCCATGG GTCTTTTCTG CAGTCACCGT CCTTGACACG CGTCTCGGGA 5880
AGCTTGCCGC CACCATGGAC TGGACCTGGC GCGTGTTTTG CCTGCTCGCC GTGGCTCCTG 5940
GGGCCCACAG CCAGGTGCAA CTGGTGCAGT CCGGCGCCGA AGTGAAGAAA CCCGGTGCTT 6000
CCGTGAAAGT CAGCTGTAAA ACTAGTAGAT ACACCTTCAC TGAATACACC ATACACTGGG 6060
TTAGACAGGC CCCTGGCCAA AGGCTGGAGT GGATAGGAGG TATTAATCCT AACAATGGTA 6120
TTCCTAACTA CAACCAGAAG TTCAAGGGCC GGGCCACCTT GACCGTAGGC AAGTCTGCCA 6180
GCACCGCCTA CATGGAACTG TCCAGCCTGC GCTCCGAGGA CACTGCAGTC TACTACTGCG 6240
CCAGAAGAAG AATCGCCTAT GGTTACGACG AGGGCCATGC TATGGACTAC TGGGGTCAAG 6300
GAACCCTTGT CACCGTCTCC TCAGGTGAGT GGATCCTCTG CGCCTGGGCC CAGCTCTGTC 6360
CCACACCGCG GTCACATGGC ACCACCTCTC TTGCAGCCTC CACCAAGGGC CCATCGGTCT 6420

```
TCCCCCTGGC ACCCTCCTCC AAGAGCACCT CTGGGGGCAC AGCGGCCCTG GGCTGCCTGG    6480
TCAAGGACTA CTTCCCCGAA CCGGTGACGG TGTCGTGGAA CTCAGGCGCC CTGACCAGCG    6540
GCGTGCACAC CTTCCCGGCT GTCCTACAGT CCTCAGGACT CTACTCCCTC AGCAGCGTGG    6600
TGACCGTGCC CTCCAGCAGC TTGGGCACCC AGACCTACAT CTGCAACGTG AATCACAAGC    6660
CCAGCAACAC CAAGGTGGAC AAGAAAGTTG AGCCCAAATC TTGTGACAAA ACTCACACAT    6720
GCCCACCGTG CCCAGCACCT GAACTCCTGG GGGGACCGTC AGTCTTCCTC TTCCCCCCAA    6780
AACCCAAGGA CACCCTCATG ATCTCCCGGA CCCCTGAGGT CACATGCGTG GTGGTGGACG    6840
TGAGCCACGA AGACCCTGAG GTCAAGTTCA ACTGGTACGT GGACGGCGTG GAGGTGCATA    6900
ATGCCAAGAC AAAGCCGCGG GAGGAGCAGT ACAACAGCAC GTACCGGGTG GTCAGCGTCC    6960
TCACCGTCCT GCACCAGGAC TGGCTGAATG GCAAGGAGTA CAAGTGCAAG GTCTCCAACA    7020
AAGCCCTCCC AGCCCCCATC GAGAAAACCA TCTCCAAAGC CAAAGGGCAG CCCCGAGAAC    7080
CACAGGTGTA CACCCTGCCC CCATCCCGGG AGGAGATGAC CAAGAACCAG GTCAGCCTGA    7140
CCTGCCTGGT CAAAGGCTTC TATCCCAGCG ACATCGCCGT GGAGTGGGAG AGCAATGGGC    7200
AGCCGGAGAA CAACTACAAG ACCACGCCTC CCGTGCTGGA CTCCGACGGC TCCTTCTTCC    7260
TCTACAGCAA GCTCACCGTG GACAAGAGCA GGTGGCAGCA GGGGAACGTC TTCTCATGCT    7320
CCGTGATGCA TGAGGCTCTG CACAACCACT ACACGCAGAA GAGCCTCTCC CTGTCTCCGG    7380
GTAAATGAGT GCGACGGCCG GCAAGCCCCG CTCCCCGGGC TCTCGCGGTC GCACGAGGAT    7440
GCTTGGCACG TACCCCCTGT ACATACTTCC CGGGCGCCCA GCATGGAAAT AAAGCACCGG    7500
ATCTAATAAA AGATATTTAT TTTCATTAGA TATGTGTGTT GGTTTTTTGT GTGCAGTGCC    7560
TCTATCTGGA GGCCAGGTAG GGCTGGCCTT GGGGGAGGGG GAGGCCAGAA TGACTCCAAG    7620
AGCTACAGGA AGGCAGGTCA GAGACCCCAC TGGACAAACA GTGGCTGGAC TCTGCACCAT    7680
AACACACAAT CAACAGGGGA GTGAGCTGGA AATTTGCTAG CGAATTAATT C             7731
```

(2) INFORMATION FOR SEQ ID NO: 43:

(i) SEQUENCE CHARACTERISTICS:

(A) LENGTH: 472 amino acids
(B) TYPE: amino acid
(C) STRANDEDNESS: single
(D) TOPOLOGY: linear

(ii) MOLECULE TYPE: protein
(xi) SEQUENCE DESCRIPTION: SEQ ID NO: 43:

```
Met Asp Trp Thr Trp Arg Val Phe Cys Leu Leu Ala Val Ala Pro Gly
```

1 5 10 15

Ala His Ser Gln Val Gln Leu Val Gln Ser Gly Ala Glu Val Lys Lys
20 25 30

Pro Gly Ala Ser Val Lys Val Ser Cys Lys Thr Ser Arg Tyr Thr Phe
35 40 45

Thr Glu Tyr Thr Ile His Trp Val Arg Gln Ala Pro Gly Gln Arg Leu
50 55 60

Glu Trp Ile Gly Gly Ile Asn Pro Asn Asn Gly Ile Pro Asn Tyr Asn
65 70 75 80

Gln Lys Phe Lys Gly Arg Ala Thr Leu Thr Val Gly Lys Ser Ala Ser
85 90 95

Thr Ala Tyr Met Glu Leu Ser Ser Leu Arg Ser Glu Asp Thr Ala Val
100 105 110

Tyr Tyr Cys Ala Arg Arg Arg Ile Ala Tyr Gly Tyr Asp Glu Gly His
115 120 125

Ala Met Asp Tyr Trp Gly Gln Gly Thr Leu Val Thr Val Ser Ser Ser
130 135 140

Thr Lys Gly Pro Ser Val Phe Pro Leu Ala Pro Ser Ser Lys Ser Thr
145 150 155 160

Ser Gly Gly Thr Ala Ala Leu Gly Cys Leu Val Lys Asp Tyr Phe Pro
165 170 175

Glu Pro Val Thr Val Ser Trp Asn Ser Gly Ala Leu Thr Ser Gly Val
180 185 190

His Thr Phe Pro Ala Val Leu Gln Ser Ser Gly Leu Tyr Ser Leu Ser
195 200 205

Ser Val Val Thr Val Pro Ser Ser Ser Leu Gly Thr Gln Thr Tyr Ile
210 215 220

Cys Asn Val Asn His Lys Pro Ser Asn Thr Lys Val Asp Lys Lys Val
225 230 235 240

Glu Pro Lys Ser Cys Asp Lys Thr His Thr Cys Pro Pro Cys Pro Ala
245 250 255

Pro Glu Leu Leu Gly Gly Pro Ser Val Phe Leu Phe Pro Pro Lys Pro
260 265 270

Lys Asp Thr Leu Met Ile Ser Arg Thr Pro Glu Val Thr Cys Val Val
275 280 285

Val Asp Val Ser His Glu Asp Pro Glu Val Lys Phe Asn Trp Tyr Val
290 295 300

Asp Gly Val Glu Val His Asn Ala Lys Thr Lys Pro Arg Glu Glu Gln
305 310 315 320

Tyr Asn Ser Thr Tyr Arg Val Val Ser Val Leu Thr Val Leu His Gln
325 330 335

```
Asp Trp Leu Asn Gly Lys Glu Tyr Lys Cys Lys Val Ser Asn Lys Ala
            340                 345                 350

Leu Pro Ala Pro Ile Glu Lys Thr Ile Ser Lys Ala Lys Gly Gln Pro
        355                 360                 365

Arg Glu Pro Gln Val Tyr Thr Leu Pro Pro Ser Arg Glu Glu Met Thr
    370                 375                 380

Lys Asn Gln Val Ser Leu Thr Cys Leu Val Lys Gly Phe Tyr Pro Ser
385                 390                 395                 400

Asp Ile Ala Val Glu Trp Glu Ser Asn Gly Gln Pro Glu Asn Asn Tyr
                405                 410                 415

Lys Thr Thr Pro Pro Val Leu Asp Ser Asp Gly Ser Phe Phe Leu Tyr
            420                 425                 430

Ser Lys Leu Thr Val Asp Lys Ser Arg Trp Gln Gln Gly Asn Val Phe
        435                 440                 445

Ser Cys Ser Val Met His Glu Ala Leu His Asn His Tyr Thr Gln Lys
    450                 455                 460

Ser Leu Ser Leu Ser Pro Gly Lys
465                 470
```

(2) INFORMATION FOR SEQ ID NO: 44:

(i) SEQUENCE CHARACTERISTICS:

(A) LENGTH: 25 base pairs
(B) TYPE: nucleic acid
(C) STRANDEDNESS: double
(D) TOPOLOGY: linear

(ii) MOLECULE TYPE: DNA (genomic)
(xi) SEQUENCE DESCRIPTION: SEQ ID NO: 44:
ACCGTCTCCT CAGGTGAGTG GATCC 25

(2) INFORMATION FOR SEQ ID NO: 45:

(i) SEQUENCE CHARACTERISTICS:

(A) LENGTH: 14 base pairs
(B) TYPE: nucleic acid
(C) STRANDEDNESS: double
(D) TOPOLOGY: linear

(ii) MOLECULE TYPE: DNA (genomic)
(xi) SEQUENCE DESCRIPTION: SEQ ID NO: 45:
CCTCTCTTGC AGCC 14

(2) INFORMATION FOR SEQ ID NO: 46:

(i) SEQUENCE CHARACTERISTICS:

(A) LENGTH: 14 base pairs
(B) TYPE: nucleic acid
(C) STRANDEDNESS: double

(D) TOPOLOGY: linear

(ii) MOLECULE TYPE: DNA (genomic)
(xi) SEQUENCE DESCRIPTION: SEQ ID NO: 46:
CCTCTCTTGC AGCC 14

(2) INFORMATION FOR SEQ ID NO: 47:

(i) SEQUENCE CHARACTERISTICS:

(A) LENGTH: 4 amino acids
(B) TYPE: amino acid
(C) STRANDEDNESS: single
(D) TOPOLOGY: linear

(ii) MOLECULE TYPE: peptide
(xi) SEQUENCE DESCRIPTION: SEQ ID NO: 47:

```
Thr Val Ser Ser
1
```

(2) INFORMATION FOR SEQ ID NO: 48:

(i) SEQUENCE CHARACTERISTICS:

(A) LENGTH: 4 amino acids
(B) TYPE: amino acid
(C) STRANDEDNESS: single
(D) TOPOLOGY: linear

(ii) MOLECULE TYPE: peptide
(xi) SEQUENCE DESCRIPTION: SEQ ID NO: 48:

```
Ser Thr Lys Gly
1
```

(2) INFORMATION FOR SEQ ID NO: 49:

(i) SEQUENCE CHARACTERISTICS:

(A) LENGTH: 27 base pairs
(B) TYPE: nucleic acid
(C) STRANDEDNESS: double
(D) TOPOLOGY: linear

(ii) MOLECULE TYPE: DNA (genomic)
(xi) SEQUENCE DESCRIPTION: SEQ ID NO: 49:
ACCGTCTCCT CAGCCTCCAC CAAGGGC 27

(2) INFORMATION FOR SEQ ID NO: 50:

(i) SEQUENCE CHARACTERISTICS:

(A) LENGTH: 8 amino acids
(B) TYPE: amino acid

(C) STRANDEDNESS: single
(D) TOPOLOGY: linear

(ii) MOLECULE TYPE: peptide
(xi) SEQUENCE DESCRIPTION: SEQ ID NO: 50:

Thr Val Ser Ser Ser Thr Lys Gly
1 5

(2) INFORMATION FOR SEQ ID NO: 51:

(i) SEQUENCE CHARACTERISTICS:

(A) LENGTH: 27 base pairs
(B) TYPE: nucleic acid
(C) STRANDEDNESS: double
(D) TOPOLOGY: linear

(ii) MOLECULE TYPE: cDNA
(xi) SEQUENCE DESCRIPTION: SEQ ID NO: 51:
ACCGTCTCCT CAGCCTCCAC CAAGGGC 27

(2) INFORMATION FOR SEQ ID NO: 52:

(i) SEQUENCE CHARACTERISTICS:

(A) LENGTH: 9 amino acids
(B) TYPE: amino acid
(C) STRANDEDNESS: single
(D) TOPOLOGY: linear

(ii) MOLECULE TYPE: peptide
(xi) SEQUENCE DESCRIPTION: SEQ ID NO: 52:

Thr Val Ser Ser Ala Ser Thr Lys Gly

1 5

(2) INFORMATION FOR SEQ ID NO: 53:

(i) SEQUENCE CHARACTERISTICS:

(A) LENGTH: 22 base pairs
(B) TYPE: nucleic acid
(C) STRANDEDNESS: double
(D) TOPOLOGY: linear

(ii) MOLECULE TYPE: DNA (genomic)
(xi) SEQUENCE DESCRIPTION: SEQ ID NO: 53:
GAAATAAAAC GTGAGTGGAT CC 22

(2) INFORMATION FOR SEQ ID NO: 54:

(i) SEQUENCE CHARACTERISTICS:

(A) LENGTH: 27 base pairs
(B) TYPE: nucleic acid
(C) STRANDEDNESS: double
(D) TOPOLOGY: linear

(ii) MOLECULE TYPE: DNA (genomic)
(xi) SEQUENCE DESCRIPTION: SEQ ID NO: 54:
CTTCTTTCCT CAGGAACTGT GGCTGCA 27

(2) INFORMATION FOR SEQ ID NO: 55:

(i) SEQUENCE CHARACTERISTICS:

(A) LENGTH: 4 amino acids
(B) TYPE: amino acid
(C) STRANDEDNESS: single
(D) TOPOLOGY: linear

(ii) MOLECULE TYPE: peptide
(xi) SEQUENCE DESCRIPTION: SEQ ID NO: 55:

Thr Val Ala Ala
1

(2) INFORMATION FOR SEQ ID NO: 56:

(i) SEQUENCE CHARACTERISTICS:

(A) LENGTH: 24 base pairs
(B) TYPE: nucleic acid
(C) STRANDEDNESS: double
(D) TOPOLOGY: linear

(ii) MOLECULE TYPE: DNA (genomic)
(xi) SEQUENCE DESCRIPTION: SEQ ID NO: 56:
GAAATAAAAC GAACTGTGGC TGCA 24

(2) INFORMATION FOR SEQ ID NO: 57:

(i) SEQUENCE CHARACTERISTICS:

(A) LENGTH: 7 amino acids
(B) TYPE: amino acid
(C) STRANDEDNESS: single
(D) TOPOLOGY: linear

(ii) MOLECULE TYPE: peptide
(xi) SEQUENCE DESCRIPTION: SEQ ID NO: 57:

Glu Ile Lys Thr Val Ala Ala
1 5

(2) INFORMATION FOR SEQ ID NO: 58:

(i) SEQUENCE CHARACTERISTICS:

(A) LENGTH: 24 base pairs
(B) TYPE: nucleic acid
(C) STRANDEDNESS: double
(D) TOPOLOGY: linear

(ii) MOLECULE TYPE: DNA (genomic)
(xi) SEQUENCE DESCRIPTION: SEQ ID NO: 58:
GAAATAAAAC GAACTGTGGC TGCA 24

(2) INFORMATION FOR SEQ ID NO: 59:

(i) SEQUENCE CHARACTERISTICS:

(A) LENGTH: 8 amino acids
(B) TYPE: amino acid
(C) STRANDEDNESS: single
(D) TOPOLOGY: linear

(ii) MOLECULE TYPE: peptide
(xi) SEQUENCE DESCRIPTION: SEQ ID NO: 59:

```
Glu Ile Lys Arg Thr Val Ala Ala

1               5
```

(2) INFORMATION FOR SEQ ID NO: 60:

(i) SEQUENCE CHARACTERISTICS:

(A) LENGTH: 20 amino acids
(B) TYPE: amino acid
(C) STRANDEDNESS: single
(D) TOPOLOGY: linear

(ii) MOLECULE TYPE: peptide
(xi) SEQUENCE DESCRIPTION: SEQ ID NO: 60:

```
Met Asp Ser Gln Ala Gln Val Leu Met Leu Leu Leu Leu Trp Val Ser
1               5                   10                  15

Gly Thr Cys Gly
            20
```

(2) INFORMATION FOR SEQ ID NO: 61:

(i) SEQUENCE CHARACTERISTICS:

(A) LENGTH: 19 amino acids
(B) TYPE: amino acid
(C) STRANDEDNESS: single
(D) TOPOLOGY: linear

(ii) MOLECULE TYPE: peptide
(xi) SEQUENCE DESCRIPTION: SEQ ID NO: 61:

```
Met Gly Trp Ser Trp Val Phe Leu Phe Leu Leu Ser Gly Thr Ala Gly
1               5                   10                  15

Val Leu Ser
```

(2) INFORMATION FOR SEQ ID NO: 62:

(i) SEQUENCE CHARACTERISTICS:

(A) LENGTH: 9 base pairs
(B) TYPE: nucleic acid
(C) STRANDEDNESS: double
(D) TOPOLOGY: linear

(ii) MOLECULE TYPE: DNA (genomic)
(xi) SEQUENCE DESCRIPTION: SEQ ID NO: 62:
GCCGCCACC 9

(2) INFORMATION FOR SEQ ID NO: 63:

(i) SEQUENCE CHARACTERISTICS:

(A) LENGTH: 37 base pairs
(B) TYPE: nucleic acid
(C) STRANDEDNESS: double
(D) TOPOLOGY: linear

(ii) MOLECULE TYPE: other nucleic acid

(A) DESCRIPTION: /desc = "PRIMER"

(xi) SEQUENCE DESCRIPTION: SEQ ID NO: 63:
CAGAAAGCTT GCCGCCACCA TGGATTCACA GGCCCAG 37

(2) INFORMATION FOR SEQ ID NO: 64:

(i) SEQUENCE CHARACTERISTICS:

(A) LENGTH: 6 amino acids
(B) TYPE: amino acid
(C) STRANDEDNESS: single
(D) TOPOLOGY: linear

(ii) MOLECULE TYPE: peptide
(xi) SEQUENCE DESCRIPTION: SEQ ID NO: 64:

```
Met Asp Ser Gln Ala Gln
1               5
```

(2) INFORMATION FOR SEQ ID NO: 65:

(i) SEQUENCE CHARACTERISTICS:

(A) LENGTH: 35 base pairs
(B) TYPE: nucleic acid

(C) STRANDEDNESS: single
(D) TOPOLOGY: linear

(ii) MOLECULE TYPE: other nucleic acid

(A) DESCRIPTION: /desc = "PRIMER"

(xi) SEQUENCE DESCRIPTION: SEQ ID NO: 65:
CCGAGGATCC ACTCACGTTT CAGCTCCAGC TTGGT 35

(2) INFORMATION FOR SEQ ID NO: 66:

(i) SEQUENCE CHARACTERISTICS:

(A) LENGTH: 37 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "PRIMER"

(xi) SEQUENCE DESCRIPTION: SEQ ID NO: 66:
CAGAAAGCTT GCCGCCACCA TGGGATGGAG CTGGGTC 37

(2) INFORMATION FOR SEQ ID NO: 67:

(i) SEQUENCE CHARACTERISTICS:

(A) LENGTH: 6 amino acids
(B) TYPE: amino acid
(C) STRANDEDNESS: single
(D) TOPOLOGY: linear

(ii) MOLECULE TYPE: peptide
(xi) SEQUENCE DESCRIPTION: SEQ ID NO: 67:

```
Met Gly Trp Ser Trp Val
1               5
```

(2) INFORMATION FOR SEQ ID NO: 68:

(i) SEQUENCE CHARACTERISTICS:

(A) LENGTH: 35 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "PRIMER"
(xi) SEQUENCE DESCRIPTION: SEQ ID NO: 68:
CCGAGGATCC ACTCACCTGA GGAGACGGTG ACTGA 35

(2) INFORMATION FOR SEQ ID NO: 69:

(i) SEQUENCE CHARACTERISTICS:

(A) LENGTH: 36 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "PRIMER"

(xi) SEQUENCE DESCRIPTION: SEQ ID NO: 69:
GTCATCACAA TGTCTCCGGA GGAACCTGGA ACCCAG 36

(2) INFORMATION FOR SEQ ID NO: 70:

(i) SEQUENCE CHARACTERISTICS:

(A) LENGTH: 29 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "PRIMER"

(xi) SEQUENCE DESCRIPTION: SEQ ID NO: 70:
CTCCGGAGAC ATTGTGATGA CCCAATCTC 29

(2) INFORMATION FOR SEQ ID NO: 71:

(i) SEQUENCE CHARACTERISTICS:

(A) LENGTH: 29 base_pairs
(B) TYPE: nucleic acid
(C) STRANDEDNESS: single
(D) TOPOLOGY: linear

(ii) MOLECULE TYPE: other nucleic acid

(A) DESCRIPTION: /desc = "PRIMER"

(xi) SEQUENCE DESCRIPTION: SEQ ID NO: 71:
CTCCGGAGAC ATTGTGATGA CCCAATCTC 29

(2) INFORMATION FOR SEQ ID NO: 72:

(i) SEQUENCE CHARACTERISTICS:

(A) LENGTH: 72 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "PRIMER"

(xi) SEQUENCE DESCRIPTION: SEQ ID NO: 72:
CAGTCAGAGC CTTTTATATT CTAGAAATCA AAAGAACTAC TTGGCCTGGT ATCAGCAGAA 60
ACCAGGACAG CC 72

(2) INFORMATION FOR SEQ ID NO: 73:

(i) SEQUENCE CHARACTERISTICS:

(A) LENGTH: 44 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "PRIMER"

(xi) SEQUENCE DESCRIPTION: SEQ ID NO: 73:
ACCCCAGATT CCCTAGTGCT AGCCCAAAAG ATGAGGAGTT TGGG 44

(2) INFORMATION FOR SEQ ID NO: 74:

(i) SEQUENCE CHARACTERISTICS:

(A) LENGTH: 67 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "PRIMER"

(xi) SEQUENCE DESCRIPTION: SEQ ID NO: 74:
TAGCACTAGG GAATCTGGGG TACCTGATAG GTTCAGTGGC AGTGGGTTTG GGACAGACTT 60
CACCCTC 67

(2) INFORMATION FOR SEQ ID NO: 75:

(i) SEQUENCE CHARACTERISTICS:

(A) LENGTH: 53 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "PRIMER"

(xi) SEQUENCE DESCRIPTION: SEQ ID NO: 75:
GTCCCTTGTC CGAACGTGAG CGGATAGCTA AAATATTGCT GACAGTAATA AAC 53

(2) INFORMATION FOR SEQ ID NO: 76:

(i) SEQUENCE CHARACTERISTICS:

(A) LENGTH: 33 base pairs

(B) TYPE: nucleic acid
(C) STRANDEDNESS: single
(D) TOPOLOGY: linear

(ii) MOLECULE TYPE: other nucleic acid

(A) DESCRIPTION: /desc = "PRIMER"

(xi) SEQUENCE DESCRIPTION: SEQ ID NO: 76:
GCTCACGTTC GGACAAGGGA CCAAGGTGGA AAT 33

(2) INFORMATION FOR SEQ ID NO: 77:

(i) SEQUENCE CHARACTERISTICS:

(A) LENGTH: 72 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "PRIMER"

(xi) SEQUENCE DESCRIPTION: SEQ ID NO: 77:
CAGTCAGAGC CTTTTATATT CTAGAAATCA AAAGAACTAC TTGGCCTGGT TCCAGCAGAA 60
ACCAGGACAG CC 72

(2) INFORMATION FOR SEQ ID NO: 78:

(i) SEQUENCE CHARACTERISTICS:

(A) LENGTH: 57 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "PRIMER"

(xi) SEQUENCE DESCRIPTION: SEQ ID NO: 78:
GTCCCTTGTC CGAACGTGAG CGGATAGCTA AAATATTGCT GACAGTCATA AACTGCC 57

(2) INFORMATION FOR SEQ ID NO: 79:

(i) SEQUENCE CHARACTERISTICS:

(A) LENGTH: 34 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "PRIMER"

(xi) SEQUENCE DESCRIPTION: SEQ ID NO: 79:

CCCAAACTCC TCATCTATTG GGCTAGCACT AGGG 34

(2) INFORMATION FOR SEQ ID NO: 80:

(i) SEQUENCE CHARACTERISTICS:

(A) LENGTH: 34 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "PRIMER"

(xi) SEQUENCE DESCRIPTION: SEQ ID NO: 80:
CCCTAGTGCT AGCCCAATAG ATGAGGAGTT TGGG 34

(2) INFORMATION FOR SEQ ID NO: 81:

(i) SEQUENCE CHARACTERISTICS:

(A) LENGTH: 17 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "PRIMER"

(xi) SEQUENCE DESCRIPTION: SEQ ID NO: 81:
TACGCAAACC GCCTCTC 17

(2) INFORMATION FOR SEQ ID NO: 82:

(i) SEQUENCE CHARACTERISTICS:

(A) LENGTH: 18 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "PRIMER"

(xi) SEQUENCE DESCRIPTION: SEQ ID NO: 82:
GAGTGCACCA TATGCGGT 18

(2) INFORMATION FOR SEQ ID NO: 83:

(i) SEQUENCE CHARACTERISTICS:

(A) LENGTH: 16 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "PRIMER"

(xi) SEQUENCE DESCRIPTION: SEQ ID NO: 83:
AACAGCTATG ACCATG 16

(2) INFORMATION FOR SEQ ID NO: 84:

(i) SEQUENCE CHARACTERISTICS:

(A) LENGTH: 17 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "PRIMER"

(xi) SEQUENCE DESCRIPTION: SEQ ID NO: 84:
GTTTTCCCAG TCACGAC 17

(2) INFORMATION FOR SEQ ID NO: 85:

(i) SEQUENCE CHARACTERISTICS:

(A) LENGTH: 47 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "PRIMER"

(xi) SEQUENCE DESCRIPTION: SEQ ID NO: 85:
GTGTATTCAG TGAAGGTGTA TCTACTAGTT TTACAGCTGA CTTTCAC 47

(2) INFORMATION FOR SEQ ID NO: 86:

(i) SEQUENCE CHARACTERISTICS:

(A) LENGTH: 53 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "PRIMER"

(xi) SEQUENCE DESCRIPTION: SEQ ID NO: 86:
TAGTAGATAC ACCTTCACTG AATACACCAT ACACTGGGTT AGACAGGCCC CTG 53

(2) INFORMATION FOR SEQ ID NO: 87:

(i) SEQUENCE CHARACTERISTICS:

(A) LENGTH: 71 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "PRIMER"

(xi) SEQUENCE DESCRIPTION: SEQ ID NO: 87:
CCCTTGAACT TCTGGTTGTA GTTAGGAATA CCATTGTTAG GATTAATACC TCCTATCCAC 60
TCCAGCCTTT G 71

(2) INFORMATION FOR SEQ ID NO: 88:

(i) SEQUENCE CHARACTERISTICS:

(A) LENGTH: 71 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "PRIMER"

(xi) SEQUENCE DESCRIPTION: SEQ ID NO: 88:
TAACTACAAC CAGAAGTTCA AGGGCCGGGC CACCTTGACC GTAGGCAAGT CTGCCAGCAC 60
CGCCTACATG G 71

(2) INFORMATION FOR SEQ ID NO: 89:

(i) SEQUENCE CHARACTERISTICS:

(A) LENGTH: 63 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "PRIMER"

(xi) SEQUENCE DESCRIPTION: SEQ ID NO: 89:
GCATGGCCCT CGTCGTAACC ATAGGCGATT CTTCTTCTGG CGCAGTAGTA GACTGCAGTG 60
TCC 63

(2) INFORMATION FOR SEQ ID NO: 90:

(i) SEQUENCE CHARACTERISTICS:

(A) LENGTH: 48 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "PRIMER"

(xi) SEQUENCE DESCRIPTION: SEQ ID NO: 90:
CTATGGTTAC GACGAGGGCC ATGCTATGGA CTACTGGGGT CAAGGAAC 48

(2) INFORMATION FOR SEQ ID NO: 91:

(i) SEQUENCE CHARACTERISTICS:

(A) LENGTH: 71 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "PRIMER"

(xi) SEQUENCE DESCRIPTION: SEQ ID NO: 91:
TAACTACAAC CAGAAGTTCA AGGGCCGGGT CACCATCACC GTAGACACCT CTGCCAGCAC 60
CGCCTACATG G 71

(2) INFORMATION FOR SEQ ID NO: 92:

(i) SEQUENCE CHARACTERISTICS:

(A) LENGTH: 27 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "PRIMER"

(xi) SEQUENCE DESCRIPTION: SEQ ID NO: 92:
GGACACTGCA GTCTACTTCT GCGCCAG 27

(2) INFORMATION FOR SEQ ID NO: 93:

(i) SEQUENCE CHARACTERISTICS:

(A) LENGTH: 17 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "PRIMER"

(xi) SEQUENCE DESCRIPTION: SEQ ID NO: 93:
TACGCAAACC GCCTCTC 17

(2) INFORMATION FOR SEQ ID NO: 94:

(i) SEQUENCE CHARACTERISTICS:

(A) LENGTH: 18 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "PRIMER"

(xi) SEQUENCE DESCRIPTION: SEQ ID NO: 94:
GAGTGCACCA TATGCGGT 18

(2) INFORMATION FOR SEQ ID NO: 95:

(i) SEQUENCE CHARACTERISTICS:

(A) LENGTH: 76 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "PRIMER"

(xi) SEQUENCE DESCRIPTION: SEQ ID NO: 95:
CCTTTGGCCA GGGGCCTGTC TAACCCAGTG TATGGTGTAT TCAGTGAAGG TGCTATCCAC 60
TAGTTTCCAC TAGTTT 76

(2) INFORMATION FOR SEQ ID NO: 96:

(i) SEQUENCE CHARACTERISTICS:

(A) LENGTH: 28 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "PRIMER"
(xi) SEQUENCE DESCRIPTION: SEQ ID NO: 96:
GTCACCGTCC TTGACACGCG TCTCGGGA 28

(2) INFORMATION FOR SEQ ID NO: 97:

(i) SEQUENCE CHARACTERISTICS:

(A) LENGTH: 17 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "PRIMER"

(xi) SEQUENCE DESCRIPTION: SEQ ID NO: 97:

TTGGAGGAGG GTGCCAG 17

(2) INFORMATION FOR SEQ ID NO: 98:

(i) SEQUENCE CHARACTERISTICS:

(A) LENGTH: 22 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "PRIMER"

(xi) SEQUENCE DESCRIPTION: SEQ ID NO: 98:
GAGACATTGT GACCCAATCT CC 22

(2) INFORMATION FOR SEQ ID NO: 99:

(i) SEQUENCE CHARACTERISTICS:

(A) LENGTH: 25 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "PRIMER"

(xi) SEQUENCE DESCRIPTION: SEQ ID NO: 99:
GACAGTCATA AACTGCCACA TCTTC 25

(2) INFORMATION FOR SEQ ID NO: 100:

(i) SEQUENCE CHARACTERISTICS:

(A) LENGTH: 23 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "PRIMER"

(xi) SEQUENCE DESCRIPTION: SEQ ID NO: 100:
TTGACACGCG TCTCGGGAAG CTT 23

(2) INFORMATION FOR SEQ ID NO: 101:

(i) SEQUENCE CHARACTERISTICS:

(A) LENGTH: 22 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "PRIMER"

(xi) SEQUENCE DESCRIPTION: SEQ ID NO: 101:
GGCGCAGAGG ATCCACTCAC CT 22

SEQUENCE LISTING

<110> Boehringer Ingelheim International GmbH

<120> FAP alpha-specific antibody with improved producibility

<130> 12-196-PCT

<140> PCT/EP99/02711
<141> 1999-04-22

<150> EP 98107925.4
<151> 1998-04-30

<160> 101

<170> PatentIn Ver. 2.1

<210> 1
<211> 339
<212> DNA
<213> Homo sapiens

<400> 1

```
gacattgtga tgacccaatc tccagactct ttggctgtgt ctctagggga gagggccacc 60
atcaactgca agtccagtca gagcctttta tattctagaa atcaaaagaa ctacttggcc 120
tggtatcagc agaaaccagg acagccaccc aaactcctca tcttttgggc tagcactagg 180
gaatctgggg tacctgatag gttcagtggc agtgggtttg ggacagactt caccctcacc 240
attagcagcc tgcaggctga agatgtggca gtttattact gtcagcaata ttttagctat 300
ccgctcacgt tcggacaagg gaccaaggtg gaaataaaa                        339
```

<210> 2
<211> 113
<212> PRT
<213> Homo sapiens

<400> 2

```
Asp Ile Val Met Thr Gln Ser Pro Asp Ser Leu Ala Val Ser Leu Gly
1               5                   10                  15

Glu Arg Ala Thr Ile Asn Cys Lys Ser Ser Gln Ser Leu Leu Tyr Ser
            20                  25                  30

Arg Asn Gln Lys Asn Tyr Leu Ala Trp Tyr Gln Gln Lys Pro Gly Gln
        35                  40                  45

Pro Pro Lys Leu Leu Ile Phe Trp Ala Ser Thr Arg Glu Ser Gly Val
    50                  55                  60

Pro Asp Arg Phe Ser Gly Ser Gly Phe Gly Thr Asp Phe Thr Leu Thr
65                  70                  75                  80

Ile Ser Ser Leu Gln Ala Glu Asp Val Ala Val Tyr Tyr Cys Gln Gln
                85                  90                  95

Tyr Phe Ser Tyr Pro Leu Thr Phe Gly Gln Gly Thr Lys Val Glu Ile
            100                 105                 110

Lys
```

<210> 3
<211> 339
<212> DNA
<213> Homo sapiens

<400> 3

```
gacattgtga tgacccaatc tccagactct ttggctgtgt ctctagggga gagggccacc 60
atcaactgca agtccagtca gagcctttta tattctagaa atcaaaagaa ctacttggcc 120
tggttccagc agaaaccagg acagccaccc aaactcctca tcttttgggc tagcactagg 180
gaatctgggg tacctgatag gttcagtggc agtgggtttg ggacagactt caccctcacc 240
attagcagcc tgcaggctga agatgtggca gtttatgact gtcaacaata ttttagctat 300
ccgctcacgt tcggacaagg gaccaaggtg gaaataaaa                        339
```

<210> 4
<211> 113
<212> PRT
<213> Homo sapiens

<400> 4

```
Asp Ile Val Met Thr Gln Ser Pro Asp Ser Leu Ala Val Ser Leu Gly
1               5                   10                  15

Glu Arg Ala Thr Ile Asn Cys Lys Ser Ser Gln Ser Leu Leu Tyr Ser
            20                  25                  30

Arg Asn Gln Lys Asn Tyr Leu Ala Trp Phe Gln Gln Lys Pro Gly Gln
        35                  40                  45

Pro Pro Lys Leu Leu Ile Phe Trp Ala Ser Thr Arg Glu Ser Gly Val
    50                  55                  60

Pro Asp Arg Phe Ser Gly Ser Gly Phe Gly Thr Asp Phe Thr Leu Thr
65                  70                  75                  80

Ile Ser Ser Leu Gln Ala Glu Asp Val Ala Val Tyr Asp Cys Gln Gln
                85                  90                  95

Tyr Phe Ser Tyr Pro Leu Thr Phe Gly Gln Gly Thr Lys Val Glu Ile
            100                 105                 110

Lys
```

<210> 5
<211> 339
<212> DNA
<213> Homo sapiens

<400> 5

```
gacattgtga tgacccaatc tccagactct ttggctgtgt ctctagggga gagggccacc 60
atcaactgca agtccagtca gagcctttta tattctagaa atcaaaagaa ctacttggcc 120
tggtatcagc agaaaccagg acagccaccc aaactcctca tctattgggc tagcactagg 180
gaatctgggg tacctgatag gttcagtggc agtgggtttg ggacagactt caccctcacc 240
attagcagcc tgcaggctga agatgtggca gtttattact gtcagcaata ttttagctat 300
ccgctcacgt tcggacaagg gaccaaggtg gaaataaaa                        339
```

<210> 6
<211> 113
<212> PRT
<213> Homo sapiens

<400> 6

```
Asp Ile Val Met Thr Gln Ser Pro Asp Ser Leu Ala Val Ser Leu Gly
1               5                   10                  15

Glu Arg Ala Thr Ile Asn Cys Lys Ser Ser Gln Ser Leu Leu Tyr Ser
            20                  25                  30

Arg Asn Gln Lys Asn Tyr Leu Ala Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Ser Gly Ser Gly Phe Gly Thr Asp Phe Thr Leu Thr
65                  70                  75                  80

Ile Ser Ser Leu Gln Ala Glu Asp Val Ala Val Tyr Tyr Cys Gln Gln
                85                  90                  95

Tyr Phe Ser Tyr Pro Leu Thr Phe Gly Gln Gly Thr Lys Val Glu Ile
            100                 105                 110

Lys
```

<210> 7
<211> 372
<212> DNA
<213> Homo sapiens

<400> 7

```
caggtgcaac tagtgcagtc cggcgccgaa gtgaagaaac ccggtgcttc cgtgaaagtc 60
agctgtaaaa ctagtagata caccttcact gaatacacca tacactgggt tagacaggcc 120
cctggccaaa ggctggagtg gataggaggt attaatccta acaatggtat tcctaactac 180
aaccagaagt tcaagggccg ggccaccttg accgtaggca agtctgccag caccgcctac 240
atggaactgt ccagcctgcg ctccgaggac actgcagtct actactgcgc cagaagaaga 300
atcgcctatg gttacgacga gggccatgct atggactact ggggtcaagg aacccttgtc 360
accgtctcct ca                                                     372
```

<210> 8
<211> 124
<212> PRT
<213> Homo sapiens

<400> 8

```
Gln Val Gln Leu Val Gln Ser Gly Ala Glu Val Lys Lys Pro Gly Ala
1               5                   10                  15

Ser Val Lys Val Ser Cys Lys Thr Ser Arg Tyr Thr Phe Thr Glu Tyr
            20                  25                  30

Thr Ile His Trp Val Arg Gln Ala Pro Gly Gln Arg Leu Glu Trp Ile
        35                  40                  45

Gly Gly Ile Asn Pro Asn Asn Gly Ile Pro Asn Tyr Asn Gln Lys Phe
    50                  55                  60

Lys Gly Arg Ala Thr Leu Thr Val Gly Lys Ser Ala Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Arg Arg Ile Ala Tyr Gly Tyr Asp Glu Gly His Ala Met Asp
            100                 105                 110

Tyr Trp Gly Gln Gly Thr Leu Val Thr Val Ser Ser
        115                 120
```

<210> 9
<211> 372
<212> DNA
<213> Homo sapiens

<400> 9

```
caggtgcaac tagtgcagtc cggcgccgaa gtgaagaaac ccggtgcttc cgtgaaagtc 60
agctgtaaaa ctagtagata caccttcact gaatacacca tacactgggt tagacaggcc 120
cctggccaaa ggctggagtg gataggaggt attaatccta acaatggtat tcctaactac 180
aaccagaagt tcaagggccg ggccaccttg accgtaggca agtctgccag caccgcctac 240
atggaactgt ccagcctgcg ctccgaggac actgcagtct acttctgcgc cagaagaaga 300
atcgcctatg gttacgacga gggccatgct atggactact ggggtcaagg aacccttgtc 360
accgtctcct ca                                                     372
```

<210> 10
<211> 124
<212> PRT
<213> Homo sapiens

<400> 10

```
Gln Val Gln Leu Val Gln Ser Gly Ala Glu Val Lys Lys Pro Gly Ala
1               5                   10                  15

Ser Val Lys Val Ser Cys Lys Thr Ser Arg Tyr Thr Phe Thr Glu Tyr
            20                  25                  30

Thr Ile His Trp Val Arg Gln Ala Pro Gly Gln Arg Leu Glu Trp Ile
        35                  40                  45

Gly Gly Ile Asn Pro Asn Asn Gly Ile Pro Asn Tyr Asn Gln Lys Phe
    50                  55                  60

Lys Gly Arg Ala Thr Leu Thr Val Gly Lys Ser Ala Ser Thr Ala Tyr
65                  70                  75                  80

Met Glu Leu Ser Ser Leu Arg Ser Glu Asp Thr Ala Val Tyr Phe Cys
                85                  90                  95

Ala Arg Arg Arg Ile Ala Tyr Gly Tyr Asp Glu Gly His Ala Met Asp
            100                 105                 110

Tyr Trp Gly Gln Gly Thr Leu Val Thr Val Ser Ser
        115                 120
```

<210> 11
<211> 372
<212> DNA
<213> Homo sapiens

<400> 11

```
caggtgcaac tagtgcagtc cggcgccgaa gtgaagaaac ccggtgcttc cgtgaaagtc 60

agctgtaaaa ctagtagata caccttcact gaatacacca tacactgggt tagacaggcc 120
cctggccaaa ggctggagtg gataggaggt attaatccta acaatggtat tcctaactac 180
aaccagaagt tcaagggccg ggtcaccatc accgtagaca cctctgccag caccgcctac 240
atggaactgt ccagcctgcg ctccgaggac actgcagtct actactgcgc cagaagaaga 300
atcgcctatg gttacgacga gggccatgct atggactact ggggtcaagg aacccttgtc 360
accgtctcct ca                                                     372
```

<210> 12
<211> 124
<212> PRT
<213> Homo sapiens

<400> 12

```
Gln Val Gln Leu Val Gln Ser Gly Ala Glu Val Lys Lys Pro Gly Ala
1               5                   10                  15

Ser Val Lys Val Ser Cys Lys Thr Ser Arg Tyr Thr Phe Thr Glu Tyr
            20                  25                  30

Thr Ile His Trp Val Arg Gln Ala Pro Gly Gln Arg Leu Glu Trp Ile
        35                  40                  45

Gly Gly Ile Asn Pro Asn Asn Gly Ile Pro Asn Tyr Asn Gln Lys Phe
    50                  55                  60

Lys Gly Arg Val Thr Ile Thr Val Asp Thr Ser Ala Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Arg Arg Ile Ala Tyr Gly Tyr Asp Glu Gly His Ala Met Asp
            100                 105                 110

Tyr Trp Gly Gln Gly Thr Leu Val Thr Val Ser Ser
        115                 120
```

<210> 13
<211> 372
<212> DNA
<213> Homo sapiens

<400> 13

```
caggtgcaac tagtgcagtc cggcgccgaa gtgaagaaac ccggtgcttc cgtgaaagtc 60
agctgtaaaa ctagtagata caccttcact gaatacacca tacactgggt tagacaggcc 120
cctggccaaa ggctggagtg gataggaggt attaatccta acaatggtat tcctaactac 180
aaccagaagt tcaagggccg ggtcaccatc accgtagaca cctctgccag caccgcctac 240
atggaactgt ccagcctgcg ctccgaggac actgcagtct acttctgcgc cagaagaaga 300
atcgcctatg gttacgacga gggccatgct atggactact ggggtcaagg aacccttgtc 360
accgtctcct ca                                                     372
```

<210> 14
<211> 124
<212> PRT
<213> Homo sapiens

<400> 14

```
Gln Val Gln Leu Val Gln Ser Gly Ala Glu Val Lys Lys Pro Gly Ala
1               5                   10                  15
```

```
Ser Val Lys Val Ser Cys Lys Thr Ser Arg Tyr Thr Phe Thr Glu Tyr
            20                  25                  30

Thr Ile His Trp Val Arg Gln Ala Pro Gly Gln Arg Leu Glu Trp Ile
        35                  40                  45

Gly Gly Ile Asn Pro Asn Asn Gly Ile Pro Asn Tyr Asn Gln Lys Phe
    50                  55                  60

Lys Gly Arg Val Thr Ile Thr Val Asp Thr Ser Ala Ser Thr Ala Tyr
65                  70                  75                  80

Met Glu Leu Ser Ser Leu Arg Ser Glu Asp Thr Ala Val Tyr Phe Cys
                85                  90                  95

Ala Arg Arg Arg Ile Ala Tyr Gly Tyr Asp Glu Gly His Ala Met Asp
            100                 105                 110

Tyr Trp Gly Gln Gly Thr Leu Val Thr Val Ser Ser
        115                 120
```

<210> 15
<211> 372
<212> DNA
<213> Homo sapiens

<400> 15

```
caggtgcaac tagtgcagtc cggcgccgaa gtgaagaaac ccggtgcttc cgtgaaagtc 60
agctgtaaaa ctagtggata caccttcact gaatacacca tacactgggt tagacaggcc 120
cctggccaaa ggctggagtg gataggaggt attaatccta acaatggtat tcctaactac 180
aaccagaagt tcaagggccg ggtcaccatc accgtagaca cctctgccag caccgcctac 240
atggaactgt ccagcctgcg ctccgaggac actgcagtct actactgcgc cagaagaaga 300
atcgcctatg gttacgacga gggccatgct atggactact ggggtcaagg aacccttgtc 360
accgtctcct ca                                                      372
```

<210> 16
<211> 124
<212> PRT
<213> Homo sapiens

<400> 16

```
Gln Val Gln Leu Val Gln Ser Gly Ala Glu Val Lys Lys Pro Gly Ala
 1               5                  10                  15

Ser Val Lys Val Ser Cys Lys Thr Ser Gly Tyr Thr Phe Thr Glu Tyr
            20                  25                  30

Thr Ile His Trp Val Arg Gln Ala Pro Gly Gln Arg Leu Glu Trp Ile
        35                  40                  45

Gly Gly Ile Asn Pro Asn Asn Gly Ile Pro Asn Tyr Asn Gln Lys Phe
    50                  55                  60

Lys Gly Arg Val Thr Ile Thr Val Asp Thr Ser Ala Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Arg Arg Ile Ala Tyr Gly Tyr Asp Glu Gly His Ala Met Asp
            100                 105                 110

Tyr Trp Gly Gln Gly Thr Leu Val Thr Val Ser Ser
        115                 120
```

<210> 17
<211> 220
<212> PRT
<213> Artificial Sequence

<220>
<223> Description of Artificial Sequence:Chimeric light chain variable region

<400> 17

```
Asp Ile Val Met Ser Gln Ser Pro Ser Ser Leu Ala Val Ser Val Gly
 1               5                  10                  15

Glu Lys Val Thr Met Ser Cys Lys Ser Ser Gln Ser Leu Leu Tyr Ser
            20                  25                  30

Arg Asn Gln Lys Asn Tyr Leu Ala Trp Phe Gln Gln Lys Pro Gly Gln
        35                  40                  45

Ser Pro Lys Leu Leu Ile Phe Trp Ala Ser Thr Arg Glu Ser Gly Val
    50                  55                  60

Pro Asp Arg Phe Thr Gly Ser Gly Phe Gly Thr Asp Phe Asn Leu Thr
65                  70                  75                  80

Ile Ser Ser Val Gln Ala Glu Asp Leu Ala Val Tyr Asp Cys Gln Gln
                85                  90                  95

Tyr Phe Ser Tyr Pro Leu Thr Phe Gly Ala Gly Thr Lys Leu Glu Leu
            100                 105                 110

Lys Arg Thr Val Ala Ala Pro Ser Val Phe Ile Phe Pro Pro Ser Asp
        115                 120                 125

Glu Gln Leu Lys Ser Gly Thr Ala Ser Val Val Cys Leu Leu Asn Asn
    130                 135                 140

Phe Tyr Pro Arg Glu Ala Lys Val Gln Trp Lys Val Asp Asn Ala Leu
145                 150                 155                 160

Gln Ser Gly Asn Ser Gln Glu Ser Val Thr Glu Gln Asp Ser Lys Asp
                165                 170                 175

Ser Thr Tyr Ser Leu Ser Ser Thr Leu Thr Leu Ser Lys Ala Asp Tyr
            180                 185                 190

Glu Lys His Lys Val Tyr Ala Cys Glu Val Thr His Gln Gly Leu Ser
        195                 200                 205

Ser Pro Val Thr Lys Ser Phe Asn Arg Gly Glu Cys
    210                 215                 220
```

<210> 18
<211> 453
<212> PRT
<213> Artificial Sequence

<220>
<223> Description of Artificial Sequence:chimeric heavy chain variable region

<400> 18

```
Val Gln Leu Gln Gln Ser Gly Pro Glu Leu Val Lys Pro Gly Ala Ser
 1               5                  10                  15

Val Lys Met Ser Cys Lys Thr Ser Arg Tyr Thr Phe Thr Glu Tyr Thr
            20                  25                  30

Ile His Trp Val Arg Gln Ser His Gly Lys Ser Leu Glu Trp Ile Gly
        35                  40                  45

Gly Ile Asn Pro Asn Asn Gly Ile Pro Asn Tyr Asn Gln Lys Phe Lys
    50                  55                  60

Gly Arg Ala Thr Leu Thr Val Gly Lys Ser Ser Ser Thr Ala Tyr Met
65                  70                  75                  80

Glu Leu Arg Ser Leu Thr Ser Glu Asp Ser Ala Val Tyr Phe Cys Ala
                85                  90                  95

Arg Arg Arg Ile Ala Tyr Gly Tyr Asp Glu Gly His Ala Met Asp Tyr
            100                 105                 110

Trp Gly Gln Gly Thr Ser Val Thr Val Ser Ser Ala Ser Thr Lys Gly
        115                 120                 125

Pro Ser Val Phe Pro Leu Ala Pro Ser Ser Lys Ser Thr Ser Gly Gly
    130                 135                 140

Thr Ala Ala Leu Gly Cys Leu Val Lys Asp Tyr Phe Pro Glu Pro Val
145                 150                 155                 160

Thr Val Ser Trp Asn Ser Gly Ala Leu Thr Ser Gly Val His Thr Phe
                165                 170                 175

Pro Ala Val Leu Gln Ser Ser Gly Leu Tyr Ser Leu Ser Ser Val Val
            180                 185                 190

Thr Val Pro Ser Ser Ser Leu Gly Thr Gln Thr Tyr Ile Cys Asn Val
        195                 200                 205

Asn His Lys Pro Ser Asn Thr Lys Val Asp Lys Lys Val Glu Pro Lys
    210                 215                 220

Ser Cys Asp Lys Thr His Thr Cys Pro Pro Cys Pro Ala Pro Glu Leu
225                 230                 235                 240

Leu Gly Gly Pro Ser Val Phe Leu Phe Pro Pro Lys Pro Lys Asp Thr
                245                 250                 255

Leu Met Ile Ser Arg Thr Pro Glu Val Thr Cys Val Val Val Asp Val
            260                 265                 270

Ser His Glu Asp Pro Glu Val Lys Phe Asn Trp Tyr Val Asp Gly Val
        275                 280                 285

Glu Val His Asn Ala Lys Thr Lys Pro Arg Glu Glu Gln Tyr Asn Ser
    290                 295                 300

Thr Tyr Arg Val Val Ser Val Leu Thr Val Leu His Gln Asp Trp Leu
```

305 310 315 320

Asn Gly Lys Glu Tyr Lys Cys Lys Val Ser Asn Lys Ala Leu Pro Ala
325 330 335

Pro Ile Glu Lys Thr Ile Ser Lys Ala Lys Gly Gln Pro Arg Glu Pro
340 345 350

Gln Val Tyr Thr Leu Pro Pro Ser Arg Glu Glu Met Thr Lys Asn Gln
355 360 365

Val Ser Leu Thr Cys Leu Val Lys Gly Phe Tyr Pro Ser Asp Ile Ala
370 375 380

Val Glu Trp Glu Ser Asn Gly Gln Pro Glu Asn Asn Tyr Lys Thr Thr
385 390 395 400

Pro Pro Val Leu Asp Ser Asp Gly Ser Phe Phe Leu Tyr Ser Lys Leu
405 410 415

Thr Val Asp Lys Ser Arg Trp Gln Gln Gly Asn Val Phe Ser Cys Ser
420 425 430

Val Met His Glu Ala Leu His Asn His Tyr Thr Gln Lys Ser Leu Ser
435 440 445

Leu Ser Pro Gly Lys
450

<210> 19
<211> 321
<212> DNA
<213> Homo sapiens

<400> 19

cgtactgtgg ctgcaccatc tgtcttcatc ttcccgccat ctgatgagca gttgaaatct 60
ggaactgcct ctgttgtgtg cctgctgaat aacttctatc ccagagaggc caaagtacag 120
tggaaggtgg ataacgccct ccaatcgggt aactcccagg agagtgtcac agagcaggac 180
agcaaggaca gcacctacag cctcagcagc accctgacgc tgagcaaagc agactacgag 240
aaacacaaag tctacgcctg cgaagtcacc catcagggcc tgagctcgcc cgtcacaaag 300
agcttcaaca ggggagagtg t 321

<210> 20
<211> 107
<212> PRT
<213> Homo sapiens

<400> 20

```
Arg Thr Val Ala Ala Pro Ser Val Phe Ile Phe Pro Pro Ser Asp Glu
 1               5                  10                  15

Gln Leu Lys Ser Gly Thr Ala Ser Val Val Cys Leu Leu Asn Asn Phe
            20                  25                  30

Tyr Pro Arg Glu Ala Lys Val Gln Trp Lys Val Asp Asn Ala Leu Gln
        35                  40                  45

Ser Gly Asn Ser Gln Glu Ser Val Thr Glu Gln Asp Ser Lys Asp Ser
    50                  55                  60

Thr Tyr Ser Leu Ser Ser Thr Leu Thr Leu Ser Lys Ala Asp Tyr Glu
65                  70                  75                  80

Lys His Lys Val Tyr Ala Cys Glu Val Thr His Gln Gly Leu Ser Ser
                85                  90                  95

Pro Val Thr Lys Ser Phe Asn Arg Gly Glu Cys
            100                 105
```

<210> 21
<211> 990
<212> DNA
<213> Homo sapiens

<400> 21

```
gcctccacca agggcccatc ggtcttcccc ctggcaccct cctccaagag cacctctggg 60
ggcacagcgg ccctgggctg cctggtcaag gactacttcc ccgaaccggt gacggtgtcg 120
tggaactcag gcgccctgac cagcggcgtg cacaccttcc cggctgtcct acagtcctca 180
ggactctact ccctcagcag cgtggtgacc gtgccctcca gcagcttggg cacccagacc 240
tacatctgca acgtgaatca caagcccagc aacaccaagg tggacaagaa agttgagccc 300
aaatcttgtg acaaaactca cacatgccca ccgtgcccag cacctgaact cctgggggga 360
ccgtcagtct tcctcttccc cccaaaaccc aaggacaccc tcatgatctc ccggacccct 420
gaggtcacat gcgtggtggt ggacgtgagc cacgaagacc ctgaggtcaa gttcaactgg 480
tacgtggacg gcgtggaggt gcataatgcc aagacaaagc cgcgggagga gcagtacaac 540
agcacgtacc gggtggtcag cgtcctcacc gtcctgcacc aggactggct gaatggcaag 600
gagtacaagt gcaaggtctc caacaaagcc ctcccagccc ccatcgagaa aaccatctcc 660
aaagccaaag ggcagccccg agaaccacag gtgtacaccc tgcccccatc ccgggaggag 720
atgaccaaga accaggtcag cctgacctgc ctggtcaaag gcttctatcc cagcgacatc 780
gccgtggagt gggagagcaa tgggcagccg gagaacaact acaagaccac gcctcccgtg 840
ctggactccg acggctcctt cttcctctac agcaagctca ccgtggacaa gagcaggtgg 900
cagcagggga acgtcttctc atgctccgtg atgcatgagg ctctgcacaa ccactacacg 960
cagaagagcc tctccctgtc tccgggtaaa                                  990
```

<210> 22
<211> 330
<212> PRT
<213> Homo sapiens

<400> 22

```
Ala Ser Thr Lys Gly Pro Ser Val Phe Pro Leu Ala Pro Ser Ser Lys
 1               5                  10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125
```

```
Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330
```

<210> 23
<211> 427
<212> DNA
<213> Homo sapiens

<400> 23

```
aagcttgccg ccaccatgga ttcacaggcc caggttctta tgttactgcc gctatgggta 60
tctggtacct gtggggacat tgtgatgtca cagtctccat cctccctagc tgtgtcagtt 120
ggagagaagg ttactatgag ctgcaagtcc agtcagagcc ttttatatag tcgtaatcaa 180
aagaactact tggcctggtt ccagcagaag ccagggcagt ctcctaaact gctgattttc 240
tgggcatcca ctagggaatc tggggtccct gatcgcttca caggcagtgg atttgggacg 300
gatttcaatc tcaccatcag cagtgtgcag gctgaggacc tggcagttta tgactgtcag 360
caatatttta gctatccgct cacgttcggt gctgggacca agctggagct gaaacgtgag 420
tggatcc                                                           427
```

<210> 24
<211> 133

<212> PRT
<213> Homo sapiens

<400> 24

```
Met Asp Ser Gln Ala Gln Val Leu Met Leu Leu Pro Leu Trp Val Ser
 1               5                  10                  15

Gly Thr Cys Gly Asp Ile Val Met Ser Gln Ser Pro Ser Ser Leu Ala
            20                  25                  30

Val Ser Val Gly Glu Lys Val Thr Met Ser Cys Lys Ser Ser Gln Ser
        35                  40                  45

Leu Leu Tyr Ser Arg Asn Gln Lys Asn Tyr Leu Ala Trp Phe Gln Gln
    50                  55                  60

Lys Pro Gly Gln Ser Pro Lys Leu Leu Ile Phe Trp Ala Ser Thr Arg
65                  70                  75                  80

Glu Ser Gly Val Pro Asp Arg Phe Thr Gly Ser Gly Phe Gly Thr Asp
                85                  90                  95

Phe Asn Leu Thr Ile Ser Ser Val Gln Ala Glu Asp Leu Ala Val Tyr
            100                 105                 110

Asp Cys Gln Gln Tyr Phe Ser Tyr Pro Leu Thr Phe Gly Ala Gly Thr
        115                 120                 125

Lys Leu Glu Leu Lys
    130
```

<210> 25
<211> 457
<212> DNA
<213> Homo sapiens

<400> 25

```
aagcttgccg ccaccatggg atggagctgg gtctttctct ttctcctgtc aggaactgca 60
ggtgtcctct ctgaggtcca gctgcaacag tctggacctg agctggtgaa gcctggggct 120
tcagtaaaga tgtcctgcaa gacttctaga tacacattca ctgaatacac catacactgg 180
gtgagacaga gccatggaaa gagccttgag tggattggag gtattaatcc taacaatggt 240
attcctaact acaaccagaa gttcaagggc agggccacat tgactgtagg caagtcctcc 300
agcaccgcct acatggagct ccgcagcctg acatctgagg attctgcggt ctatttctgt 360
gcaagaagaa gaatcgccta tggttacgac gagggccatg ctatggacta ctggggtcaa 420
ggaacctcag tcaccgtctc ctcaggtgag tggatcc                          457
```

<210> 26
<211> 143
<212> PRT
<213> Homo sapiens

<400> 26

```
Met Gly Trp Ser Trp Val Phe Leu Phe Leu Leu Ser Gly Thr Ala Gly
 1               5                  10                  15

Val Leu Ser Glu Val Gln Leu Gln Gln Ser Gly Pro Glu Leu Val Lys
            20                  25                  30

Pro Gly Ala Ser Val Lys Met Ser Cys Lys Thr Ser Arg Tyr Thr Phe
        35                  40                  45

Thr Glu Tyr Thr Ile His Trp Val Arg Gln Ser His Gly Lys Ser Leu
    50                  55                  60

Glu Trp Ile Gly Gly Ile Asn Pro Asn Asn Gly Ile Pro Asn Tyr Asn
65                  70                  75                  80

Gln Lys Phe Lys Gly Arg Ala Thr Leu Thr Val Gly Lys Ser Ser Ser
                85                  90                  95

Thr Ala Tyr Met Glu Leu Arg Ser Leu Thr Ser Glu Asp Ser Ala Val
            100                 105                 110

Tyr Phe Cys Ala Arg Arg Arg Ile Ala Tyr Gly Tyr Asp Glu Gly His
        115                 120                 125

Ala Met Asp Tyr Trp Gly Gln Gly Thr Ser Val Thr Val Ser Ser
    130                 135                 140
```

<210> 27
<211> 8068
<212> DNA
<213> Homo sapiens

<400> 27

```
gaattccagc acactggcgg ccgttactag ttattaatag taatcaatta cggggtcatt 60
agttcatagc ccatatatgg agttccgcgt tacataactt acggtaaatg gcccgcctgg 120
ctgaccgccc aacgaccccc gcccattgac gtcaataatg acgtatgttc ccatagtaac 180
gccaataggg actttccatt gacgtcaatg ggtggagtat ttacggtaaa ctgcccactt 240
ggcagtacat caagtgtatc atatgccaag tacgccccct attgacgtca atgacggtaa 300
atggcccgcc tggcattatg cccagtacat gaccttatgg gactttccta cttggcagta 360
catctacgta ttagtcatcg ctattaccat ggtgatgcgg ttttggcagt acatcaatgg 420
gcgtggatag cggtttgact cacggggatt tccaagtctc caccccattg acgtcaatgg 480
gagtttgttt tggcaccaaa atcaacggga ctttccaaaa tgtcgtaaca actccgcccc 540
attgacgcaa atgggcggta ggcgtgtacg gtgggaggtc tatataagca gagctcgttt 600
agtgaaccgt cagatcgcct ggagacgcca tccacgctgt tttgacctcc atagaagaca 660
ccgggaccga tccagcctcc gcggccggga acggtgcatt ggaacgcgga ttccccgtgc 720
caagagtgac gtaagtaccg cctatagagt ctataggccc acccccttgg cttcttatgc 780
atgctatact gtttttggct tggggtctat acacccccgc ttcctcatgt tataggtgat 840
ggtatagctt agcctatagg tgtgggttat tgaccattat tgaccactcc cctattggtg 900
acgatacttt ccattactaa tccataacat ggctctttgc cacaactctc tttattggct 960
atatgccaat acactgtcct tcagagactg acacggactc tgtattttta caggatgggg 1020
tctcatttat tatttacaaa ttcacatata caacaccacc gtccccagtg cccgcagttt 1080
ttattaaaca taacgtggga tctccacgcg aatctcgggt acgtgttccg gacatgggct 1140
cttctccggt agcggcggag cttctacatc cgagccctgc tcccatgcct ccagcgactc 1200
atggtcgctc ggcagctcct tgctcctaac agtggaggcc agacttaggc acagcacgat 1260
gcccaccacc accagtgtgc cgcacaaggc cgtggcggta gggtatgtgt ctgaaaatga 1320
gctcggggag cgggcttgca ccgctgacgc atttggaaga cttaaggcag cggcagaaga 1380
agatgcaggc agctgagttg ttgtgttctg ataagagtca gaggtaactc ccgttgcggt 1440
gctgttaacg gtggagggca gtgtagtctg agcagtactc gttgctgccg cgcgcgccac 1500
cagacataat agctgacaga ctaacagact gttcctttcc atgggtcttt tctgcagtca 1560
ccgtccttga cacgcgtctc gggaagcttg ccgccaccat ggattcacag gcccaggttc 1620
ttatgttact gccgctatgg gtatctggta cctgtgggga cattgtgatg tcacagtctc 1680
catcctccct agctgtgtca gttggagaga aggttactat gagctgcaag tccagtcaga 1740
gccttttata ttctagaaat caaaagaact acttggcctg gttccagcag aagccagggc 1800
agtctcctaa actgctgatt ttctgggcat ccactaggga atctggggtc cctgatcgct 1860
tcacaggcag tggatttggg acggatttca atctcaccat cagcagtgtg caggctgagg 1920
acctggcagt ttatgactgt cagcaatatt ttagctatcc gctcacgttc ggtgctggga 1980
ccaagctgga gctgaaacgt gagtggatcc atctgggata agcatgctgt ttctgtctg 2040
tccctaacat gccctgtgat tatgcgcaaa caacacaccc aagggcagaa ctttgttact 2100
taaacaccat cctgtttgct tctttcctca ggaactgtgg ctgcaccatc tgtcttcatc 2160
ttcccgccat ctgatgagca gttgaaatct ggaactgcct ctgttgtgtg cctgctgaat 2220
aacttctatc ccagagaggc caaagtacag tggaaggtgg ataacgccct ccaatcgggt 2280
aactcccagg agagtgtcac agagcaggac agcaaggaca gcacctacag cctcagcagc 2340
accctgacgc tgagcaaagc agactacgag aaacacaaag tctacgcctg cgaagtcacc 2400
catcagggcc tgagctcgcc cgtcacaaag agcttcaaca ggggagagtg ttagagggag 2460
aagtgccccc acctgctcct cagttccagc ctgacccct cccatccttt ggcctctgac 2520
```

cctttttcca caggggacct accoctattg cggtcctcca gctcatcttt cacctcaccc 2580
ccctcctcct ccttggcttt aattatgcta atgttggagg agaatgaata aataaagtga 2640
atctttgcac ctgtggtgga tctaataaaa gatatttatt ttcattagat atgtgtgttg 2700
gtttttgtg tgcagtgcct ctatctggag gccaggtagg gctggccttg ggggaggggg 2760
aggccagaat gactccaaga gctacaggaa ggcaggtcag agaccccact ggacaaacag 2820
tggctggact ctgcaccata acacacaatc aacaggggag tgagctggaa atttgctagc 2880
gaattcttga agacgaaagg gcctcgtgat acgcctattt ttataggtta atgtcatgat 2940
aataatggtt tcttagacgt caggtggcac ttttcgggga aatgtgcgcg gaacccctat 3000
ttgtttattt ttctaaatac attcaaatat gtatccgctc atgagacaat aaccctgata 3060
aatgcttcaa taatattgaa aaaggaagag tatgagtatt caacatttcc gtgtcgccct 3120
tattcccttt tttgcggcat tttgccttcc tgtttttgct cacccagaaa cgctggtgaa 3180
agtaaaagat gctgaagatc agttgggtgc acgagtgggt tacatcgaac tggatctcaa 3240
cagcggtaag atccttgaga gttttcgccc cgaagaacgt tttccaatga tgagcacttt 3300
taaagttctg ctatgtggcg cggtattatc ccgtgttgac gccgggcaag agcaactcgg 3360
tcgccgcata cactattctc agaatgactt ggttgagtac tcaccagtca cagaaaagca 3420
tcttacggat ggcatgacag taagagaatt atgcagtgct gccataacca tgagtgataa 3480
cactgcggcc aacttacttc tgacaacgat cggaggaccg aaggagctaa ccgctttttt 3540
gcacaacatg ggggatcatg taactcgcct tgatcgttgg gaaccggagc tgaatgaagc 3600
cataccaaac gacgagcgtg acaccacgat gcctgcagca atggcaacaa cgttgcgcaa 3660
actattaact ggcgaactac ttactctagc ttcccggcaa caattaatag actggatgga 3720
ggcggataaa gttgcaggac cacttctgcg ctcggccctt ccggctggct ggtttattgc 3780
tgataaatct ggagccggtg agcgtgggtc tcgcggtatc attgcagcac tggggccaga 3840
tggtaagccc tcccgtatcg tagttatcta cacgacgggg agtcaggcaa ctatggatga 3900
acgaaataga cagatcgctg agataggtgc ctcactgatt aagcattggt aactgtcaga 3960
ccaagtttac tcatatatac tttagattga tttaaaactt catttttaat ttaaaaggat 4020
ctaggtgaag atcctttttg ataatctcat gaccaaaatc ccttaacgtg agttttcgtt 4080
ccactgagcg tcagaccccg tagaaaagat caaaggatct tcttgagatc cttttttct 4140
gcgcgtaatc tgctgcttgc aaacaaaaaa accaccgcta ccagcggtgg tttgtttgcc 4200
ggatcaagag ctaccaactc tttttccgaa ggtaactggc ttcagcagag cgcagatacc 4260
aaatactgtc cttctagtgt agccgtagtt aggccaccac ttcaagaact ctgtagcacc 4320
gcctacatac ctcgctctgc taatcctgtt accagtggct gctgccagtg gcgataagtc 4380
gtgtcttacc gggttggact caagacgata gttaccggat aaggcgcagc ggtcgggctg 4440
aacggggggt tcgtgcacac agcccagctt ggagcgaacg acctacaccg aactgagata 4500
cctacagcgt gagctatgag aaagcgccac gcttcccgaa gggagaaagg cggacaggta 4560
tccggtaagc ggcagggtcg gaacaggaga gcgcacgagg gagcttccag ggggaaacgc 4620
ctggtatctt tatagtcctg tcgggtttcg ccacctctga cttgagcgtc gatttttgtg 4680
atgctcgtca ggggggcgga gcctatggaa aaacgccagc aacgcggcct ttttacggtt 4740
cctggccttt tgctggcctt ttgctcacat gttctttcct gcgttatccc ctgattctgt 4800
ggataaccgt attaccgcct ttgagtgagc tgataccgct cgccgcagcc gaacgaccga 4860
gcgcagcgag tcagtgagcg aggaagcgga agagcgcctg atgcggtatt ttctccttac 4920
gcatctgtgc ggtatttcac accgcatatg gtgcactctc agtacaatct gctctgatgc 4980
cgcatagtta agccagtata cactccgcta tcgctacgtg actgggtcat ggctgcgccc 5040
cgacacccgc caacacccgc tgacgcgccc tgacgggctt gtctgctccc ggcatccgct 5100
tacagacaag ctgtgaccgt ctccgggagc tgcatgtgtc agaggttttc accgtcatca 5160
ccgaaacgcg cgaggcagct gtggaatgtg tgtcagttag ggtgtggaaa gtccccaggc 5220
tccccagcag gcagaagtat gcaaagcatg catctcaatt agtcagcaac caggctcccc 5280
agcaggcaga agtatgcaaa gcatgcatct caattagtca gcaaccatag tcccgcccct 5340
aactccgccc atcccgcccc taactccgcc cagttccgcc cattctccgc cccatggctg 5400
actaattttt tttatttatg cagaggccga ggccgcctcg gcctctgagc tattccagaa 5460
gtagtgagga ggcttttttg gaggcctagg cttttgcaaa aagctagctt cacgctgccg 5520
caagcactca gggcgcaagg gctgctaaag gaagcggaac acgtagaaag ccagtccgca 5580
gaaacggtgc tgaccccgga tgaatgtcag ctactgggct atctggacaa gggaaaacgc 5640
aagcgcaaag agaaagcagg tagcttgcag tgggcttaca tggcgatagc tagactgggc 5700
ggttttatgg acagcaagcg aaccggaatt gccagctggg gcgccctctg gtaaggttgg 5760
gaagccctgc aaagtaaact ggatggcttt cttgccgcca aggatctgat ggcgcagggg 5820
atcaagatct gatcaagaga caggatgagg atcgtttcgc atgattgaac aagatggatt 5880
gcacgcaggt tctccggccg cttgggtgga gaggctattc ggctatgact gggcacaaca 5940
gacaatcggc tgctctgatg ccgccgtgtt ccggctgtca gcgcaggggc gcccggttct 6000
ttttgtcaag accgacctgt ccggtgccct gaatgaactg caggacgagg cagcgcggct 6060
atcgtggctg gccacgacgg gcgttccttg cgcagctgtg ctcgacgttg tcactgaagc 6120
gggaagggac tggctgctat tgggcgaagt gccggggcag gatctcctgt catctcacct 6180
tgctcctgcc gagaaagtat ccatcatggc tgatgcaatg cggcggctgc atacgcttga 6240
tccggctacc tgcccattcg accaccaagc gaaacatcgc atcgagcgag cacgtactcg 6300

```
gatggaagcc ggtcttgtcg atcaggatga tctggacgaa gagcatcagg ggctcgcgcc 6360
agccgaactg ttcgccaggc tcaaggcgcg catgcccgac ggcgaggatc tcgtcgtgac 6420
ccatggcgat gcctgcttgc cgaatatcat ggtggaaaat ggccgctttt ctggattcat 6480
cgactgtggc cggctgggtg tggcggaccg ctatcaggac atagcgttgg ctacccgtga 6540
tattgctgaa gagcttggcg gcgaatgggc tgaccgcttc ctcgtgcttt acggtatcgc 6600
cgctcccgat tcgcagcgca tcgccttcta tcgccttctt gacgagttct tctgagcggg 6660
actctggggt tcgaaatgac cgaccaagcg acgcccaacc tgccatcacg agatttcgat 6720
tccaccgccg ccttctatga aaggttgggc ttcggaatcg ttttccggga cgccggctgg 6780
atgatcctcc agcgcgggga tctcatgctg gagttcttcg cccaccccgg gctcgatccc 6840
ctcgcgagtt ggttcagctg ctgcctgagg ctggacgacc tcgcggagtt ctaccggcag 6900
tgcaaatccg tcggcatcca ggaaaccagc agcggctatc cgcgcatcca tgcccccgaa 6960
ctgcaggagt ggggaggcac gatggccgct ttggtcccgg atctttgtga aggaacctta 7020
cttctgtggt gtgacataat tggacaaact acctacagag atttaaagct ctaaggtaaa 7080
tataaaattt ttaagtgtat aatgtgttaa actactgatt ctaattgttt gtgtatttta 7140
gattccaacc tatggaactg atgaatggga gcagtggtgg aatgccttta atgaggaaaa 7200
cctgttttgc tcagaagaaa tgccatctag tgatgatgag gctactgctg actctcaaca 7260
ttctactcct ccaaaaaaga agagaaaggt agaagacccc aaggactttc cttcagaatt 7320
gctaagtttt ttgagtcatg ctgtgtttag taatagaact cttgcttgct ttgctattta 7380
caccacaaag gaaaaagctg cactgctata caagaaaatt atggaaaaat attctgtaac 7440
ctttataagt aggcataaca gttataatca taacatactg ttttttctta ctccacacag 7500
gcatagagtg tctgctatta ataactatgc tcaaaaattg tgtaccttta gctttttaat 7560
ttgtaaaggg gttaataagg aatatttgat gtatagtgcc ttgactagag atcataatca 7620
gccataccac atttgtagag gttttacttg ctttaaaaaa cctcccacac ctccccctga 7680
acctgaaaca taaaatgaat gcaattgttg ttgttaactt gtttattgca gcttataatg 7740
gttacaaata aagcaatagc atcacaaatt tcacaaataa agcatttttt tcactgcatt 7800
ctagttgtgg tttgtccaaa ctcatcaatg tatcttatca tgtctggatc taataaaaga 7860
tatttatttt cattagatat gtgtgttggt ttttgtgtg cagtgcctct atctggaggc 7920
caggtagggc tggccttggg ggaggggggag gccagaatga ctccaagagc tacaggaagg 7980
caggtcagag accccactgg acaaacagtg gctggactct gcaccataac acacaatcaa 8040
caggggagtg agctggaaat ttgctagc                                    8068
```

<210> 28
<211> 239
<212> PRT
<213> Homo sapiens

<400> 28

```
Asp Ser Gln Ala Gln Val Leu Met Leu Leu Pro Leu Trp Val Ser Gly
 1               5                  10                  15

Thr Cys Gly Asp Ile Val Met Ser Gln Ser Pro Ser Ser Leu Ala Val
            20                  25                  30

Ser Val Gly Glu Lys Val Thr Met Ser Cys Lys Ser Ser Gln Ser Leu
        35                  40                  45

Leu Tyr Ser Arg Asn Gln Lys Asn Tyr Leu Ala Trp Phe Gln Gln Lys
    50                  55                  60

Pro Gly Gln Ser Pro Lys Leu Leu Ile Phe Trp Ala Ser Thr Arg Glu
65                  70                  75                  80

Ser Gly Val Pro Asp Arg Phe Thr Gly Ser Gly Phe Gly Thr Asp Phe
                85                  90                  95

Asn Leu Thr Ile Ser Ser Val Gln Ala Glu Asp Leu Ala Val Tyr Asp
            100                 105                 110

Cys Gln Gln Tyr Phe Ser Tyr Pro Leu Thr Phe Gly Ala Gly Thr Lys
        115                 120                 125

Leu Glu Leu Lys Arg Thr Val Ala Ala Pro Ser Val Phe Ile Phe Pro

    130                 135                 140

Pro Ser Asp Glu Gln Leu Lys Ser Gly Thr Ala Ser Val Val Cys Leu
145                 150                 155                 160

Leu Asn Asn Phe Tyr Pro Arg Glu Ala Lys Val Gln Trp Lys Val Asp
                165                 170                 175

Asn Ala Leu Gln Ser Gly Asn Ser Gln Glu Ser Val Thr Glu Gln Asp
            180                 185                 190

Ser Lys Asp Ser Thr Tyr Ser Leu Ser Ser Thr Leu Thr Leu Ser Lys
        195                 200                 205

Ala Asp Tyr Glu Lys His Lys Val Tyr Ala Cys Glu Val Thr His Gln
    210                 215                 220

Gly Leu Ser Ser Pro Val Thr Lys Ser Phe Asn Arg Gly Glu Cys
225                 230                 235
```

<210> 29
<211> 7731
<212> DNA
<213> Homo sapiens

<400> 29

```
ttgaagacga aagggcctcg tgatacgcct atttttatag gttaatgtca tgataataat 60
ggtttcttag acgtcaggtg gcacttttcg gggaaatgtg cgcggaaccc ctatttgttt 120
atttttctaa atacattcaa atatgtatcc gctcatgaga caataaccct gataaatgct 180
tcaataatat tgaaaaagga agagtatgag tattcaacat ttccgtgtcg cccttattcc 240
cttttttgcg gcattttgcc ttcctgtttt tgctcaccca gaaacgctgg tgaaagtaaa 300
agatgctgaa gatcagttgg gtgcacgagt gggttacatc gaactggatc tcaacagcgg 360
taagatcctt gagagttttc gccccgaaga acgttttcca atgatgagca cttttaaagt 420
tctgctatgt ggcgcggtat tatcccgtgt tgacgccggg caagagcaac tcggtcgccg 480
catacactat tctcagaatg acttggttga gtactcacca gtcacagaaa agcatcttac 540
ggatggcatg acagtaagag aattatgcag tgctgccata accatgagtg ataacactgc 600
ggccaactta cttctgacaa cgatcggagg accgaaggag ctaaccgctt ttttgcacaa 660
catgggggat catgtaactc gccttgatcg ttgggaaccg gagctgaatg aagccatacc 720
aaacgacgag cgtgacacca cgatgcctgc agcaatggca acaacgttgc gcaaactatt 780
aactggcgaa ctacttactc tagcttcccg gcaacaatta atagactgga tggaggcgga 840
taaagttgca ggaccacttc tgcgctcggc ccttccggct ggctggttta ttgctgataa 900
atctggagcc ggtgagcgtg ggtctcgcgg tatcattgca gcactggggc cagatggtaa 960
gccctcccgt atcgtagtta tctacacgac ggggagtcag gcaactatgg atgaacgaaa 1020
tagacagatc gctgagatag gtgcctcact gattaagcat tggtaactgt cagaccaagt 1080
ttactcatat atactttaga ttgatttaaa acttcatttt taatttaaaa ggatctaggt 1140
gaagatcctt tttgataatc tcatgaccaa aatcccttaa cgtgagtttt cgttccactg 1200
agcgtcagac cccgtagaaa agatcaaagg atcttcttga gatccttttt ttctgcgcgt 1260
aatctgctgc ttgcaaacaa aaaaaccacc gctaccagcg gtggtttgtt tgccggatca 1320
agagctacca actctttttc cgaaggtaac tggcttcagc agagcgcaga taccaaatac 1380
tgtccttcta gtgtagccgt agttaggcca ccacttcaag aactctgtag caccgcctac 1440
atacctcgct ctgctaatcc tgttaccagt ggctgctgcc agtggcgata agtcgtgtct 1500
taccgggttg gactcaagac gatagttacc ggataaggcg cagcggtcgg gctgaacggg 1560
gggttcgtgc acacagccca gcttggagcg aacgacctac accgaactga gatacctaca 1620
gcgtgagcta tgagaaagcg ccacgcttcc cgaagggaga aaggcggaca ggtatccggt 1680
aagcggcagg gtcggaacag gagagcgcac gagggagctt ccagggggaa acgcctggta 1740
tctttatagt cctgtcgggt ttcgccacct ctgacttgag cgtcgatttt tgtgatgctc 1800
gtcagggggg cggagcctat ggaaaaacgc cagcaacgcg gcctttttac ggttcctggc 1860
cttttgctgg ccttttgctc acatgttctt tcctgcgtta tcccctgatt ctgtggataa 1920
ccgtattacc gcctttgagt gagctgatac cgctcgccgc agccgaacga ccgagcgcag 1980
cgagtcagtg agcgaggaag cggaagagcg cctgatgcgg tattttctcc ttacgcatct 2040
gtgcggtatt tcacaccgca tatggtgcac tctcagtaca atctgctctg atgccgcata 2100
gttaagccag tatacactcc gctatcgcta cgtgactggg tcatggctgc gccccgacac 2160
```

```
ccgccaacac ccgctgacgc gccctgacgg gcttgtctgc tcccggcatc cgcttacaga 2220
caagctgtga ccgtctccgg gagctgcatg tgtcagaggt tttcaccgtc atcaccgaaa 2280
cgcgcgaggc agcatgcatc tcaattagtc agcaaccata gtcccgcccc taactccgcc 2340
catcccgccc ctaactccgc ccagttccgc ccattctccg ccccatggct gactaatttt 2400
ttttatttat gcagaggccg aggccgcctc ggcctctgag ctattccaga agtagtgagg 2460
aggctttttt ggaggcctag gcttttgcaa aaagctagct tacagctcag ggctgcgatt 2520
tcgcgccaaa cttgacggca atcctagcgt gaaggctggt aggattttat ccccgctgcc 2580
atcatggttc gaccattgaa ctgcatcgtc gccgtgtccc aaaatatggg gattggcaag 2640
aacggagacc taccctggcc tccgctcagg aacgagttca agtacttcca aagaatgacc 2700
acaacctctt cagtggaagg taaacagaat ctggtgatta tgggtaggaa aacctggttc 2760
tccattcctg agaagaatcg acctttaaag gacagaatta atatagttct cagtagagaa 2820
ctcaaagaac caccacgagg agctcatttt cttgccaaaa gtttggatga tgccttaaga 2880
cttattgaac aaccggaatt ggcaagtaaa gtagacatgg tttggatagt cggaggcagt 2940
tctgtttacc aggaagccat gaatcaacca ggccacctca gactctttgt gacaaggatc 3000
atgcaggaat ttgaaagtga cacgtttttc ccagaaattg atttggggaa atataaactt 3060
ctcccagaat acccaggcgt cctctctgag gtccaggagg aaaaaggcat caagtataag 3120
tttgaagtct acgagaagaa agactaacag gaagatgctt tcaagttctc tgctcccctc 3180
ctaaagctat gcatttttat aagaccatgg gacttttgct ggctttagat ctttgtgaag 3240
gaaccttact tctgtggtgt gacataattg gacaaactac ctacagagat ttaaagctct 3300
aaggtaaata taaaattttt aagtgtataa tgtgttaaac tactgattct aattgtttgt 3360
gtattttaga ttccaaccta tggaactgat gaatgggagc agtggtggaa tgcctttaat 3420
gaggaaaacc tgttttgctc agaagaaatg ccatctagtg atgatgaggc tactgctgac 3480
tctcaacatt ctactcctcc aaaaaagaag agaaaggtag aagaccccaa ggactttcct 3540
tcagaattgc taagtttttt gagtcatgct gtgtttagta atagaactct tgcttgcttt 3600
gctatttaca ccacaaagga aaaagctgca ctgctataca agaaaattat ggaaaaatat 3660
tctgtaacct ttataagtag gcataacagt tataatcata acatactgtt ttttcttact 3720
ccacacaggc atagagtgtc tgctattaat aactatgctc aaaaattgtg tacctttagc 3780
tttttaattt gtaaaggggt taataaggaa tatttgatgt atagtgcctt gactagagat 3840
cataatcagc cataccacat ttgtagaggt tttacttgct ttaaaaaacc tcccacacct 3900
ccccctgaac ctgaaacata aaatgaatgc aattgttgtt gttaacttgt ttattgcagc 3960
ttataatggt tacaaataaa gcaatagcat cacaaatttc acaaataaag catttttttc 4020
actgcattct agttgtggtt tgtccaaact catcaatgta tcttatcatg tctggatcta 4080
ataaaagata tttattttca ttagatatgt gtgttggttt tttgtgtgca gtgcctctat 4140
ctggaggcca ggtagggctg gccttggggg agggggaggc cagaatgact ccaagagcta 4200
caggaaggca ggtcagagac cccactggac aaacagtggc tggactctgc accataacac 4260
acaatcaaca ggggagtgag ctggaaattt gctagcgaat tccagcacac tggcggccgt 4320
tactagttat taatagtaat caattacggg gtcattagtt catagcccat atatggagtt 4380
ccgcgttaca taacttacgg taaatggccc gcctggctga ccgcccaacg acccccgccc 4440
attgacgtca ataatgacgt atgttcccat agtaacgcca atagggactt tccattgacg 4500
tcaatgggtg gagtatttac ggtaaactgc ccacttggca gtacatcaag tgtatcatat 4560
gccaagtacg ccccctattg acgtcaatga cggtaaatgg cccgcctggc attatgccca 4620
gtacatgacc ttatgggact ttcctacttg gcagtacatc tacgtattag tcatcgctat 4680
taccatggtg atgcggtttt ggcagtacat caatgggcgt ggatagcggt ttgactcacg 4740
gggatttcca agtctccacc ccattgacgt caatgggagt ttgttttggc accaaaatca 4800
acgggacttt ccaaaatgtc gtaacaactc cgccccattg acgcaaatgg gcggtaggcg 4860
tgtacggtgg gaggtctata taagcagagc tcgtttagtg aaccgtcaga tcgcctggag 4920
acgccatcca cgctgttttg acctccatag aagacaccgg gaccgatcca gcctccgcgg 4980
ccgggaacgg tgcattggaa cgcggattcc ccgtgccaag agtgacgtaa gtaccgccta 5040
tagagtctat aggcccaccc ccttggcttc ttatgcatgc tatactgttt ttggcttggg 5100
gtctatacac ccccgcttcc tcatgttata ggtgatggta tagcttagcc tataggtgtg 5160
ggttattgac cattattgac cactccccta ttggtgacga tactttccat tactaatcca 5220
taacatggct ctttgccaca actctcttta ttggctatat gccaatacac tgtccttcag 5280
agactgacac ggactctgta tttttacagg atggggtctc atttattatt tacaaattca 5340
catatacaac accaccgtcc ccagtgcccg cagtttttat taaacataac gtgggatctc 5400
cacgcgaatc tcgggtacgt gttccggaca tgggctcttc tccggtagcg gcggagcttc 5460
tacatccgag ccctgctccc atgcctccag cgactcatgg tcgctcggca gctccttgct 5520
cctaacagtg gaggccagac ttaggcacag cacgatgccc accaccacca gtgtgccgca 5580
caaggccgtg gcggtagggt atgtgtctga aaatgagctc ggggagcggg cttgcaccgc 5640
tgacgcattt ggaagactta aggcagcggc agaagaagat gcaggcagct gagttgttgt 5700
gttctgataa gagtcagagg taactcccgt tgcggtgctg ttaacggtgg agggcagtgt 5760
agtctgagca gtactcgttg ctgccgcgcg cgccaccaga cataatagct gacagactaa 5820
cagactgttc ctttccatgg gtcttttctg cagtcaccgt ccttgacacg cgtctcggga 5880
agcttgccgc caccatggga tggagctggg tctttctctt tctcctgtca ggaactgcag 5940
```

```
gtgtcctctc tgaggtccag ctgcaacagt ctggacctga gctggtgaag cctggggctt 6000
cagtaaagat gtcctgcaag acttctagat acacattcac tgaatacacc atacactggg 6060
tgagacagag ccatggaaag agccttgagt ggattggagg tattaatcct aacaatggta 6120
ttcctaacta caaccagaag ttcaagggca gggccacatt gactgtaggc aagtcctcca 6180
gcaccgccta catggagctc cgcagcctga catctgagga ttctgcggtc tatttctgtg 6240
caagaagaag aatcgcctat ggttacgacg agggccatgc tatggactac tggggtcaag 6300
gaacctcagt caccgtctcc tcaggtgagt ggatcctctg cgcctgggcc cagctctgtc 6360
ccacaccgcg gtcacatggc accacctctc ttgcagcctc caccaagggc ccatcggtct 6420
tccccctggc accctcctcc aagagcacct ctgggggcac agcggccctg ggctgcctgg 6480
tcaaggacta cttccccgaa ccggtgacgg tgtcgtggaa ctcaggcgcc ctgaccagcg 6540
gcgtgcacac cttcccggct gtcctacagt cctcaggact ctactccctc agcagcgtgg 6600
tgaccgtgcc ctccagcagc ttgggcaccc agacctacat ctgcaacgtg aatcacaagc 6660
ccagcaacac caaggtggac aagaaagttg agcccaaatc ttgtgacaaa actcacacat 6720
gcccaccgtg cccagcacct gaactcctgg ggggaccgtc agtcttcctc ttccccccaa 6780
aacccaagga caccctcatg atctcccgga cccctgaggt cacatgcgtg gtggtggacg 6840
tgagccacga agaccctgag gtcaagttca actggtacgt ggacggcgtg gaggtgcata 6900
atgccaagac aaagccgcgg gaggagcagt acaacagcac gtaccgggtg gtcagcgtcc 6960
tcaccgtcct gcaccaggac tggctgaatg gcaaggagta caagtgcaag gtctccaaca 7020
aagccctccc agcccccatc gagaaaacca tctccaaagc caaagggcag ccccgagaac 7080
cacaggtgta caccctgccc ccatcccggg aggagatgac caagaaccag gtcagcctga 7140
cctgcctggt caaaggcttc tatcccagcg acatcgccgt ggagtgggag agcaatgggc 7200
agccggagaa caactacaag accacgcctc ccgtgctgga ctccgacggc tccttcttcc 7260
tctacagcaa gctcaccgtg gacaagagca ggtggcagca ggggaacgtc ttctcatgct 7320
ccgtgatgca tgaggctctg cacaaccact acacgcagaa gagcctctcc ctgtctccgg 7380
gtaaatgagt gcgacggccg gcaagccccg ctccccgggc tctcgcggtc gcacgaggat 7440
gcttggcacg taccccctgt acatacttcc cgggcgccca gcatggaaat aaagcaccgg 7500
atctaataaa agatatttat tttcattaga tatgtgtgtt ggtttttgt gtgcagtgcc 7560
tctatctgga ggccaggtag ggctggcctt gggggagggg gaggccagaa tgactccaag 7620
agctacagga aggcaggtca gagaccccac tggacaaaca gtggctggac tctgcaccat 7680
aacacacaat caacagggga gtgagctgga aatttgctag cgaattaatt c          7731
```

<210> 30
<211> 472
<212> PRT
<213> Homo sapiens

<400> 30

```
Met Gly Trp Ser Trp Val Phe Leu Phe Leu Leu Ser Gly Thr Ala Gly
 1               5                  10                  15

Val Leu Ser Glu Val Gln Leu Gln Gln Ser Gly Pro Glu Leu Val Lys
            20                  25                  30

Pro Gly Ala Ser Val Lys Met Ser Cys Lys Thr Ser Arg Tyr Thr Phe
        35                  40                  45

Thr Glu Tyr Thr Ile His Trp Val Arg Gln Ser His Gly Lys Ser Leu
    50                  55                  60

Glu Trp Ile Gly Gly Ile Asn Pro Asn Asn Gly Ile Pro Asn Tyr Asn
65                  70                  75                  80

Gln Lys Phe Lys Gly Arg Ala Thr Leu Thr Val Gly Lys Ser Ser Ser
                85                  90                  95

Thr Ala Tyr Met Glu Leu Arg Ser Leu Thr Ser Glu Asp Ser Ala Val
            100                 105                 110

Tyr Phe Cys Ala Arg Arg Arg Ile Ala Tyr Gly Tyr Asp Glu Gly His
        115                 120                 125

Ala Met Asp Tyr Trp Gly Gln Gly Thr Ser Val Thr Val Ser Ser Ser
```

130 135 140

Thr Lys Gly Pro Ser Val Phe Pro Leu Ala Pro Ser Ser Lys Ser Thr
145 150 155 160

Ser Gly Gly Thr Ala Ala Leu Gly Cys Leu Val Lys Asp Tyr Phe Pro
165 170 175

Glu Pro Val Thr Val Ser Trp Asn Ser Gly Ala Leu Thr Ser Gly Val
180 185 190

His Thr Phe Pro Ala Val Leu Gln Ser Ser Gly Leu Tyr Ser Leu Ser
195 200 205

Ser Val Val Thr Val Pro Ser Ser Ser Leu Gly Thr Gln Thr Tyr Ile
210 215 220

Cys Asn Val Asn His Lys Pro Ser Asn Thr Lys Val Asp Lys Lys Val
225 230 235 240

Glu Pro Lys Ser Cys Asp Lys Thr His Thr Cys Pro Pro Cys Pro Ala
245 250 255

Pro Glu Leu Leu Gly Gly Pro Ser Val Phe Leu Phe Pro Pro Lys Pro
260 265 270

Lys Asp Thr Leu Met Ile Ser Arg Thr Pro Glu Val Thr Cys Val Val
275 280 285

Val Asp Val Ser His Glu Asp Pro Glu Val Lys Phe Asn Trp Tyr Val
290 295 300

Asp Gly Val Glu Val His Asn Ala Lys Thr Lys Pro Arg Glu Glu Gln
305 310 315 320

Tyr Asn Ser Thr Tyr Arg Val Val Ser Val Leu Thr Val Leu His Gln
325 330 335

Asp Trp Leu Asn Gly Lys Glu Tyr Lys Cys Lys Val Ser Asn Lys Ala
340 345 350

Leu Pro Ala Pro Ile Glu Lys Thr Ile Ser Lys Ala Lys Gly Gln Pro
355 360 365

Arg Glu Pro Gln Val Tyr Thr Leu Pro Pro Ser Arg Glu Glu Met Thr
370 375 380

Lys Asn Gln Val Ser Leu Thr Cys Leu Val Lys Gly Phe Tyr Pro Ser
385 390 395 400

Asp Ile Ala Val Glu Trp Glu Ser Asn Gly Gln Pro Glu Asn Asn Tyr
405 410 415

Lys Thr Thr Pro Pro Val Leu Asp Ser Asp Gly Ser Phe Phe Leu Tyr
420 425 430

Ser Lys Leu Thr Val Asp Lys Ser Arg Trp Gln Gln Gly Asn Val Phe
435 440 445

Ser Cys Ser Val Met His Glu Ala Leu His Asn His Tyr Thr Gln Lys
450 455 460

Ser Leu Ser Leu Ser Pro Gly Lys

465 470

<210> 31
<211> 339
<212> DNA
<213> Homo sapiens

<400> 31

```
gacattgtga tgacccaatc tccagactct ttggctgtgt ctctagggga gagggccacc 60
atcaactgca agtccagtca gagcctttta tattctagaa atcaaaagaa ctacttggcc 120
tggtatcagc agaaaccagg acagccaccc aaactcctca tcttttgggc tagcactagg 180
gaatctgggg tacctgatag gttcagtggc agtgggtttg ggacagactt caccctcacc 240
attagcagcc tgcaggctga agatgtggca gtttattact gtcagcaata ttttagctat 300
ccgctcacgt tcggacaagg gaccaaggtg gaaataaaa                        339
```

<210> 32
<211> 113
<212> PRT
<213> Homo sapiens

<400> 32

```
Asp Ile Val Met Thr Gln Ser Pro Asp Ser Leu Ala Val Ser Leu Gly
1               5                   10                  15

Glu Arg Ala Thr Ile Asn Cys Lys Ser Ser Gln Ser Leu Leu Tyr Ser
            20                  25                  30

Arg Asn Gln Lys Asn Tyr Leu Ala Trp Tyr Gln Gln Lys Pro Gly Gln
        35                  40                  45

Pro Pro Lys Leu Leu Ile Phe Trp Ala Ser Thr Arg Glu Ser Gly Val
    50                  55                  60

Pro Asp Arg Phe Ser Gly Ser Gly Phe Gly Thr Asp Phe Thr Leu Thr
65                  70                  75                  80

Ile Ser Ser Leu Gln Ala Glu Asp Val Ala Val Tyr Tyr Cys Gln Gln
                85                  90                  95

Tyr Phe Ser Tyr Pro Leu Thr Phe Gly Gln Gly Thr Lys Val Glu Ile
            100                 105                 110

Lys
```

<210> 33
<211> 113
<212> PRT
<213> Homo sapiens

<400> 33

```
Asp Ile Val Met Thr Gln Ser Pro Asp Ser Leu Ala Val Ser Leu Gly
1               5                   10                  15

Glu Arg Ala Thr Ile Asn Cys Lys Ser Ser Gln Ser Leu Leu Tyr Ser
            20                  25                  30

Arg Asn Gln Lys Asn Tyr Leu Ala Trp Phe Gln Gln Lys Pro Gly Gln
        35                  40                  45

Pro Pro Lys Leu Leu Ile Phe Trp Ala Ser Thr Arg Glu Ser Gly Val
    50                  55                  60

Pro Asp Arg Phe Ser Gly Ser Gly Phe Gly Thr Asp Phe Thr Leu Thr
65                  70                  75                  80

Ile Ser Ser Leu Gln Ala Glu Asp Val Ala Val Tyr Asp Cys Gln Gln
                85                  90                  95

Tyr Phe Ser Tyr Pro Leu Thr Phe Gly Gln Gly Thr Lys Val Glu Ile
            100                 105                 110

Lys
```

<210> 34
<211> 113
<212> PRT
<213> Homo sapiens

<400> 34

Asp Ile Val Met Thr Gln Ser Pro Asp Ser Leu Ala Val Ser Leu Gly
1 5 10 15

Glu Arg Ala Thr Ile Asn Cys Lys Ser Ser Gln Ser Leu Leu Tyr Ser
20 25 30

Arg Asn Gln Lys Asn Tyr Leu Ala Trp Tyr Gln Gln Lys Pro Gly Gln
35 40 45

Pro Pro Lys Leu Leu Ile Tyr Trp Ala Ser Thr Arg Glu Ser Gly Val
50 55 60

Pro Asp Arg Phe Ser Gly Ser Gly Phe Gly Thr Asp Phe Thr Leu Thr
65 70 75 80

Ile Ser Ser Leu Gln Ala Glu Asp Val Ala Val Tyr Tyr Cys Gln Gln
85 90 95

Tyr Phe Ser Tyr Pro Leu Thr Phe Gly Gln Gly Thr Lys Val Glu Ile
100 105 110

Lys

<210> 35
<211> 8068
<212> DNA
<213> Homo sapiens

<400> 35

gaattccagc acactggcgg ccgttactag ttattaatag taatcaatta cggggtcatt 60
agttcatagc ccatatatgg agttccgcgt tacataactt acggtaaatg gcccgcctgg 120
ctgaccgccc aacgaccccc gcccattgac gtcaataatg acgtatgttc ccatagtaac 180
gccaataggg actttccatt gacgtcaatg ggtggagtat ttacggtaaa ctgcccactt 240
ggcagtacat caagtgtatc atatgccaag tacgccccct attgacgtca atgacggtaa 300
atggcccgcc tggcattatg cccagtacat gaccttatgg gactttccta cttggcagta 360
catctacgta ttagtcatcg ctattaccat ggtgatgcgg ttttggcagt acatcaatgg 420
gcgtggatag cggtttgact cacggggatt tccaagtctc caccccattg acgtcaatgg 480
gagtttgttt tggcaccaaa atcaacggga ctttccaaaa tgtcgtaaca actccgcccc 540
attgacgcaa atgggcggta ggcgtgtacg gtgggaggtc tatataagca gagctcgttt 600

agtgaaccgt cagatcgcct ggagacgcca tccacgctgt tttgacctcc atagaagaca 660
ccgggaccga tccagcctcc gcggccggga acggtgcatt ggaacgcgga ttccccgtgc 720
caagagtgac gtaagtaccg cctatagagt ctataggccc acccccttgg cttcttatgc 780
atgctatact gtttttggct tggggtctat acacccccgc ttcctcatgt tataggtgat 840
ggtatagctt agcctatagg tgtgggttat tgaccattat tgaccactcc cctattggtg 900
acgatacttt ccattactaa tccataacat ggctctttgc cacaactctc tttattggct 960
atatgccaat acactgtcct tcagagactg acacggactc tgtattttta caggatgggg 1020
tctcatttat tatttacaaa ttcacatata caacaccacc gtccccagtg cccgcagttt 1080
ttattaaaca taacgtggga tctccacgcg aatctcgggt acgtgttccg gacatgggct 1140
cttctccggt agcggcggag cttctacatc cgagccctgc tcccatgcct ccagcgactc 1200
atggtcgctc ggcagctcct tgctcctaac agtggaggcc agacttaggc acagcacgat 1260
gcccaccacc accagtgtgc cgcacaaggc cgtggcggta gggtatgtgt ctgaaaatga 1320
gctcggggag cgggcttgca ccgctgacgc atttggaaga cttaaggcag cggcagaaga 1380
agatgcaggc agctgagttg ttgtgttctg ataagagtca gaggtaactc ccgttgcggt 1440
gctgttaacg gtggagggca gtgtagtctg agcagtactc gttgctgccg cgcgcgccac 1500
cagacataat agctgacaga ctaacagact gttcctttcc atgggtcttt tctgcagtca 1560
ccgtccttga cacgcgtctc gggaagcttg ccgccaccat ggagacagac acactcctgc 1620
tatgggtgct gctgctctgg gttccaggtt cctccggaga cattgtgatg acccaatctc 1680
cagactcttt ggctgtgtct ctaggggaga gggccaccat caactgcaag tccagtcaga 1740
gccttttata ttctagaaat caaaagaact acttggcctg gtatcagcag aaaccaggac 1800
agccacccaa actcctcatc ttttgggcta gcactaggga atctggggta cctgataggt 1860
tcagtggcag tgggtttggg acagacttca ccctcaccat tagcagcctg caggctgaag 1920
atgtggcagt ttattactgt cagcaatatt ttagctatcc gctcacgttc ggacaaggga 1980
ccaaggtgga aataaaacgt gagtggatcc atctgggata agcatgctgt tttctgtctg 2040
tccctaacat gccctgtgat tatgcgcaaa caacacaccc aagggcagaa ctttgttact 2100
taaacaccat cctgtttgct tctttcctca ggaactgtgg ctgcaccatc tgtcttcatc 2160
ttcccgccat ctgatgagca gttgaaatct ggaactgcct ctgttgtgtg cctgctgaat 2220
aacttctatc ccagagaggc caaagtacag tggaaggtgg ataacgccct ccaatcgggt 2280
aactcccagg agagtgtcac agagcaggac agcaaggaca gcacctacag cctcagcagc 2340
accctgacgc tgagcaaagc agactacgag aaacacaaag tctacgcctg cgaagtcacc 2400
catcagggcc tgagctcgcc cgtcacaaag agcttcaaca ggggagagtg ttagagggag 2460
aagtgccccc acctgctcct cagttccagc ctgaccccct cccatccttt ggcctctgac 2520
ccttttcca caggggacct acccctattg cggtcctcca gctcatcttt cacctcaccc 2580
ccctcctcct ccttggcttt aattatgcta atgttggagg agaatgaata aataaagtga 2640
atctttgcac ctgtggtgga tctaataaaa gatatttatt ttcattagat atgtgtgttg 2700
gtttttttgtg tgcagtgcct ctatctggag gccaggtagg gctggccttg ggggaggggg 2760
aggccagaat gactccaaga gctacaggaa ggcaggtcag agaccccact ggacaaacag 2820
tggctggact ctgcaccata acacacaatc aacaggggag tgagctggaa atttgctagc 2880
gaattcttga agacgaaagg gcctcgtgat acgcctattt ttataggtta atgtcatgat 2940
aataatggtt tcttagacgt caggtggcac ttttcgggga aatgtgcgcg gaacccctat 3000
ttgtttattt ttctaaatac attcaaatat gtatccgctc atgagacaat aaccctgata 3060
aatgcttcaa taatattgaa aaaggaagag tatgagtatt caacatttcc gtgtcgccct 3120
tattcccttt tttgcggcat tttgccttcc tgtttttgct cacccagaaa cgctggtgaa 3180
agtaaaagat gctgaagatc agttgggtgc acgagtgggt tacatcgaac tggatctcaa 3240
cagcggtaag atccttgaga gttttcgccc cgaagaacgt tttccaatga tgagcacttt 3300
taaagttctg ctatgtggcg cggtattatc ccgtgttgac gccgggcaag agcaactcgg 3360
tcgccgcata cactattctc agaatgactt ggttgagtac tcaccagtca cagaaaagca 3420
tcttacggat ggcatgacag taagagaatt atgcagtgct gccataacca tgagtgataa 3480
cactgcggcc aacttacttc tgacaacgat cggaggaccg aaggagctaa ccgctttttt 3540
gcacaacatg ggggatcatg taactcgcct tgatcgttgg gaaccggagc tgaatgaagc 3600
cataccaaac gacgagcgtg acaccacgat gcctgcagca atggcaacaa cgttgcgcaa 3660
actattaact ggcgaactac ttactctagc ttcccggcaa caattaatag actggatgga 3720
ggcggataaa gttgcaggac cacttctgcg ctcggccctt ccggctggct ggtttattgc 3780
tgataaatct ggagccggtg agcgtgggtc tcgcggtatc attgcagcac tggggccaga 3840
tggtaagccc tcccgtatcg tagttatcta cacgacgggg agtcaggcaa ctatggatga 3900
acgaaataga cagatcgctg agataggtgc ctcactgatt aagcattggt aactgtcaga 3960
ccaagtttac tcatatatac tttagattga tttaaaactt catttttaat ttaaaaggat 4020
ctaggtgaag atcctttttg ataatctcat gaccaaaatc ccttaacgtg agttttcgtt 4080
ccactgagcg tcagaccccg tagaaaagat caaaggatct tcttgagatc cttttttct 4140
gcgcgtaatc tgctgcttgc aaacaaaaaa accaccgcta ccagcggtgg tttgtttgcc 4200
ggatcaagag ctaccaactc tttttccgaa ggtaactggc ttcagcagag cgcagatacc 4260
aaatactgtc cttctagtgt agccgtagtt aggccaccac ttcaagaact ctgtagcacc 4320
gcctacatac ctcgctctgc taatcctgtt accagtggct gctgccagtg gcgataagtc 4380

gtgtcttacc gggttggact caagacgata gttaccggat aaggcgcagc ggtcgggctg 4440
aacggggggt tcgtgcacac agcccagctt ggagcgaacg acctacaccg aactgagata 4500
cctacagcgt gagctatgag aaagcgccac gcttcccgaa gggagaaagg cggacaggta 4560
tccggtaagc ggcagggtcg gaacaggaga gcgcacgagg gagcttccag ggggaaacgc 4620
ctggtatctt tatagtcctg tcgggtttcg ccacctctga cttgagcgtc gatttttgtg 4680
atgctcgtca ggggggcgga gcctatggaa aaacgccagc aacgcggcct ttttacggtt 4740
cctggccttt tgctggcctt ttgctcacat gttctttcct gcgttatccc ctgattctgt 4800
ggataaccgt attaccgcct ttgagtgagc tgataccgct cgccgcagcc gaacgaccga 4860
gcgcagcgag tcagtgagcg aggaagcgga agagcgcctg atgcggtatt ttctccttac 4920
gcatctgtgc ggtatttcac accgcatatg gtgcactctc agtacaatct gctctgatgc 4980
cgcatagtta agccagtata cactccgcta tcgctacgtg actgggtcat ggctgcgccc 5040
cgacacccgc caacacccgc tgacgcgccc tgacgggctt gtctgctccc ggcatccgct 5100
tacagacaag ctgtgaccgt ctccgggagc tgcatgtgtc agaggttttc accgtcatca 5160
ccgaaacgcg cgaggcagct gtggaatgtg tgtcagttag ggtgtggaaa gtccccaggc 5220
tccccagcag gcagaagtat gcaaagcatg catctcaatt agtcagcaac caggctcccc 5280
agcaggcaga agtatgcaaa gcatgcatct caattagtca gcaaccatag tcccgcccct 5340
aactccgccc atcccgcccc taactccgcc cagttccgcc cattctccgc cccatggctg 5400
actaattttt tttatttatg cagaggccga ggccgcctcg gcctctgagc tattccagaa 5460
gtagtgagga ggcttttttg gaggcctagg cttttgcaaa aagctagctt cacgctgccg 5520
caagcactca gggcgcaagg gctgctaaag gaagcggaac acgtagaaag ccagtccgca 5580
gaaacggtgc tgaccccgga tgaatgtcag ctactgggct atctggacaa gggaaaacgc 5640
aagcgcaaag agaaagcagg tagcttgcag tgggcttaca tggcgatagc tagactgggc 5700
ggttttatgg acagcaagcg aaccggaatt gccagctggg gcgccctctg gtaaggttgg 5760
gaagccctgc aaagtaaact ggatggcttt cttgccgcca aggatctgat ggcgcagggg 5820
atcaagatct gatcaagaga caggatgagg atcgtttcgc atgattgaac aagatggatt 5880
gcacgcaggt tctccggccg cttgggtgga gaggctattc ggctatgact gggcacaaca 5940
gacaatcggc tgctctgatg ccgccgtgtt ccggctgtca gcgcaggggc gcccggttct 6000
ttttgtcaag accgacctgt ccggtgccct gaatgaactg caggacgagg cagcgcggct 6060
atcgtggctg gccacgacgg gcgttccttg cgcagctgtg ctcgacgttg tcactgaagc 6120
gggaagggac tggctgctat tgggcgaagt gccggggcag gatctcctgt catctcacct 6180
tgctcctgcc gagaaagtat ccatcatggc tgatgcaatg cggcggctgc atacgcttga 6240
tccggctacc tgcccattcg accaccaagc gaaacatcgc atcgagcgag cacgtactcg 6300
gatggaagcc ggtcttgtcg atcaggatga tctggacgaa gagcatcagg ggctcgcgcc 6360
agccgaactg ttcgccaggc tcaaggcgcg catgcccgac ggcgaggatc tcgtcgtgac 6420
ccatggcgat gcctgcttgc cgaatatcat ggtggaaaat ggccgctttt ctggattcat 6480
cgactgtggc cggctgggtg tggcggaccg ctatcaggac atagcgttgg ctacccgtga 6540
tattgctgaa gagcttggcg gcgaatgggc tgaccgcttc ctcgtgcttt acggtatcgc 6600
cgctcccgat tcgcagcgca tcgccttcta tcgccttctt gacgagttct tctgagcggg 6660
actctggggt tcgaaatgac cgaccaagcg acgcccaacc tgccatcacg agatttcgat 6720
tccaccgccg ccttctatga aaggttgggc ttcggaatcg ttttccggga cgccggctgg 6780
atgatcctcc agcgcgggga tctcatgctg gagttcttcg cccaccccgg gctcgatccc 6840
ctcgcgagtt ggttcagctg ctgcctgagg ctggacgacc tcgcggagtt ctaccggcag 6900
tgcaaatccg tcggcatcca ggaaaccagc agcggctatc cgcgcatcca tgcccccgaa 6960
ctgcaggagt ggggaggcac gatggccgct ttggtcccgg atctttgtga aggaacctta 7020
cttctgtggt gtgacataat tggacaaact acctacagag atttaaagct ctaaggtaaa 7080
tataaaattt ttaagtgtat aatgtgttaa actactgatt ctaattgttt gtgtatttta 7140
gattccaacc tatggaactg atgaatggga gcagtggtgg aatgccttta atgaggaaaa 7200
cctgttttgc tcagaagaaa tgccatctag tgatgatgag gctactgctg actctcaaca 7260
ttctactcct ccaaaaaaga agagaaaggt agaagacccc aaggactttc cttcagaatt 7320
gctaagtttt ttgagtcatg ctgtgtttag taatagaact cttgcttgct ttgctattta 7380
caccacaaag gaaaaagctg cactgctata caagaaaatt atggaaaaat attctgtaac 7440
ctttataagt aggcataaca gttataatca taacatactg ttttttctta ctccacacag 7500
gcatagagtg tctgctatta ataactatgc tcaaaaattg tgtaccttta gctttttaat 7560
ttgtaaaggg gttaataagg aatatttgat gtatagtgcc ttgactagag atcataatca 7620
gccataccac atttgtagag gttttacttg ctttaaaaaa cctcccacac ctccccctga 7680
acctgaaaca taaaatgaat gcaattgttg ttgttaactt gtttattgca gcttataatg 7740
gttacaaata aagcaatagc atcacaaatt tcacaaataa agcatttttt tcactgcatt 7800
ctagttgtgg tttgtccaaa ctcatcaatg tatcttatca tgtctggatc taataaaaga 7860
tatttatttt cattagatat gtgtgttggt tttttgtgtg cagtgcctct atctggaggc 7920
caggtagggc tggccttggg ggagggggag gccagaatga ctccaagagc tacaggaagg 7980
caggtcagag accccactgg acaaacagtg gctggactct gcaccataac acacaatcaa 8040
caggggagtg agctggaaat ttgctagc 8068

<210> 36
<211> 234
<212> PRT
<213> Homo sapiens

<400> 36

```
Met Glu Thr Asp Thr Leu Leu Leu Trp Val Leu Leu Leu Trp Val Pro
 1               5                  10                  15

Gly Ser Ser Gly Asp Ile Val Met Thr Gln Ser Pro Asp Ser Leu Ala
            20                  25                  30

Val Ser Leu Gly Glu Arg Ala Thr Ile Asn Cys Lys Ser Ser Gln Ser
        35                  40                  45

Leu Leu Tyr Ser Arg Asn Gln Lys Asn Tyr Leu Ala Trp Tyr Gln Gln
    50                  55                  60

Lys Pro Gly Gln Pro Pro Lys Leu Leu Ile Phe Trp Ala Ser Thr Arg
65                  70                  75                  80

Glu Ser Gly Val Pro Asp Arg Phe Ser Gly Ser Gly Phe Gly Thr Asp
                85                  90                  95

Phe Thr Leu Thr Ile Ser Ser Leu Gln Ala Glu Asp Val Ala Val Tyr
            100                 105                 110

Tyr Cys Gln Gln Tyr Phe Ser Tyr Pro Leu Thr Phe Gly Gln Gly Thr
        115                 120                 125

Lys Val Glu Ile Lys Arg Val Phe Ile Phe Pro Pro Ser Asp Glu Gln
    130                 135                 140

Leu Lys Ser Gly Thr Ala Ser Val Val Cys Leu Leu Asn Asn Phe Tyr
145                 150                 155                 160

Pro Arg Glu Ala Lys Val Gln Trp Lys Val Asp Asn Ala Leu Gln Ser
                165                 170                 175

Gly Asn Ser Gln Glu Ser Val Thr Glu Gln Asp Ser Lys Asp Ser Thr
            180                 185                 190

Tyr Ser Leu Ser Ser Thr Leu Thr Leu Ser Lys Ala Asp Tyr Glu Lys
        195                 200                 205

His Lys Val Tyr Ala Cys Glu Val Thr His Gln Gly Leu Ser Ser Pro
    210                 215                 220

Val Thr Lys Ser Phe Asn Arg Gly Glu Cys
225                 230
```

<210> 37
<211> 372
<212> DNA
<213> Homo sapiens

<400> 37

```
caggtgcaac tagtgcagtc cggcgccgaa gtgaagaaac ccggtgcttc cgtgaaagtc 60
agctgtaaaa ctagtagata caccttcact gaatacacca tacactgggt tagacaggcc 120
cctggccaaa ggctggagtg gataggaggt attaatccta acaatggtat tcctaactac 180
aaccagaagt tcaagggccg ggccaccttg accgtaggca agtctgccag caccgcctac 240

atggaactgt ccagcctgcg ctccgaggac actgcagtct actactgcgc cagaagaaga 300
atcgcctatg gttacgacga gggccatgct atggactact ggggtcaagg aacccttgtc 360
accgtctcct ca                                                     372
```

<210> 38
<211> 124
<212> PRT
<213> Homo sapiens

<400> 38

```
Gln Val Gln Leu Val Gln Ser Gly Ala Glu Val Lys Lys Pro Gly Ala
1               5                   10                  15

Ser Val Lys Val Ser Cys Lys Thr Ser Arg Tyr Thr Phe Thr Glu Tyr
            20                  25                  30

Thr Ile His Trp Val Arg Gln Ala Pro Gly Gln Arg Leu Glu Trp Ile
        35                  40                  45

Gly Gly Ile Asn Pro Asn Asn Gly Ile Pro Asn Tyr Asn Gln Lys Phe
    50                  55                  60

Lys Gly Arg Ala Thr Leu Thr Val Gly Lys Ser Ala Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Arg Arg Ile Ala Tyr Gly Tyr Asp Glu Gly His Ala Met Asp
            100                 105                 110

Tyr Trp Gly Gln Gly Thr Leu Val Thr Val Ser Ser
        115                 120
```

<210> 39
<211> 124
<212> PRT
<213> Homo sapiens

<400> 39

```
Gln Val Gln Leu Val Gln Ser Gly Ala Glu Val Lys Lys Pro Gly Ala
 1               5                  10                  15

Ser Val Lys Val Ser Cys Lys Thr Ser Arg Tyr Thr Phe Thr Glu Tyr
            20                  25                  30

Thr Ile His Trp Val Arg Gln Ala Pro Gly Gln Arg Leu Glu Trp Ile
        35                  40                  45

Gly Gly Ile Asn Pro Asn Asn Gly Ile Pro Asn Tyr Asn Gln Lys Phe
    50                  55                  60

Lys Gly Arg Ala Thr Leu Thr Val Gly Lys Ser Ala Ser Thr Ala Tyr
65                  70                  75                  80

Met Glu Leu Ser Ser Leu Arg Ser Glu Asp Thr Ala Val Tyr Phe Cys
                85                  90                  95

Ala Arg Arg Arg Ile Ala Tyr Gly Tyr Asp Glu Gly His Ala Met Asp
            100                 105                 110

Tyr Trp Gly Gln Gly Thr Leu Val Thr Val Ser Ser
        115                 120
```

<210> 40
<211> 124
<212> PRT
<213> Homo sapiens

<400> 40

```
Gln Val Gln Leu Val Gln Ser Gly Ala Glu Val Lys Lys Pro Gly Ala
1               5                   10                  15

Ser Val Lys Val Ser Cys Lys Thr Ser Arg Tyr Thr Phe Thr Glu Tyr
            20                  25                  30

Thr Ile His Trp Val Arg Gln Ala Pro Gly Gln Arg Leu Glu Trp Ile
        35                  40                  45

Gly Gly Ile Asn Pro Asn Asn Gly Ile Pro Asn Tyr Asn Gln Lys Phe
    50                  55                  60

Lys Gly Arg Val Thr Ile Thr Val Asp Thr Ser Ala Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Arg Arg Ile Ala Tyr Gly Tyr Asp Glu Gly His Ala Met Asp
            100                 105                 110

Tyr Trp Gly Gln Gly Thr Leu Val Thr Val Ser Ser
        115                 120
```

<210> 41
<211> 124
<212> PRT
<213> Homo sapiens

<400> 41

```
Gln Val Gln Leu Val Gln Ser Gly Ala Glu Val Lys Lys Pro Gly Ala
1               5                   10                  15

Ser Val Lys Val Ser Cys Lys Thr Ser Gly Tyr Thr Phe Thr Glu Tyr
            20                  25                  30

Thr Ile His Trp Val Arg Gln Ala Pro Gly Gln Arg Leu Glu Trp Ile
        35                  40                  45

Gly Gly Ile Asn Pro Asn Asn Gly Ile Pro Asn Tyr Asn Gln Lys Phe
    50                  55                  60

Lys Gly Arg Val Thr Ile Thr Val Asp Thr Ser Ala Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Arg Arg Ile Ala Tyr Gly Tyr Asp Glu Gly His Ala Met Asp
            100                 105                 110

Tyr Trp Gly Gln Gly Thr Leu Val Thr Val Ser Ser
```

115 120

<210> 42
<211> 7731
<212> DNA
<213> Homo sapiens

<400> 42

```
ttgaagacga aagggcctcg tgatacgcct atttttatag gttaatgtca tgataataat 60
ggtttcttag acgtcaggtg gcacttttcg gggaaatgtg cgcggaaccc ctatttgttt 120
atttttctaa atacattcaa atatgtatcc gctcatgaga caataaccct gataaatgct 180
tcaataatat tgaaaaagga agagtatgag tattcaacat ttccgtgtcg cccttattcc 240
cttttttgcg gcattttgcc ttcctgtttt tgctcaccca gaaacgctgg tgaaagtaaa 300
agatgctgaa gatcagttgg gtgcacgagt gggttacatc gaactggatc tcaacagcgg 360
taagatcctt gagagttttc gccccgaaga acgttttcca atgatgagca cttttaaagt 420
tctgctatgt ggcgcggtat tatcccgtgt tgacgccggg caagagcaac tcggtcgccg 480
catacactat tctcagaatg acttggttga gtactcacca gtcacagaaa agcatcttac 540
ggatggcatg acagtaagag aattatgcag tgctgccata accatgagtg ataacactgc 600
ggccaactta cttctgacaa cgatcggagg accgaaggag ctaaccgctt ttttgcacaa 660
catgggggat catgtaactc gccttgatcg ttgggaaccg gagctgaatg aagccatacc 720
aaacgacgag cgtgacacca cgatgcctgc agcaatggca acaacgttgc gcaaactatt 780
aactggcgaa ctacttactc tagcttcccg gcaacaatta atagactgga tggaggcgga 840
taaagttgca ggaccacttc tgcgctcggc ccttccggct ggctggttta ttgctgataa 900
atctggagcc ggtgagcgtg ggtctcgcgg tatcattgca gcactggggc cagatggtaa 960
gccctcccgt atcgtagtta tctacacgac ggggagtcag gcaactatgg atgaacgaaa 1020
tagacagatc gctgagatag gtgcctcact gattaagcat tggtaactgt cagaccaagt 1080
ttactcatat atactttaga ttgatttaaa acttcatttt taatttaaaa ggatctaggt 1140
gaagatcctt tttgataatc tcatgaccaa aatcccttaa cgtgagtttt cgttccactg 1200
agcgtcagac cccgtagaaa agatcaaagg atcttcttga gatccttttt ttctgcgcgt 1260
aatctgctgc ttgcaaacaa aaaaaccacc gctaccagcg gtggtttgtt tgccggatca 1320
agagctacca actctttttc cgaaggtaac tggcttcagc agagcgcaga taccaaatac 1380
tgtccttcta gtgtagccgt agttaggcca ccacttcaag aactctgtag caccgcctac 1440
atacctcgct ctgctaatcc tgttaccagt ggctgctgcc agtggcgata agtcgtgtct 1500
taccgggttg gactcaagac gatagttacc ggataaggcg cagcggtcgg gctgaacggg 1560
gggttcgtgc acacagccca gcttggagcg aacgacctac accgaactga gatacctaca 1620
gcgtgagcta tgagaaagcg ccacgcttcc cgaagggaga aaggcggaca ggtatccggt 1680
aagcggcagg gtcggaacag gagagcgcac gagggagctt ccagggggaa acgcctggta 1740
tctttatagt cctgtcgggt ttcgccacct ctgacttgag cgtcgatttt tgtgatgctc 1800
gtcagggggg cggagcctat ggaaaaacgc cagcaacgcg gcctttttac ggttcctggc 1860
cttttgctgg ccttttgctc acatgttctt tcctgcgtta tcccctgatt ctgtggataa 1920
ccgtattacc gcctttgagt gagctgatac cgctcgccgc agccgaacga ccgagcgcag 1980
cgagtcagtg agcgaggaag cggaagagcg cctgatgcgg tattttctcc ttacgcatct 2040
gtgcggtatt tcacaccgca tatggtgcac tctcagtaca atctgctctg atgccgcata 2100
gttaagccag tatacactcc gctatcgcta cgtgactggg tcatggctgc gccccgacac 2160
ccgccaacac ccgctgacgc gccctgacgg gcttgtctgc tcccggcatc cgcttacaga 2220
caagctgtga ccgtctccgg gagctgcatg tgtcagaggt tttcaccgtc atcaccgaaa 2280
cgcgcgaggc agcatgcatc tcaattagtc agcaaccata gtcccgcccc taactccgcc 2340
catcccgccc ctaactccgc ccagttccgc ccattctccg ccccatggct gactaatttt 2400
ttttatttat gcagaggccg aggccgcctc ggcctctgag ctattccaga agtagtgagg 2460
aggctttttt ggaggcctag gcttttgcaa aaagctagct tacagctcag ggctgcgatt 2520
tcgcgccaaa cttgacggca atcctagcgt gaaggctggt aggattttat ccccgctgcc 2580
atcatggttc gaccattgaa ctgcatcgtc gccgtgtccc aaaatatggg gattggcaag 2640
aacggagacc taccctggcc tccgctcagg aacgagttca agtacttcca aagaatgacc 2700
acaacctctt cagtggaagg taaacagaat ctggtgatta tgggtaggaa aacctggttc 2760
tccattcctg agaagaatcg acctttaaag gacagaatta atatagttct cagtagagaa 2820
ctcaaagaac caccacgagg agctcatttt cttgccaaaa gtttggatga tgccttaaga 2880
cttattgaac aaccggaatt ggcaagtaaa gtagacatgg tttggatagt cggaggcagt 2940
tctgtttacc aggaagccat gaatcaacca ggccacctca gactctttgt gacaaggatc 3000
atgcaggaat ttgaaagtga cacgtttttc ccagaaattg atttggggaa atataaactt 3060
ctcccagaat acccaggcgt cctctctgag gtccaggagg aaaaaggcat caagtataag 3120
tttgaagtct acgagaagaa agactaacag gaagatgctt tcaagttctc tgctcccctc 3180
ctaaagctat gcatttttat aagaccatgg gacttttgct ggctttagat ctttgtgaag 3240
```

gaaccttact tctgtggtgt gacataattg gacaaactac ctacagagat ttaaagctct 3300
aaggtaaata taaaattttt aagtgtataa tgtgttaaac tactgattct aattgtttgt 3360
gtattttaga ttccaaccta tggaactgat gaatgggagc agtggtggaa tgcctttaat 3420
gaggaaaacc tgttttgctc agaagaaatg ccatctagtg atgatgaggc tactgctgac 3480
tctcaacatt ctactcctcc aaaaaagaag agaaaggtag aagaccccaa ggactttcct 3540
tcagaattgc taagtttttt gagtcatgct gtgtttagta atagaactct tgcttgcttt 3600
gctatttaca ccacaaagga aaaagctgca ctgctataca agaaaattat ggaaaaatat 3660
tctgtaacct ttataagtag gcataacagt tataatcata acatactgtt ttttcttact 3720
ccacacaggc atagagtgtc tgctattaat aactatgctc aaaaattgtg tacctttagc 3780
tttttaattt gtaaaggggt taataaggaa tatttgatgt atagtgcctt gactagagat 3840
cataatcagc cataccacat ttgtagaggt tttacttgct ttaaaaaacc tcccacacct 3900
ccccctgaac ctgaaacata aaatgaatgc aattgttgtt gttaacttgt ttattgcagc 3960
ttataatggt tacaaataaa gcaatagcat cacaaatttc acaaataaag catttttttc 4020
actgcattct agttgtggtt tgtccaaact catcaatgta tcttatcatg tctggatcta 4080
ataaaagata tttattttca ttagatatgt gtgttggttt tttgtgtgca gtgcctctat 4140
ctggaggcca ggtagggctg gccttggggg agggggaggc cagaatgact ccaagagcta 4200
caggaaggca ggtcagagac cccactggac aaacagtggc tggactctgc accataacac 4260
acaatcaaca ggggagtgag ctggaaattt gctagcgaat tccagcacac tggcggccgt 4320
tactagttat taatagtaat caattacggg gtcattagtt catagcccat atatggagtt 4380
ccgcgttaca taacttacgg taaatggccc gcctggctga ccgcccaacg acccccgccc 4440
attgacgtca ataatgacgt atgttcccat agtaacgcca atagggactt tccattgacg 4500
tcaatgggtg gagtatttac ggtaaactgc ccacttggca gtacatcaag tgtatcatat 4560
gccaagtacg ccccctattg acgtcaatga cggtaaatgg cccgcctggc attatgccca 4620
gtacatgacc ttatgggact ttcctacttg gcagtacatc tacgtattag tcatcgctat 4680
taccatggtg atgcggtttt ggcagtacat caatgggcgt ggatagcggt ttgactcacg 4740
gggatttcca agtctccacc ccattgacgt caatgggagt ttgttttggc accaaaatca 4800
acgggacttt ccaaaatgtc gtaacaactc cgccccattg acgcaaatgg gcggtaggcg 4860
tgtacggtgg gaggtctata taagcagagc tcgtttagtg aaccgtcaga tcgcctggag 4920
acgccatcca cgctgttttg acctccatag aagacaccgg gaccgatcca gcctccgcgg 4980
ccgggaacgg tgcattggaa cgcggattcc ccgtgccaag agtgacgtaa gtaccgccta 5040
tagagtctat aggcccaccc ccttggcttc ttatgcatgc tatactgttt ttggcttggg 5100
gtctatacac ccccgcttcc tcatgttata ggtgatggta tagcttagcc tataggtgtg 5160
ggttattgac cattattgac cactccccta ttggtgacga tactttccat tactaatcca 5220
taacatggct ctttgccaca actctcttta ttggctatat gccaatacac tgtccttcag 5280
agactgacac ggactctgta tttttacagg atggggtctc atttattatt tacaaattca 5340
catatacaac accaccgtcc ccagtgcccg cagtttttat taaacataac gtgggatctc 5400
cacgcgaatc tcgggtacgt gttccggaca tgggctcttc tccggtagcg gcggagcttc 5460
tacatccgag ccctgctccc atgcctccag cgactcatgg tcgctcggca gctccttgct 5520
cctaacagtg gaggccagac ttaggcacag cacgatgccc accaccacca gtgtgccgca 5580
caaggccgtg gcggtagggt atgtgtctga aaatgagctc ggggagcggg cttgcaccgc 5640
tgacgcattt ggaagactta aggcagcggc agaagaagat gcaggcagct gagttgttgt 5700
gttctgataa gagtcagagg taactcccgt tgcggtgctg ttaacggtgg agggcagtgt 5760
agtctgagca gtactcgttg ctgccgcgcg cgccaccaga cataatagct gacagactaa 5820
cagactgttc ctttccatgg gtcttttctg cagtcaccgt ccttgacacg cgtctcggga 5880
agcttgccgc caccatggac tggacctggc gcgtgttttg cctgctcgcc gtggctcctg 5940
gggcccacag ccaggtgcaa ctggtgcagt ccggcgccga agtgaagaaa cccggtgctt 6000
ccgtgaaagt cagctgtaaa actagtagat acaccttcac tgaatacacc atacactggg 6060
ttagacaggc ccctggccaa aggctggagt ggataggagg tattaatcct aacaatggta 6120
ttcctaacta caaccagaag ttcaagggcc gggccacctt gaccgtaggc aagtctgcca 6180
gcaccgccta catggaactg tccagcctgc gctccgagga cactgcagtc tactactgcg 6240
ccagaagaag aatcgcctat ggttacgacg agggccatgc tatggactac tggggtcaag 6300
gaacccttgt caccgtctcc tcaggtgagt ggatcctctg cgcctgggcc cagctctgtc 6360
ccacaccgcg gtcacatggc accacctctc ttgcagcctc caccaagggc ccatcggtct 6420
tcccccctggc accctcctcc aagagcacct ctgggggcac agcggccctg ggctgcctgg 6480
tcaaggacta cttccccgaa ccggtgacgg tgtcgtggaa ctcaggcgcc ctgaccagcg 6540
gcgtgcacac cttcccggct gtcctacagt cctcaggact ctactccctc agcagcgtgg 6600
tgaccgtgcc ctccagcagc ttgggcaccc agacctacat ctgcaacgtg aatcacaagc 6660
ccagcaacac caaggtggac aagaaagttg agcccaaatc ttgtgacaaa actcacacat 6720
gcccaccgtg cccagcacct gaactcctgg ggggaccgtc agtcttcctc ttccccccaa 6780
aacccaagga caccctcatg atctcccgga cccctgaggt cacatgcgtg gtggtggacg 6840
tgagccacga agaccctgag gtcaagttca actggtacgt ggacggcgtg gaggtgcata 6900
atgccaagac aaagccgcgg gaggagcagt acaacagcac gtaccgggtg gtcagcgtcc 6960
tcaccgtcct gcaccaggac tggctgaatg gcaaggagta caagtgcaag gtctccaaca 7020

```
aagccctccc agcccccatc gagaaaacca tctccaaagc caaagggcag ccccgagaac 7080
cacaggtgta caccctgccc ccatcccggg aggagatgac caagaaccag gtcagcctga 7140
cctgcctggt caaaggcttc tatcccagcg acatcgccgt ggagtgggag agcaatgggc 7200
agccggagaa caactacaag accacgcctc ccgtgctgga ctccgacggc tccttcttcc 7260
tctacagcaa gctcaccgtg gacaagagca ggtggcagca ggggaacgtc ttctcatgct 7320
ccgtgatgca tgaggctctg cacaaccact acacgcagaa gagcctctcc ctgtctccgg 7380
gtaaatgagt gcgacggccg gcaagccccg ctccccgggc tctcgcggtc gcacgaggat 7440
gcttggcacg taccccctgt acatacttcc cgggcgccca gcatggaaat aaagcaccgg 7500
atctaataaa agatatttat tttcattaga tatgtgtgtt ggtttttttgt gtgcagtgcc 7560
tctatctgga ggccaggtag ggctggcctt gggggagggg gaggccagaa tgactccaag 7620
agctacagga aggcaggtca gagaccccac tggacaaaca gtggctggac tctgcaccat 7680
aacacacaat caacagggga gtgagctgga aatttgctag cgaattaatt c          7731
```

<210> 43
<211> 472
<212> PRT
<213> Homo sapiens

<400> 43

Met Asp Trp Thr Trp Arg Val Phe Cys Leu Leu Ala Val Ala Pro Gly
1 5 10 15

Ala His Ser Gln Val Gln Leu Val Gln Ser Gly Ala Glu Val Lys Lys
20 25 30

Pro Gly Ala Ser Val Lys Val Ser Cys Lys Thr Ser Arg Tyr Thr Phe
35 40 45

Thr Glu Tyr Thr Ile His Trp Val Arg Gln Ala Pro Gly Gln Arg Leu
50 55 60

Glu Trp Ile Gly Gly Ile Asn Pro Asn Asn Gly Ile Pro Asn Tyr Asn
65 70 75 80

Gln Lys Phe Lys Gly Arg Ala Thr Leu Thr Val Gly Lys Ser Ala Ser
85 90 95

Thr Ala Tyr Met Glu Leu Ser Ser Leu Arg Ser Glu Asp Thr Ala Val
100 105 110

Tyr Tyr Cys Ala Arg Arg Arg Ile Ala Tyr Gly Tyr Asp Glu Gly His
115 120 125

Ala Met Asp Tyr Trp Gly Gln Gly Thr Leu Val Thr Val Ser Ser Ser
130 135 140

Thr Lys Gly Pro Ser Val Phe Pro Leu Ala Pro Ser Ser Lys Ser Thr
145 150 155 160

Ser Gly Gly Thr Ala Ala Leu Gly Cys Leu Val Lys Asp Tyr Phe Pro
165 170 175

Glu Pro Val Thr Val Ser Trp Asn Ser Gly Ala Leu Thr Ser Gly Val
180 185 190

His Thr Phe Pro Ala Val Leu Gln Ser Ser Gly Leu Tyr Ser Leu Ser
195 200 205

Ser Val Val Thr Val Pro Ser Ser Ser Leu Gly Thr Gln Thr Tyr Ile
210 215 220

Cys Asn Val Asn His Lys Pro Ser Asn Thr Lys Val Asp Lys Lys Val

```
225                 230                 235                 240

Glu Pro Lys Ser Cys Asp Lys Thr His Thr Cys Pro Pro Cys Pro Ala
                245                 250                 255

Pro Glu Leu Leu Gly Gly Pro Ser Val Phe Leu Phe Pro Pro Lys Pro
            260                 265                 270

Lys Asp Thr Leu Met Ile Ser Arg Thr Pro Glu Val Thr Cys Val Val
        275                 280                 285

Val Asp Val Ser His Glu Asp Pro Glu Val Lys Phe Asn Trp Tyr Val
    290                 295                 300

Asp Gly Val Glu Val His Asn Ala Lys Thr Lys Pro Arg Glu Glu Gln
305                 310                 315                 320

Tyr Asn Ser Thr Tyr Arg Val Val Ser Val Leu Thr Val Leu His Gln
                325                 330                 335

Asp Trp Leu Asn Gly Lys Glu Tyr Lys Cys Lys Val Ser Asn Lys Ala
            340                 345                 350

Leu Pro Ala Pro Ile Glu Lys Thr Ile Ser Lys Ala Lys Gly Gln Pro
        355                 360                 365

Arg Glu Pro Gln Val Tyr Thr Leu Pro Pro Ser Arg Glu Glu Met Thr
    370                 375                 380

Lys Asn Gln Val Ser Leu Thr Cys Leu Val Lys Gly Phe Tyr Pro Ser
385                 390                 395                 400

Asp Ile Ala Val Glu Trp Glu Ser Asn Gly Gln Pro Glu Asn Asn Tyr
                405                 410                 415

Lys Thr Thr Pro Pro Val Leu Asp Ser Asp Gly Ser Phe Phe Leu Tyr
            420                 425                 430

Ser Lys Leu Thr Val Asp Lys Ser Arg Trp Gln Gln Gly Asn Val Phe
        435                 440                 445

Ser Cys Ser Val Met His Glu Ala Leu His Asn His Tyr Thr Gln Lys
    450                 455                 460

Ser Leu Ser Leu Ser Pro Gly Lys
465                 470
```

<210> 44
<211> 25
<212> DNA
<213> Homo sapiens

<400> 44
accgtctcct caggtgagtg gatcc 25

<210> 45
<211> 14

<212> DNA
<213> Homo sapiens

<400> 45
cctctcttgc agcc 14

<210> 46
<211> 14
<212> DNA
<213> Homo sapiens

<400> 46
cctctcttgc agcc 14

<210> 47
<211> 4
<212> PRT
<213> Homo sapiens

<400> 47

Thr Val Ser Ser
1

<210> 48
<211> 4
<212> PRT
<213> Homo sapiens

<400> 48

Ser Thr Lys Gly
1

<210> 49
<211> 27
<212> DNA
<213> Homo sapiens

<400> 49
accgtctcct cagcctccac caagggc 27

<210> 50
<211> 8
<212> PRT
<213> Homo sapiens

<400> 50

Thr Val Ser Ser Ser Thr Lys Gly
1 5

<210> 51
<211> 27

<212> DNA
<213> Homo sapiens

<400> 51
accgtctcct cagcctccac caagggc 27

<210> 52
<211> 9
<212> PRT
<213> Homo sapiens

<400> 52

Thr Val Ser Ser Ala Ser Thr Lys Gly
1 5

<210> 53
<211> 22
<212> DNA
<213> Homo sapiens

<400> 53
gaaataaaac gtgagtggat cc 22

<210> 54
<211> 27
<212> DNA
<213> Homo sapiens

<400> 54
cttctttcct caggaactgt ggctgca 27

<210> 55
<211> 4
<212> PRT
<213> Homo sapiens

<400> 55

Thr Val Ala Ala
1

<210> 56
<211> 24
<212> DNA
<213> Homo sapiens

<400> 56
gaaataaaac gaactgtggc tgca 24

<210> 57
<211> 7
<212> PRT
<213> Homo sapiens

<400> 57

Glu Ile Lys Thr Val Ala Ala
1 5

<210> 58
<211> 24
<212> DNA
<213> Homo sapiens

<400> 58
gaaataaaac gaactgtggc tgca 24

<210> 59
<211> 8
<212> PRT
<213> Homo sapiens

<400> 59

Glu Ile Lys Arg Thr Val Ala Ala
1 5

<210> 60
<211> 20
<212> PRT
<213> Homo sapiens

<400> 60

Met Asp Ser Gln Ala Gln Val Leu Met Leu Leu Leu Leu Trp Val Ser
1 5 10 15

Gly Thr Cys Gly
20

<210> 61
<211> 19
<212> PRT
<213> Homo sapiens

<400> 61

Met Gly Trp Ser Trp Val Phe Leu Phe Leu Leu Ser Gly Thr Ala Gly
1 5 10 15

Val Leu Ser

<210> 62
<211> 9
<212> DNA
<213> Homo sapiens

<400> 62
gccgccacc 9

<210> 63
<211> 37
<212> DNA
<213> Artificial Sequence

<220>
<223> Description of Artificial Sequence:PRIMER

<400> 63
cagaaagctt gccgccacca tggattcaca ggcccag 37

<210> 64
<211> 6
<212> PRT
<213> Homo sapiens

<400> 64

Met Asp Ser Gln Ala Gln
1 5

<210> 65
<211> 35
<212> DNA
<213> Artificial Sequence

<220>
<223> Description of Artificial Sequence:primer

<400> 65
ccgaggatcc actcacgttt cagctccagc ttggt 35

<210> 66
<211> 37
<212> DNA
<213> Artificial Sequence

<220>
<223> Description of Artificial Sequence:primer

<400> 66
cagaaagctt gccgccacca tgggatggag ctgggtc 37

<210> 67
<211> 6
<212> PRT
<213> Homo sapiens

<400> 67

Met Gly Trp Ser Trp Val
1 5

<210> 68

<211> 35
<212> DNA
<213> Artificial Sequence

<220>
<223> Description of Artificial Sequence:primer

<400> 68
ccgaggatcc actcacctga ggagacggtg actga 35

<210> 69
<211> 36
<212> DNA
<213> Artificial Sequence

<220>
<223> Description of Artificial Sequence:primer

<400> 69
gtcatcacaa tgtctccgga ggaacctgga acccag 36

<210> 70
<211> 29
<212> DNA
<213> Artificial Sequence

<220>
<223> Description of Artificial Sequence:primer

<400> 70
ctccggagac attgtgatga cccaatctc 29

<210> 71
<211> 45
<212> DNA
<213> Artificial Sequence

<220>
<223> Description of Artificial Sequence:primer

<400> 71
gaatataaaa ggctctgact ggacttgcag ttgatggtgg ccctc 45

<210> 72
<211> 72
<212> DNA
<213> Artificial Sequence

<220>
<223> Description of Artificial Sequence:primer

<400> 72
cagtcagagc cttttatatt ctagaaatca aaagaactac ttggcctggt atcagcagaa 60
accaggacag cc 72

<210> 73
<211> 44
<212> DNA

<213> Artificial Sequence

<220>
<223> Description of Artificial Sequence:primer

<400> 73
accccagatt ccctagtgct agcccaaaag atgaggagtt tggg 44

<210> 74
<211> 67
<212> DNA
<213> Artificial Sequence

<220>
<223> Description of Artificial Sequence:primer

<400> 74
tagcactagg gaatctgggg tacctgatag gttcagtggc agtgggtttg ggacagactt 60
caccctc 67

<210> 75
<211> 53
<212> DNA
<213> Artificial Sequence

<220>
<223> Description of Artificial Sequence:primer

<400> 75
gtcccttgtc cgaacgtgag cggatagcta aaatattgct gacagtaata aac 53

<210> 76
<211> 33
<212> DNA
<213> Artificial Sequence

<220>
<223> Description of Artificial Sequence:primer

<400> 76
gctcacgttc ggacaaggga ccaaggtgga aat 33

<210> 77
<211> 72
<212> DNA
<213> Artificial Sequence

<220>
<223> Description of Artificial Sequence:primer

<400> 77
cagtcagagc cttttatatt ctagaaatca aaagaactac ttggcctggt tccagcagaa 60
accaggacag cc 72

<210> 78
<211> 57
<212> DNA
<213> Artificial Sequence

<220>
<223> Description of Artificial Sequence:primer

<400> 78
gtcccttgtc cgaacgtgag cggatagcta aaatattgct gacagtcata aactgcc 57

<210> 79
<211> 34
<212> DNA
<213> Artificial Sequence

<220>
<223> Description of Artificial Sequence:primer

<400> 79
cccaaactcc tcatctattg ggctagcact aggg 34

<210> 80
<211> 34
<212> DNA
<213> Artificial Sequence

<220>
<223> Description of Artificial Sequence:primer

<400> 80
ccctagtgct agcccaatag atgaggagtt tggg 34

<210> 81
<211> 17
<212> DNA
<213> Artificial Sequence

<220>
<223> Description of Artificial Sequence:primer

<400> 81
tacgcaaacc gcctctc 17

<210> 82
<211> 18
<212> DNA
<213> Artificial Sequence

<220>
<223> Description of Artificial Sequence:primer

<400> 82
gagtgcacca tatgcggt 18

<210> 83
<211> 16
<212> DNA
<213> Artificial Sequence

<220>
<223> Description of Artificial Sequence:primer

<400> 83
aacagctatg accatg 16

<210> 84
<211> 17
<212> DNA
<213> Artificial Sequence

<220>
<223> Description of Artificial Sequence:primer

<400> 84
gttttcccag tcacgac 17

<210> 85
<211> 47
<212> DNA
<213> Artificial Sequence

<220>
<223> Description of Artificial Sequence:primer

<400> 85
gtgtattcag tgaaggtgta tctactagtt ttacagctga ctttcac 47

<210> 86
<211> 53
<212> DNA
<213> Artificial Sequence

<220>
<223> Description of Artificial Sequence:primer

<400> 86
tagtagatac accttcactg aatacaccat acactgggtt agacaggccc ctg 53

<210> 87
<211> 71
<212> DNA
<213> Artificial Sequence

<220>
<223> Description of Artificial Sequence:primer

<400> 87
cccttgaact tctggttgta gttaggaata ccattgttag gattaatacc tcctatccac 60
tccagccttt g 71

<210> 88
<211> 71
<212> DNA
<213> Artificial Sequence

<220>
<223> Description of Artificial Sequence:primer

<400> 88
taactacaac cagaagttca agggccgggc caccttgacc gtaggcaagt ctgccagcac 60

cgcctacatg g 71

<210> 89
<211> 63
<212> DNA
<213> Artificial Sequence

<220>
<223> Description of Artificial Sequence:primer

<400> 89
gcatggccct cgtcgtaacc ataggcgatt cttcttctgg cgcagtagta gactgcagtg 60
tcc 63

<210> 90
<211> 48
<212> DNA
<213> Artificial Sequence

<220>
<223> Description of Artificial Sequence:primer

<400> 90
ctatggttac gacgagggcc atgctatgga ctactggggt caaggaac 48

<210> 91
<211> 71
<212> DNA
<213> Artificial Sequence

<220>
<223> Description of Artificial Sequence:primer

<400> 91
taactacaac cagaagttca agggccgggt caccatcacc gtagacacct ctgccagcac 60
cgcctacatg g 71

<210> 92
<211> 27
<212> DNA
<213> Artificial Sequence

<220>
<223> Description of Artificial Sequence:primer

<400> 92
ggacactgca gtctacttct gcgccag 27

<210> 93
<211> 17
<212> DNA
<213> Artificial Sequence

<220>
<223> Description of Artificial Sequence:primer

<400> 93
tacgcaaacc gcctctc 17

<210> 94
<211> 18
<212> DNA
<213> Artificial Sequence

<220>
<223> Description of Artificial Sequence:primer

<400> 94
gagtgcacca tatgcggt 18

<210> 95
<211> 75
<212> DNA
<213> Artificial Sequence

<220>
<223> Description of Artificial Sequence:primer

<400> 95
cctttggcca ggggcctgtc taacccagtg tatggtgtat tcagtgaagg tgtatccact 60
agtttccact agttt 75

<210> 96
<211> 28
<212> DNA
<213> Artificial Sequence

<220>
<223> Description of Artificial Sequence:primer

<400> 96
gtcaccgtcc ttgacacgcg tctcggga 28

<210> 97
<211> 17
<212> DNA
<213> Artificial Sequence

<220>
<223> Description of Artificial Sequence:primer

<400> 97
ttggaggagg gtgccag 17

<210> 98
<211> 22
<212> DNA
<213> Artificial Sequence

<220>
<223> Description of Artificial Sequence:primer

<400> 98
gagacattgt gacccaatct cc 22

<210> 99
<211> 25

<212> DNA
<213> Artificial Sequence

<220>
<223> Description of Artificial Sequence:primer

<400> 99
gacagtcata aactgccaca tcttc 25

<210> 100
<211> 23
<212> DNA
<213> Artificial Sequence

<220>
<223> Description of Artificial Sequence:primer

<400> 100
ttgacacgcg tctcgggaag ctt 23

<210> 101
<211> 22
<212> DNA
<213> Artificial Sequence

<220>
<223> Description of Artificial Sequence:primer

<400> 101
ggcgcagagg atccactcac ct 22

## Description

### *Field of the invention*

The present invention relates to antibody proteins that specifically bind fibroblast activation protein alpha (FAP$\alpha$). The invention also relates to the use of said antibodies for diagnostic and therapeutic purposes and methods of producing said antibodies.

### *Background of the invention*

The invasive growth of epithelial cancers is associated with a number of characteristic cellular and molecular changes in the supporting stroma. A highly consistent molecular trait of the reactive stroma of many types of epithelial cancer is induction of the fibroblast activation protein alpha (from now on referred to as FAP), a cell surface molecule of reactive stromal fibroblasts originally identified with monoclonal antibody F19 (Garin-Chesa P., Old L. J. and Rettig W. J. (1990) Cell surface glycoprotein of reactive stromal fibroblasts as a potential antibody target in human epithelial cancers. *Proc. Natl. Acad. Sci.* **87:** 7235). Since the FAP antigen is selectively expressed in the stroma of a range of epithelial carcinomas, independent of location and histological type, an FAP-targeting concept has been developed for imaging, diagnosis and treatment of epithelial cancers and certain other conditions. For this purpose a monoclonal antibody termed F19 that specifically binds to FAP was developed and described in US Patents 5,059,523 and WO 93/05804.

One serious problem that arises when using non-human antibodies for applications *in vivo* in humans is that they quickly raise a human anti-non-human response that reduces the efficacy of the antibody in patients and impairs continued administration. Humanization of non-human antibodies is commonly achieved in one of two ways: (1) by constructing non-human/human chimeric antibodies, wherein the non-human variable regions are joined to human constant regions (Boulianne G. L., Hozumi N. and Shulman, M .J. (1984) Production of functional chimeric mouse/human antibody *Nature* **312**: 643) or (2) by grafting the complementarity determining regions (CDRs) from the non-human variable regions to human variable regions and then joining these "reshaped human" variable regions to human constant regions (Riechmann L., Clark M., Waldmann H. and Winter G. (1988) Reshaping human antibodies for therapy. *Nature* **332**: 323). Chimeric antibodies, although significantly better than mouse antibodies, can still elicit an anti-mouse response in humans (LoBuglio A. F., Wheeler R. H., Trang J., Haynes A., Rogers K., Harvey E. B., Sun L., Ghrayeb J. and Khazaeli M. B. (1989) Mouse/human chimeric monoclonal antibody in man: Kinetics and immune response. *Proc. Natl. Acad. Sci.* **86**: 4220). CDR-grafted or reshaped human antibodies contain little or no protein sequences that can be identified as being derived from mouse antibodies. Although an antibody humanised by CDR-grafting may still be able to elicit some immune reactions, such as an anti-allotype or an anti-idiotypic response, as seen even with natural human antibodies, the CDR-grafted antibody will be significantly less immunogenic than a mouse antibody thus enabling a more prolonged treatment of patients.

Another serious limitation relating to the commercial use of antibodies for diagnosis, imaging and therapy is their producibility in large amounts. In many instances recombinant expression of native, chimeric and/or CDR-grafted antibodies in cell culture systems is poor. Factors contributing to poor producibility may include the choice of leader sequences and the choice of host cells for production as well as improper folding and reduced secretion. Improper folding can lead to poor assembly of heavy and light chains or a transport incompetent conformation that forbids secretion of one or both chains. It is generally accepted that the L-chain confers the ability of secretion of the assembled protein. In some instances multiple or even single substitutions can result in the increased producibility of antibodies.

Because of the clinical importance of specific immunological targeting *in vitro* and *in vivo* of specific disease-related antigens for diagnosis and therapy in humans, there is a growing need for antibodies that combine the features of antigen specificity, low immunogenicity and high producibility.

Therefore, the problem underlying the present invention was to provide antibody proteins that combine the properties of specific binding to FAP, low immunogenicity in humans, and high producibility in recombinant systems.

### *Disclosure of the invention*

The technical problem is solved by the embodiments characterized in the claims.

The present invention provides new antibody proteins having the complementary determining regions of the monoclonal antibody F19 (ATCC Accession No. HB 8269), said new antibody proteins specifically binding to fibroblast activation protein (FAP), characterised in that they have framework modifications resulting in the improved producibility in host cells as compared to a chimeric antibody having the variable regions of F19 and foreign constant regions.

As used herein, an "antibody protein" is a protein with the antigen binding specificity of a monoclonal antibody.

"Complementarity determining regions of a monoclonal antibody" are understood to be those amino acid sequences involved in specific antigen binding according to Kabat (Kabat E. A., Wu T. T., Perry H. M., Gottesman K. S. and Foeller C. (1991) *Sequences of Proteins of Immunological Interest* (5th Ed.). NIH Publication No. 91-3242. U.S. Department of Health and Human Services, Public Health Service, National Institutes of Health, Bethesda, MD.) in connection with Chothia and Lesk (Chothia and Lesk (1987) *J. Mol. Biol.* 196:901-917).

As used herein, the term "framework modifications" refers to the exchange, deletion or addition of single or multiple amino acids in the variable regions surrounding the individual complementarity determining regions. Framework modifications may have an impact on the immunogenicity, producibility or binding specificity of an antibody protein.

"Fibroblast activation protein (FAP)", also designated fibroblast activation protein alpha (FAP$\alpha$), is a membrane-bound glycoprotein belonging to the serine protease gene family (WO 97/34927). No shed or secreted form of FAP is known. FAP can be characterized by its binding to the monoclonal antibody F19 (F19 is obtainable from the hybridoma cell line with the accession No. HB 8269 deposited at the ATCC).

The term ""fibroblast activation protein specific binding" of an antibody protein is defined herein by its ability to specifically recognise and bind FAP-expressing human cells. The binding specificity of the proteins of the invention can be determined by standard methods for the evaluation of binding specificity such as described in an exemplary fashion in example 6, 8 and example 12.

The term "chimeric antibody" refers to an antibody protein having the light and heavy chain variable regions as described in figures 17 and 18 and foreign constant regions. "Foreign constant regions" as defined herein are constant regions which are different from the constant regions of F19. For comparing an antibody protein of the invention to a chimeric antibody it is to be understood that such a chimeric antibody must contain the same constant regions as said antibody protein. For the purpose of demonstration and comparison alone the human constant heavy and light chains as described in Figures 19 to 22 are used in an exemplary fashion.

To provide the antibody proteins of the present invention, the nucleic acid sequences of the heavy and light chain genes of the murine antibody designated F19 were determined from RNA extracted from F19 hybridoma cells (ATCC Accession No. HB 8269).

In one embodiment the present invention relates to antibody proteins having the complementary determining regions of the monoclonal antibody F19 (ATCC Accession No. HB 8269), said new antibody proteins specifically binding to fibroblast activation protein (FAP), characterized in that they have framework modifications resulting in the improved producibility in host cells as compared to a chimeric antibody having the variable regions of F19 and foreign constant regions, wherein said antibody protein is derived from the murine antibody designated F19 (ATCC Accession No. HB 8269).

To generate humanised FAP-specific antibody proteins a chimeric antibody was constructed, having variable regions of the light and heavy chains of F19 and human light and heavy regions, respectively. The construction and production of chimeric mouse/human antibodies is well known (Boulianne et al. (1984), referenced above) and demonstrated in an exemplary fashion in examples 1 and 2.

The variable regions of the antibody proteins of the present invention are typically linked to at least a portion of the immunoglobulin constant region ($F_C$), typically that of a human immunoglobulin. Human constant region DNA sequences can be isolated in accordance with well-known procedures from a variety of human cells, but preferably immortalized B cells (see Kabat et al., *supra,* and WO 87/02671). Hence the antibody proteins of the invention may contain all or only a portion of the constant region as long as they exhibit specific binding to the FAP antigen. The choice of the type and extent of the constant region depends on whether effector functions like complement fixation or antibody dependent cellular toxicity are desired, and on the desired pharmacological properties of the antibody protein. The antibody protein of the invention will typically be a tetramer consisting of two light chain/heavy chain pairs, but may also be dimeric, i.e. consisting of a light chain/heavy chain pair, e.g. a Fab or Fv fragment.

Therefore, in a further embodiment the invention relates to antibody proteins according to the invention, characterised in that they have a variable light chain region and a variable heavy chain region, each joined to a human constant region. In particular, the variable region of the light chain was joined to a human kappa constant region and the variable region of the heavy chain was joined to a human gamma-1 constant region. Other human constant regions for humanising light and heavy chains are also available to the expert.

Therefore, in one particular embodiment the antibody proteins of the invention contain a human kappa constant region.

Also, in another particular embodiment the antibody proteins of the invention contain a human gamma-1 constant region.

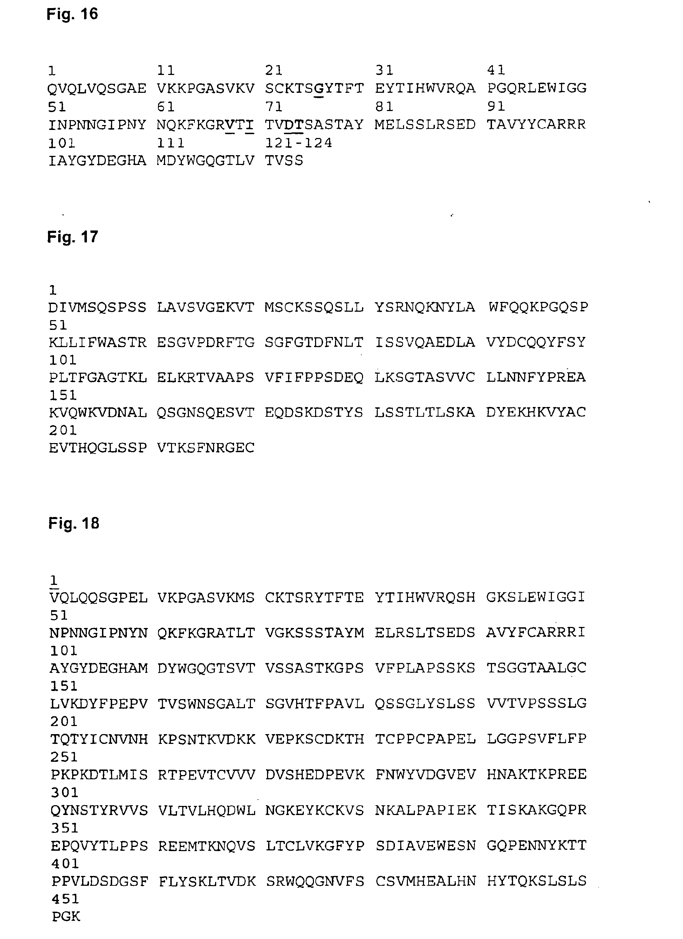

One particular "chimeric F19 antibody" protein (cF19) consists of the light and heavy chain variable and constant regions described in Figures 17 to 22. cF19 demonstrates specific binding and high avidity to the FAP antigen. As demonstrated in example 2, the expression of cF19 in COS cells (cells derived from the kidney of an African green monkey) is poor, ranging from about 10 to 60 ng/ml, which is at least 10 fold less than most antibodies.

In an attempt to increase expression levels of cF19, the leader sequence of the F19 $V_L$ region was changed by substitution of proline to leucine at position -9. This single change in amino acid in the leader sequence resulted in at least doubling the amount of chimeric antibody produced in COS cells. For the expression of this particular chimeric antibody in COS cells the following mutated leader sequence of the light chain: MDSQAQVLMLLLLWVSGTCG, and the following leader sequence of the heavy chain: MGWSWVFLFLLSGTAGVLS were used.

According to the invention the term "improved producibility" in host cells refers to the substantial improvement of expression levels and/or purified antibody yields when compared with the expression levels and/or antibody yields of a chimeric antibody without framework modifications as defined above. Two particular but not limiting examples for demonstrating improved producibility are exemplified for the COS cell expression system (in examples 2 and 5) and for the CHO cell expression system (in example 10 and 11).

While the mutation of the leader sequence only leads to a doubling of the expression yield of the chimeric F19 antibody, a substantial improvement as defined herein refers to an improvement in expression level and/or purification yield of at least a factor of 10.

In a preferred embodiment, the invention refers to antibody proteins, characterised in that their expression levels in crude media samples as determined by ELISA and/or purified antibody yields exceed the expression levels and/or purification yields of the chimeric antibodies without framework modifications by at least a factor of 10.

In more preferred embodiment, the invention refers to antibody proteins, characterised in that their expression levels in crude media samples as determined by ELISA and/or purified antibody yields exceed the expression levels and/or purification yields of the chimeric antibodies without framework modifications by at least a factor of 20.

In a most preferred embodiment, antibody proteins, characterised in that their expression levels in crude media samples as determined by ELISA and/or purified antibody yields exceed the expression levels and/or purification yields of the chimeric antibodies without framework modifications by at least a factor of 100.

Improved producibility of the recombinant antibody proteins of the invention can be demonstrated for eukaryotic cells in general as shown for COS and CHO (Chinese hamster ovary derived cells) eukaryotic cells (see examples 5 and 11). In a further embodiment, the present invention relates to recombinant antibody proteins characterised in that they display improved producibility in eukaryotic cells.

In a preferred embodiment the present invention relates to antibody proteins, wherein said eukaryotic cell is a Chinese hamster ovary cell (CHO cell).

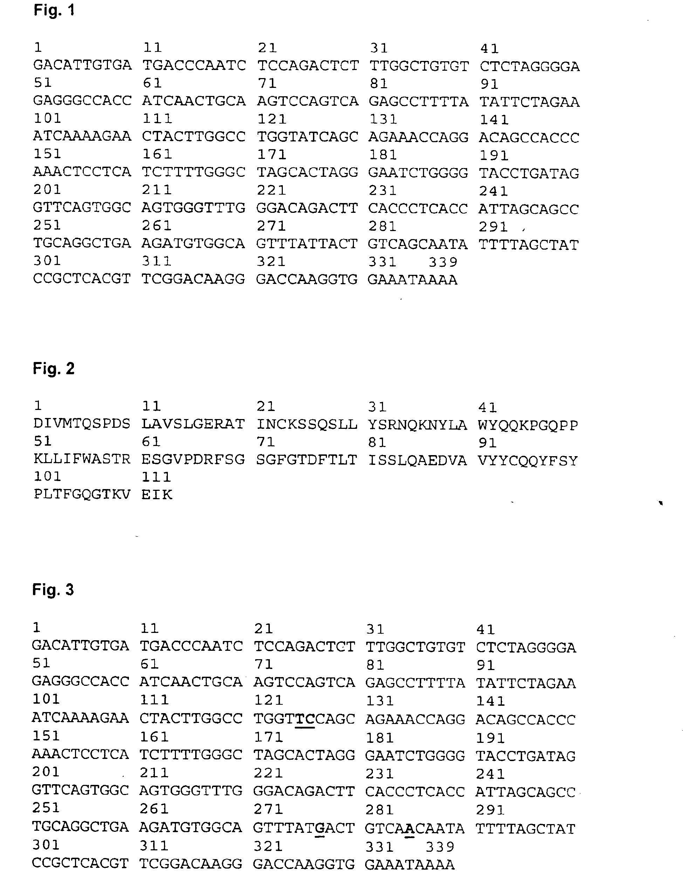

It was unexpectedly found that certain framework modifications of the light chain variable regions determine the improved producibility of the antibody proteins of the invention. Three versions of reshaped light chain variable regions, designated version A, B, and C, as described in Figures 1 to 6, were prepared.

Light chain variable region versions A, B, and C demonstrate substantially improved producibility in CHO cells (see example 11). While light chain variable region versions A and C differ from light chain variable region version B by only two common amino acid residues they display an even further substantial improvement in producibility. There is at least another 10 fold difference in antibody secretion levels between the human reshaped F19 light chain version B and versions A or C. Reshaped human F19 light chain version A and B only differ in their amino acid sequences by two residues at positions 36 (Tyr to Phe mutation) and 87 (Tyr to Asp mutation) (nomenclature according to Kabat). This negative effect on the secretory capability of antibodies containing the light chain variable region version B could have been indirect if the Tyr to Asp and Tyr to Phe mutations, considered individually or together, merely caused improper folding of the protein. But this is unlikely to be the case since antigen binding assays show that immunoglobulins containing F19 light chain version B have similar avidities to those paired with F19 light chain version A or C, suggesting that they were not grossly misfolded.

Residue 87 in reshaped human F19 light chain version B seems particularly responsible for the reduction of secretion when compared to versions A and C.

In a preferred embodiment, the present invention relates to antibody proteins according to the invention, wherein the amino acid in Kabat position 87 of the light chain region is not asparagine.

In a more preferred embodiment, the invention relates to antibody proteins according to the invention, wherein the amino acid in Kabat position 87 of the light chain region is selected from aromatic or aliphatic amino acids.

In a most preferred embodiment, the present invention relates to antibody proteins according to the invention, wherein the aromatic amino acid in Kabat position 87 of the light chain region is a tyrosine or phenylalanine.

In a further embodiment, the present invention also pertains to antibody proteins according to the invention, wherein the amino acid in Kabat position 36 of the light chain region is selected from aromatic amino acids.

In a particular embodiment the invention relates to the specific antibody proteins that may be prepared from the individually disclosed reshaped variable regions of the light and heavy chains.

Especially light chain variable region versions A and C are particularly suitable to practice the invention because of their exceptionally high producibility, while retaining full FAP-binding specificity and achieving low immunogenicity. This holds especially true when compared to the chimeric antibody having the variable regions of F19 and the same constant regions but also when compared to light chain version B.

Therefore, in one embodiment the present invention relates to antibody proteins that contain the variable region of the light chain as set forth in SEQ ID NO: 2.

In a further embodiment the invention also relates to antibody proteins, characterised in that the variable region of the light chain is encoded by a nucleotide sequence as set forth in SEQ ID NO: 1.

In one embodiment the present invention relates to antibody proteins that contain the variable region of the light chain as set forth in SEQ ID NO: 6.

In a further embodiment the invention also relates to antibody proteins characterised in that the variable region of the light chain is encoded by a nucleotide sequence as set forth in SEQ ID NO: 5.

The present invention also discloses several different variable regions of the heavy chain that work particularly well with the variable regions of the light chain versions A and C in terms of improved producibility.

In one embodiment the invention relates to antibody proteins containing a variable region of the heavy chain as set forth in any one of SEQ ID NOs: 8, 10, 12, 14.

In another embodiment the invention relates to antibody proteins characterised in that the variable region of the heavy chain is encoded by a nucleotide sequence as set forth in any one of SEQ ID NOs: 7, 9, 11, 13.

In a very particular embodiment the invention relates to antibody proteins containing the variable region of the light chain as set forth in SEQ ID NO: 2 and the variable region of the heavy chain as set forth in SEQ ID NO: 12. Most preferably, this antibody protein additionally contains the constant region of the light chain as set forth in SEQ ID NO: 20 and the constant region of the heavy chain as set forth in SEQ ID NO: 22.

Thus a further aspect of the present invention is an antibody protein containing an amino acid sequence as set forth in SEQ ID NO: 2. More preferably, such an antibody protein further contains an amino acid sequence as set forth in SEQ ID NO: 12. More preferably, said antibody protein further contains an amino acid sequence as set forth in SEQ ID NO: 20 and an amino acid sequence as set forth in SEQ ID NO: 22. A further aspect of the invention is an antibody protein as described in this paragraph which is conjugated to a radioisotope, preferably $^{131}I$, $^{125}I$, $^{186}Re$, $^{188}Re$, or $^{90}Y$. An additional aspect of the present invention is a DNA molecule coding for an antibody protein as described in this paragraph. A further aspect of the invention is a host cell carrying such a DNA molecule. Accordingly, a further aspect of the invention is a method of producing an antibody protein as described in this paragraph, said method comprising the steps of cultivating such a host cell under conditions where said antibody protein is expressed by said host cell, and isolating said protein. A further aspect of the invention is a pharmaceutical composition comprising an antibody protein of the present invention and a pharmaceutically acceptable carrier. In a further particular embodiment the invention relates to antibody proteins characterised in that the variable region of the light chain is encoded by a nucleotide sequence as set forth in SEQ ID NO: 1 and the variable region of the heavy chain is encoded by a nucleotide sequence as set forth in SEQ ID NO:11.

In a further particular embodiment the invention relates to antibody proteins containing the variable region of the light chain as set forth in SEQ ID NO: 2 and the variable region of the heavy chain as set forth in SEQ ID NO: 8.

In a further particular embodiment the invention relates to antibody proteins characterised in that the variable region of the light chain is encoded by a nucleotide sequence as set forth in SEQ ID NO: 1 and the variable region of the heavy chain is encoded by a nucleotide sequence as set forth in SEQ ID NO: 7.

Humanization of the variable region of a murine antibody may be achieved employing methods known in the art. EP 0239400 discloses grafting of the CDRs of a murine variable region into the framework of a human variable region. WO 90/07861 discloses methods of reshaping a CDR-grafted variable region by introducing additional framework modifications. WO 92/11018 discloses methods of producing humanized Ig combining donor CDRs with an acceptor framework that has a high homology to the donor framework. WO 92/05274 discloses the preparation of framework mutated antibodies starting from a murine antibody. Further prior art references related to humanization of murine monoclonal antibodies are EP 0368684; EP 0438310; WO 92/07075, or WO 92/22653. Thus, the expert can produce the antibodies of the present invention starting from the publicly available murine monoclonal antibody F19 and employing techniques known in the art, e.g. from WO 92/05274. DNA molecules coding for the antibody proteins of the present invention may of course also be obtained by state-of-the-art synthetic procedures, e.g. by chemical synthesis of appropriate oligonucleotides and subsequent ligation and amplification procedures (see e.g. Frank et al. (1987) *Methods Enzymol.* **154:** 221-249).

In a further aspect, the present invention relates to nucleic acid molecules containing the coding information for the antibody proteins according to the invention as disclosed above. Preferably, a nucleic acid molecule according to the present invention is a nucleic acid molecule containing a nucleotide sequence selected from SEQ ID NOs: 1, 3, 5, 7, 9, 11, 13, or 15.

A further aspect of the present invention is a recombinant DNA vector containing the nucleotide sequence of any one of the above-mentioned nucleic acids, especially when said nucleotide sequence is operationally linked to an expression control sequence as in expression vectors. Preferred is a recombinant DNA vector, said vector being an expression vector.

A further aspect of the present invention is a host cell carrying a vector as described, especially an expression vector. Such a host cell can be a prokaryotic or eukaryotic cell. Preferably, such a host cell is an eukaryotic cell, a yeast cell, or a mammalian cell. More preferably, said host cell is a CHO (Chinese hamster ovary) cell or a COS cell.

Accordingly, a still further aspect of the present invention is a method of producing antibody proteins according to the invention. Such a method comprises the steps of:

(a) cultivating a host cell as described above under conditions where said antibody protein is expressed by said host cell, and
(b) isolating said antibody protein.

Mammalian host cells, preferably CHO or COS cells are preferred. Host cells for producing the antibody proteins of the invention may be transfected with a single vector containing the expression units for both, the light and the heavy chain (see e.g. WO 94/11523). In one particular embodiment the method of producing antibody proteins according to the invention pertains to host cells, wherein said host cells are cotransfected with two plasmids carrying the expression units for the light and heavy chains respectively (See e.g. EP 0481790).

The antibody proteins of the invention provide a highly specific tool for targeting therapeutic agents to the FAP antigen. Therefore, in a further aspect, the invention relates to antibody proteins according to the invention, wherein said antibody protein is conjugated to a therapeutic agent. Of the many therapeutic agents known in the art, therapeutic agents selected from the group consisting of radioisotopes, toxins, toxoids, inflammatogenic agents, enzymes, antisense molecules, peptides, cytokines, and chemotherapeutic agents are preferred. Among the radioisotopes, gamma, beta and alpha-emitting radioisotopes may be used as a therapeutic agent. β-emitting radioisotopes are preferred as therapeutic radioisotopes. $^{186}$ Rhenium, $^{188}$Rhenium, $^{131}$Iodine and $^{90}$Yttrium have been proven to be particularly useful β-emitting isotopes to achieve localized irradiation and destruction of malignant tumor cells. Therefore, radioisotopes selected from the group consisting of $^{186}$Rhenium, $^{188}$Rhenium, $^{131}$ Iodine and $^{90}$Ytirium are particularly preferred as therapeutic agents conjugated to the antibody proteins of the invention. For example, for the radioiodination of an antibody of the invention, a method as disclosed in WO 93/05804 may be employed.

A further aspect of the present invention pertains to antibody proteins according to the invention, characterised in that they are labelled. Such an FAP-specific labelled antibody allows for the localisation and/or detection of the FAP antigen *in vitro* and/or *in vivo.* A label is defined as a marker that may be directly or indirectly detectable. An indirect marker is defined as a marker that cannot be detected by itself but needs a further directly detectable marker specific for the indirect marker. Preferred labels for practicing the invention are detectable markers. From the large variety of detectable markers, a detectable marker selected from the group consisting of enzymes, dyes, radioisotopes, digoxygenin, and biotin is most preferred.

A further aspect of the present invention relates to antibody proteins according to the invention, characterised in that they are conjugated to an imageable agent. A large variety of imageable agents, especially radioisotopes, are available from the state of the art. For practising the invention gamma-emitting isotopes are more preferred. Most preferred is $^{125}$Iodine.

One aspect of the present invention relates to pharmaceutical compositions containing an antibody protein according to the present invention as described above and a pharmaceutically acceptable carrier. Such pharmaceutical compositions are useful for treating tumors, wherein said tumors are associated with activated stromal fibroblasts. There are two possible effector principles for an antitumor stroma immunotherapy that may act synergistically: (a) An unmodified (unconjugated, 'naked') antibody according to the invention may induce immune destruction or inflammatory reactions in the tumor stroma while (b) an antibody conjugated to a therapeutic agent, such as for example, a radioisotope or other toxic substance, may achieve localized irradiation and destruction of the malignant tumor cells. Accordingly, a further aspect of the present invention is the use of an antibody protein as described for the manufacture of a pharmaceutical composition, especially for the treatment of tumors.

One further embodiment are pharmaceutical compositions containing an antibody protein according to the invention conjugated to a therapeutic agent as described above and a pharmaceutically acceptable carrier useful for treating tumors, wherein said tumors are associated with activated stromal fibroblasts. Another embodiment pertains to pharmaceutical compositions containing an antibody protein according to the present invention conjugated to an imageable agent as described above and a pharmaceutically acceptable carrier useful for imaging the presence of activated stromal fibroblasts in a healing wound, inflamed skin or a tumor, in a human patient. A most preferred embodiment relates to the pharmaceutical compositions mentioned above, wherein said tumors are tumors selected from the cancer group consisting of colorectal cancers, non-small cell lung cancers, breast cancers, head and neck cancer, ovarian cancers, lung cancers, invasive bladder cancers, pancreatic cancers and cancers metastatic of the brain.

In an animal or human body, it can prove advantageous to apply the pharmaceutical compositions as described above via an intravenous or other route, e.g. systemically, locally or topically to the tissue or organ of interest, depending on the type and origin of the disease or problem treated, e.g. a tumor. For example, a systemic mode of action is desired when different organs or organ systems are in need of treatment as in e.g. systemic autoimmune diseases, or allergies, or transplantations of foreign organs or tissues, or tumors that are diffuse or difficult to localise. A local mode of action would be considered when only local manifestations of neoplastic or immunologic action are expected, such as, for example local tumors.

The antibody proteins of the present invention may be applied by different routes of application known to the expert, notably intravenous injection or direct injection into target tissues. For systemic application, the intravenous, intravascular, intramuscular, intraarterial, intraperitoneal, oral, or intrathecal routes are preferred. A more local application can be effected subcutaneously, intracutaneously, intracardially, intralobally, intramedullarly, intrapulmonarily or directly in or near the tissue to be treated (connective-, bone-, muscle-, nerve-, epithelial tissue). Depending on the desired duration and effectiveness of the treatment, pharmaceutical antibody compositions may be administered once or several times, also intermittently, for instance on a daily basis for several days, weeks or months and in different dosages.

For preparing suitable antibody preparations for the applications described above, the expert may use known injectable, physiologically acceptable sterile solutions. For preparing a ready-to-use solution for parenteral injection or infusion, aqueous isotonic solutions, such as e.g. saline or corresponding plasma protein solutions are readily available. The pharmaceutical compositions may be present as lyophylisates or dry preparations, which can be reconstituted with a known injectable solution directly before use under sterile conditions, e.g. as a kit of parts. The final preparation of the antibody compositions of the present invention are prepared for injection, infusion or perfusion by mixing purified antibodies according to the invention with a sterile physiologically acceptable solution, that may be supplemented with known carrier substances or/and additives (e.g. serum albumin, dextrose, sodium bisulfite, EDTA).

The amount of the antibody applied depends on the nature of the disease. In cancer patients, the applied dose of a 'naked' antibody may be between 0.1 and 100 mg/m$^2$, preferably between 5 and 50 mg/m$^2$ per application. For radiolabeled antibodies, e.g. with iodine-131, the maximally tolerated dose (MTD) has to be determined which must not be exceeded in therapeutic settings. Application of radiolabeled antibody to cancer patients may then be carried out by repeated (monthly or weekly) intravenous infusion of a dose which is below the MTD (See e.g. Welt et al. (1994) *J. Clin. Oncol.* **12:** 1193-1203).

Furthermore, one aspect of the present invention relates to the use of the antibody proteins according to the invention for the treatment of cancer. In a preferred embodiment the present invention relates to the use of antibody proteins according to the invention conjugated to a therapeutic agent as described above for the treatment of cancer. In another preferred embodiment the present invention relates to the use of antibody proteins according to the invention conjugated to an imageable agent for imaging activated stromal fibroblasts. In a further preferred embodiment the present invention relates to the use of labelled antibody proteins according to the invention for detecting the presence of activated stromal fibroblasts in a sample.

One aspect of the invention relates to a method of treating tumors, wherein the tumor is associated with activated stromal fibroblasts capable of specifically forming a complex with antibody proteins according to the invention, present as naked/unmodified antibodies, modified antibody proteins, such as e.g. fusion proteins, or antibody proteins conjugated to a therapeutic agent, which comprises contacting the tumor with an effective amount of said antibodies. In a preferred embodiment the present invention relates to a method of treating tumors as mentioned above, wherein the tumor is a tumor having cancer cells selected from the cancer group consisting of colorectal cancers, non-small cell lung cancers, breast cancers, head and neck cancer, ovarian cancers, lung cancers, invasive bladder cancers, pancreatic cancers and metastatic cancers of the brain. The method of treating tumors as described above may be effected *in vitro or in vivo.*

A further aspect of the invention relates to a method of detecting the presence of activated stromal fibroblasts in wound healing, inflammation or in tumors, characterised in that

(a) a sample, possibly containing activated stromal fibroblasts, is contacted with an antibody protein according to the invention under conditions suitable for the formation of a complex between said antibody and antigen,
(b) detecting the presence of said complex, thereby detecting the presence of activated stromal fibroblasts in wound healing, inflammation or a tumor.

In a preferred embodiment, the present invention relates to a method of detecting the presence of activated stromal fibroblasts in a tumor, wherein the tumor is a tumor having cancer cells selected from the cancer group consisting of colorectal cancers, non-small cell lung cancers, breast cancers, head and neck cancer, ovarian cancers, lung cancers, bladder cancers, pancreatic cancers and metastatic cancers of the brain. Most preferred antibody proteins of the invention are those which are characterised in that they are labelled as mentioned above.

A further aspect of the invention relates to a method of imaging the presence of activated stromal fibroblasts in a healing wound, inflamed tissue (rheumatoid arthritis and cirrhosis are also positive) or a tumor, in a human patient, characterised in that

(a) an antibody protein according to the present invention conjugated to an imageable agent is administered to a human patient under conditions suitable for the formation of an antibody-antigen complex,
(b) imaging any complex formed in this manner,
(c) thereby imaging the presence of activated stromal fibroblasts in a human patient.

In a preferred embodiment the present invention relates to a method of imaging the presence of activated stromal fibroblasts as described above in tumors, wherein the tumor is a tumor having cancer cells selected from the cancer group consisting of colorectal cancers, non-small cell lung cancers, breast cancers, head and neck cancer, ovarian cancers, lung cancers, bladder cancers, pancreatic cancers and metastatic cancers of the brain.

In a further aspect the present invention relates to a method of detecting tumor-stroma, characterised in that

(a) a suitable sample is contacted with an antibody protein according to the present invention, under conditions suitable for the formation of an antibody-antigen complex,
(b) detecting the presence of any complex so formed,
(c) relating the presence of said complex to the presence of tumor-stroma.

Antibody proteins for practicing the invention are preferably labelled with a detectable marker.

In a further aspect the present invention relates to a method of imaging tumor-stroma in a human patient, which comprises

(a) administering to the patient an antibody according to the invention conjugated to an imageable agent as described above under conditions suitable for the formation of an antibody-antigen complex,
(b) imaging any complex so formed, and thereby imaging the presence of tumor-stroma in a human patient.

***Figure legends***

***Fig. 1.*** *DNA sequence of F19 human reshaped light chain variable region version A (hF19$L_A$) SEQ ID N0:1.*

***Fig. 2.*** *Amino acid sequence of F19 human reshaped light chain variable region version A (hF19$L_A$) SEQ ID NO: 2.*

***Fig. 3.*** *DNA sequence of F19 human reshaped light chain variable region version B (hF19$L_B$) SEQ ID NO:* 3. Nucleotides differing from version A are underlined and in bold type.

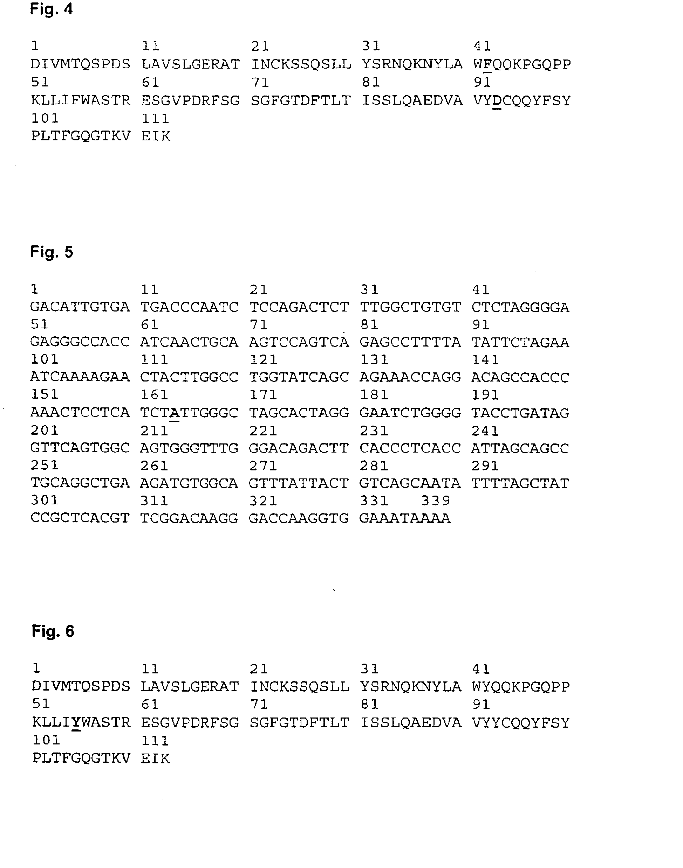

***Fig. 4.*** *Amino acid sequence of F19 human reshaped light chain variable region version B (hF19$L_B$) SEQ ID NO:* 4. Amino acids differing from version A are underlined and in bold type.

***Fig. 5.*** *DNA sequence of F19 human reshaped light chain variable region version* C *(hF19$L_C$) SEQ ID NO:5.* Nucleotides differing from version A are underlined and in bold type.

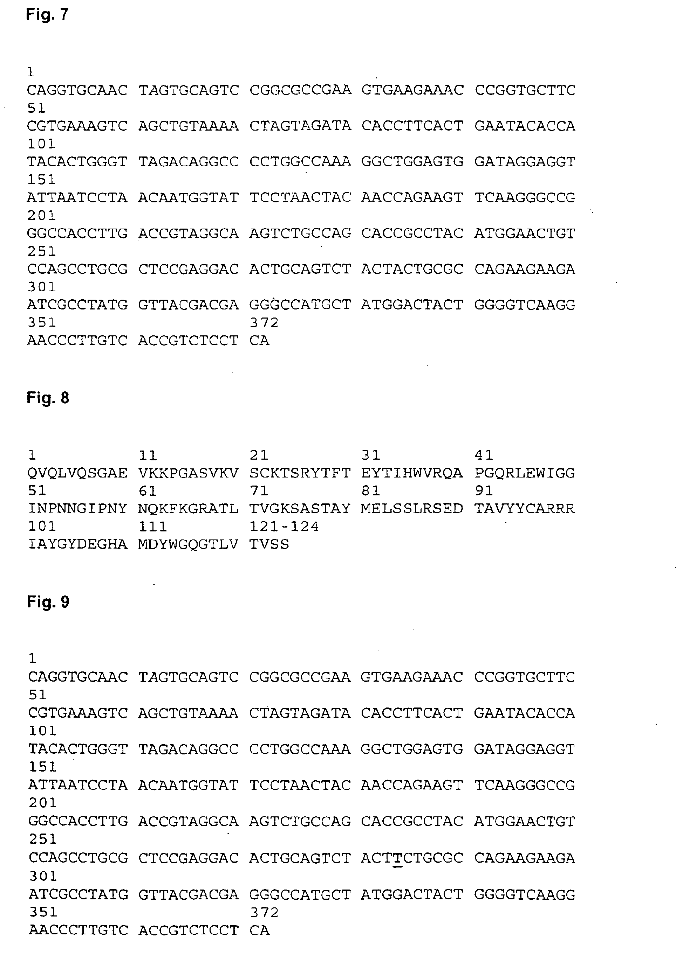

***Fig. 6.*** *Amino acid sequence of F19 human reshaped light chain variable region version C (hF19$L_C$) SEQ ID NO:* 6. Amino acids differing from version A are underlined and in bold type.

***Fig. 7.*** *DNA sequence of F19 human reshaped variable region heavy chain version A (hF19$H_A$) SEQ ID NO: 7.*

***Fig. 8.*** *Amino acid sequence of F19 human reshaped heavy chain variable region version A (hF19$H_A$) SEQ ID NO: 8*

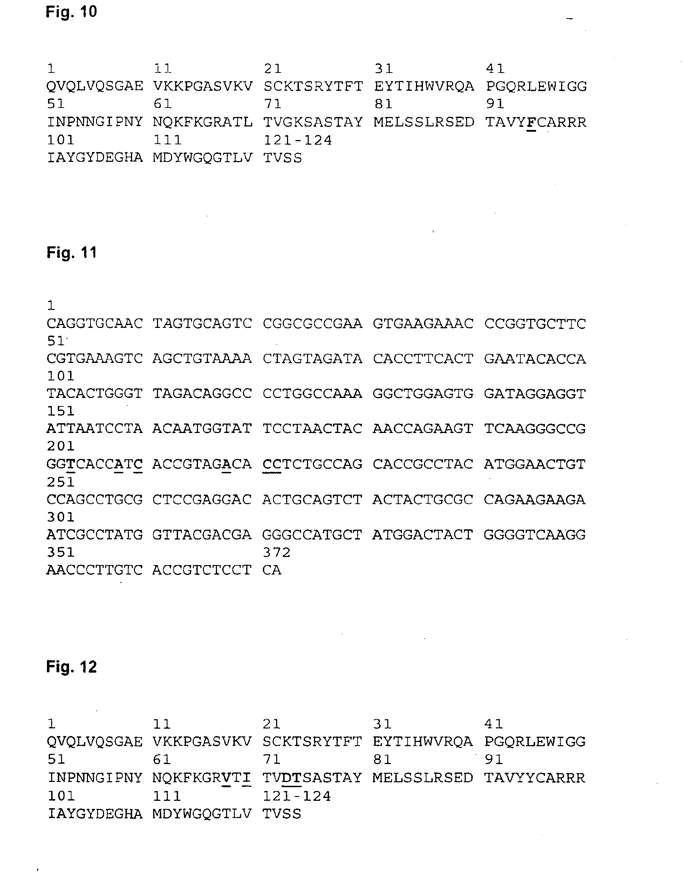

***Fig. 9.*** *DNA sequence of F19 human reshaped heavy chain variable region version B (hF19$H_B$) SEQ ID NO:* 9. Nucleotides differing from version A are underlined and in bold type.

***Fig. 10.*** *Amino acid sequence of F19 human reshaped heavy chain variable region version B (hF19$H_B$) SEQ ID NO: 10.* Amino acids differing from version A are underlined and in bold type.

***Fig. 11.*** *DNA sequence of F19 human reshaped heavy chain variable region version C (hF19$H_C$) SEQ ID NO:* 11. Nucleotides differing from version A are underlined and in bold type.

***Fig. 12.*** *Amino acid sequence of F19 human reshaped heavy chain variable region version C (hF19$H_C$) SEQ ID NO:* 12. Amino acids differing from version A are underlined and in bold type.

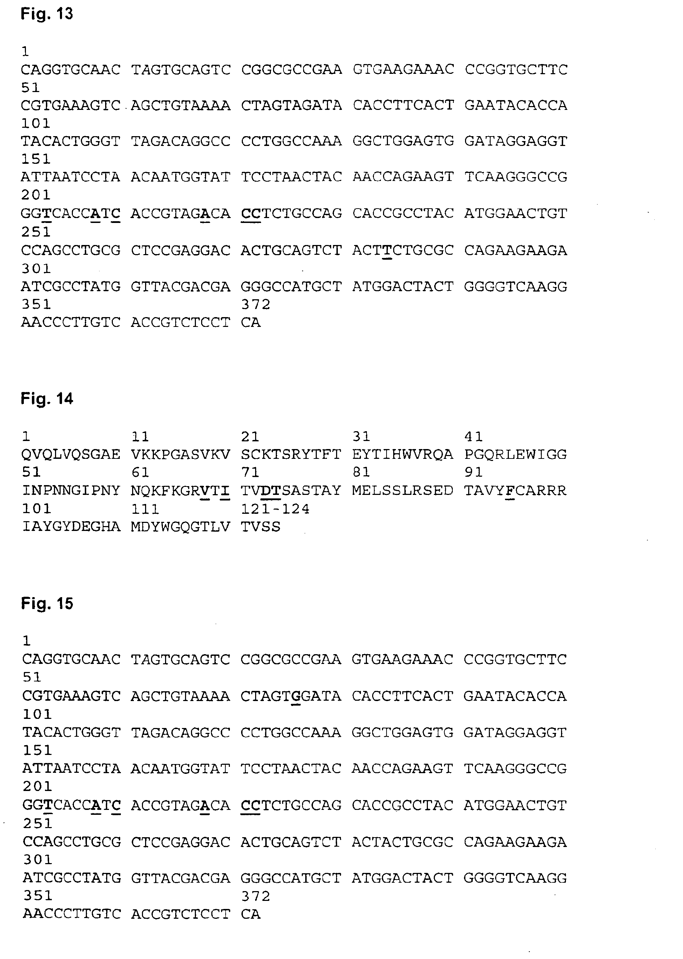

***Fig. 13.*** *DNA sequence of F19 human reshaped heavy chain variable region version D (hF19$H_D$) SEQ ID NO:* 13. Nucleotides differing from version A are underlined and in bold type.

**Fig.** ***14.*** *Amino acid sequence of F19 human reshaped heavy chain variable region version D (hFl9$H_D$) SEQ ID NO: 14.* Amino acids differing from version A are underlined and in bold type.

**Fig.** ***15.*** *DNA sequence of F19 human reshaped heavy chain variable region version E (hF19$H_E$) SEQ ID NO:* 15. Nucleotides differing from version A are underlined and in bold type.

**Fig.** ***16.*** *Amino acid sequence of F19 human reshaped heavy chain variable region version E (hF19$H_E$) SEQ ID NO: 16.* Amino acids differing from version A are underlined and in bold type

**Fig.** ***17.*** *Amino acid sequence of F19 chimeric light chain variable region (chF19LC) SEQ ID NO: 17.*

**Fig.** ***18.*** *Amino acid sequence of F19 chimeric heavy chain variable region (chF19HC) SEQ ID NO: 18.*

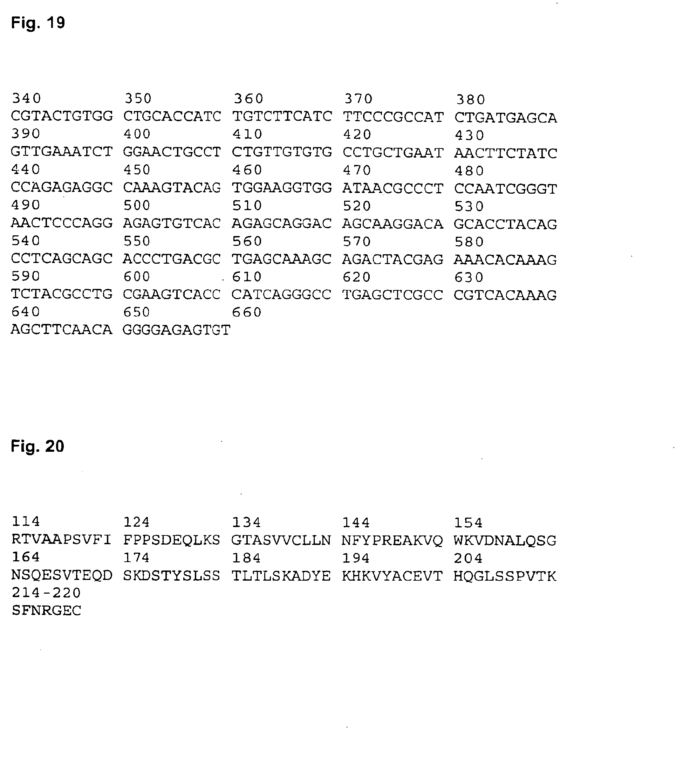

**Fig.** ***19.*** *DNA sequence of human kappa light constant chain SEQ ID NO: 19.*

**Fig.** ***20.*** *Amino acid sequence of human light constant chain SEQ ID NO: 20*.

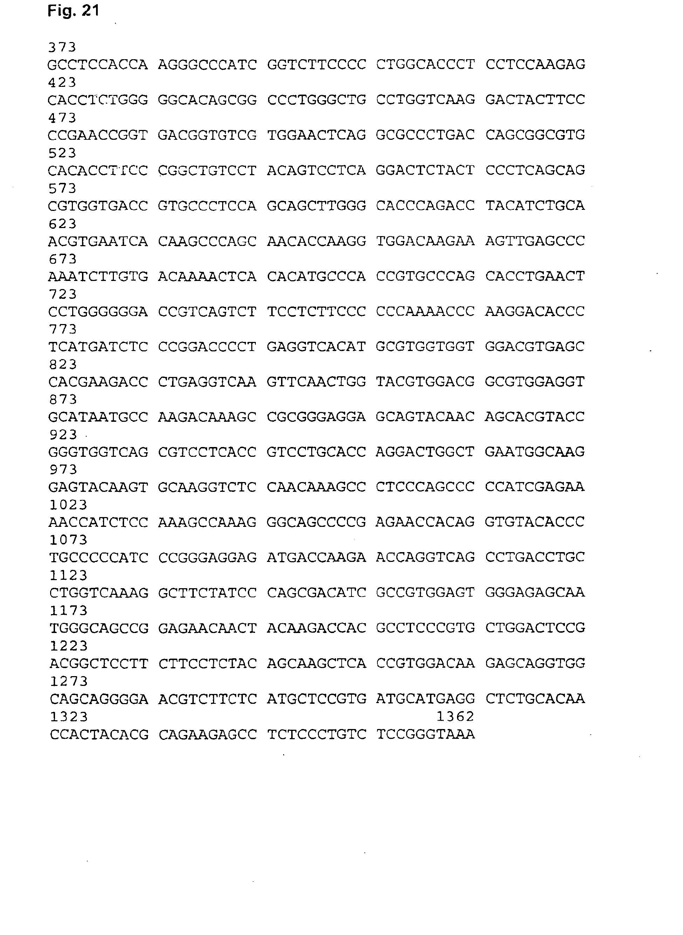

**Fig.** ***21.*** *DNA sequence of human heavy constant chain SEQ ID NO: 21.*

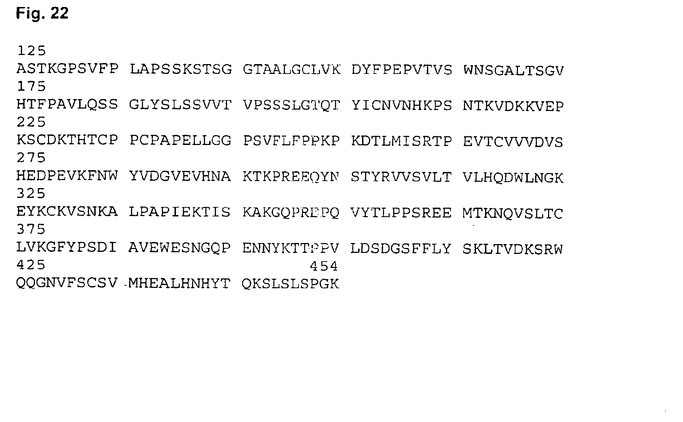

**Fig.** ***22.*** *Amino acid sequence of human heavy constant chain SEQ ID NO: 22.*

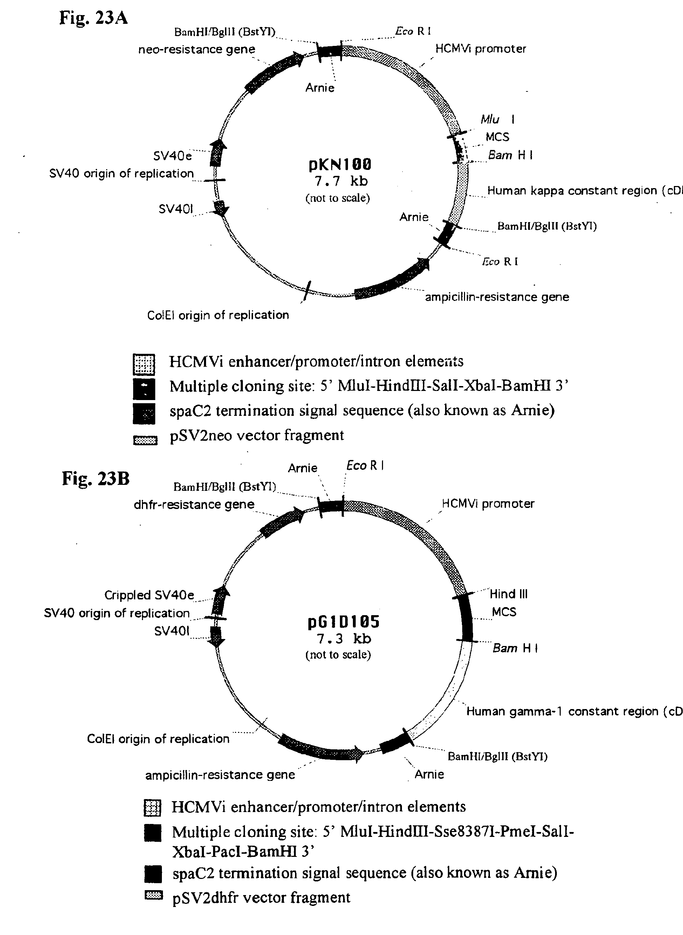

***Fig. 23.*** *Mammalian cell expression vectors used to produce chimeric and reshaped human antibodies with human kappa light chains and human gamma-1 heavy chains.*

A. Light chain expression vector: pKN 100
B. Heavy chain expression vector: pG1D105

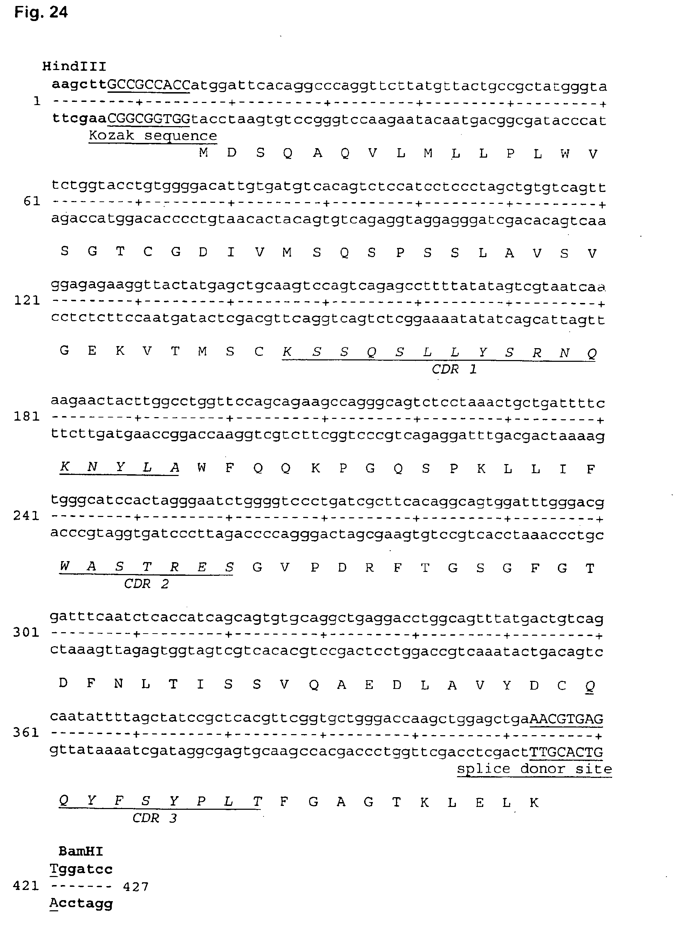

***Fig 24.*** *DNA and amino acid sequences of mouse F19 light chain variable region* as *modified for use in the construction of chimeric F19 light chain.* Restriction sites are indicated by bold letters. The Kozak sequence, CDRs 1 to 3 and the splice donor site are underlined.

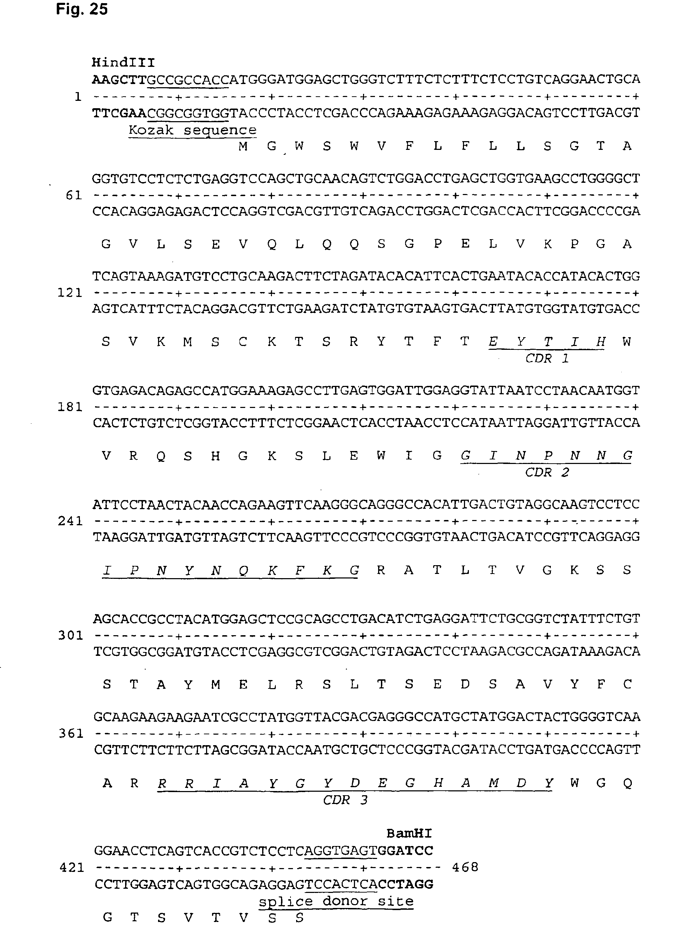

***Fig 25.*** *DNA and amino acid sequences of mouse F19 heavy chain variable region as modified for use in the construction of chimeric F19 heavy chain.* Restriction sites are indicated by bold letters. The Kozak sequence and the splice donor site are underlined.

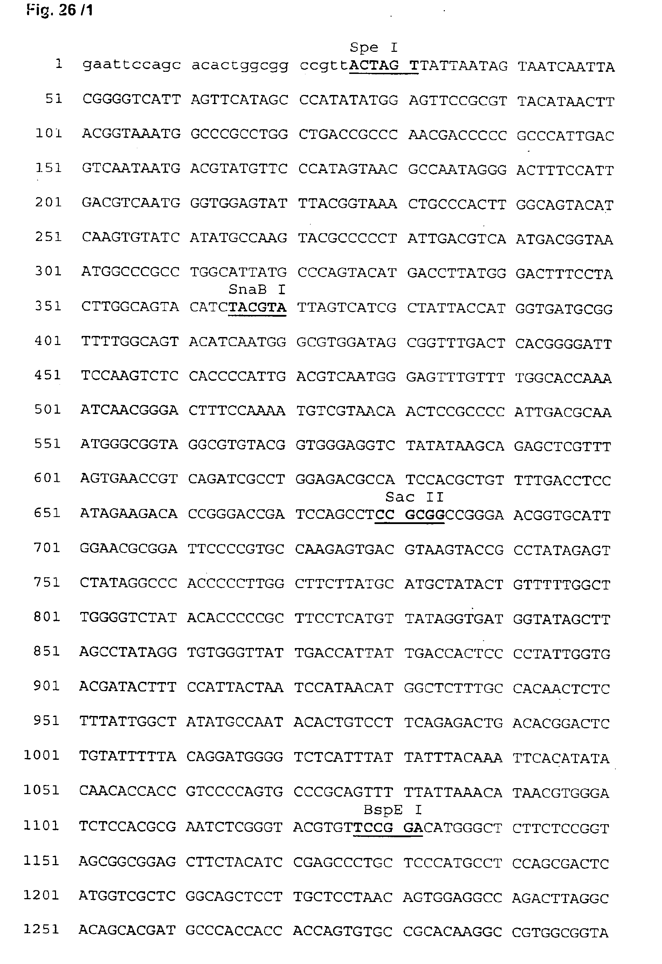

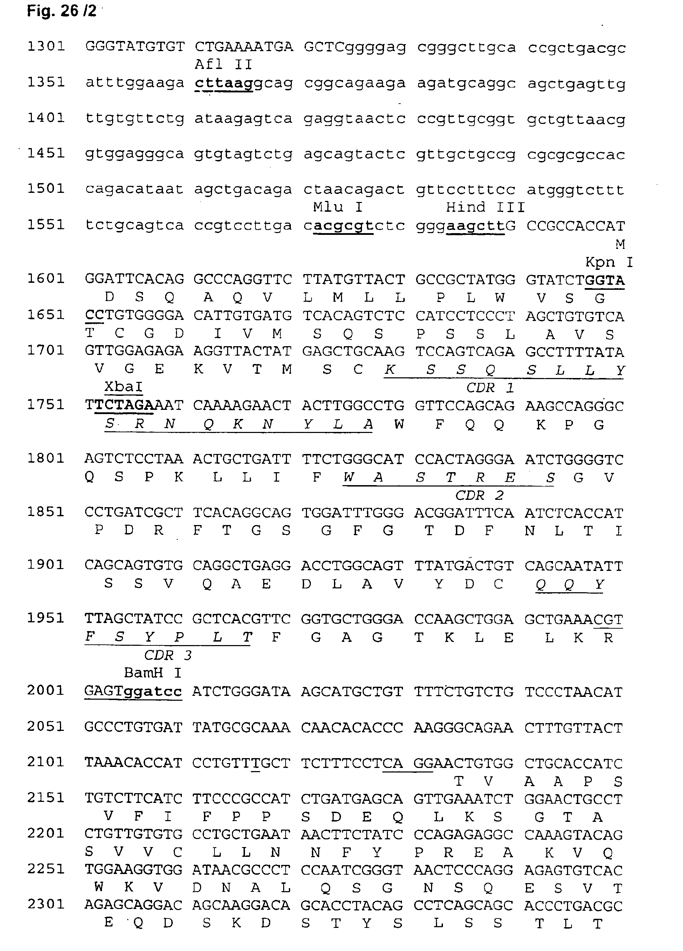

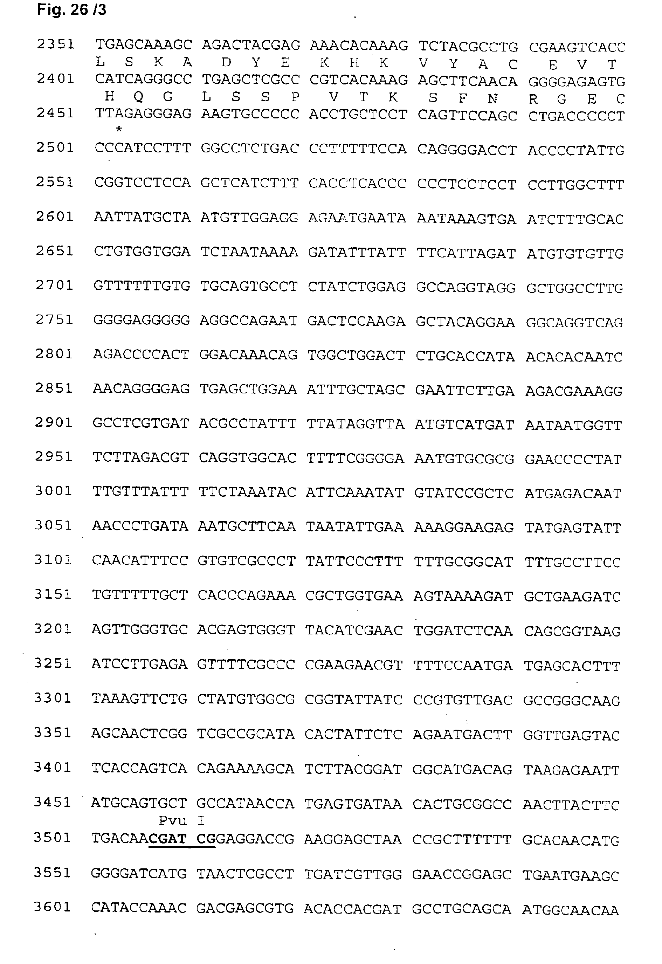

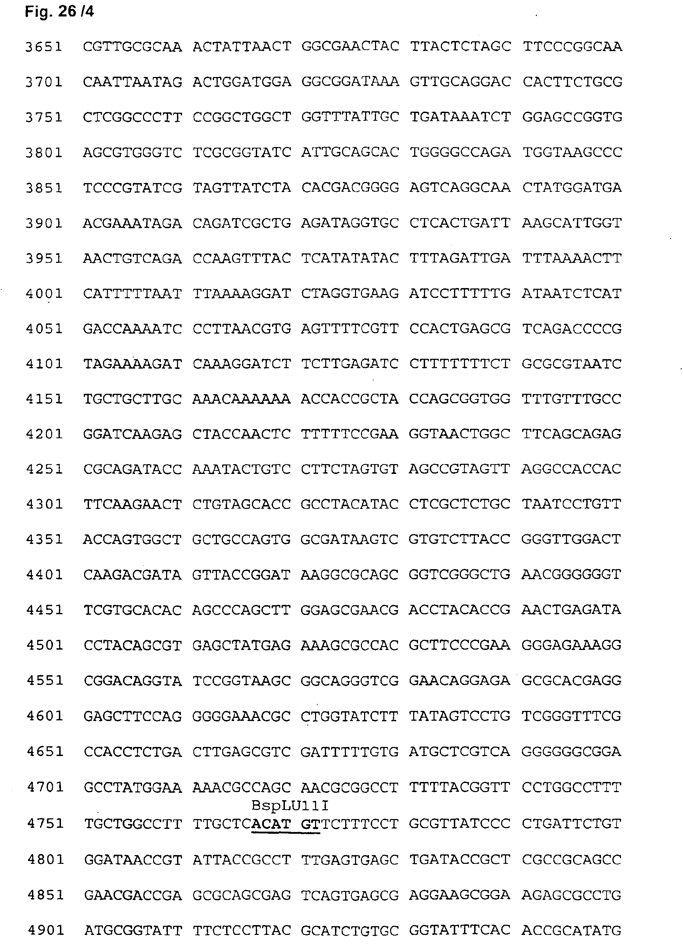

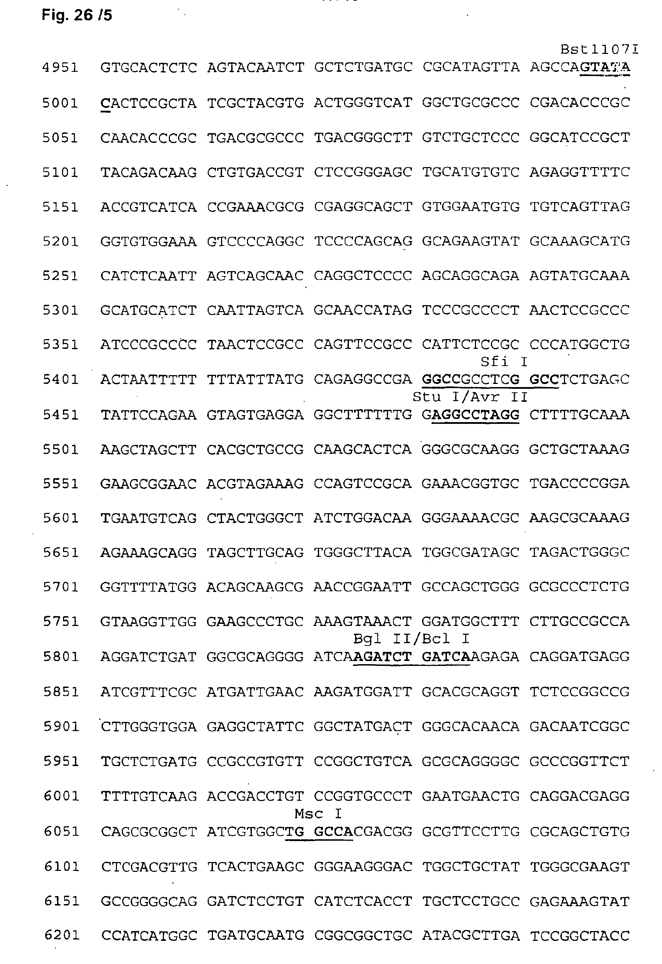

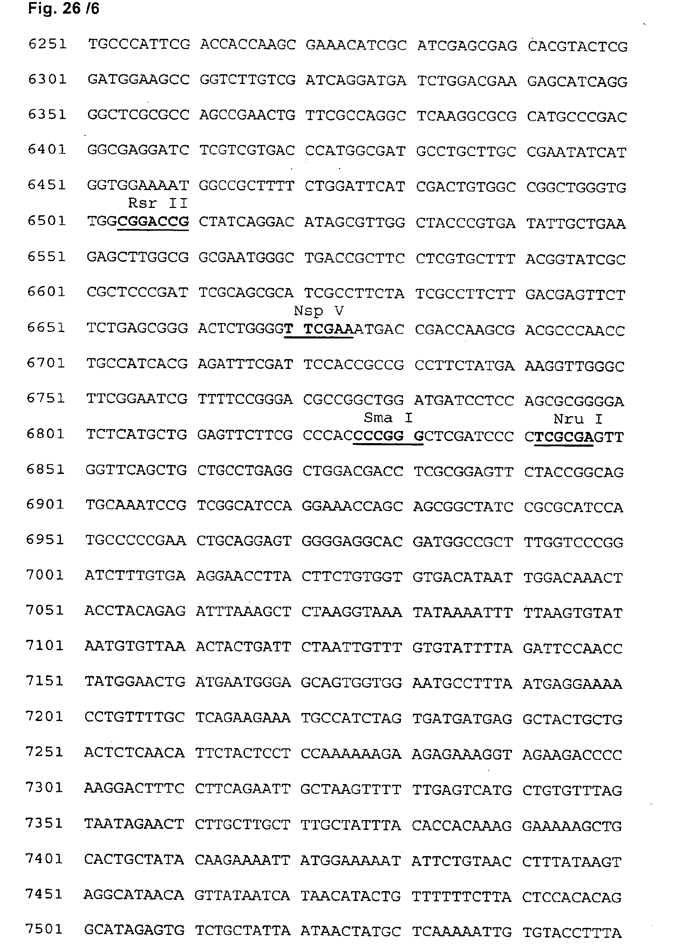

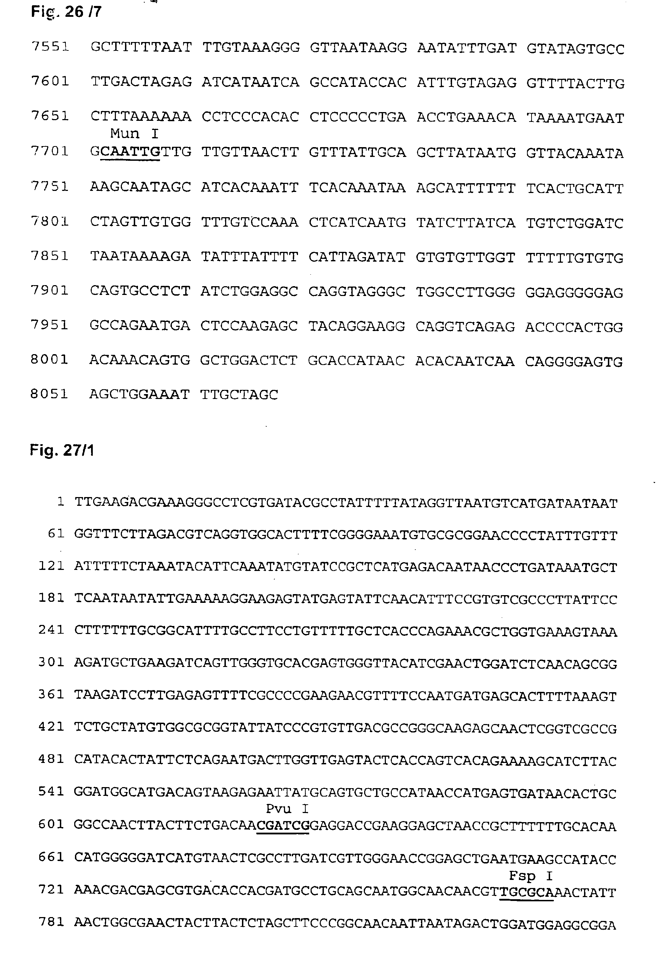

***Fig. 26.*** *DNA sequence of F19 chimeric antibody cloned into pKN100 mammalian expression vector.* Restriction sites are indicated by bold letters and underlined. CDRs 1 to 3 and the splice donor site are underlined. This is the DNA sequence of the mouse F19 light chain inside the pKN100 eukaryotic expression vector. This vector has a cDNA version of the human kappa constant region gene (allotype Km(3)) terminated by a strong artificial termination sequence. In addition, the Neo selection gene is also terminated by this artificial sequence and is also in the same orientation as the kappa light chain expression cassette.

The essential components of the pKN100 eukaryotic expression vector are:
1 - 6 = EcoRl site
7 - 1571 = HCMVi promoter/enhancer
583 - 587 = TATAA box
610 = Start of transcription
728 - 736 = Splice donor site
731 = Beginning of intron
1557 = End of intron
1544 - 1558 = Splice acceptor site
1590 - 1598 = Kozak sequence
1599 - 1658 = peptide leader sequence
1659 - 1997 = mouse F19 light chain
1996 - 2004 = splice donor site
2011 - 2657 = cDNA copy of human Kappa constant region (Km(3)) gene
2664 - 2880 = Artificial spaC2 termination sequence
2887 - 7845 = This is the pSV2neo vector DNA fragment comprising of
the Amp-resistance gene (in the opposite orientation),

the ColEl and SV40 origins of replication and the
Neo-resistance gene (in the same orientation as the
HCMVi-KCT cassette)
7852 - 8068 = Artificial spaC2 termination signal
This sequence ends immediately upstream of the EcoRl site (position 1-6) at the beginning of the sequence. As a vector this DNA sequence would be circular.

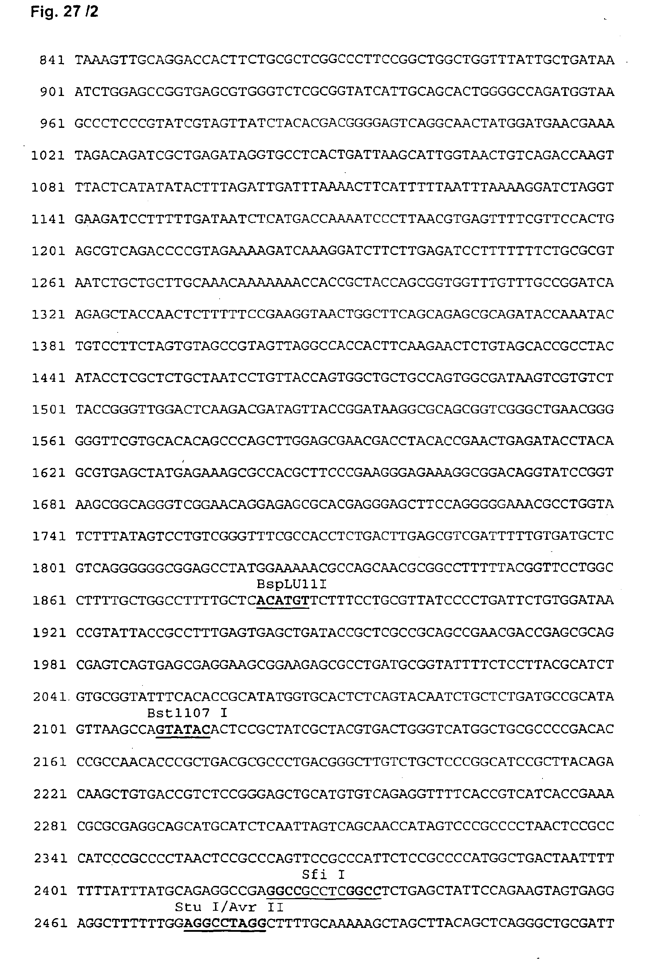

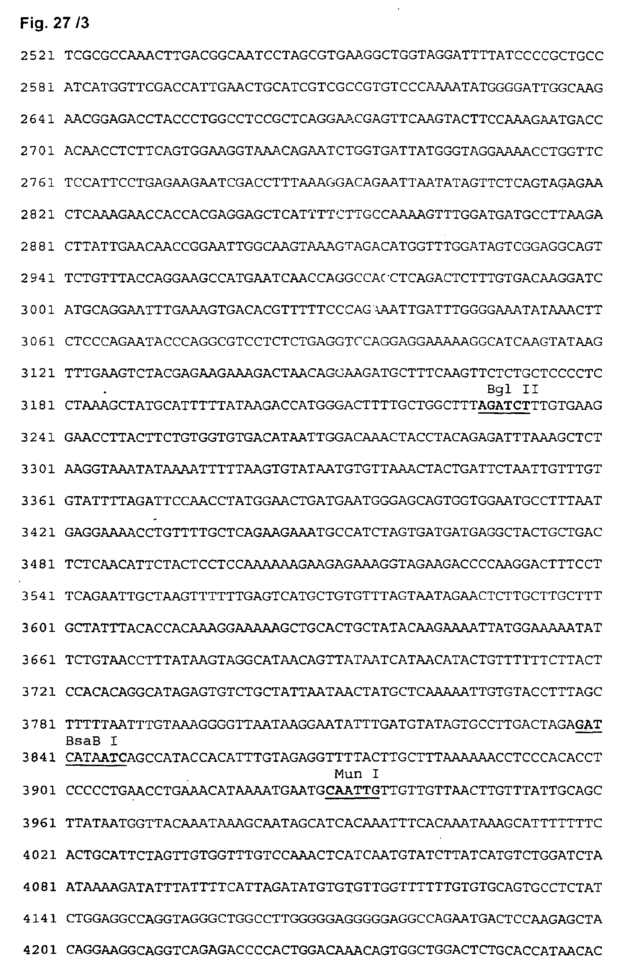

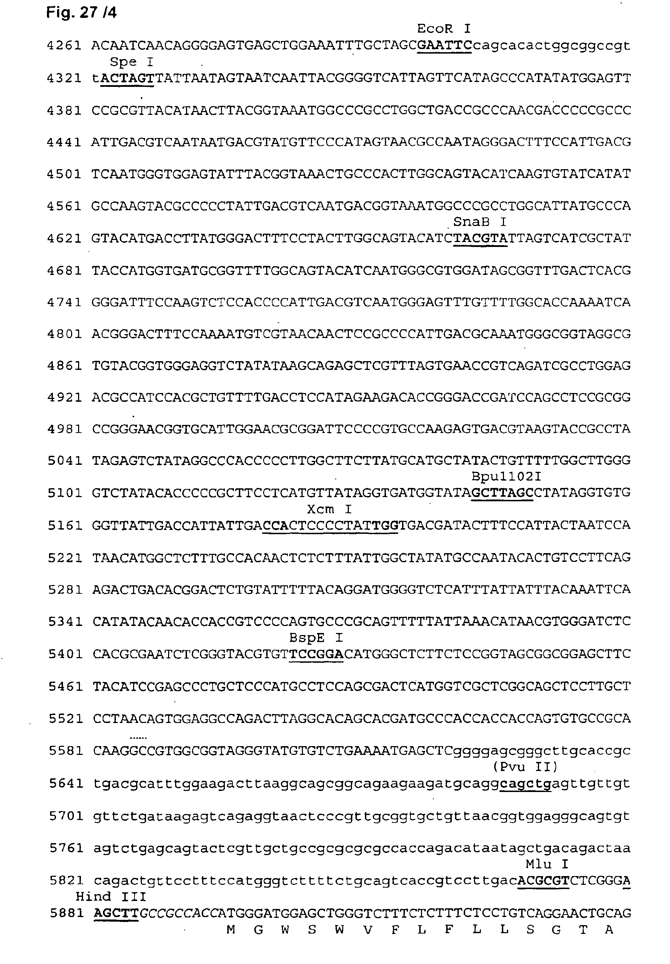

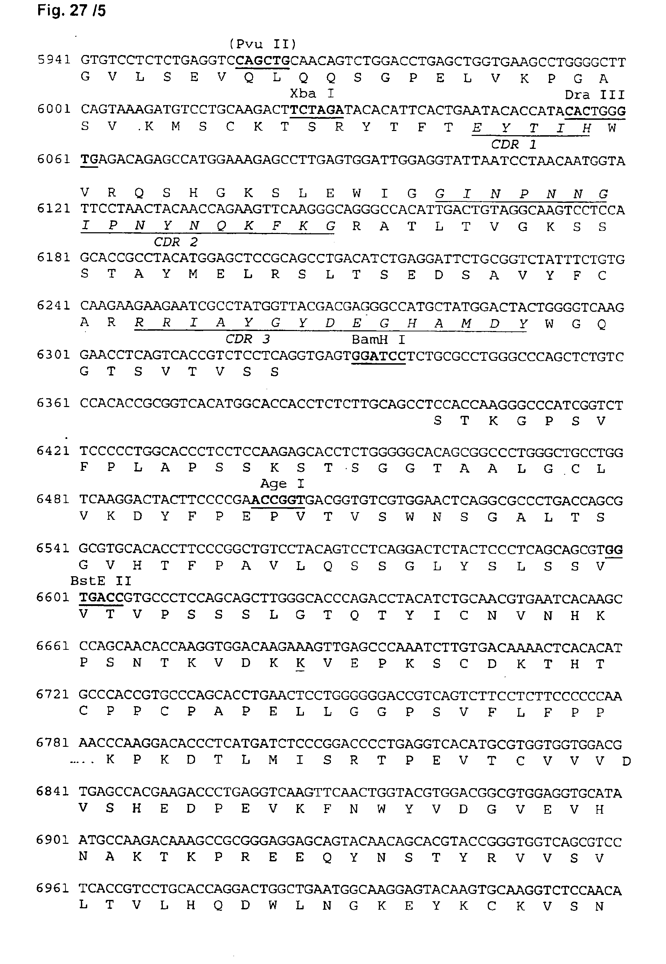

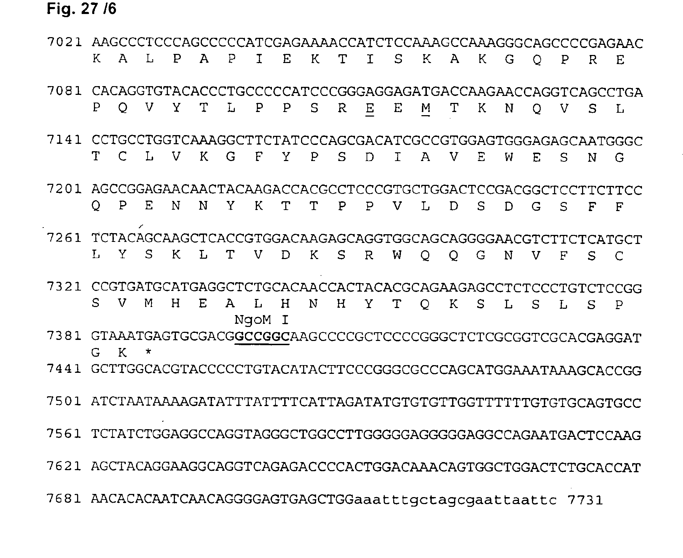

**Fig. 27.** *DNA sequence of F19 chimeric antibody cloned into pgldl05 mammalian expression vector.* Restriction sites are indicated by bold letters and underlined. CDRs 1 to 3 and the splice donor site are underlined. This is the DNA sequence of the eukaryotic expression vector pG1 D105 containing the mouse F19 heavy chain variable region. This vector contains a cDNA version of the human gamma-1 constant region (allotype G1m (non-a, z, -x) also known as Gm1 (17) allotype).
The essential components of the construct are:
1 - 2501 = pBR322 based sequence including Ampicillin resistance
gene and ColEl origin plus the SV40 origin and the
crippled SV40 early promoter
2502 - 3226 = dhfr gene
3233 - 4073 = SV40 poly A sequence etc.
4074 - 4079 = ligated BamHI and BgIII site (BstYl)
4080 - 4302 = SPA site plus C2 termination signal
4303 - 5867 = HCMVi promoter
5879 - 5885 = unique HindIII restriction site for cloning of immunoglobulin variable genes
5886 - 5894 = Kozak sequence
5895 - 5951 = signal peptide
5952 - 6323 = mouse F19 heavy chain
6323 - 6330 = splice donor site
6331 - 6336 = unique BamHI restriction site for cloning of immunoglobulin variable genes
6337 - 7388 = cDNA copy of human gamma-1 constant regions preceded by a 62 bp intron
7389 - 7709 = Arnie termination sequence
The human gamma-1 constant region used in this construct has a G1 m (non-a, z, -x) also known as Gm1 (17) allotype which is defined by a glutamic acid (E) residue at position 356 (according to Eu numbering), a methionine (M) residue at position 358 (according to Eu numbering) and a lysine (K) residue at position 214 (according to Eu numbering). These three residues are underlined in the sequence above.

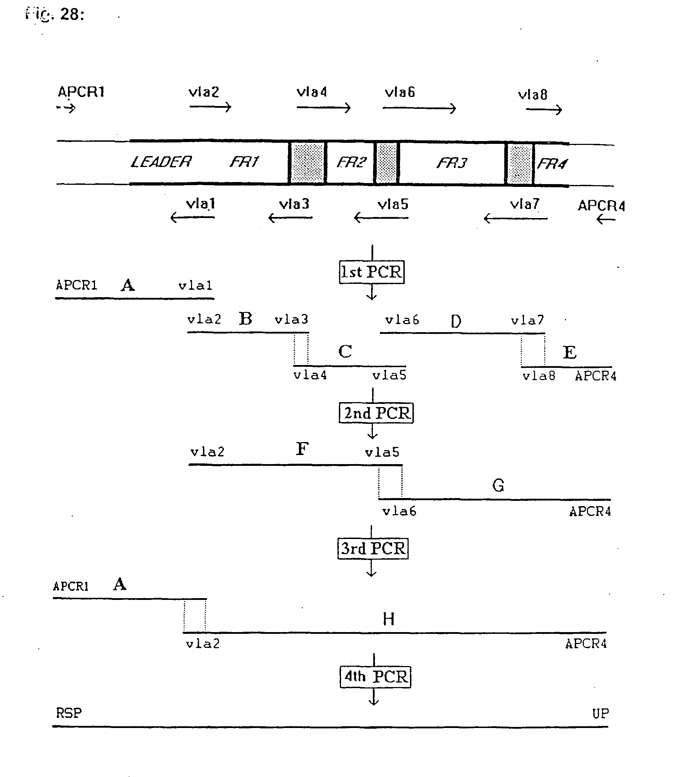

**Fig. 28.** *PCR-based method for the construction of human reshaped F19 light chain.*
This figure provides a schematic overview of the strategy of construction. The dotted lines indicate a complementary sequence of at least 21 bases between the primers.

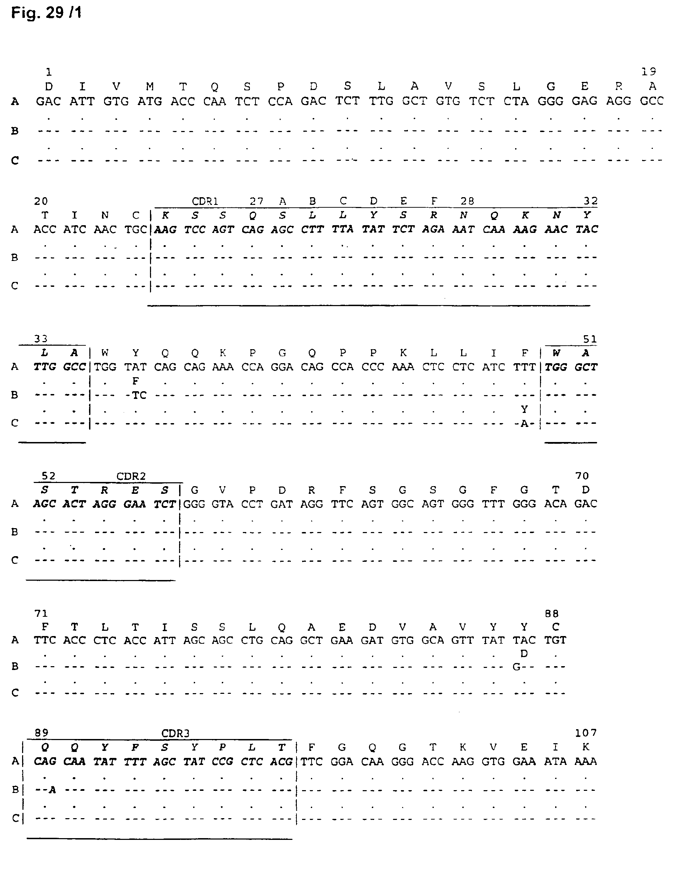

**Fig. 29.** *Nucleotide and deduced amino acid sequences of reshaped human F19 light chain variable regions version A, B and C.* Nucleotide and deduced amino acid sequences are aligned and compared with that of version A, dashes indicate nucleotide identity, dots indicate amino acid identity with this sequence. Amino acids are numbered according to Kabat *et al.* (1991). The locations of CDRs are indicated in boxes.

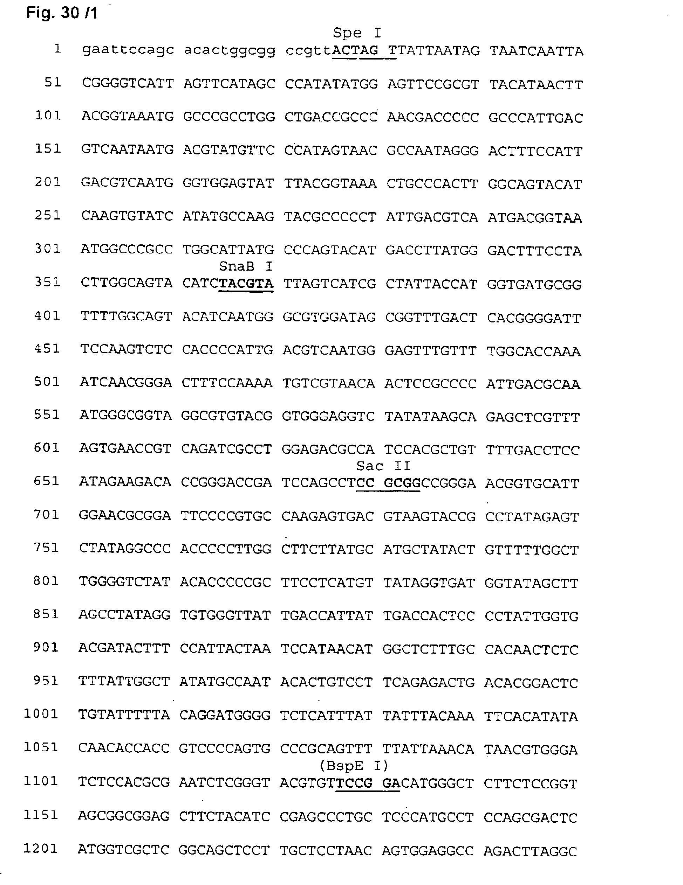

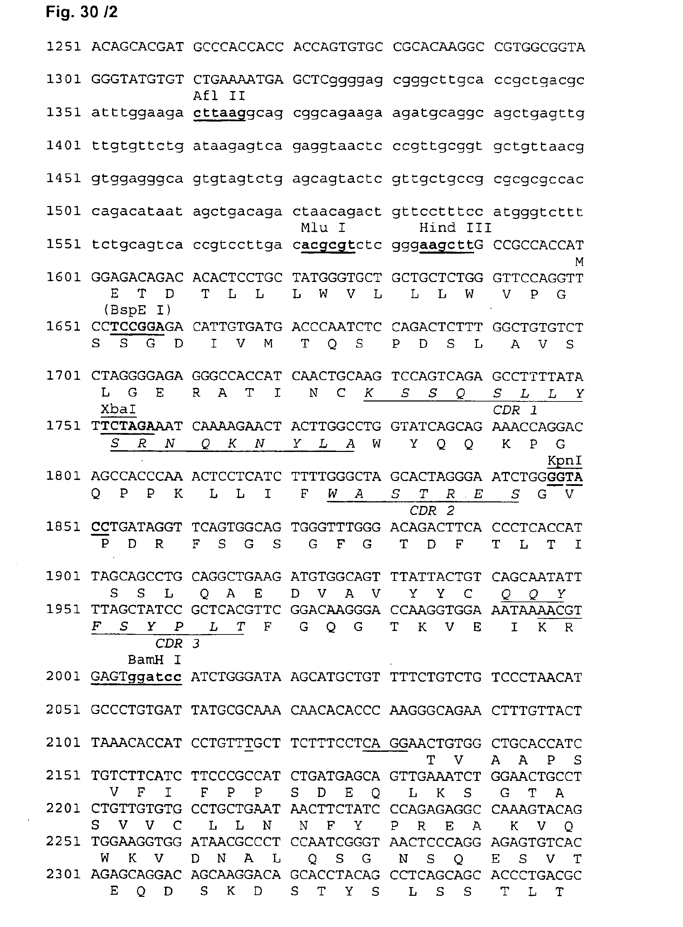

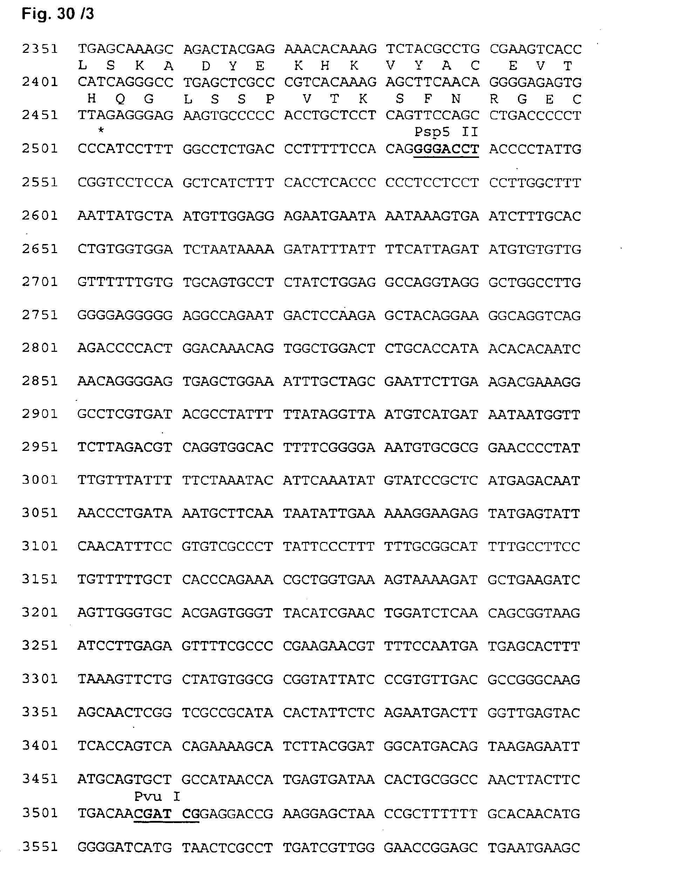

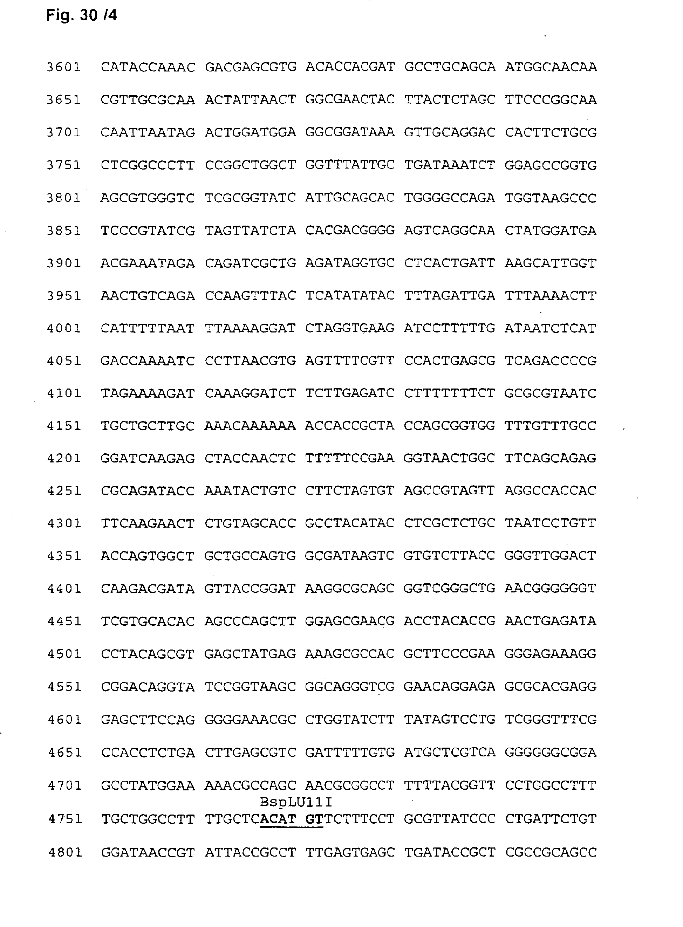

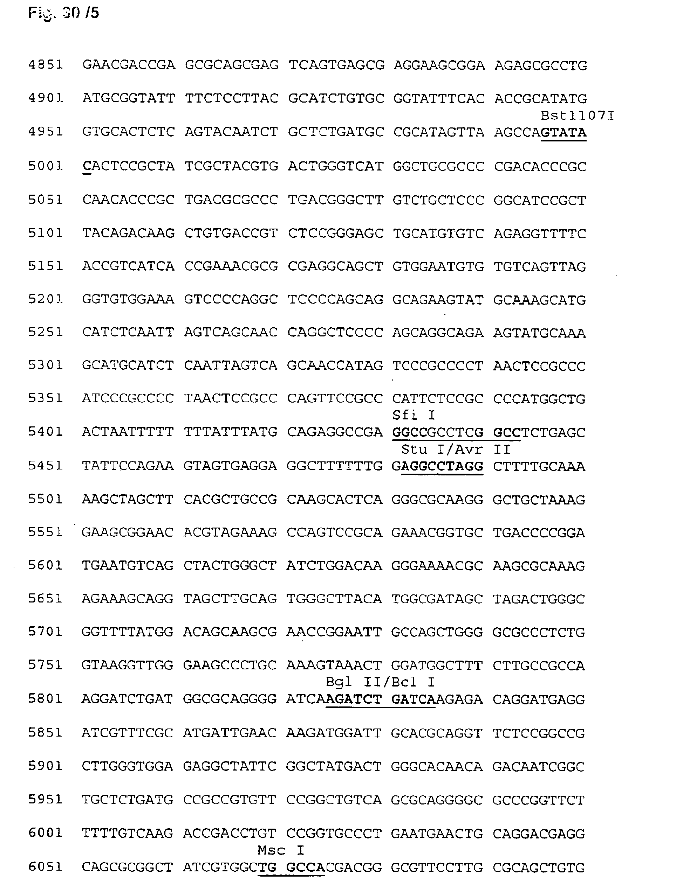

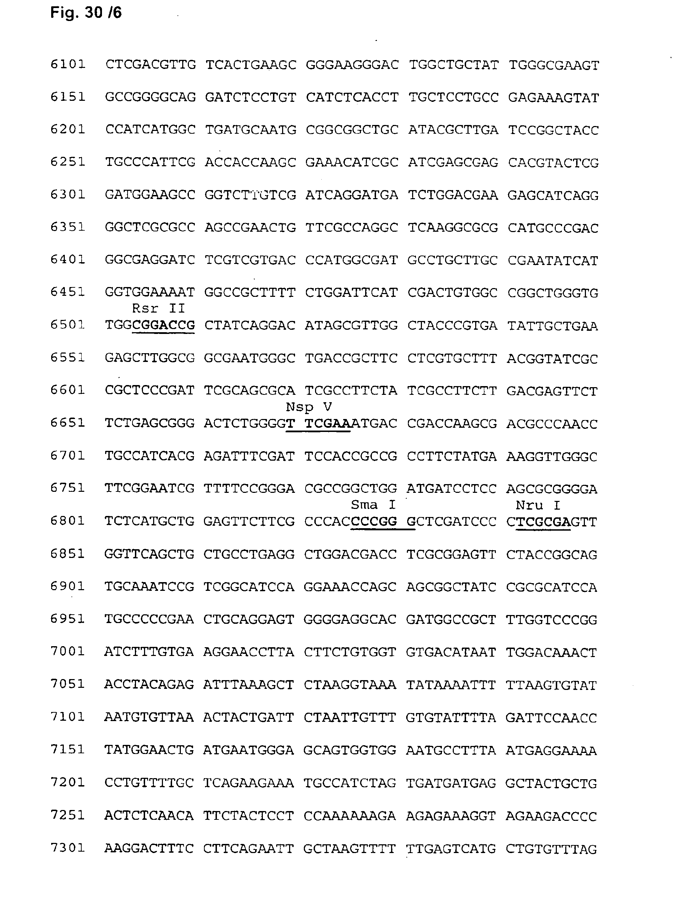

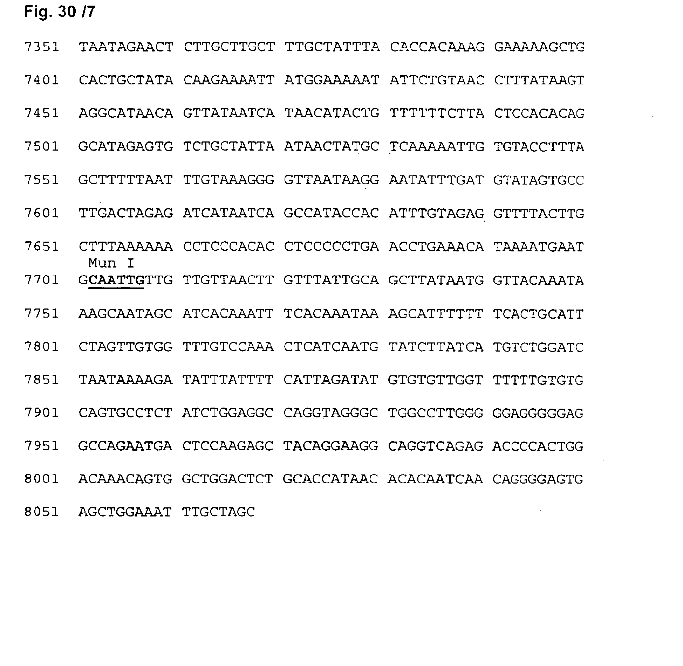

**Fig. 30.** *DNA sequence of F19 $L_A$ (human reshaped light chain version A) cloned into pKN100 mammalian expression vector.* Restriction sites are indicated by bold letters and underlined. CDRs 1 to 3 and the splice donor site are underlined.
This is the DNA sequence of the reshaped F19 light chain version A cloned into pKN100 eukaryotic expression vector. This vector has a cDNA version of the human kappa constant region gene (allotype Km(3)) terminated by a strong artificial termination sequence. In addition, the Neo selection gene is also terminated by this artificial sequence and is also in the same orientation as the kappa light chain expression cassette.
The components of the vector are:
7 - 1571 = HCMVi promoter/enhancer
583 - 587 = TATAA box.
610 = Start of transcription.
728 - 736 = Splice donor site.
731 = Beginning of intron.
1557 = End of intron.
1544 - 1558 = Splice acceptor site.

1590 - 1598 = Kozak sequence
1599 - 1658 = peptide leader sequence
1659 - 1997 = reshaped F19 light chain version A
1996 - 2004 = splice donor site
2011 - 2657 = cDNA copy of human kappa constant region (Km(3)) gene.
2664 - 2880 = Artificial spaC2 termination sequence.
2887 - 7845 = This is the pSV2neo vector DNA fragment comprising of the Amp-resistance gene (in the opposite orientation), the ColEl and SV40 origins of replication and the Neo-resistance gene (in the same orientation as the HCMVi-KCT cassette).
7852 - 8068 = Artificial spaC2 termination signal.
This sequence ends immediately upstream of the EcoRl site (position 1-6) at the beginning of the sequence below. As a vector this DNA sequence would be circular.

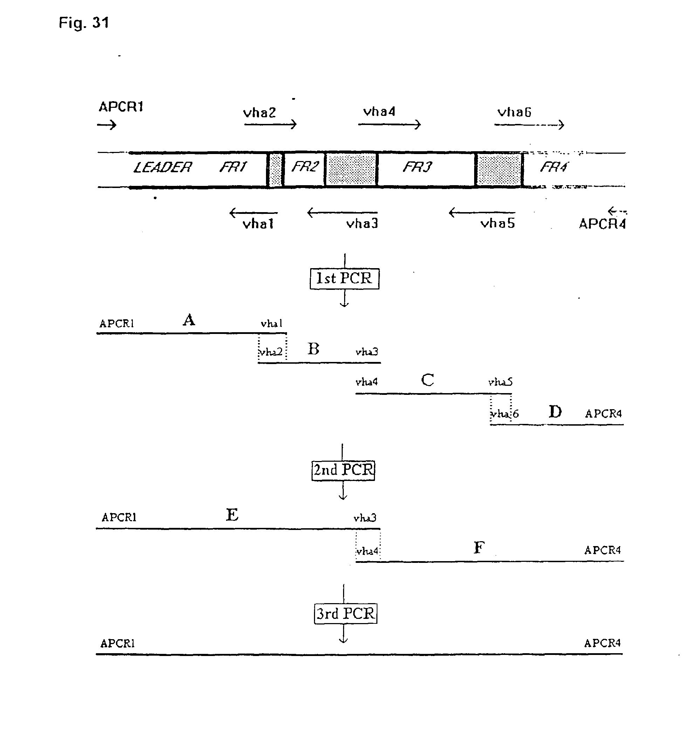

***Fig. 31.*** *PCR-based method for the construction of human reshaped F19 heavy chain.*
This figure provides a schematic overview of the strategy of construction. The dotted lines indicate a complementary sequence of at least 21 bases between the primers.

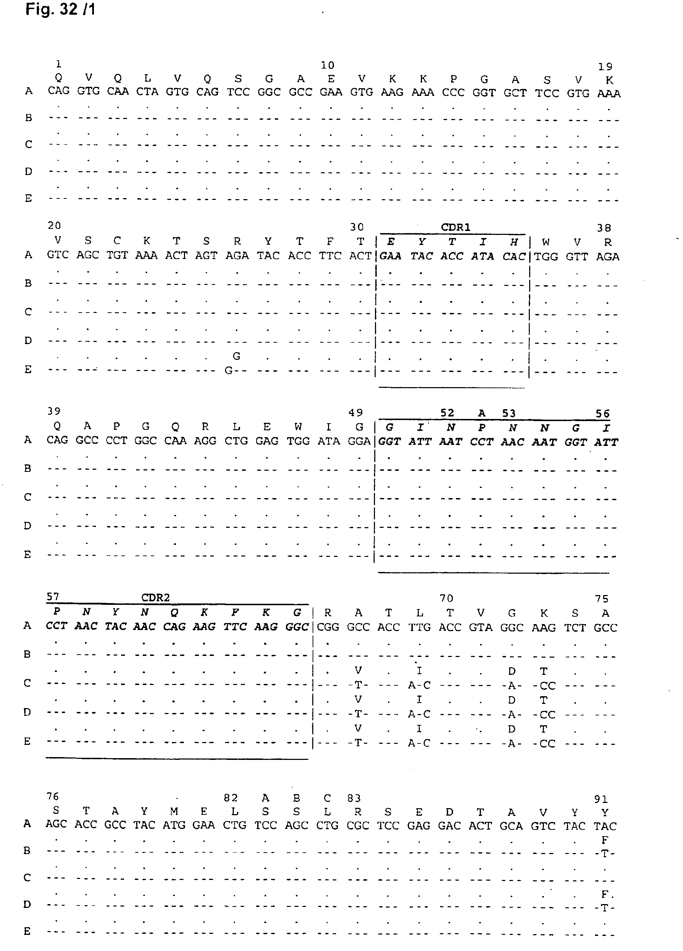

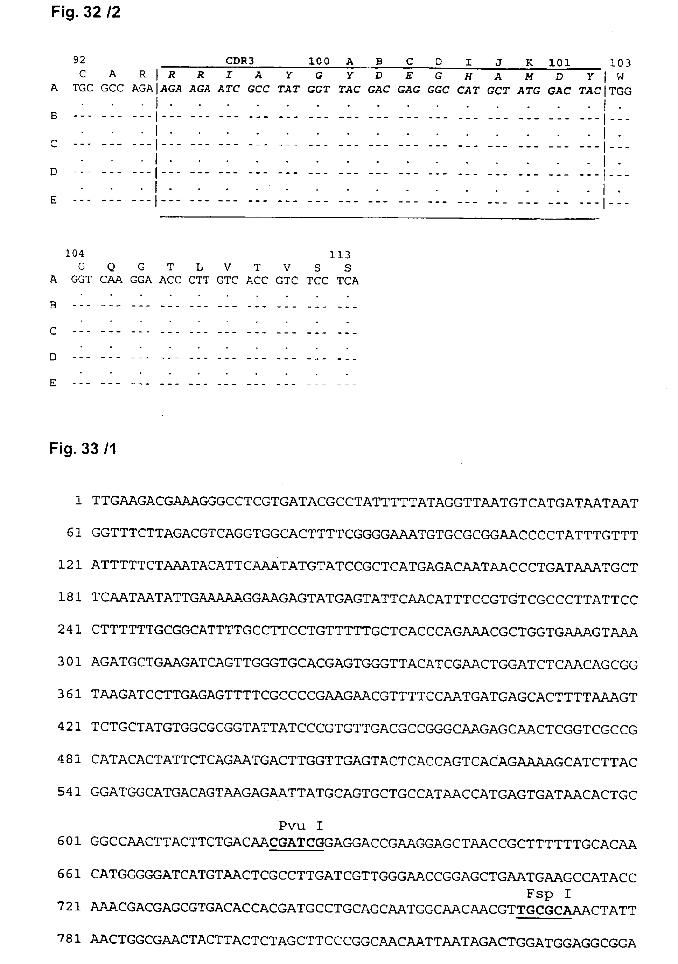

***Fig. 32.*** *Nucleotide and deduced amino acid sequences of reshaped human F19 heavy chain variable region versions a to* e. Nucleotide and deduced amino acid sequences are aligned and compared with that of version A, dashes indicate nucleotide identity, dots indicate amino acid identity with this sequence. Amino acids are numbered according to Kabat *et al.* (1991). The location of CDRs is indicated by boxes.

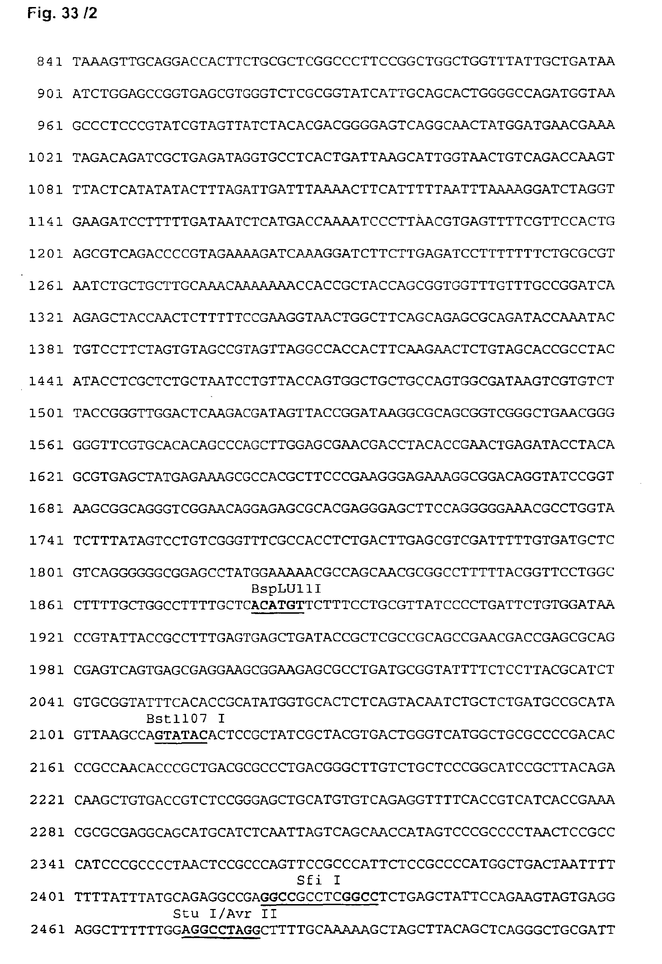

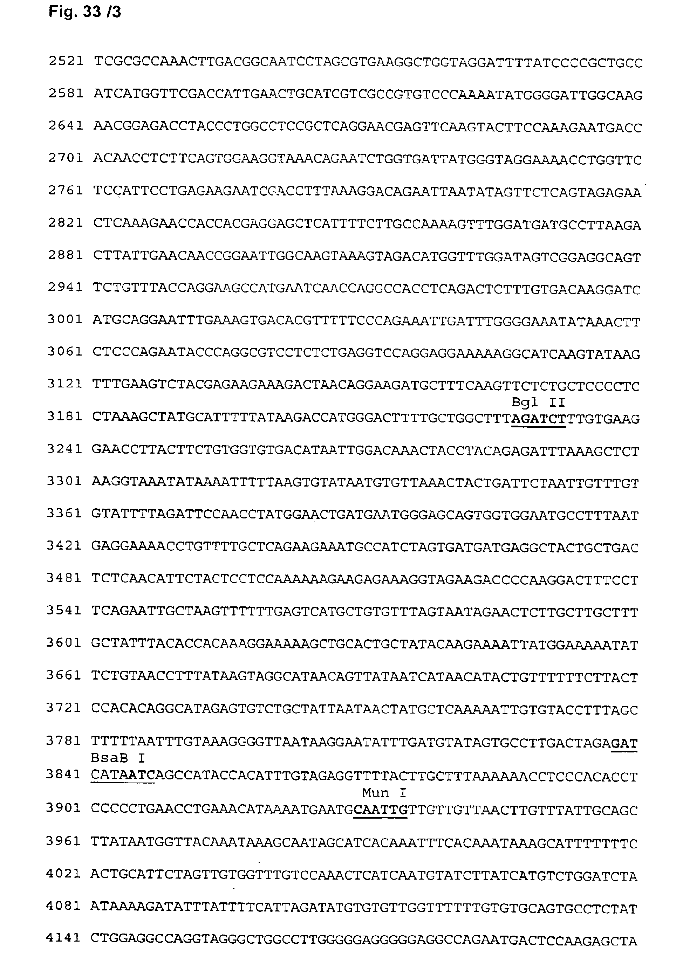

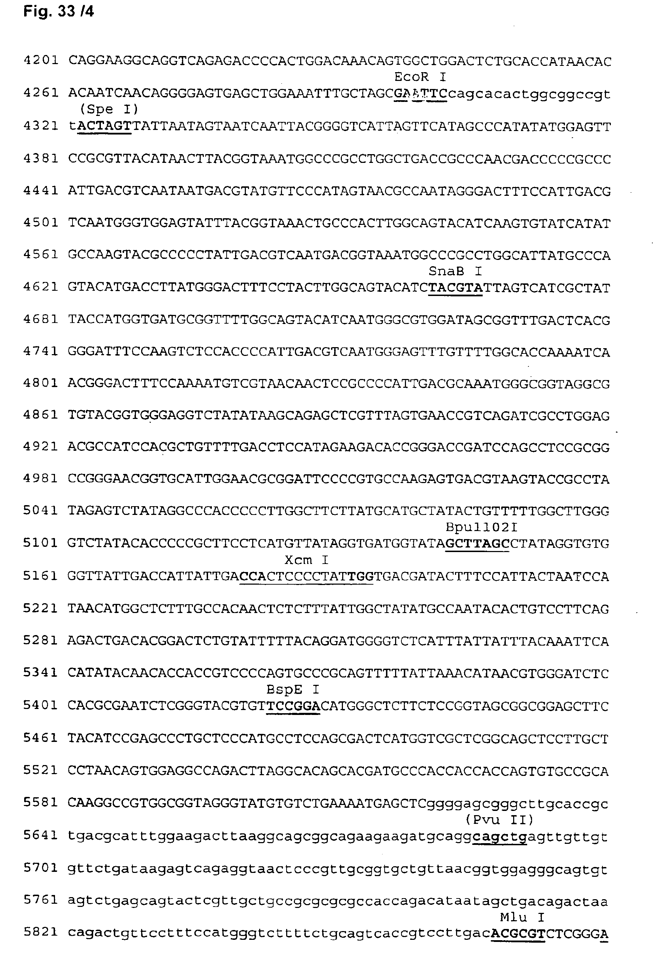

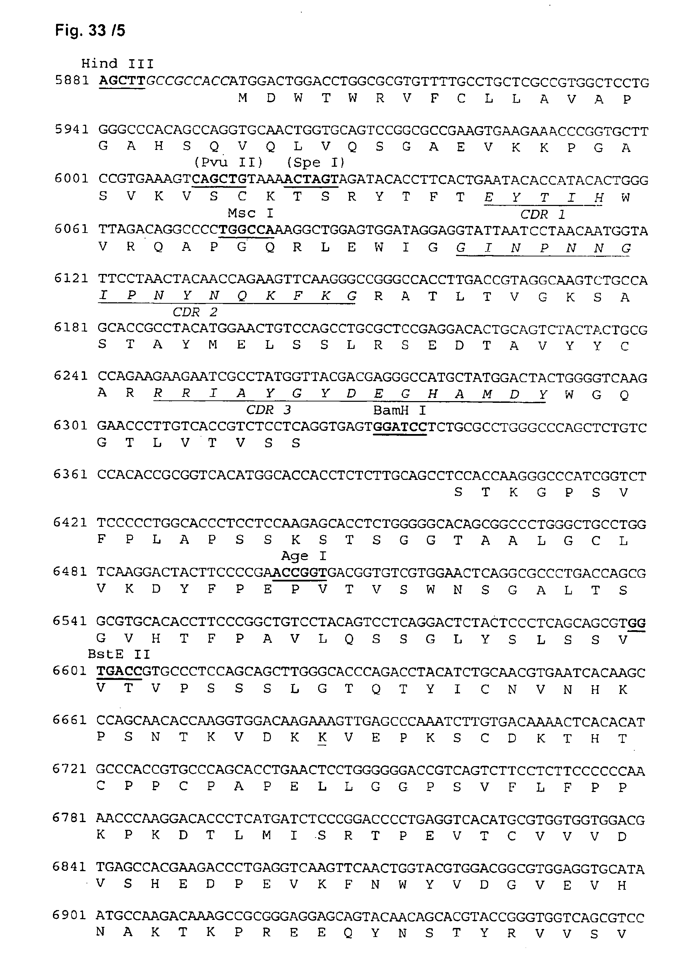

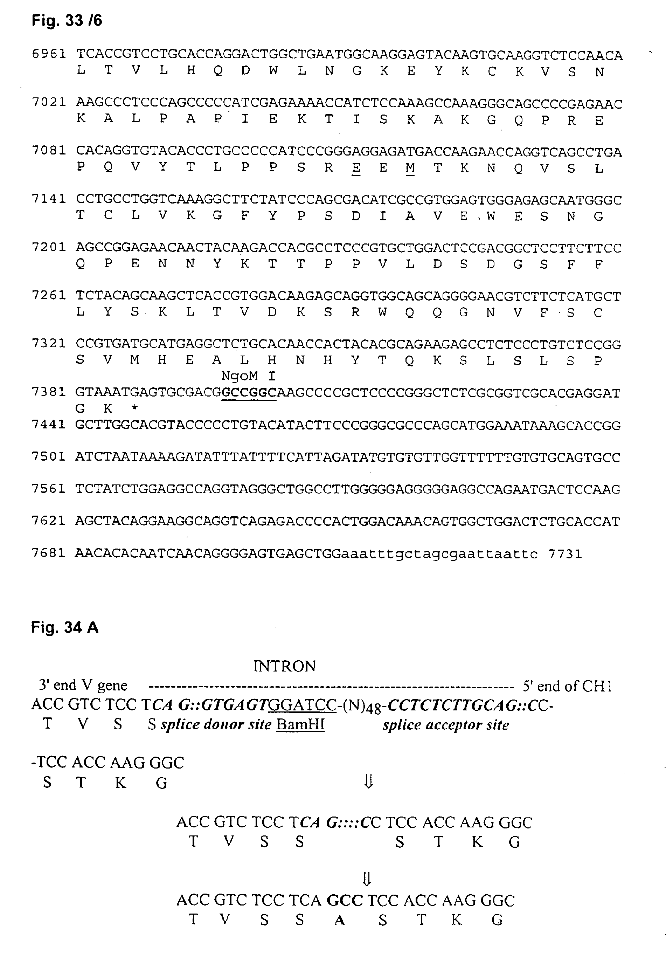

***Fig. 33.*** *DNA sequence of F19Ha (human reshaped heavy chain version a) cloned into pg1d105 mammalian expression vector.* Restriction sites are indicated by bold letters and underlined. CDRs 1 to 3 and the splice donor site are underlined.
This is the DNA sequence of the eukaryotic expression vector pG1D105 containing the reshaped version A of F19 heavy chain variable region. This vector contains a cDNA version of the human gamma-1 constant region (allotype G1m (non-a, z, -x) also known as Gm1(17) allotype).
The essential components of the construct are:
1 - 2501 = pBR322 based sequence including Ampicillin resistance gene and ColEl origin plus the SV40 origin and the crippled SV40 early promoter
2502 - 3226 = dhfr gene
3233 - 4073 = SV40 poly A sequence etc.
4080 - 4302 = SPA site plus C2 termination signal
4303 - 5867 = HCMVi promoter/enhancer
5879 - 5885 = unique HindIII restriction site for cloning of immunoglobulin variable genes
5886 - 5894 = Kozak sequence
5895 - 5951 = signal peptide
5952 - 6323 = reshaped F19 heavy chain version A
6323 - 6330 = splice donor site
6331 - 6336 = unique BamHl restriction site for cloning of immunoglobulin variable genes
6337 - 7388 = cDNA copy of human gamma-1 constant regions preceded by a 62 bp intron
7389 - 7709 = Arnie termination sequence
The human gamma-1 constant region used in this construct has a G1 m (non-a, z, -x) also known as Gm1 (17) allotype which is defined by a glutamic acid (E) residue at position 356 (according to Eu numbering), a methionine (M) residue at position 358 (according to Eu numbering) and a lysine (K) residue at position 214 (according to Eu numbering). These three residues are underlined in the sequence above.

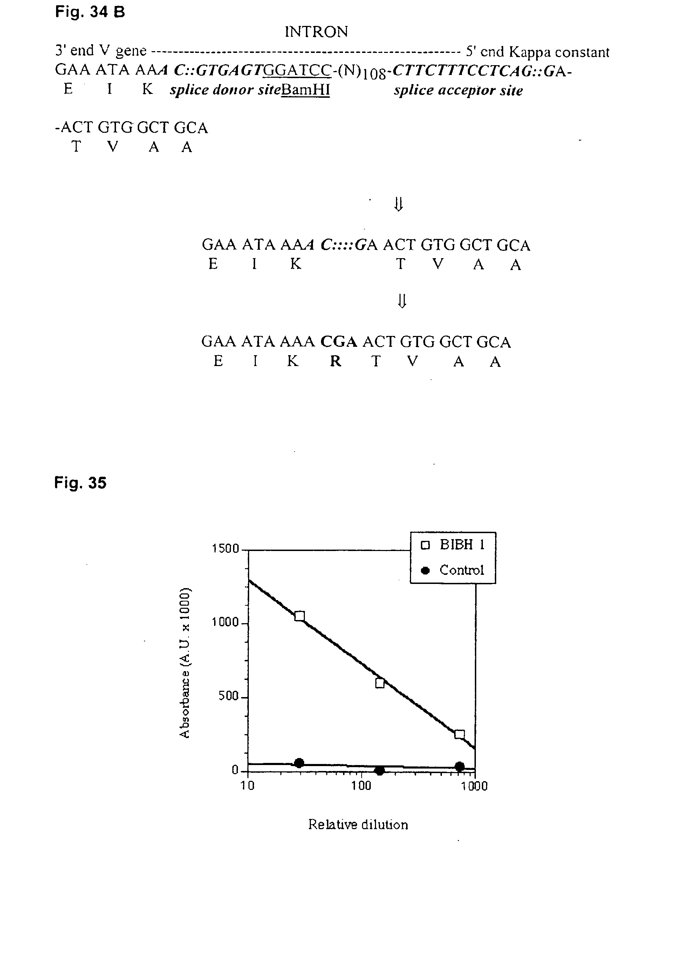

***Fig. 34.*** *Heavy (panel A) and light (panel B) chains RNA splicing events taking place during antibody F19 expression in mammalian cells* - schematic overview.

A. Heavy chain RNA splicing
B. Kappa light chain RNA splicing

***Fig. 35.*** *Concentration dependence of $L_AH_C$ supernatant binding to CD8-FAP.*

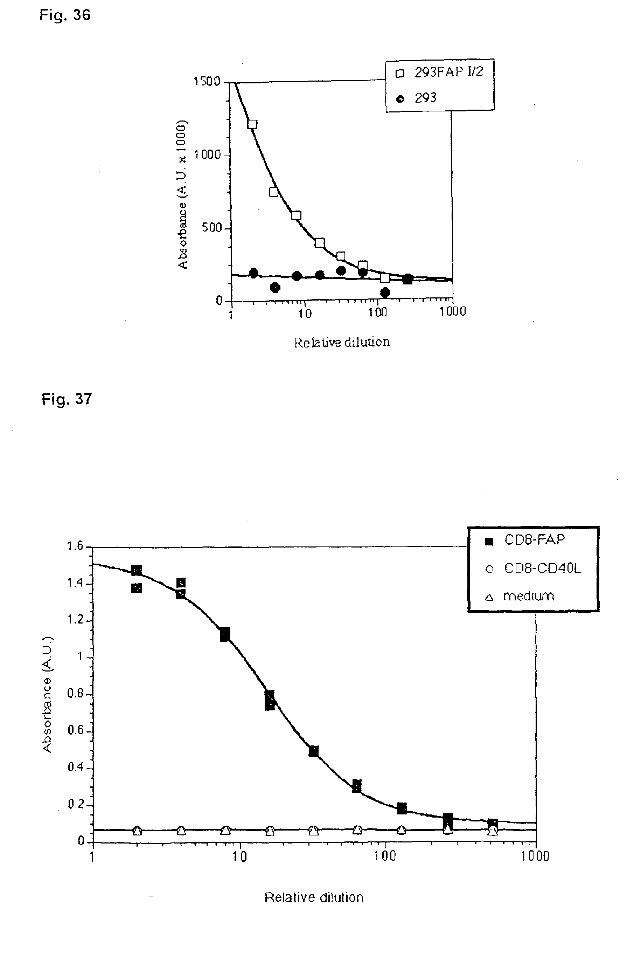

***Fig. 36***. *Binding of biotinylated $L_AH_C$ to human FAP.*

**Fig. 37**. *CD8-FAP carries the F19 epitope* as *detected with cF19.*

## *Examples*

### *Example 1:* Construction of mouse - human chimeric genes

The chimeric F19 (cF19) antibody was designed to have the mouse F19 $V_L$ and $V_H$ regions linked to human kappa and gamma-1 constant regions, respectively. PCR primers were used to modify the 5'- and 3'- sequences flanking the cDNA sequences coding for the mouse F19 $V_L$ and $V_H$ regions (Table 1). PCR primers specific for F19 light chain V-region were designed. These adapted mouse F19 variable regions were then subcloned into mammalian cell expression vectors already containing the human kappa (pKN100 vector) or gamma-1 (pG1 D105 vector) constant regions (Figure 23). These vectors employ the human cytomegalovirus (HCMV) promoter/enhancer to efficiently transcribe the light and heavy chains. The vectors also contain the SV40 origin of replication to permit efficient DNA replication and subsequent protein expression in COS cells. The expression vectors were designed to have the variable regions inserted as HindIII-BamHI DNA fragments. PCR primers were designed to introduce these restrictions sites at the 5'- (HindIII) and 3'- (BamHI) ends of the cDNAs coding for the V-regions. In addition the PCR primers were designed to introduce the Kozak sequence (GCCGCCACC) at the 5'-ends of both the light and heavy chain cDNAs to allow efficient translation (Kozak M.: At least six nucleotides preceding the AUG initiator codon enhance translation in mammalian cells. (1987) *J. Mol. Biol.* **196:** 947), and to introduce splice donor sites at the 3'-ends of both the light and heavy chain cDNAs for the variable regions to be spliced to the constant regions. The PCR primers used in the construction of the chimeric F19 light and heavy chains are shown in Table 1. The DNA and amino acid sequences of the mouse F19 $V_L$ and $V_H$ regions as adapted for use in the construction of chimeric F19 light and heavy chains are shown in Figures 24 and 25. The DNA sequences of mouse F19 light and heavy chains cloned into the eukaryotic expression vectors pKN100 and pG1D105, respectively, are shown in Figures 26 and 27.

**TABLE 1: PCR primers for the construction of chimeric F19 antibody.**

| A. Light chain variable region |
|---|
| 1. Primer for the construction of the 5'-end (37mer) |
| 5' CAGA **AAGCTT** GCCGCCACC ATG GAT TCA CAG GCC CAG 3' |
| **HindIII** Kozak sequence M D S Q A Q |
| 2. Primer for the construction of the 3'-end (35mer) |
| 5' CCGA **GGATCC** ACTCACG TTT CAG CTC CAG CTT GGT 3' |
| **BamHI** Splice donor site |
| B. Heavy chain variable region |
| 1. Primer for the construction of the 5'-end (37mer) |
| 5' CAGA **AAGCTT** GCCGCCACC ATG GGA TGG AGC TGG GTC 3' |
| **HindIII** Kozak sequence M G W S W V |
| 2. Primer for the construction of the 3'-end (35mer) |
| 5' CCGA **GGATCC** ACTCACC TGA GGA GAC GGT GAC TGA 3' |
| **BamHI** Splice donor site |

### *Example 2:* Expression and binding activity of chimeric F19 antibody

The two plasmid DNAs coding for the chimeric F19 light and heavy chains (see example 1) were co-transfected into *COS* cells to look for transient expression of chimeric F19 antibody as described below. After incubation for 72 hours, the medium was collected, centrifuged to remove cellular debris, and analysed by ELISA for the production of a human IgG1-like antibody. The *COS* cell supernatant containing the chimeric F19 antibody was analysed for its ability to bind to HT 1080 cells (see example 13) expressing the FAP antigen on their surface.

Transfection of COS cells using electroporation

The mammalian expression vectors pg1d105 and pKN100 containing the chimeric or reshaped human heavy and light chains versions, respectively, were tested in COS cells to look for transient expression of F19 antibodies. COS7 cells were passaged routinely in DMEM (Gibco BRL cat. #41966) containing penicillin (50 IU/ml), streptomycin (50 μg/ml), L-glutamine and 10% heat-inactivated gamma globulin-free foetal calf serum (FCS, Harlan Sera-Lab cat. # D0001). The DNA was introduced into the *COS* cells by electroporation using the Gene Pulsar apparatus (BioRad). DNA (10μg of each vector) was added to a 0.8 ml aliquot of $1\times10^7$ cells/ml in Phosphate-buffered saline (PBS, $Ca^{2+}$ and $Mg^{2+}$ free). A pulse was delivered at 1,900 volts, 25 μF capacitance. After a 10 min recovery period at ambient temperature the electroporated cells were added to 8 ml of DMEM containing 5% FCS. After incubation at 37°C for 72 hours, the medium was collected, centrifuged to remove cellular debris, and stored under sterile conditions at 4°C for short periods of time, or at -20°C for longer periods.

ELISA method for measuring assembled IgG1/kappa antibody concentrations in *COS* cell supernatants

Samples of antibodies produced in transfected *COS* cells were assayed by ELISA to determine how much chimeric or reshaped human antibody had been produced. For the detection of antibody, plates were coated with goat anti-human IgG (Fcγ fragment specific) antibody (Jackson ImmunoResearch Laboratories, Inc., #109-005-098). The samples from *COS* cells were serially diluted and added io each well. After incubation for 1 h at 37°C and washing, horseradish peroxidase conjugated goat anti-human kappa light chain (Sigma, A-7164) was added. After incubation for 30 minutes at 37°C and washing, K-blue substrate (a mixture of 3,3',5,5' tetramethylbenzidine and hydrogen peroxide, Bionostics Limited, #KB175) was added for 30 minutes at room temperature. The reaction was stopped using Red Stop solution (Bionostics Limited, #RS20) and the optical density read on a microplate reader at 650 nm. Purified human IgG1/Kappa antibody (Sigma, I-3889) of known concentration was used as a standard.

The expression of chimeric F19 antibody in *COS* cells was poor (Table 2), between 10 and 60 ng/ml, which is at least 10 fold less than most antibodies.

In an attempt to increase expression levels of the chimeric F19 antibody, the leader sequence of F19 $V_L$ region was changed by substitution of leucine to proline at position -9. This single change in amino acid in the leader sequence resulted in at least doubling the amount of chimeric antibody produced in COS cells.

Cell binding results show that chimeric F19 binds specifically and with the expected avidity to the FAP target.

**TABLE 2: Chimeric F19 antibody concentrations in *COS* cell supernatants(These are the results of three independent transfections)**

| Transfected Antibody components | | Human γ1/K |
|---|---|---|
| Heavy chain | Kappa light chain | [in μg/ml] |
| cF19 | cFI9 (F19 leader sequence) | 0.060 |
| cF19 | cF19 (mutated leader sequence) | 0.212 |
| cF19 | cF19 (F19 leader sequence) | 0.056 |
| cF19 | cF19 (mutated leader sequence) | 0.108 |
| cF19 | cF19 (F19 leader sequence) | 0.011 |
| cF19 | cF19 (mutated leader sequence) | 0.087 |

### *Example 3:* Construction of the reshaped human F19 light chain versions A to C ($L_A$ - $L_B$)

The construction of the first version of reshaped human F19 $V_L$ region ($L_A$) was carried out using overlapping PCR fragments in a method similar to that described by Daugherty B. L., DeMartino J. A., Law M. F., Kawka D. W., Singer I. I. and Mark G. E. (1991) Polymerase chain reaction (PCR) facilitates the cloning, CDR-grafting, and rapid expression of a murine monoclonal antibody directed against the CD18 component of leukocyte integrins. *Nucl. Acids Res.* **19**: 2471. Ten oligonucleotides were synthesised that consisted of five primer pairs, APCR1-vla1, vla2-vla3, vla4-vla5, vla6-vla7, and vla8-APCR4 (Table 3 and Figure 28). There was an overlapping sequence of at least 21 bases between adjacent pairs (Figure 28). APCR1 and APCR4 hybridised to the flanking pUC19 vector sequences. The mutagenic primers were designed such that their 5' end immediately followed the wobble position of a codon. This strategy was used to counteract the gratuitous addition of one nucleotide to the 3' end of the strand complementary to the mutagenic primer by the DNA polymerase during PCR (Sharrocks A. D. and Shaw P. E. (1992) Improved primer design for PCR-based, site-directed mutagenesis. *Nucl. Acids Res.* **20**: 1147). The appropriate primer pairs (0.2 μM of each) were combined with 10ng of version "B" of reshaped human L25$V_L$ region cDNA, and 1 unit of AmpliTaq (Perkin Elmer Cetus) DNA polymerase in 50μl of PCR buffer containing 10 mM Tris-HCl (pH 8.3), 50 mM KCl, 200 μM dNTPs, and 1.5 mM $MgCl_2$. This was overlaid with mineral oil and PCR was performed for 25 cycles, each cycle consisting of a denaturation step at 94°C for 1 min, a primer annealing step at 55°C for 1 min, and an extension step at 72°C for 2 minutes. This was followed by a single cycle consisting of a further elongation step at 72°C for 10 minutes followed by cooling to 4°C. The ramp time between the primer-annealing and extension steps was 2.5 minutes. The PCR products of the five reactions (A, B, C, D and E) were then purified by gel electrophoresis followed by DNA elution using Wizard PCR preps (Promega). PCR products A, B, C, D, and E were assembled by their complementarity to one another. In the second set of PCR reactions, PCR products B and C, and D and E, (50 ng of each) were added to 50 μl PCR reactions (as described above) each containing 1 unit of AmpliTaq (Perkin Elmer Cetus) DNA polymerase. The reactions were cycled for 20 cycles as described above with the exception that the annealing temperature was raised to 60°C. In the third set of PCR reactions, PCR products F and G were PCR-amplified using 1 μl of each prior PCR reaction and the appropriate pair of PCR primers (vla2-vla5 or vla6-APCR4). The PCR reactions contained 1 unit of AmpliTaq DNA polymerase in 50 μl PCR reaction (as described above) and were amplified for 25 cycles as in the first stage. In the fourth set of PCR reactions, the PCR product H was PCR-amplified using 1 μl of each prior PCR reaction and the vla2-APCR4 pair of PCR primers. Finally, PCR products A and H were assembled by their own complementarity in a two step-PCR reaction similar to that described above using RSP and UP as the terminal primers. The fully assembled fragment representing the entire reshaped human F19 $V_L$ region including a leader sequence was digested with HindIII and BamHI and cloned into pUC19 for sequencing. A clone having the correct DNA sequence was designated reshF19La (Figure 29) and was then subcloned into the eukaryotic expression vector pKN100. The DNA sequence of reshF19La cloned into pKN100 is shown in Figure 30.

The second version of reshaped human F19 $V_L$ region ($L_B$) was constructed using the same scheme as that described for La but where vla4 and vla7 primers were substituted by vlb4 and vlb7 respectively (Table 3). The DNA sequence of $L_B$ is shown in Figure 29.

The third version of reshaped human F19 $V_L$ region ($L_C$) was constructed using the QuikChange™ site-directed mutagenesis kit from Stratagene. The QuikChange site-directed mutagenesis method was performed according to the manufacturer's instructions, using reshF19La in pKN100 vector as double stranded DNA template. The mutagenic oligonucleotide primers F19Lc-sense and F19Lc-antisense (Table 3) for use in this protocol were designed according to the manufacturer's instructions. Briefly, both the mutagenic primers contained the desired point mutation (codon TTT at Kabat residue position 49 (Phe) changed to TAT coding for Tyr) and annealed to the same sequence on opposite strands of $L_A$ in pKN100 vector. The point mutation was verified by DNA sequencing the entire $V_L$ region. The DNA sequence of $L_C$ is shown in Figure 29. To eliminate the possibility that random mutations occurred in the pKN100 during the PCR reaction, the $V_L$ region was cut out of the pKN100 vector as an HindIII/BamHI fragment and re-subcloned into an unmodified pKN100 vector cut with the same two restriction enzymes beforehand.

**TABLE 3: PCR primers for the construction of reshaped human F19 light chain variable regions**

**1. Primers for the synthesis of version "A"**

F19vla1 (36 mer):
5' GTCATCACAATGTCTCCGGAGGAACCTGGAACCCAG 3'
F19vla2 (29 mer):
5' CTCCGGAGACATTGTGATGACCCAATCTC 3'
F19vla3 (45 mer):
5' GAATATAAAAGGCTCTGACTGGACTTGCAGTTGATGGTGGCCCTC 3'
F19vla4 (72 mer):
5' CAGTCAGACCCTTTTATATTCTAGAAATCAAAAGAACTACTTGGCCTGGTAT CAGCAGAAACCAGGACAGCC 3'
F19vla5 (44 mer): 5' ACCCCAGATTCCCTAGTGCTAGCCCAAAAGATGAGGAGTTTGGG 3'
F19vla6 (67 mer):
5' TAGCACTAGGGAATCTGGGGTACCTGATAGGTTCAGTGGCAGTGGGTTTG GGACAGACTTCACCCTC 3'
F19vla7 (53 mer):
5'GTCCCTTGTCCGAACGTGAGCGGATAGCTAAAATATTGCTGACAGTAA TAAAC 3'
F19vla8 (33 mer):
5' GCTCACGTTCGGACAAGGGACCAAGGTGGAAAT 3'

2. Primers for the synthesis of version "B"

F19vlb4 (72 mer):
5' CAGTCAGAGCCTTTTATATTCTAGAAATCAAAAGAACTACTTGGCCTGG TTCCAGCAGAAACCAGGACAGCC 3'
F19vlb7 (57 mer):

5'GTCCCTTGTCCGAACGTGAGCGGATAGCTAAAATATTGCTGACAGTCATA AACTGCC 3'

3. Primers for the synthesis of version "C"

5' CCCAAACTCCTCATCTATTGGGCTAGCACTAGGG 3'

F19Lc-antisense (34 mer):

5' CCCTAGTGCTAGCCCAATAGATGAGGAGTTTGGG 3'

F19Lc-sense (34 mer):

4. Primers hybridizing to the flanking PUC19 vector sequences

APCR1 (17 mer, sense primer): 5' TACGCAAACCGCCTCTC 3'

APCR4 (18 mer, anti-sense primer): 5' GAGTGCACCATATGCGGT 3'

RSP (-24) (16 mer, sense primer): 5' AACAGCTATGACCATG 3'

UP (-40) (17 mer, anti-sense primer): 5' GTTTTCCCAGTCACGAC 3'

***Example 4:*** **Construction of the reshaped human F19 heavy chain versions A to E ($H_A$ - $H_E$)**

Version "A" of reshaped human F19 $V_H$ regions ($H_A$) was constructed using the same PCR methods as described for the construction of version "A" of reshaped human F19 $V_L$ region ($L_A$) (Figure 31). The template DNA was version "A" of reshaped human 226 $V_H$ (Léger O. J. P., Yednock T. A., Tanner L., Horner H. C., Hines D. K., Keen S., Saldanha J., Jones T., Fritz L. C. and Bendig M. M. (1997). Humanization of a mouse antibody against human alpha-4 integrin: a potential therapeutic for the treatment of multiple sclerosis. *Hum. Antibod.* **8**: 3). Six PCR primers were designed and synthesized for the construction of version "A" of reshaped human F19 $V_H$ region (Table 4). PCR products A, B, C, and D were obtained using APCR1-Vha1, Vha2-Vha3, Vha4-Vha5 and Vha6-APCR4 as PCR primer pairs, respectively. The PCR conditions were essentially as described for the construction of reshaped human F19 $V_L$ region. A clone having the correct DNA sequence was designated reshF19Ha (Figure 32) and was then subcloned into the eukaryotic expression vector pG1D105. The DNA sequence of reshF19Ha cloned into pG1D105 is shown in Figure 33.

The third version of reshaped human F19 $V_H$ region ($H_C$) was constructed using the same scheme as that described for $H_A$ but where Vha4 primer was substituted by Vhc4 (Table 4). The DNA sequence of $H_C$ is shown in Figure 32. The second ($H_B$) and fourth ($H_D$) version of reshaped human F19 $V_H$ region were constructed based on the PCR-mutagenesis methods of Kamman et al. (Kamman M., Laufs J., Schell J. and Gronenborn B. (1989) Rapid insertional mutagenesis of DNA by polymerase chain reaction (PCR). *Nucl. Acids Res.* **17**: 5404). For $H_B$ and $H_D$, a mutagenic primer F19VHbd6 (Tyr-91 to Phe-91, Table 4) was used paired with APCR4 in PCR reactions with $H_A$ and $H_C$ as the template DNA, respectively. The PCR products VHb and VHd were restriction enzyme digested with PstI and BamHI and subcloned into reshF19Ha and reshF19Hc, respectively, previously digested with the same two restriction enzymes. The DNA sequences of $H_B$ and $H_D$ are shown in Figure 32.

Version "E" of reshaped human F19 $V_H$ region ($H_E$) was constructed based on the PCR-mutagenesis methods of Kamman *et al.* (1989) already mentioned above:

For reshF19He mutagenic primer F19MscIHe (Table 5) was used paired with primer F19$V_H$HindIII (Table 5) in PCR reactions with $H_C$ cloned in pg1d105 mammalian expression vector as the template DNA. The appropriate primer pairs (0.2 μM of each) were combined with 10 ng of cDNA of version "A" of reshaped human 226 $V_H$ region in 100 μl of PCR buffer containing 10 mM KCl, 10 mM $(NH_4)_2SO_4$, 20 mM Tris-HCl (pH 8.8) 2 mM $MgSO_4$, 0.1 % Triton X-100 and 200 μM dNTPs. Reaction mixtures were overlaid with mineral oil and kept at 94°C for 5 minutes. Then 1 unit of Deep Vent DNA polymerase (New England Biolabs) was added ("Hot Start" PCR; Chou Q., Russell M., Birch D., Raymond J. and Bloch W. (1992) Prevention of pre-PCR mis-priming and primer dimerization improves low-copy-number amplifications. *Nucl. Acids Res.* **20**: 1717) and PCR was performed for 25 cycles on a TRIO-Thermoblock Thermal Cycler (Biometra, Göttingen, Germany). Each cycle consisting of a denaturation step at 94°C for 1 min, a primer-annealing step at 70°C for 1 min, and an extension step at 72°C for 2 minutes. This was followed by a single cycle consisting of a further elongation step at 72°C for 10 minutes followed by cooling at 4°C. The PCR products were then extracted and purified from a TAE 1.4% standard agarose gel using a QIAquick™ gel extraction kit, following the protocol supplied by the manufacturer (QIAGEN Ltd., UK). The PCR product $V_H$e was then restriction enzyme digested with MscI and HindIII and ligated into reshF19Hc cloned in pg1d105 previously digested with the same two restriction en zymes. The MscI restriction recognition site is unique to all the reshaped human F19 $V_H$ region versions and is not present in the pg1d105 expression vector. The HindIII restriction recognition site is a unique site in pg1d105 for cloning of $V_H$ immunoglobulin genes. Electroporation-competent XL-1 Blue E. coli cells were transformed with 1 μl of the ligated DNA and plated on agarose plates containing Ampicillin. Colonies were then screened for the presence and correct size of inserts by direct PCR on colonies (Güssow D. and Clackson T. (1989) Direct clone characterization from plaques and colonies by the polymerase chain reaction. *Nucl. Acids Res.* **17**: 4000) with primers HCMi and Hucγ1 hybridising to the flanking pg1d105 vector sequences (Table 5). DNA from positive colonies was prepared using a Plasmid Midi kit, following the protocol supplied by the manufacturer (QIAGEN Ltd., UK). DNA sequencing was performed by the dide-oxy chain termination method (Sanger F., Nicklen S. and Coulson A. (1977) DNA sequencing with chain-terminating inhibitors. *Proc. Natl. Acad. Sci. U. S. A.* **74**: 5463) directly from circular vector DNA using conventional heat denaturation (Andersen A., Pettersson A. and Kieldsen T. (1992) A fast and simple technique for sequencing plasmid DNA with sequenase using heat denaturation. *Biotechniques* **13**: 678) and Sequenase 2.0 (USB, Cleveland, OH). The DNA sequence of reshF19He is shown in Figure 32.

**TABLE 4: PCR primers for the construction of reshaped human F19 heavy chain variable regions versions A to D.**

1. Primers for the synthesis of version "A"

F19vha1 (47mer):
5' GTGTATTCAGTGAAGGTGTATCTACTAGTTTTACAGCTGACTTTCAC 3'
F19vha2 (53 mer):
5' TAGTAGATACACCTTCACTGAATACACCATACACTGGGTTAGACAGG CCCCTG 3'
F19vha3 (71 mer):
5' CCCTTGAACTTCTGGTTGTAGTTAGGAATACCATTGTTAGGATTAATACC TCCTATCCACTCCAGCCTTTG 3'
F19vha4 (71 mer):
5' TAACTACAACCAGAAGTTCAAGGGCCGGGCCACCTTGACCGTAGGCAA GTCTGCCAGCACCGCCTACATGG 3'
F19vha5 (63 mer):
5'GCATGGCCCTCGTCGTAACCATAGGCGATTCTTCTTCTGGCGCAGTAGT AGACTGCAGTGTCC 3'
F19vha6 (48 mer):
5' CTATGGTTACGACGAGGGCCATGCTATGGACTACTGGGGTCAAGGAAC 3'

2. Primers for the synthesis of version "c"

F19vhc4 (71 mer):
5'TAACTACAACCAGAAGTTCAAGGGCCGGGTCACCATCACCGTAGACACCTCTGCCAGCACCGCCTACATGG 3'

3. Primers for the synthesis of version "B" and "D"

F19vhbd6 (27 mer):
5' GGACACTGCAGTCTACTTCTGCGCCAG 3'

4. Primers hybridizing to the flanking PUC19 vector sequences

APCR1 (17 mer, sense primer): 5' TACGCAAACCGCCTCTC 3'
APCR4 (18 mer, anti-sense primer): 5' GAGTGCACCATATGCGGT 3'

**TABLE 5: PCR primer for the construction of reshaped human F19 heavy chain variable regions version E**

1. Primer for the synthesis of version "E"

F19MsclHe (65 mer, anti-sense):
5' CCTT*TGGCCA*GGGGCCTGTCTAACCCAGTGTATGGTGTATTCAGTGAAGGTG *MscI* TATCCACTAGTTTCCACTAGTTT 3'

2. Primers hybridizing to the flanking pg1d105 mammalian expression vector sequences

HCMi (28 mer, sense): 5' GTCACCGTCCTTGACACGCGTCTCGGGA 3'
Hucγ1 (17 mer, anti-sense): 5' TTGGAGGAGGGTGCCAG 3'

### *Example 5:* Reshaped human F19 antibody concentrations in COS cells supernatants

*COS* cells were transfected with one pair of a series of reshaped human F19 antibody constructs and the human antibody concentration was measured using the IgG1/Kappa ELISA as described in example 2.

TABLE 6 Reshaped human F19 antibody concentrations in *COS* cell supernatants

| Transfected Antibody components | | Human γ1/K |
|---|---|---|
| Heavy chain | Kappa light chain | Concentration [μg/ml] |
| $H_A$ | $L_A$ | 2.50 |
| $H_A$ | $L_B$ | 0.18 |
| $H_B$ | $L_A$ | 1.25 |
| $H_B$ | $L_B$ | 0.10 |
| $H_D$ | $L_A$ | 1.15 |
| $H_D$ | $L_B$ | 0.18 |
| $H_A$ | $L_A$ | 1.50 |
| $H_A$ | $L_C$ | 1.56 |
| $H_C$ | $L_A$ | 1.47 |
| $H_C$ | $L_C$ | 1.97 |
| cF19 | $L_A$ | 1.54 |
| cF19 | $L_B$ | 0.07 |
| cF19 | $L_C$ | 2.14 |

TABLE 7: Reshaped human F19 antibody concentrations in COS cell supernatants

| Transfected Antibody components | | Human γ1/K |
|---|---|---|
| Heavy chain | Kappa light chain | concentration [μg/ml] |
| $H_A$ | $L_A$ | 2.00 |
| $H_A$ | $L_C$ | 2.50 |
| $H_C$ | $L_A$ | 2.90 |
| $H_C$ | $L_C$ | 3.00 |
| $H_E$ | $L_A$ | 2.80 |
| $H_E$ | $L_C$ | 3.50 |

RNA splicing events required for the expression of immunoglobulin genes in mammalian cells

Both mammalian expression vectors pKN100 and pg1d105 have an intron between the variable and the constant regions which is removed during the process of gene expression to give rise to an messenger RNA. The splicing event which consists of a DNA recombination between the heavy or light chain splice donor sites and the immunoglobulin splice acceptor site is described in Figure 34.

***Example* 6: Flow cytometric analysis of the binding of cF19 and $L_AH_C$ to FAP-expressing human cells**

The ability of $L_AH_C$ to bind to both recombinant and endogenously expressed FAP on cell surface was tested.

The example was conducted to determine the binding of $L_AH_C$ to cellular FAP. Both naturally FAP expressing MF-SH human tumour cells (Shirasuma, K, et al., Cancer 1985, 55:2521-32) and FAP-transfected human tumour cell lines were used as cellular targets. $L_AH_C$ was studied in cytofluorometric assays evaluating direct binding to target cells as well as by the inhibitory effect on the binding of either murine F19 or chimeric cF19 anti-FAP antibodies.

Antibodies and cell lines used were F19 (murine monoclonal anti-human FAP antibody, IgG2 subclass), mIgG (murine immunoglobulin, IgG class), cF19 (chimeric monoclonal anti-human FAP antibody, IgG1 subclass), $L_AH_C$ (reshaped monoclonal anti-human FAP antibody, IgG1 subclass), hIgG1 (human immunoglobulin, IgG1 subclass), MF-SH (human malignant fibrous histiocytoma cell line), HT-1080 (human fibrosarcoma cell line), HT-1080FAP clone 33 (HT-1080 cell line transfected with cDNA encoding human FAP). Antibodies were biotinylated as described in examples 8 and 12. Direct binding of $L_AH_C$ to FAP on the surface of human tumour cell lines

5x10$^5$ cells of the tumour cell line under investigation were incubated with the indicated concentration of test or control antibody in a total volume of 0.2 ml phosphate-buffered saline (PBS) supplemented with 1% bovine serum albumin (BSA) for 30 min on ice. Subsequently, cells were washed twice with 2 ml of PBS, resuspended in 0.2 ml of PBS supplemented with 1% BSA, a 1:20 dilution of mouse anti-human IgG FITC-labelled [Dianova] as secondary reagent was added) and incubated for another 30 min on ice.

Alternatively, 5x10$^5$ cells of the tumour cell line under investigation were incubated with the indicated concentration of biotin-labelled cF19 in a total volume of 0.2 ml PBS supplemented with 1 % BSA for 30 min on ice. Subsequently, cells were washed twice with 2 ml of PBS, resuspended in 0.2 ml of PBS supplemented with 1 % BSA, and incubated for another 30 min on ice with 1:40 dilution of streptavidin-FITC (Dianova) as secondary reagent.

Cells were again washed twice with 2 ml of PBS, resuspended in a total volume of 0.5 ml of PBS supplemented with 1% paraformaldehyde (PFA) and kept on ice. Single cell fluorescence was determined cytofluorometrically by analysing the cellular green fluorescence at 488nm in an EPICS XL (Coulter) fluorescence-activated cell analyzer.

Competition of $L_AH_C$ for binding of biotinylated cF19 to cell-surface FAP on FAP-expressing human cell lines

5x10$^5$ cells of the tumour cell line under investigation were incubated with the indicated amounts of unlabelled test or control antibody added together with 1 μg/ml biotin-labelled cF19 antibody. Subsequently, cells were washed twice with 2 ml of PBS, resuspended in 0.2 ml of PBS supplemented with 1 % BSA, 1:40 diluted streptavidin-FITC (Dianova) as secondary reagent and incubated for another 30 min on ice.

Cells were then washed twice with 2 ml of PBS, resuspended in a total volume of 0.5 ml PBS supplemented with 1 % PFA and kept on ice. Single cell fluorescence was determined cytofluorometrically by analysing the cellular green fluorescence at 488nm in an EPICS XL (Coulter) fluorescence-activated cell analyzer.

Both, cF19 and $L_AH_C$ bind in a concentration dependent manner specifically to FAP-transfected HT-1080FAP clone33 human tumour cells (Table 8). No binding to FAP-negative HT-1080 cells was detectable (Table 9). Both cF19 and $L_AH_C$ bound in a concentration dependent manner to human MF-SH cells endogenously expressing FAP (Table 10).

Biotinylated cF19 bound to human HT-1080FAP clone 33 (Table 11) in a concentration dependent manner. No binding was detectable to FAP-negative HT-1080 cells (Table 12).

Binding of biotinylated cF19 to HT-1080FAP clone 33 cells was inhibited by both unlabelled cF19 and unlabelled $L_AH_C$ (Table 13).

Chimeric anti-human FAP monoclonal antibody cF19 as well as reshaped human anti-human FAP monoclonal antibody $L_AH_C$ (example 10) were shown to bind directly to FAP expressed on human cell lines either endogenously expressing this protein or transfected with cDNA encoding for it. This binding was shown to be concentration dependent. Binding of biotinylated cF19 could be inhibited by both unlabelled cF19 and unlabelled $L_AH_C$.

Using cytofluorometric technology, direct binding as well as inhibition of specifically binding reagents showed specificity of chimeric cF19 and reshaped $L_AH_C$ human monoclonal antibodies to cell surface expressed FAP.

**Table 8: Binding of anti-FAP antibodies to HT-1080FAP clone 33 cells**

| Concentration of antibody | Mean fluorescence intensity | | |
|---|---|---|---|
| [ng/ml] | hlgG1 | cF19 | $L_AH_C$ |
| 500.0 | 0.12 | 6.65 | 2.76 |
| 100.0 | 0.12 | 1.63 | 0.66 |
| 20.0 | 0.12 | 0.43 | 0.22 |
| 4.0 | 0.12 | 0.17 | 0.15 |
| 0.8 | 0.12 | 0.14 | 0.13 |

**Table 9: Binding of anti-FAP antibodies to non-transfected HT-1080 cells**

| Concentration of antibody | Mean fluorescence intensity | | |
|---|---|---|---|
| [ng/ml] | hlgG1 | cF19 | $L_AH_C$ |
| 500.0 | 0.11 | 0.11 | 0.12 |

(continued)

| Concentration of antibody | Mean fluorescence intensity | | |
|---|---|---|---|
| [ng/ml] | hIgG1 | cF19 | $L_AH_C$ |
| 100.0 | 0.11 | 0.11 | 0.11 |
| 20.0 | 0.11 | 0.11 | 0.12 |
| 4.0 | 0.11 | 0.11 | 0.12 |
| 0.8 | 0.11 | 0.11 | 0.11 |

**Table 10: Binding of anti-FAP antibodies to MF-SH cells**

| Concentration of antibody | Mean fluorescence intensity | | |
|---|---|---|---|
| [ng/ml] | hIgG1 | cF 19 | $L_AH_C$ |
| 4000 | 0.6 | 3.6 | 2.8 |
| 2000 | n.d. | 3.3 | 2.5 |
| 1000 | n.d. | 2.4 | 1.9 |
| 500 | n.d. | 1.8 | 1.3 |
| n.d.: not done | | | |

**Table 11: Binding of biotinylated cF19 antibody to HT-1080FAP clone 33 cells**

| Concentration of antibody | Mean fluorescence intensity | |
|---|---|---|
| [ng/ml] | Biotinylated hIgG1 | Biotinylated cF19 |
| 5,000.0 | 0.2 | 36.5 |
| 1,000.0 | 0.2 | 18.1 |
| 200.0 | 0.2 | 4.5 |
| 40.0 | 0.2 | 1.3 |
| 8.0 | 0.2 | 0.5 |
| 1.6 | 0.3 | 0.3 |

**Table 12: Binding of biotinylated cF19 antibody to non-transfected HT-1080 cells**

| Concentration of antibody | Mean fluorescence intensity | |
|---|---|---|
| [ng/ml] | Biotinylated hIgG1 | Biotinylated cF19 |
| 5,000.0 | 0.1 | 0.1 |
| 1,000.0 | 0.1 | 0.1 |
| 200.0 | 0.1 | 0.1 |
| 40.0 | 0.1 | 0.1 |
| 8.0 | 0.1 | 0.1 |
| 1.6 | 0.1 | 0.1 |

**Table 13: Competition of anti-FAP antibodies with the binding of biotinylated cF19 to HT-1080FAP clone 33 cells**

| | Concentration of competitor antibody | Mean fluorescence concentration |
|---|---|---|
| Competitor antibody | [μg/ml] | |
| No | 0.00 | 11.2 |
| hlgG1 | 1.00 | 9.0 |
| hlgG1 | 3.16 | 11.3 |
| hlgG1 | 10.00 | 9.8 |
| hlgG1 | 31.66 | 10.3 |
| cF19 | 1.00 | 7.5 |
| cF19 | 3.16 | 4.8 |
| cF19 | 10.00 | 1.3 |
| cF19 | 31.66 | 1.2 |
| $L_AH_C$ | 1.00 | 8.0 |
| $L_AH_C$ | 3.16 | 5.5 |
| $L_AH_C$ | 10.00 | 2.9 |
| $L_AH_C$ | 31.66 | 1.7 |
| Biotinylated cF19 was used at a concentration of 1 μg/ml in all tests shown in the Table 13. | | |

***Example* 7: In vitro immune effector functions of monoclonal antibody $L_AH_C$**

This experiment was conducted to determine the potential of the monoclonal antibody (mAb) $L_AH_C$ with specificity for fibroblast activation antigen (FAP) to lyse FAP-expressing targets in the presence of human complement or human mononuclear leukocytes, respectively.

In particular, the ability of $L_AH_C$ to mediate cytotoxic effects against HT-1080FAP clone 33 cells, which expressed human FAP on the surface, was studied. Cytotoxicity was determined in vitro using the following approach: $^{51}Cr$ -labelled target cells were incubated in the presence of $L_AH_C$ with human serum as source of complement or human MNC (peripheral blood mononuclear cells) as effectors. Release of $^{51}Cr$ was measured as measure of target-cell lysis.

Antibodies and cell lines used were $L_AH_C$ (reshaped human anti-human FAP IgG1 antibody), hlgG1 (human IgG1 isotype control), 3S193 (murine monoclonal anti-Lewis$^y$ IgG3 antibody), mlgG (murine IgG control), HT-1080 (human fibrosarcoma), HT-1 080FAP clone 33, (HT1080 transfected with cDNA encoding human FAP), MCF-7 (human breast adenocarcinoma cell line).

<u>Complement-mediated lysis of target cells by $L_AH_C$</u>

Tumour cells were radiolabelled by incubation in RPM!1640 medium with 100 μCi $^{51}Cr$ (NEN) at 37° C for one hour. Subsequently, cells were washed twice in $^{51}Cr$ -free medium and resuspended at a concentration of $2x10^5$ cells per ml.

Human serum as source of complement was freshly prepared from blood of different volunteers. Blood was taken by puncturing the arm vein, remained at room temperature for one hour to allow clotting to occur, and was kept at 4° C over night. Serum was separated by centrifugation and taken off from the sediment.

The antibody under study was diluted from the stock solution to the appropriate concentration in RPM11640 cell culture medium.

$1x10^5$ radiolabelled tumor cells of the indicated cell line were incubated for 2 h at 37° C in an incubator (95% air and 5% $CO_2$) in the presence of different concentrations of test or control antibody and 25% (v/v) human serum as the source of human complement. Incubations were performed in U-shaped 96-well plates in a total volume of 200 μl RPMI1640 and done in triplicate. After the incubation period, plates were centrifuged, 100 μl supernatant was removed and radioactivity was counted in a gamma-counter. The total amount of incorporated radioactivity was determined by measuring $10^4$ target cells. Spontaneous release was defined as activity released from the target cells in the absence of both antibody and complement during the described incubation period.

Specific lysis was calculated as follows:

$$\text{specific lysis (in \%)} = \frac{[\text{activity sample}] - [\text{activity spontaneous release}]}{[\text{maximum activity}] - [\text{activity spontaneous release}]} \times 100$$

Antibody-dependent cellular cytotoxicity (ADCC) of $L_AH_C$

Tumour cells were radiolabelled by incubation in RPMI1640 medium with 100 µCi $^{51}$Cr at 37°C for one hour. Subsequently, cells were washed twice in $^{51}$ Cr -free medium and resuspended at a concentration of 2x10$^5$ cells per ml.

MNC (peripheral blood mononuclear cells) were prepared from peripheral blood taken by puncturing the arm vein of healthy human volunteers. Clotting was prevented by the addition of 20% citrate buffer. MNC from 4 ml of this blood preparation were purified by centrifugation (30 min at 400 G and room temperature) on 3 ml of lymphocyte preparation medium (Boehringer Mannheim, Germany). MNC (peripheral blood mononuclear cells) were taken off from the gradient, washed three times and diluted with RPMI1640 to the appropriate concentration. Lymphocyte activated killer (LAK) cells were derived from MNC (peripheral blood mononuclear cells) by incubation for 5 days at 37° C in an 95% air and 5% $CO_2$ incubator at an initial density of 1.3x10$^6$ cells per ml in the presence of 100 U recombinant human Interleukin-2 (IL-2). The antibody under study was diluted from the stock solution to the appropriate concentration in RPMI1640 cell culture medium.

1x10$^4$ radiolabelled tumour cells of the indicated cell line were incubated for 5 h at 37°C and 5% $CO_2$ in the presence of different concentrations of test or control antibody and MNC. MNC were added in amounts to reach the indicated effector:target cell ratio. Incubation was performed in U-shaped 96-well plates in a total volume of 200 µl RPMI1640 and done in duplicate.

After the incubation period, plates were centrifuged, 100 µl of the supernatant were taken off and radioactivity was determined in a gamma-counter. The total amount of incorporated radioactivity was determined by measuring 10$^4$ target cells. Spontaneous release was defined as activity released from the target cells in the absence of both antibody and effector cells during the described incubation period.
Specific lysis was calculated as follows:

$$\text{specific lysis (in \%)} = \frac{[\text{activity sample}] - [\text{activity spontaneous release}]}{[\text{maximum activity}] - [\text{activity spontaneous release}]} \times 100$$

Antibody-mediated complement lysis of tumor cells

No $L_AH_C$ -specific complement-mediated lysis (above that seen with an isotype control) was observed in HT-1080FAP clone 33 cells treated with $L_AH_C$ at concentrations up to 50 µg/ml (Table 14, Table 15a)

Lytic activity of human serum used as source of complement was shown by lysis of MCF-7 human breast carcinoma cells in the presence of 12.5 µg/ml 3S193, a murine monoclonal anti-Lewis$^y$ antibody with known complement activating ability (Table 15b)

Antibody-mediated cellular lysis of tumor cells

In the presence of $L_AH_C$ at concentrations up to 10 µg/ml, no ADCC (antibody-dependent cellular toxicity) mediated by human MNC (Table 16) or human LAK cells (lymphokine activated killer cell, Table 17) of $L_AH_C$ on HT-1080FAP clone 33 as measured by lysis was detectable above that seen with an isotype control at an effector:target ratio of 50:1.

In appropriate in vitro assays with either human complement or with human MNC - as effector mechanisms, human anti-FAP monoclonal antibody $L_AH_C$ revealed no - detectable cytotoxic effects above isotype controls on FAP-expressing tumor cell line HT-1080FAP clone 33.

**Table 14: Specific complement lysis (in %) of HT-1080FAP clone 33 tumor cell targets mediated by $L_AH_C$**

| Source of human serum: | HT-1080 clone 33: | |
|---|---|---|
| Concentration of antibody | hIgG1 isotype control | $L_AH_C$ |
| A 50 μg/ml | 5 | 4 |
| A 10 μg/ml | 5 | 3 |
| B 50 μg/ml | 7 | 5 |
| B 10 μg/ml | 6 | 5 |
| 0 μg/ml | 0 | 0 |
| Incubation: 2 hours at 37°C, 25% serum from human volunteers A or B, respectively, as source of complement. | | |

**Table 15a: Specific complement lysis (in %) of HT-1080FAP clone 33 tumor cell targets mediated by human anti-FAP monoclonal antibody $L_AH_C$**

| Source of human serum: | | HT1080ctone 33: | |
|---|---|---|---|
| concentration of antibody | | hIgG1 | $L_AH_C$ |
| A | 10.00 μg/ml | 2 | 1 |
| A | 2.50 μg/ml | 2 | 2 |
| A | 0.60 μg/ml | 1 | 1 |
| A | 0.15 μg/ml | 1 | 2 |
| A | 0.00 μg/ml | 2 | 2 |
| B | 10.00 μg/ml | 2 | 2 |
| B | 2.50 μg/ml | 2 | 2 |
| B | 0.60 μg/ml | 2 | 2 |
| B | 0. 15 μg/ml | 2 | 2 |
| B | 0.00 μg/ml | 2 | 2 |
| C | 10.00 μg/ml | 2 | 2 |
| C | 2.50 μg/ml | 1 | 1 |
| C | 0.60 μg/ml | 1 | 1 |
| C | 0.15 μg/ml | 2 | 1 |
| C | 0.00 μg/ml | 3 | 3 |
| Incubation: 2 hours at 37°C, 25% serum from human volunteers A, B or C, respectively, as source of complement. | | | |

**Table 15b: Specific complement lysis (in %) of MCF-7 tumour cell targets mediated by murine anti-Lewis$^y$ monoclonal antibody 3S193**

| Source of human serum: | | MCF-7: | |
|---|---|---|---|
| concentration of antibody | | mIgG | 3S193 |
| A | 10.00 μg/ml | 0 | 21 |
| A | 2.50 μg/ml | 1 | 21 |
| A | 0.60 μg/ml | 0 | 21 |
| A | 0.15 μg/ml | 1 | 18 |

(continued)

| Source of human serum: | | MCF-7: | |
|---|---|---|---|
| concentration of antibody | | mIgG | 3S193 |
| A | 0.00 μg/ml | 0 | 0 |
| B | 10.00 μg/ml | 1 | 13 |
| B | 2.50 μg/ml | 0 | 17 |
| B | 0.60 μg/ml | 1 | 18 |
| B | 0.15 μg/ml | 1 | 15 |
| B | 0.00 μg/ml | 0 | 0 |
| C | 10.00 μg/ml | 1 | 22 |
| C | 2.50 μg/ml | 0 | 23 |
| C | 0.60 μg/ml | 1 | 26 |
| C | 0.15 μg/ml | 1 | 20 |
| C | 0.00 μg/ml | 1 | 1 |
| Incubation: 2 hours at 37° C, 25% serum from human volunteers A, B or C, as source of complement. | | | |

**Table 16: ADCC (antibody-dependant cellular cytotoxicity) (specific lysis in %) of HT-1080FAP clone 33 target cells by human MNC (peripheral blood mononuclear cells) mediated by $L_AH_C$.**

| HT-1080FAP clone 33: | | |
|---|---|---|
| Concentration of antibody: | HT-1080FAP clone 33: | |
| [in μg/ml] | hIgG1 | $L_AH_C$ |
| 10.000 | 2 | 2 |
| 2.500 | 2 | 2 |
| 0.625 | 2 | 2 |
| 0.156 | 3 | 3 |
| 0.000 | 3 | . 3 |
| Incubation: 5 hours at 37°C, $10^4$ target cells and an effector:target cell ration of 50:1. | | |

**Table 17: ADCC (antibody-dependent cellular cytotoxicity, specific lysis in %) of HT-1080FAP clone 33 target cells by LAK cells (lymphokine activated killer cells) mediated by $L_AH_C$.**

| Concentration of antibody: | HT-1080FAP clone 33: | |
|---|---|---|
| [in μg/ml] | hIgG1 | $L_AH_C$ |
| 10.000 | 12 | 14 |
| 2.500 | 14 | 17 |
| 0.625 | 14 | 21 |
| 0.156 | 15 | 21 |
| 0.000 | 14 | 14 |
| Incubation: 5 hours at 37°C, $10^4$ target cells and an effector:target cell ration of 50:1. | | |

***Example.* 8: Immunohistochemical analysis of monoclonal antibody $L_AH_C$ binding to normal and neoplastic human tissues**

This experiment was performed to determine the binding characteristics of the humanized mAb $L_AH_C$ to normal and neoplastic human tissues.

The following antibodies were used: $L_AH_C$, cF19, and the negative control hIgG1 were directly biotinylated according to methods of the state of the art and used at concentrations of 2.5 to 0.25 mg/ml in 2% BSA/PBS (bovine serum albumin in phosphate-buffered saline). Murine mAb F19 was used as tissue culture supernatant of the F19 hybridoma, at dilutions of 1:5 to 1:10 in 2% BSA/PBS.

The following reagents were used for immunochemical assays: Streptavidin peroxidase complex (Vector Labs, Burlingame, CA, USA), Avidin-biotin peroxidase complex (Vector Labs.), Biotinylated horse anti-mouse (Vector Labs.), DAB (diaminobenzidine, Sigma Chemical Co. St. Louis, MO, USA), Harris' hematoxylin.

Fresh frozen tissue samples examined included the following: Normal colon, breast, lung, stomach, pancreas, skin, larynx, urinary bladder, smooth and skeletal muscle. Among the tumors tested were carcinomas from breast, colon, lung, esophagus, uterus, ovary, pancreas, stomach, and head and neck.

An indirect immunoperoxidase method was carried out according to state of the art methods (Garin-Chesa P, Old LJ, Rettig WJ: Cell surface glycoprotein of reactive stromal fibroblasts as a potential antibody target in human epithelial cancers. *Proc Natl Acad Sci USA* (1990) **87**:7235-7239) on five micrometer thickness fresh frozen sections. DAB was used as a substrate for the final reaction product. The sections were counterstained with Harris' hematoxylin and examined for antigen expression.

<u>$L_{AHC}$ expression in normal human tissues</u>

The normal tissues tested were negative for $L_AH_C$ expression, except for the normal pancreas in which a subset of positive endocrine cells in the islets of Langerhans (A cells) were identified with $L_AH_C$, cF19 and F19. (Table 18). No immunoreactivity was observed with the hIgG1 (human immunoglobulin IgG1 subclass) used as a negative control.

<u>$L_AH_C$ expression in tumors</u>

In the tumor samples, $L_AH_C$, cF19 and F19 showed an indistinguishable pattern of expression in the tumor stromal fibroblasts. A strong and homogeneous expression was found in the majority of the cases examined, especially in the cancer samples derived from breast, colon, lung, pancreas and in the squamous cell carcinomas (SQCC) of the head and neck tested (Table 19). No immunoreactivity was observed with the hIgG1 used as negative control.

$L_AH_C$, cF19 and F19 showed immunoreactivity with the tumor stromal fibroblasts in the epithelial cancer samples tested. No $L_AH_C$ or F19 immunoreactivity was seen with either the fibrocytes of the normal organ mesenchyme or the parenchymal cells of normal adult organs. Anti-FAP immunoreactivity was only observed in a subset of endocrine cells in the pancreatic islets, presumably glucagon-producing A cells, and in four of nine uterine samples tested, representing subsets of stromal fibroblasts in these tissues.

Immunohistochemical analysis of $L_AH_C$ in normal human tissues and FAP-expressing human carcinomas showed indistinguishable patterns of binding for $L_AH_C$, cF19 and murine mAb F19.

**Table 18: Immunoreactivity of mAbs $L_AH_C$, cF19 and F19 with normal human tissues**

| Tissue type | No. | BIBH 1 | cF19 | F19 |
|---|---|---|---|---|
| Breast | 4 | | | |
| -Epithelial cells ducts / acini | | - | - | - |
| -Myoepithelial cells | | - | - | - |
| -Connective tissue | | - | - | - |
| -Blood vessels | | - | - | - |
| Colon | 6 | | | |
| -Crypts of Lieberkuhn | | - | - | - |
| -Connective tissue | | - | - | - |
| -Lymphoid tissue | | - | - | - |
| -Smooth muscle | | - | - | - |
| -Blood vessels | | - | - | - |
| -Myenteric plexus | | - | - | - |

(continued)

| Tissue type | No. | BIBH 1 | cF19 | F19 |
|---|---|---|---|---|
| Lung | 4 | | | |
| Bronchus: | | | | |
| -Bronchial epithelium | | - | - | - |
| -Hyaline cartilage | | - | - | - |
| -Connective tissue | | - | - | - |
| -Mucous glands Alveolus: | | - | - | - |
| -Pneumocytes (type I/II) | | - | - | - |
| -Alveolar phagocytes | | - | - | - |
| -Alveolar capillaries | | - | - | - |
| Stomach | 3 | | | |
| -Surface epithelium | | - | - | - |
| -Gastric glands: | | - | - | - |
| -Chief cells | | - | - | - |
| -Parietal (oxyntic) cells | | - | - | - |
| -Mucous cells | | - | - | - |
| -Neuroendocrine cells | | - | - | - |
| -Connective tissue | | - | - | - |
| -Blood vessels | | | | |
| -Smooth muscle | | | | |
| Esophagus | 1 | | | |
| -Surface epithelium | | - | - | - |
| -Connective tissue | | - | - | - |
| Small intestine | 1 | | | |
| -Epithelium of villi&crypts | | - | - | - |
| -Connective tissue | | - | - | |
| -Smooth muscle | | - | - | - |
| -Blood vessels | | - | - | - |
| -Lymphoid tissue | | - | - | - |
| Urinary bladder | 2 | | | |
| -Urothelium | | - | - | - |
| -Connective tissue | | - | - | - |
| -Smooth muscle | | - | - | - |
| -Blood vessels | | - | - | - |
| Pancreas | 3 | | | |
| -Duct epithelium | | - | - | - |
| -Acinar epithelium | | - | - | - |
| -Islets of Langerhans: | | - | - | - |
| -B-cells | | - | - | - |
| -A-cells | | +* | +* | +* |
| -D-cells | | - | - | - |
| -Connective tissue | | - | - | - |
| -Blood vessels | | - | - | - |
| -Nerves | | - | - | - |

(continued)

| Tissue type | No. | BIBH 1 | cF19 | F19 |
|---|---|---|---|---|
| Larynx | 1 | | | |
| -Squamous epithelium | | - | - | - |
| -Mucous glands | | - | - | - |
| -Connective tissue | | - | - | - |
| -Hyaline cartilage | | - | - | - |
| -Blood vessels | | - | - | - |
| -Skeletal muscle | | - | - | - |
| Lymph node | 1 | | | |
| -Lymphoid cells | | - | - | - |
| -Lymph sinuses | | - | - | - |
| -Connective tissue | | - | - | - |
| -Blood vessels | | - | - | - |
| Spleen | 1 | | | |
| -Red & white pulp | | - | - | - |
| -Sinuses | | - | - | - |
| -Connective tissue | | - | - | - |
| Liver | 1 | | | |
| -Hepatocytes | | - | - | - |
| -Bile ducts | | - | - | - |
| -Portal triad | | - | - | - |
| Thyroid gland | 2 | | | |
| -Follicular epithelium | | - | - | - |
| -Parafollicular cells | | - | - | - |
| -Connective tissue | | - | - | - |
| Prostate gland | 1 | | | |
| -Glandular epithelium | | - | - | - |
| -Stroma | | - | - | - |
| Testicle | 1 | | | |
| -Seminiferous tubules | | - | - | - |
| -Stroma | | - | - | - |
| Ovary | 3 | | | |
| -Follicles | | - | - | - |
| -Stroma | | - | - | - |
| Uterine cervix | 1 | | | |
| -Epithelium | | - | - | - |
| -Stroma | | - | - | - |
| Uterus | 9 | | | |
| -Endometrium: | | | | |
| glands | | - | - | - |
| stroma | | +# | +# | +# |
| blood vessels | | - | - | - |
| -Myometrium | | - | - | - |
| Cerebral cortex | 1 | | | |
| -Neurons | | - | - | - |
| -Neuroglial cells | | - | - | - |
| -Blood vessels | | - | - | - |

(continued)

| Tissue type | No. | BIBH 1 | cF19 | F19 |
|---|---|---|---|---|
| Cerebellum | 1 | | | |
| -Molecular layer | | - | - | - |
| -Granular cell layer | | - | - | - |
| -Purkinje cells | | - | - | - |
| -Blood vessels | | - | - | - |
| Skin | 3 | | | |
| -Squamous epithelium | | - | - | - |
| -Melanocytes | | - | - | - |
| -Skin appendages | | - | - | - |
| -Connective tissue | | - | - | - |
| -Blood Vessels | | - | - | - |

Acetone-fixed frozen sections were tested by the avidin-biotin complex immunoperoxidase procedure.
No, Number of tissue samples derived from different individuals tested.
* identification of A-cells, based on morphology and location within the islets.
# Positive immunoreactivity in the stroma in 4 out of 9 samples tested. The positive samples represent early (x2) and intermediate (x2) phase proliferative endometrium.

***Example* 9: Species specificity of $L_AH_C$ binding in tissue sections**

This experiment was conducted to assess the reactivity of $L_AH_C$ with tissues from mouse, rat, rabbit and cynomolgus monkeys by immunohistochemical methods.

Also used in these tests were cF19 and human IgG1 (hlgG1) as negative controls. The reagents used for immunohistochemistry were Streptavidin peroxidase complex (Vector Labs., Burlingame, CA, USA), DAB (Sigma Chemical Co., St. Louis, MO, USA) and Harris' hematoxylin.

The following fresh frozen tissue samples from mouse, rat, rabbit and cynomolgus were tested: Brain, liver, lung, kidney, stomach, pancreas, intestine, thymus, skin, muscle, heart, spleen, ovary, uterus and testes. As positive control, sections from normal human pancreas and a breast carcinoma sample were included in every assay.

Immunohistochemistry

An indirect immunoperoxidase method was carried out as described in the state of the art (Garin-Chesa P, Old LJ, Rettig WJ: Cell surface glycoprotein of reactive stromal fibroblasts as a potential antibody target in human epithelial cancers. *Proc Natl Acad Sci USA* (1990) **87**:7235-7239) on five micrometer thickness fresh frozen sections. The antibodies $L_AH_C$, cF19 and hlgG1 (at 1 μg/ml) were biotinylated according to the state of the art and were detected with streptavidin peroxidase complex. DAB was used as a substrate for the final reaction product. The sections were counterstained with Harris' hematoxylin and examined for antigen expression.

The normal tissues tested did not react with either $L_AH_C$ or cF19 in the experiments (Table 1).

The normal human pancreas used as positive control showed $L_AH_C$ and cF19 binding in a subset of endocrine cells in the islets of Langerhans as previously described for F19. In addition, binding of $L_AH_C$ and cF19 was seen in the tumor stromal fibroblasts in the breast carcinoma sample.

Immunohistochemical analysis of normal tissues from mouse, rat, rabbit and cynomolgus failed to detect any binding of either $L_AH_C$ or cF19, in the experiments performed.

**Table 20: Binding of $L_AH_C$ to tissue sections of non-human species, as determined by immunohistochemistry.**

| Organ / Tissue type | Mouse | Rat | Rabbit | Cynomolgus |
|---|---|---|---|---|
| Brain -Cerebral cortex | - | - | - | |
| -Cerebellum | - | - | - | - |
| Liver -Hepatocytes | - | - | - | - |

(continued)

| Organ / Tissue type | Mouse | Rat | Rabbit | Cynomolgus |
|---|---|---|---|---|
| -Portal triad | - | - | - | - |
| Lung-Bronchi<br>-Alveoli | -<br>- | -<br>- | -<br>- | -<br>- |
| Kidney -Glomeruli<br>-Tubular epithelium | -<br>- | -<br>- | -<br>- | -<br>- |
| Stomach -Glandular epithelium<br>-Smooth muscle | - | - | - | - |
| Pancreas -Exocrine acini<br>-Endocrine islets | -<br>- | -<br>- | -<br>- | -<br>- |
| Intestine -Glandular epithelium<br>-Smooth muscle | -<br>- | -<br>- | -<br>- | -<br>- |
| Thymus -Lymphocytes | - | - | - | - |
| Skin -Keratinocytes<br>-Sweat glands<br>-Hair follicles | -<br>-<br>- | -<br>-<br>- | -<br>-<br>- | -<br>-<br>- |
| Skeletal muscle | - | - | - | - |
| Heart | - | - | - | - |
| Spleen -Lymphocytes | - | - | - | - |
| Ovary -Follicular epithelium<br>-Stroma | -<br>- | -<br>- | -<br>- | -<br>- |
| Uterus -Myometrium<br>-Cervix uteri | -<br>- | -<br>- | -<br>- | -<br>- |
| Testis -Tubular epithelium | nt | nt | nt | - |
| Connective tissue | - | - | - | - |
| nt, not tested | | | | |

***Example 10:*** **Construction of cell lines producting chimeric and reshaped anti-FAP monoclonal antibodies**

The objective of this experiment was to demonstrate stable cell lines according to the invention expressing $L_AH_C$, $L_AH_A$, $L_BH_B$, $L_B$-$H_D$, and cF19 in CHO DG44 cells. Stable cell lines transfected with humanized or chimeric F19 antibodies were produced and their identity was confirmed by PCR amplification of heavy and light variable regions using genomic DNA derived from each transfectant as template.

CHO DG44 cells maintained under serum-free conditions in SFM-II medium. Lipofectin and SFM-II serum-free medium were obtained from Gibco/BRL. Geneticin and all restriction enzymes were obtained from Boehringer Mannheim. Pfu polymerase was obtained from Stratagene.

DNA for transfections was purified from E. coli cells using QiaFilter Maxi Cartridges (Qiagen) as directed by the manufacturer. All DNA preparations were examined by restriction enzyme digestion. Sequences of $L_AH_C$ variable regions in their respective vectors were confirmed using an ABI PRISM 310 Sequencer (Perkin-Elmer).

Further information regarding the vectors and DNA sequences employed is available in the prior examples.

Transfection of CHO DG44 cells

Cells in logarithmic growth were plated into 6 well plates containing 1 ml fresh SFM-II medium. Plasmids encoding heavy and light chains of humanized or chimeric F19 versions were cotransfected into CHO DG44 cells using liposomal transfection. Liposomes were prepared using 6 μl Lipofectin reagent and 0.5 μg of each vector (one for the desired heavy chain and one for the light) as described for LipofectAMINE transfections except that SFM-II medium was used to dilute all reagents. Twenty-four hours later, cells were diluted 1:10 into SFM-II medium containing 300 μg/ml

Geneticin. After the initial phase of cell killing was over (10-14 days), the concentration of Geneticin was reduced to 200 mg/ml and methotrexate was added to a final concentration of 5 nM. Methotrexate concentrations were increased after 10-14 days to a final concentration of 20 nM.

PCR Amplification of transfectant DNA

$10^7$ CHO DG44 cells were centrifuged in an Eppendorf microcentrifuge briefly at full speed, washed once with PBS, and pelleted once again. Genomic DNA was prepared by ethanol precipitation after SDS lysis and Proteinase K treatment of the cell pellets.

A mixture containing one of the following primer pairs, dNTPs, buffer, and Pfu polymerase was used to amplify either the heavy or light chain variable region using genomic DNA as template. The resulting PCR products were digested with the appropriate restriction enzyme and analyzed by agarose gel electrophoresis to confirm their identity.

Light chain primer set:
5'- GAG ACA TTG TGA CCC AAT CTC C - 3' PKN 1690
5'- GAC AGT CAT AAA CTG CCA CAT CTT C - 3' PKN.1930.R

Heavy chain primer set:
5'- TTG ACA CGC GTC TCG GGA AGC TT - 3' PG 5863
5'- GGC GCA GAG GAT CCA CTC ACC T - 3' PG 6332.R

The undigested heavy chain PCR product has a predicted size of 469 bp while the light chain PCR product has a predicted size of 286 bp. Verification of identity was determined by restriction enzyme digest with BstEII (heavy chain) or NIaIV (light chain).

CHO cell lines were transfected with $L_AH_C$, $L_AH_A$, $L_BH_B$, $L_BH_D$, as well as cF19. Geneticin-resistant cells were obtained and these cells were further selected for resistance to methotrexate. PCR amplification, followed by restriction enzyme cleavage of the light and heavy chain DNA produced the expected bands and confirmed the identity of $L_AH_C$, $L_BH_B$, $L_AH_A$ and $L_BH_D$ transfectants..

The cells described were maintained under serum-free conditions at all times and were not treated with animal-derived products such as trypsin.

Producer cell lines transfected with expressing monoclonal $L_AH_C$, $L_AH_A$, $L_BH_B$, $L_BH_D$ and cF19 antibodies were produced. Their identities were confirmed using PCR amplification and restriction enzyme cleavage of the resulting PCR products of both their heavy and light chain variable regions.

***Example* 11: Expression of antibody proteins in Chinese hamster ovary DG44 cells and their purification**

The objective of this experiment was to express and purify $L_AH_C$, $L_AH_A$, $L_BH_B$, and $L_BH_D$ mAbs to enable their characterization. Other goals included the establishment of a quantitative ELISA to permit measurement of antibody concentrations in both crude media samples as well as purified Ig samples and determination of relative expression levels of various humanized F19 constructs using this assay.

Serum-free CHO DG44 cells and USP-grade methotrexate were obtained from the Biotechnical Production Unit of the Dr. Karl Thomae GmbH, Biberach, Germany; both products are also commercially available. Cells were maintained under serum-free conditions at all times. SFM-II serum-free medium was obtained from Gibco/BRL. Protein A agarose was from Pierce Chemical (Indianapolis, IN, USA). Human IgG1 standards (Cat. No. I 3889), p-Nitrophenyl phosphate tablets (N 2640), bovine serum albumin (BSA) (A 7906), and goat anti-human kappa chain specific alkaline phosphatase-conjugated antibody (A 3813) were obtained from Sigma Chemical (St. Louis, MO, USA). Goat anti-human gamma-chain specific alkaline phosphatase-conjugated antibody was obtained from Jackson Immunoresearch Laboratories (through Stratech Scientific). Tris-buffered saline (TBS) consisted of 150 mM NaCl, 50 mM Tris, pH 7.5.

Cell culture conditions for antibody expression

Cells were cultured and maintained in T-175 flasks in SFM-II serum-free medium without agitation. The medium contained 200 μg/ml Geneticin and 20 nM methotrexate without antibiotics. Cells were passaged by dilution, were not adherent, and grew in small clusters. When the cells reached stationary phase, the medium was collected and centrifuged to remove cells and frozen at -20°C until needed.

Purification of $L_AH_C$

All purification steps were carried out at 4°C. A C10/10 column (Pharmacia Fine Chemicals) was packed with Protein A agarose (3 ml bed volume). The column was washed with TBS and preeluted once with 0.1 M Na citrate, pH 3.0 to insure that no loosely bound material remained on the column. The column was then immediately reequilibrated with TBS and stored at 4°C. Spent culture supernatants were thawed and centrifuged at 10,000 x g for 30 minutes prior to Protein A chromatography to remove debris and diluted with an equal volume of TBS. This material was loaded onto the Protein A column at 0.5 ml/min using a P-1 peristaltic pump (Pharmacia) and washed with TBS until the absorbance at 280 nm was undetectable. Elution of the antibody was initiated with 0.1 M Na citrate pH 3.0 at approximately 0.2 ml/min. The elution was monitored at 280 nm and one ml fractions of the eluted material were collected into tubes containing sufficient Tris base pH 9 to neutralize the citrate buffer. Protein-containing fractions were pooled and concentrated using an Amicon filtration apparatus with a YM-30 filter and dialyzed against PBS. The column was immediately regenerated with TBS. Protein dye-binding assays were performed with the BioRad (Hercules, California) protein determination kit, according to the manufacturer's instructions, using bovine serum albumin as a standard.

Human IgG (gamma immunoglobulin) ELISA

ELISA plates were coated overnight with 100 μl goat anti-human gamma-chain specific alkaline phosphatase-conjugated antibody at 0.4 mg/ml in coating buffer at 4°C. Coating antibody was removed and plates were blocked with 2% BSA in PBS for 2 hours. All subsequent steps were performed at 37°C. Blocking buffer was replaced with antibody samples or human IgG1 standard diluted in dilution buffer, serially diluted in a 200 ml volume, and incubated for one hour. Negative controls included dilution buffer and/or culture medium of nontransfected cells. Wells were washed and 100 μl goat anti-human kappa chain specific alkaline phosphatase-conjugated antibody diluted 1:5000 was added and incubated for one hour. Wells were washed and 100 μl reaction buffer was added and incubated for 30 minutes. The reaction was stopped by addition of 1 M NaOH and absorbance read at 405 nm in an ELISA plate reader. Results were analyzed by four-parameter iterative curve fitting.

Amino acid analysis was performed according to methods available in the state of the art.

Monoclonal antibody $L_AH_C$ was produced and purified to homogeneity using Protein A affinity chromatography. ELISA assays using human IgG1 as standard indicated $L_AH_C$ recoveries exceeding 70%. The purity of the material was estimated to be >90% by SDS-polyacrylamide gel electrophoresis. Representative expression data and typical purification yields are shown in Table 21.

**Table 21: Expression data and purification yields FAP antibody proteins in CHO cells**

| Antibody | Expression levels in crude media samples (ELISA) | Purified antibody yields | Yield improvement [purified antibody] |
|---|---|---|---|
| $L_AH_C$ | 7 - 10 mg/l | ~ 5 - 7 mg/l | 500 - 700 |
| $L_AH_A$ | 5 - 7 mg/l | ~ 3 - 4 mg/l | 300 - 400 |
| $L_BH_B$ | 0.5 - 1 mg/l | ~ 0.2 - 0.5 mg/l | 20 - 50 |
| $L_BH_D$ | 0.8 - 1.5 mg/l | ~ 0.3 - 0.8 mg/l | 30 - 60 |
| Chimeric F19 | ~ 0.02 mg/l | < 0.01 mg/l | 1 |

Representative expression data for each of the anti-FAP antibodies produced in this study are shown. Recoveries after Protein A agarose affinity chromatography were based on protein dye-binding measurements of the purified Ig using BSA as a standard.

### *Example 12:* Binding of monoclonal antibody $L_AH_C$ to isolated recombinant human FAP

The objective of this study was to characterize binding of $L_AH_C$ to isolated recombinant human FAP.

CD8-FAP ELISA

ELISA plates were coated overnight with 100 μl of mouse anti-rat antibody (Sigma Chemical R0761) at 1:2000 in coating buffer at 4°C. Coating antibody was removed and plates were blocked with 2% BSA in PBS for one hour. All subsequent steps were performed at room temperature. Blocking buffer was replaced with 100 ml of 1 μg/ml rat anti-CD8 antibody (Pharmingen 01041 D) and incubated for one hour. Plates were washed and 100 μL CD8-FAP culture

supernatant (see Example 14) (1:2 in PBS) was added and allowed to bind for one hour. Plates were washed and antibody samples were added (two-fold serial dilutions) in a 100 μl volume and incubated for one hour. Negative controls included human IgG and/or culture medium of nontransfected cells. Wells were washed and 100 μl of horseradish peroxidase (HRP) conjugated mouse anti-human IgG1 antibody (Zymed 05-3320) diluted 1:500 in dilution buffer were added and incubated for one hour. Wells were washed and 100 μl HRP substrate, (azino-bis (3-ethylbenzthiazoline 6-sulfonic) acid, Sigma Chemical A9941), were added and incubated for 60 minutes. The reaction was stopped by addition of 1 M NaOH and absorbance read at 405/490 nm in an ELISA plate reader. Results were analyzed by four-parameter curve iterative curve fitting.

Alternatively, plates were coated directly with cF19. FAP (recombinant human FAP, see example 13) was allowed to bind to these plates as above and biotinylated $L_AH_C$ (~1 μg/ml) was then added. Antibody binding was detected with HRP-streptavidin conjugate as above.

Solubilization of membrane-bound human FAP

FAP-expressing 293FAP 1/2 cells or control 293 cells were washed with PBS and lysed with 1% Triton X-114 in Tris-buffered saline. Nuclei and debris were removed by centrifugation at 10,000 x g. The supernatant was phase-partitioned (Estreicher A, Wohlend A, Belin D, Scheuning WD Vasalli JD. Characterization of the cellular binding site for the urokinase-type plasminogen activator. (1989) *J Biol Chem* **264**:1180-1189) to enrich membrane proteins. The detergent phase was collected and diluted in buffer containing 1 % Empigen BB (Calbiochem) to prevent reaggregation of the Triton X-114. This material was subjected to Concanavalin A agarose chromatography (Rettig WJ, Garin-Chesa P, Healey JH, Su SL, Ozer HL, Schwab, M, Albino AP, Old LJ. Regulation and heteromeric structure of the fibroblast activation protein in normal and transformed cells of mesenchymal and neuroectodermal origin. (1993) *Cancer Res* **53**: 3327-3335).

Biotinylation of $L_AH_C$

$L_AH_C$ (1-2 mg) was dialyzed against 50 mM bicarbonate buffer and biotinylated with a ten-fold molar excess of sulfosuccinimidyl-6-biotinamido hexanoate (NHS-LC biotin, Pierce Chemical, Rockford, Illinois, USA) for 2 hours at room temperature. Unreacted product was removed by repeated microdialysis in a microconcentrator.

Transient transfections

COS-7 cells (American Type Tissue Culture Collection, reference number CRL 1651) were cotransfected by electroporation with the heavy and light chain vectors encoding $L_AH_C$.

Anti-CD8 monoclonal antibody was immobilized onto microtiter plates. CD8-FAP from medium of insect cells infected with CD8-FAP baculovirus was allowed to bind to these plates. Spent medium from COS-7 cell cultures transiently transfected with two separate vectors encoding $L_AH_C$ was serially diluted and added to the wells containing the immobilized CD8-FAP. $L_AH_C$ bound to isolated immobilized CD8-FAP protein (Figure 35). Culture supernatants from mock-transfected COS-7 cells failed to demonstrate binding.

Recombinant membrane-bound FAP from detergent extracts of 293FAP I/2 cells or control extracts was serially diluted and immobilized via chimeric F19 monoclonal antibody bound to microtiter plates. Biotinylated $L_AH_C$ bound recombinant human FAP immobilized with cF19 (Figure 36) in a concentration-dependent manner.

$L_AH_C$ recognized isolated immobilized recombinant human FAP carrying the epitope for murine F19. $L_AH_C$ bound to both CD8-FAP produced in insect cells, as well as FAP protein produced in 293FAP 1/2 cells.

Culture supernatants from COS7 cells transfected with either heavy and light chain vectors encoding $L_AH_C$ or without DNA (Control) were collected three days posttransfection. CD8-FAP was immobilized via an anti-CD8 antibody as described in the text. Serial dilutions of the COS7 supernatants were allowed to bind to the immobilized CD8-FAP and subsequently detected with an HRP-conjugated anti-human IgG1 antibody.

Detergent extracts of FAP-expressing 293FAP 1/2 cells or control 293 cells were serially diluted and added to cF19-coated microtiter plates. Biotinylated $L_AH_C$ was added and binding of biotinylated $L_AH_C$ was detected with HRP-conjugated streptavidin.

***Example 13:* Characterization of HT-1080 fibrosarcoma cells and 293 human embryonic kidney cells transfected with cDNA for human FAP**

Fibroblast activation protein (FAP) is a cell-surface, membrane-bound protein which carries the F19 epitope and is expressed on tumor stromal fibroblasts. Cell lines expressing recombinant FAP protein and matched controls lacking FAP were generated for the characterization of anti-FAP monoclonal antibodies.

Cells used were HT-1080 cells (reference number CCL 121) and 293 human embryonic kidney cells (reference number CRL 1573) were obtained from the American Type Culture Collection (Maryland, USA). Transfectam was obtained from Promega (Madison, WI). Geneticin and all restriction enzymes were obtained from Boehringer Mannheim. DNA for transfections was purified from E. coli cells using QiaFilter Maxi Cartridges (Qiagen) as directed by the manufacturer. All DNA preparations were examined by restriction enzyme digestion. Vector sequences were confirmed using an ABI PRISM 310 Sequencer.

Further information regarding the vectors and DNA sequences employed has been described in Scanlan MJ, Raj BK, Calvo B, Garin-Chesa P, Sanz-Moncasi MP, Healey JH, Old LJ, Rettig WJ. Molecular cloning of fibroblast activation protein alpha, a member of the serine protease family selectively expressed in stromal fibroblasts of epithelial cancers, (1992) *Proc Natl Acad Sci USA* **89**:10832-10836. The FAP cDNA sequence has been deposited in Genbank (accession number HS09287).

Cell culture and immunoassays

HT-1080 cells were transfected with 1 mg DNA using Transfectam according to the manufacturer's instructions. Human embryonic kidney 293 cells were transfected by calcium phosphate transfection (Brann MR; Buckley NJ; Jones SVP; Bonner TI. Expression of cloned muscarinic receptor in A9 L cells. (1987) *Mol Pharmacol* **32**: 450-455) with 10 mg DNA. Twenty-four hours later, cells were diluted 1:10 into fresh medium containing 200 mg/ml Geneticin. Colonies were picked and examined by immunofluorescence for FAP expression as described in Rettig WJ; Garin-Chesa P; Beresford HR; Oettgen HF; Melamed MR; Old LJ. Cell-surface glycoproteins of human sarcomas: differential expression in normal and malignant tissues and cultured cells.(1988) *Proc Natl Acad Sci USA* **85**: 3110-3114.

Immunoprecipitations with cF19 were performed with metabolically labelled cells as described in Rettig WJ, Garin-Chesa P, Healey JH, Su SL, Ozer HL, Schwab, M, Albino AP, Old LJ. Regulation and heteromeric structure of the fibroblast activation protein in normal and transformed cells of mesenchymal and neuroectodermal origin. (1993) *Cancer Res* **53**:3327-3335.

HT-1080 and 293 cells were tested for FAP antigen expression in immunofluorescence assays with anti-FAP antibodies and were found to be antigen-negative. Transfection of these cells with FAP.38 vector resulted in the generation of Geneticin-resistant colonies. Isolated colonies were picked and analyzed by immunofluorescence for FAP expression. Two cell clones were identified, designated HT-1080FAP clone 33 and 293FAP 1/2, which express cell surface-bound FAP protein, as recognized by cF19 antibody. Staining of nonpermeabilized HT-1080FAP clone 33 cells and 293FAP 1/2 with cF19 antibody confirmed the cell surface localization of the FAP protein.

Immunoprecipitation of radiolabelled FAP protein with cF19 from extracts of $^{35}$S-methionine labelled HT-1080FAP clone 33 cells or 293FAP 1/2 cells resulted in the appearance of a 93 kilodalton band after autoradiography. This band is not detectable in immunoprecipitates of parental HT-1080 or 293 cell extracts.

Two stably transfected cell lines, HT-1080FAP clone 33 and 293FAP l/2, express FAP on the cell surface as determined in immunological assays with anti-FAP mAbs. Neither parental HT-1080 cells nor parental 293 cells express detectable levels of FAP.

### *Example 14:* Generation and characterization of CD8-FAP fusion protein

A soluble form of human FAP (fibroblast activation protein) in the form of a CD8-FAP fusion protein was produced in insect cells for the characterization of $L_AH_C$ containing the binding site for anti-FAP mAbs. Murine CD8 was chosen to permit secretion of the protein and to provide an additional epitope tag.

The cDNA encoding the extracellular domain of CD8, consisting of the first 189 amino acids of murine CD8$\alpha$ (Genbank M12825), was linked to that of the extracellular domain of FAP (amino acids 27 to 760), essentially as described by Lane, et al. (Lane P, Brocker T, Hubele S, Padovan E, Lazavecchia A, McConnell. Soluble CD40 ligand can replace the normal T cell-derived CD40 ligand signal to B cells in T cell-dependent activation. (1993) *J Exp Med* **177:**1209-1213) using standard PCR protocols. The authenticity of all clones was verified by DNA sequencing. The resulting DNA was inserted into the pVL1393 vector (Invitrogen) and transfection of Sf9 cells (Invitrogen) with this vector and amplification of the resulting recombinant baculovirus were performed as described (Baculovirus Expression Vectors. A Laboratory Manual. O'Reilly DR, Miller LK, Luckow VA, (Eds.), Oxford University Press: New York, 1994). The spent medium of High Five™ cells (Invitrogen) infected with recombinant CD8-FAP baculovirus for four days was collected and cleared by ultracentrifugation.

The CD8-FAP ELISA (enzyme-linked immunosorbent assay) has been described above (Example 12).

Insect cell cultures infected with CD8-FAP virus secreted a fusion protein into the medium which carries the F19 epitope and is recognized by an anti-FAP antibody (Figure 1). Neither the cell culture medium alone nor medium from insect cells infected with CD8-CD40L fusion protein bound anti-FAP antibody.

Soluble CD8-FAP protein carrying the F19 epitope was secreted into the medium of infected insect cell cultures.

Culture supernatant from cells infected with a control construct did not contain antigen bearing the F19 epitope.

A soluble form of FAP, CD8-FAP, was produced in insect cells and CD8-FAP was shown to carry the epitope recognized by cF19.

Supernatants from insect cells infected with recombinant baculovirus encoding either CD8-FAP or CD8-CD40L fusion protein were collected four days postinfection. Cell culture medium without cells was used as an additional control (medium). Serial dilutions of these materials were added to anti-CD8 antibody-coated microtiter plates and allowed to bind. cF19 (1 mg/ml) was subsequently added and allowed to bind. Bound cF19.was detected with horseradish peroxidase-conjugated anti-human antibody.

## Claims

1. An antibody protein having the complementary determining regions of the monoclonal antibody F19 (ATCC Accession No. HB 8269), said antibody protein specifically binding to fibroblast activation protein, **characterized in that** it contains the variable region of the light chain as set forth in SEQ ID NO: 2 or SEQ ID NO: 6.

2. An antibody protein of claim 1 **characterised in that** the variable region of the light chain is encoded by a nucleotide sequence as set forth in SEQ ID NO: 1 or SEQ ID NO: 5.

3. An antibody protein according to any one of claims 1 or 2 containing a variable region of the heavy chain as set forth in any one of SEQ ID NOs: 8, 10, 12, 14,

4. An antibody protein according to claim 3 **characterised in that** the variable region of the heavy chain is encoded by a nucleotide sequence as set forth in SEQ ID NOs: 7, 9, 11, 13.

5. An antibody protein according to any one of claims 1 to 4 containing the variable region of the light chain as set forth in SEQ ID NO: 2 and the variable region of the heavy chain as set forth in SEQ ID NOs: 12.

6. The antibody protein of claim 5 **characterised in that** the variable region of the light chain is encoded by a nucleotide sequence as set forth in SEQ ID NO: 1 and the variable region of the heavy chain is encoded by a nucleotide sequence as set forth in SEQ ID NO: 11.

7. An antibody protein according to claim 5 or claim 6 containing the constant region of the light chain as set forth in SEQ ID NO: 20 and the constant region of the heavy chain as set forth in SEQ ID NO: 22.

8. An antibody protein according to any one of claims 1 to 4 containing the variable region of the light chain as set forth in SEQ ID NO; 2 and the variable region of the heavy chain as set forth in SEQ ID NOs: 8.

**9.** The antibody protein of claim 8 **characterised in that** the variable region of the light chain is encoded by a nucleotide sequence as set forth in SEQ ID NO: 1 and the variable region of the heavy chain is encoded by a nucleotide sequence as set forth in SEQ ID NO: 7.

**10.** A nucleotide sequence encoding an antibody protein according to any one of claims 1 to 9.

**11.** A recombinant DNA vector that contains a nucleotide sequence of claim 10.

**12.** The recombinant DNA vector of claim 11, said vector being an expression vector.

**13.** A host cell carrying a vector according to claims 11 or 12.

**14.** The host cell of claim 13, wherein said host cell is a eukaryotic cell.

**15.** The host cell of claim 14, wherein said eukaryotic host cell is a mammalian cell.

**16.** The host cell of claim 15. wherein said host cell is a CHO or a COS cell.

**17.** A method of producing antibody proteins according to any one of claims 1 to 9, said method comprising the steps of:

(a) cultivating a host cell according to any one of claims 13 to 16 under conditions where said antibody protein is expressed by said host cell, and
(b) isolating said antibody protein.

**18.** The method of claim 17, wherein said host cell is a mammalian cell, preferably a CHO or COS cell.

**19.** The method of claim 17 or 18, wherein said host cell is cotransfected with two plasmids carrying the expression units for light and heavy chains respectively.

**20.** An antibody protein according to any one of claims 1 to 9, wherein said antibody protein is conjugated to a therapeutic agent.

**21.** The antibody protein of claim 20, wherein said therapeutic agent is a therapeutic agent selected from the group consisting of radioisotopes, toxins, toxoids, inflammatory agents and chemotherapeutic agents.

**22.** The antibody protein of claim 21. wherein said radioisotope is a β-emitting radioisotope.

**23.** The antibody protein of claim 22, wherein said radioisotope is selected from the group consisting of $^{186}$Rhenium, $^{188}$Rhenium, $^{131}$Iodine and $^{90}$Yttrium.

**24.** An antibody protein according to any one of claims 1 to 9, **characterised in that** it is labelled.

**25.** The antibody protein of claim 24, wherein said label is a detectable marker.

**26.** The antibody protein of claim 25, wherein the detectable marker is a detectable marker selected from the group consisting of enzymes, dyes, radioisotopes, digoxygenin, and biotin.

**27.** An antibody protein according to any one of claims 1 to 9 conjugated to an imageable agent.

**28.** The antibody protein of claim 27, wherein the imageable agent is a radioisotope.

**29.** The antibody protein of claim 28, wherein said radioisotope is a y-emitting radioisotopes.

**30.** The antibody protein of claim 29, wherein said radioisotope is $^{125}$I.

**31.** A pharmaceutical composition containing an antibody protein according to any one of claims 1 to 9 and a pharmaceutically acceptable carrier.

32. A pharmaceutical composition containing an antibody protein according to any one of claims 20 to 23 and a pharmaceutically acceptable carrier.

33. A pharmaceutical composition containing an antibody protein according to any one of claims 27 to 30 and a pharmaceutically acceptable carrier.

34. The pharmaceutical composition of claims 31 to 33, for use in the treatment or imaging of tumors, wherein said tumors are associated with activated stromal fibroblasts, preferably wherein said tumors are tumors selected from the cancer group consisting of colorectal cancers, non-small cell lung cancers, breast cancers, head and neck cancer, ovarian cancers, lung cancers, bladder cancers, pancreatic cancers and metastatic cancers of the brain.

35. Use of an antibody protein according to anyone of claims 1 to 9 for the manufacture of a medicament for the treatment of cancer.

36. Use of an antibody protein according to anyone of claims 20 to 23 for the manufacture of a medicament for the treatment of cancer.

37. Use of an antibody protein according to anyone of claims 27 to 30 for the manufacture of a medicament for imaging activated stromal fibroblasts.

38. Use of an antibody protein according to anyone of claims 24 to 26 for detecting the presence of activated stromal fibroblasts in a sample.

39. The use of claim 35 or 36, wherein the cancer is selected from the cancer group consisting of colorectal cancers, non-small cell lung cancers, breast cancers, head and neck cancer, ovarian cancers, lung cancers, bladder cancers, pancreatic cancers and metastatic cancers of the brain.

40. A method of detecting the presence of activated stromal fibroblasts in wound healing, inflammation or a tumor, **characterised in that**

(a) a sample, possibly containing activated stromal fibroblasts, is contacted with an antibody protein according to any one of claims 1 to 9 or 24 to 26 under conditions suitable for the formation of a complex between said antibody and antigen,
(b) detecting the presence of said complex, thereby detecting the presence of activated stromal fibroblasts in wound healing, inflammation or a tumor.

41. The method of claim 40, wherein the tumor is a tumor having cancer cells selected from the cancer group consisting of colorectal cancers, non-small cell lung cancers, breast cancers, head and neck cancer, ovarian cancers, lung cancers, bladder cancers, pancreatic cancers and metastatic cancers of the brain.

42. A method of detecting tumor-stroma, **characterised in that**

(a) a suitable sample is contacted with an antibody protein according to any one of claims 1 to 9, under conditions suitable for the formation of an antibody-antigen complex,
(b) detecting the presence of any complex so formed,
(c) relating the presence of said complex to the presence of tumor-stroma.

43. The method of claim 42, wherein said antibody is labelled with a detectable marker.

44. An antibody protein containing an amino acid sequence as set forth in SEQ ID NO: 2.

45. An antibody protein according to claim 44 further containing an amino acid sequence as set forth in SEQ ID NO: 12.

46. An antibody protein according to claim 44 or 45 further containing an amino acid sequence as set forth in SEQ ID NO: 20 and an amino acid sequence as set forth in SEQ ID NO: 22.

47. A DNA molecule coding for an antibody protein according to any one of claims 44 to 46.

48. A host cell carrying a DNA molecule according to claim 47.

49. A method of producing an antibody protein of any-one of claims 44 to 46, said method comprising the steps of

(a) cultivating the host cell of claim 48 under conditions where said antibody protein is expressed by said host cell, and
(b) isolating said protein.

50. An antibody protein according to any one of claims 44 to 46 which is conjugated to a radioisotope, preferably $^{131}I$, $^{125}I$, $^{188}Re$, $^{188}Re$, or $^{90}Y$.

51. A pharmaceutical composition comprising an antibody protein according to any one of claims 44 to 46, or 50, and a pharmaceutically acceptable carrier.

**Patentansprüche**

1. Antikörperprotein mit den komplementaritätsbestimmenden Regionen des monoklonalen Antikörpers F19 (ATCC-Hinterlegungsnummer HB 8269), wobei dieses Antikörperprotein spezifisch an Fibroblasten-Aktivierungsprotein bindet, **dadurch gekennzeichnet, dass** es die variable Region der leichten Kette gemäß SEQ ID NO: 2 oder SEQ ID NO: 6 enthält.

2. Antikörperprotein nach Anspruch 1, **dadurch gekennzeichnet, dass** die variable Region der leichten Kette durch eine Nucleotidsequenz gemäß SEQ ID NO: 1 oder SEQ ID NO: 5 kodiert wird.

3. Antikörperprotein nach einem der Ansprüche 1 oder 2, enthaltend eine variable Region der schweren Kette gemäß einer beliebigen Sequenz von SEQ ID NOs: 8, 10, 12 und 14.

4. Antikörperprotein nach Anspruch 3, **dadurch gekennzeichnet, dass** die variable Region der schweren Kette durch eine Nucleotidsequenz gemäß SEQ ID NOs: 7, 9, 11 und 13 kodiert wird.

5. Antikörperprotein nach einem der Ansprüche 1 bis 4, enthaltend die variable Region der leichten Kette gemäß SEQ ID NO: 2 und die variable Region der schweren Kette gemäß SEQ ID NO: 12.

6. Antikörperprotein nach Anspruch 5, **dadurch gekennzeichnet, dass** die variable Region der leichten Kette durch eine Nucleotidsequenz gemäß SEQ ID NO: 1 kodiert wird und die variable Region der schweren Kette durch eine Nucleotidsequenz gemäß SEQ ID NO: 11 kodiert wird.

7. Antikörperprotein nach Anspruch 5 oder Anspruch 6, enthaltend die konstante Region der leichten Kette gemäß SEQ ID NO: 20 und die konstante Region der schweren Kette gemäß SEQ ID NO: 22.

8. Antikörperprotein nach einem der Ansprüche 1 bis 4, enthaltend die variable Region der leichten Kette gemäß SEQ ID NO: 2 und die variable Region der schweren Kette gemäß SEQ ID NO: 8.

9. Antikörperprotein nach Anspruch 8, **dadurch gekennzeichnet, dass** die variable Region der leichten Kette durch eine Nucleotidsequenz gemäß SEQ ID NO: 1 kodiert wird und die variable Region der schweren Kette durch eine Nucleotidsequenz gemäß SEQ ID NO: 7 kodiert wird.

10. Nucleotidsequenz, kodierend für ein Antikörperprotein nach einem der Ansprüche 1 bis 9.

11. Rekombinanter DNA-Vektor, der eine Nucleotidsequenz gemäß Anspruch 10 enthält.

12. Rekombinanter DNA-Vektor nach Anspruch 11, wobei es sich bei diesem Vektor um einen Expressionsvektor handelt.

13. Wirtszelle, tragend einen Vektor nach Anspruch 11 oder 12.

**14.** Wirtszelle nach Anspruch 13, wobei es sich bei der Wirtszelle um eine eukaryontische Zelle handelt.

**15.** Wirtszelle nach Anspruch 14, wobei es sich bei der eukaryontischen Wirtszelle um eine Säugetierzelle handelt.

**16.** Wirtszelle nach Anspruch 15, wobei es sich bei der Wirtszelle um eine CHO- oder COS-Zelle handelt.

**17.** Verfahren zur Herstellung von Antikörperproteinen nach einem der Ansprüche 1 bis 9, wobei das Verfahren die folgenden Stufen umfasst:

(a) das Züchten einer Wirtszelle nach einem der Ansprüche 13 bis 16 unter Bedingungen, bei denen das Antikörperprotein durch die Wirtszelle exprimiert wird, und
(b) das Isolieren des Antikörperproteins.

**18.** Verfahren nach Anspruch 17, wobei es sich bei der Wirtszelle um eine Säugetierzelle und vorzugsweise um eine CHO- oder COS-Zelle handelt.

**19.** Verfahren nach Anspruch 17 oder 18, wobei die Wirtszelle mit zwei Plasmiden kotransfiziert ist, die die Expressionseinheiten für leichte bzw. schwere Ketten tragen.

**20.** Antikörperprotein nach einem der Ansprüche 1 bis 9, wobei das Antikörperprotein mit einem therapeutischen Mittel konjugiert ist.

**21.** Antikörperprotein nach Anspruch 20, wobei es sich beim therapeutischen Mittel um ein therapeutisches Mittel handelt, das aus der Gruppe ausgewählt ist, die aus Radioisotopen, Toxinen, Toxoiden, entzündungshemmenden Mitteln und chemotherapeutischen Mitteln besteht.

**22.** Antikörperprotein nach Anspruch 21, wobei es sich beim Radioisotop um ein β-emittierendes Radioisotop handelt.

**23.** Antikörperprotein nach Anspruch 22, wobei das Radioisotop aus der Gruppe ausgewählt ist, die aus $^{186}$Rhenium, $^{188}$Rhenium, $^{131}$Iod und $^{90}$Yttrium besteht.

**24.** Antikörperprotein nach einem der Ansprüche 1 bis 9, **dadurch gekennzeichnet, dass** es markiert ist.

**25.** Antikörperprotein nach Anspruch 24, wobei es sich bei der Markierung um einen nachweisbaren Marker handelt.

**26.** Antikörperprotein nach Anspruch 25, wobei es sich beim nachweisbaren Marker um einen nachweisbaren Marker handelt, der aus der Gruppe ausgewählt ist, die aus Enzymen, Farbstoffen, Radioisotopen, Digoxygenin und Biotin besteht.

**27.** Antikörperprotein nach einem der Ansprüche 1 bis 9, das mit einem abbildbaren Mittel konjugiert ist.

**28.** Antikörperprotein nach Anspruch 27, wobei es sich beim abbildbaren Mittel um ein Radioisotop handelt.

**29.** Antikörperprotein nach Anspruch 28, wobei es sich beim Radioisotop um ein γ-emittierendes Radioisotop handelt.

**30.** Antikörperprotein nach Anspruch 29, wobei es sich beim Radioisotop um 1251 handelt.

**31.** Pharmazeutische Zusammensetzung, enthaltend ein Antikörperprotein nach einem der Ansprüche 1 bis 9 und einen pharmazeutisch verträglichen Träger.

**32.** Pharmazeutische Zusammensetzung, enthaltend ein Antikörperprotein nach einem der Ansprüche 20 bis 23 und einen pharmazeutisch verträglichen Träger.

**33.** Pharmazeutische Zusammensetzung, enthaltend ein Antikörperprotein nach einem der Ansprüche 27 bis 30 und einen pharmazeutisch verträglichen Träger.

**34.** Pharmazeutische Zusammensetzung nach den Ansprüchen 31 bis 33 zur Verwendung bei der Behandlung oder Abbildung von Tumoren, wobei die Tumoren mit aktivierten stromalen Fibroblasten assoziiert sind, wobei es sich bei den Tumoren vorzugsweise um Tumoren handelt, die aus der Krebsgruppe ausgewählt sind, die aus kolorektalem Krebs, nicht-kleinzelligem Lungenkrebs, Brustkrebs, Kopf- und Halskrebs, Ovarialkrebs, Lungenkrebs, Blasenkrebs, Pankreaskrebs und metastatischem Hirnkrebs besteht.

**35.** Verwendung eines Antikörperproteins nach einem der Ansprüche 1 bis 9 zur Herstellung eines Arzneimittels zur Behandlung von Krebs.

**36.** Verwendung eines Antikörperproteins nach einem der Ansprüche 20 bis 23 zur Herstellung eines Arzneimittels zur Behandlung von Krebs.

**37.** Verwendung eines Antikörperproteins nach einem der Ansprüche 27 bis 30 zur Herstellung eines Arzneimittels zur Abbildung von aktivierten stromalen Fibroblasten.

**38.** Verwendung eines Antikörperproteins nach einem der Ansprüche 24 bis 26 zum Nachweis der Anwesenheit von aktivierten stromalen Fibroblasten in einer Probe.

**39.** Verwendung nach Anspruch 35 oder 36, wobei der Krebs aus der Krebsgruppe ausgewählt ist, die aus kolorektalem Krebs, nicht-kleinzelligem Lungenkrebs, Brustkrebs, Kopf- und Halskrebs, Ovarialkrebs, Lungenkrebs, Blasenkrebs, Pankreaskrebs und metastatischem Hirnkrebs besteht.

**40.** Verfahren zum Nachweisen der Anwesenheit von aktivierten stromalen Fibroblasten bei der Wundheilung, bei Entzündungen oder bei einem Tumor, **dadurch gekennzeichnet, dass**

(a) eine Probe, die möglicherweise aktivierte stromale Fibroblasten enthält, mit einem Antikörperprotein nach einem der Ansprüche 1 bis 9 oder 24 bis 26 unter Bedingungen in Kontakt gebracht wird, die sich zur Bildung eines Komplexes zwischen dem Antikörper und dem Antigen eignen, und
(b) die Anwesenheit des Komplexes nachgewiesen wird, wodurch die Anwesenheit von aktivierten stromalen Fibroblasten bei der Wundheilung, bei Entzündungen oder bei einem Tumor nachgewiesen wird.

**41.** Verfahren nach Anspruch 40, wobei es sich beim Tumor um einen Tumor handelt, der Krebszellen aufweist, die aus der Krebsgruppe ausgewählt sind, die aus kolorektalem Krebs, nicht-kleinzelligem Lungenkrebs, Brustkrebs, Kopf- und Halskrebs, Ovarialkrebs, Lungenkrebs, Blasenkrebs, pankreatischem Krebs und metastatischem Hirnkrebs besteht.

**42.** Verfahren zum Nachweis eines Tumor-Stromas, **dadurch gekennzeichnet, dass**

(a) eine geeignete Probe mit einem Antikörperprotein nach einem der Ansprüche 1 bis 9 unter Bedingungen in Kontakt gebracht wird, die sich zur Bildung eines Antikörper-Antigen-Komplexes eignen,
(b) die Anwesenheit eines etwaigen, auf diese Weise gebildeten Komplexes nachgewiesen wird und
(c) die Anwesenheit des Komplexes mit der Anwesenheit von Tumor-Stroma in Zusammenhang gebracht wird.

**43.** Verfahren nach Anspruch 42, wobei der Antikörper mit einem nachweisbaren Marker markiert ist.

**44.** Antikörperprotein, enthaltend eine Aminosäuresequenz gemäß SEQ ID NO: 2.

**45.** Antikörperprotein nach Anspruch 44, ferner enthaltend eine Aminosäuresequenz gemäß SEQ ID NO: 12.

**46.** Antikörperprotein nach Anspruch 44 oder 45, ferner enthaltend eine Aminosäuresequenz gemäß SEQ ID NO: 20 und eine Aminosäuresequenz gemäß SEQ ID NO: 22.

**47.** DNA-Molekül, kodierend für ein Antikörperprotein nach einem der Ansprüche 44 bis 46.

**48.** Wirtszelle, tragend ein DNA-Molekül nach Anspruch 47.

**49.** Verfahren zur Herstellung eines Antikörperproteins nach einem der Ansprüche 44 bis 46, wobei das Verfahren die folgenden Stufen umfasst:

(a) das Züchten der Wirtszelle nach Anspruch 48 unter Bedingungen, bei denen das Antikörperprotein durch die Wirtszelle exprimiert wird, und
(b) das Isolieren des Poteins.

**50.** Antikörperprotein nach einem der Ansprüche 44 bis 46, das mit einem Radioisotop und vorzugsweise mit $^{131}I$, $^{125}I$, $^{186}Re$, $^{188}Re$ oder $^{90}Y$ konjugiert ist.

**51.** Pharmazeutische Zusammensetzung, umfassend ein Antikörperprotein nach einem der Ansprüche 44 bis 46 oder 50 und einen pharmazeutisch verträglichen Träger.

## Revendications

**1.** Protéine anticorps comportant les régions déterminantes complémentaires de l'anticorps monoclonal F 19 (accès ATCC n° HB 8 269), ladite protéine anticorps se liant spécifiquement à la protéine activatrice de fibroblaste, **caractérisée en ce qu'**elle contient la région variable de la chaîne légère telle qu'elle se trouve dans SEQ ID n° 2 ou SEQ ID n° 6.

**2.** Protéine anticorps selon la revendication 1, **caractérisée en ce que** la région variable de la chaîne légère est codée par une séquence de nucléotides telle qu'elle se trouve dans SEQ ID n° 1 ou SEQ ID n° 5.

**3.** Protéine anticorps selon l'une des revendication 1 ou 2, contenant une région variable de la chaîne lourde telle qu'elle se trouve dans l'une quelconque des SEQ ID n°8, 10, 12, 14.

**4.** Protéine anticorps selon la revendication 3, **caractérisée en ce que** la région variable de la chaîne lourde est codée par une séquence de nucléotides telle qu'elle se trouve dans SEQ ID n° 7, 9, 11, 13.

**5.** Protéine anticorps selon l'une des revendications 1 à 4, contenant la région variable de la chaîne légère telle qu'elle se trouve dans SEQ ID n° 2 et la région variable de la chaîne lourde telle qu'elle se trouve dans SEQ ID n° 12.

**6.** Protéine anticorps selon la revendication 5, **caractérisée en ce que** la région variable de la chaîne légère est codée par une séquence de nucléotides telle qu'elle se trouve dans SEQ ID n° 1 et la région variable de la chaîne lourde est codée par une séquence de nucléotides telle qu'elle se trouve dans SEQ ID n° 11.

**7.** Protéine anticorps selon la revendication 5 ou 6, contenant la région constante de la chaîne légère telle qu'elle se trouve dans SEQ ID n° 20 et la région constante de la chaîne lourde telle qu'elle se trouve dans SEQ ID n° 22.

**8.** Protéine anticorps selon l'une des revendications 1 à 4, contenant la région variable de la chaîne légère telle qu'elle se trouve dans SEQ ID n° 2 et la région variable de la chaîne lourde telle qu'elle se trouve dans SEQ ID n° 8.

**9.** Protéine anticorps selon la revendication 8, **caractérisée en ce que** la région variable de la chaîne légère est codée par une séquence de nucléotides telle qu'elle se trouve dans SEQ ID n° 1 et la région variable de la chaîne lourde est codée par une séquence de nucléotides telle qu'elle se trouve dans SEQ ID n° 7.

**10.** Séquence de nucléotides codant une protéine anticorps d'après l'une des revendications 1 à 9.

**11.** Vecteur d'ADN recombinant qui contient une séquence de nucléotides d'après la revendication 10.

**12.** Vecteur d'ADN recombinant selon la revendication 11, ledit vecteur étant un vecteur d'expression.

**13.** Cellule hôte qui porte un vecteur selon les revendications 11 ou 12.

**14.** Cellule hôte selon la revendication 13, ladite cellule hôte étant une cellule eucaryote.

**15.** Cellule hôte selon la revendication 14., ladite cellule hôte eucaryote étant une cellule de mammifère.

**16.** Cellule hôte selon la revendication 15, ladite cellule hôte étant une cellule CHO ou COS.

**17.** Procédé pour préparer des protéines anticorps selon l'une des revendications 1 à 9, ledit procédé comprend les étapes de

(a) cultiver une cellule hôte selon l'une des revendications 13 à 16 dans des conditions où ladite protéine anticorps est exprimée dans ladite cellule hôte et
(b) isoler ladite protéine anticorps.

**18.** Procédé selon la revendication 17, **caractérisé en ce que** ladite cellule hôte est une cellule de mammifère, de préférence une cellule CHO ou COS.

**19.** Procédé selon la revendication 17 ou 18, d'après lequel ladite cellule hôte est co-transfectée avec deux plasmides qui portent les unités d'expression pour les chaînes légère et lourde respectivement.

**20.** Protéine anticorps selon l'une des revendications 1 à 9, ladite protéine anticorps étant conjuguée à un agent thérapeutique.

**21.** Protéine anticorps selon la revendication 20, ledit agent thérapeutique étant un agent thérapeutique choisi parmi le groupe formé par les radio-isotopes, les toxines, les toxoïdes, les agents inflammatoires et les agents chimiothérapeutiques.

**22.** Protéine anticorps selon la revendication 21, ledit radio-isotope étant un radio-isotope émetteur bêta.

**23.** Protéine anticorps selon la revendication 22, ledit radio-isotope étant choisi dans le groupe formé par $^{186}$rhénium, $^{188}$rhénium, $^{131}$iode et $^{90}$yttrium.

**24.** Protéine anticorps selon l'une des revendications 1 à 9, **caractérisée en ce qu'**elle est marquée.

**25.** Protéine anticorps selon la revendication 24, dans laquelle ladite marque est un marqueur détectable.

**26.** Protéine anticorps selon la revendication 25, dans laquelle le marqueur détectable est un marqueur détectable choisi parmi le groupe formé des enzymes, des teintures, des radio-isotopes, de la digoxigénine et de la biotine.

**27.** Protéine anticorps selon l'une des revendications 1 à 9 conjuguée à un agent d'imagerie.

**28.** Protéine anticorps selon la revendication 27, l'agent d'imagerie étant un radio-isotope.

**29.** Protéine anticorps selon la revendication 28, ledit radio-isotope étant un radio-isotope émetteur gamma.

**30.** Protéine anticorps selon la revendication 29, ledit radio-isotope étant $^{125}$I.

**31.** Composition pharmaceutique qui contient une protéine anticorps selon l'une des revendications 1 à 9 et un porteur admissible en pharmacie.

**32.** Composition pharmaceutique qui contient une protéine anticorps selon l'une des revendications 20 à 23 et un porteur admissible en pharmacie.

**33.** Composition pharmaceutique qui contient une protéine anticorps selon l'une des revendications 27 à 30 et un porteur admissible en pharmacie.

**34.** Composition pharmaceutique selon les revendications 31 à 33 à utiliser pour le traitement ou l'imagerie des tumeurs, lesdites tumeurs sont alors associées à des fibroblastes stromaux activés, lesdites tumeurs sont de préférence des tumeurs choisies parmi le groupe des cancers formés par les cancers colo-rectaux, les cancers du poumon à grandes cellules, les cancers du sein, le cancer de la tête et de la nuque, les cancers des ovaires, les cancers du poumon, les cancers de la vessie, les cancers du pancréas et les cancers métastatiques du cerveau.

**35.** Utilisation de la protéine anticorps selon l'une des revendications 1 à 9 pour préparer un médicament destiné au traitement du cancer.

**36.** Utilisation de la protéine anticorps selon l'une des revendications 20 à 23 pour préparer un médicament destiné au traitement du cancer.

**37.** Utilisation de la protéine anticorps selon l'une des revendications 27 à 30 pour préparer un médicament destiné à l'imagerie des fibroblastes stromaux activés.

**38.** Utilisation de la protéine anticorps selon l'une des revendications 24 à 26 pour détecter la présence de fibroblastes stromaux activés dans un échantillon.

**39.** Utilisation selon la revendication 35 ou 36 d'après laquelle le cancer est choisi parmi le groupe des cancers formés par les cancers colo-rectaux, les cancers du poumon à grandes cellules, les cancers du sein, le cancer de la tête et de la nuque, les cancers des ovaires, les cancers du poumon, les cancers de la vessie, les cancers du pancréas et les cancers métastatiques du cerveau.

**40.** Procédé pour détecter la présence de fibroblastes activés dans la guérison des plaies, l'inflammation ou une tumeur, **caractérisé en ce que**

(a) un échantillon qui contient peut-être des fibroblastes stromaux activés est mis en contact avec une protéine anticorps selon l'une des revendications 1 à 9 ou 24 à 26 dans des conditions appropriées à la formation d'un complexe entre ledit anticorps et l'antigène,
(b) on détecte la présence dudit complexe, ce qui détecte la présence de fibroblastes stromaux activés dans la guérison des plaies, l'inflammation ou une tumeur.

**41.** Procédé selon la revendication 40, d'après lequel la tumeur est une tumeur qui comporte des cellules cancéreuses choisies parmi le groupe des cancers formés par les cancers colo-rectaux, les cancers du poumon à grandes cellules, les cancers du sein, le cancer de la tête et de la nuque, les cancers des ovaires, les cancers du poumon, les cancers de la vessie, les cancers du pancréas et les cancers métastatiques du cerveau.

**42.** Procédé pour détecter le stroma de tumeur, **caractérisé en ce que**

(a) un échantillon adapté est mis en contact avec une protéine anticorps selon l'une des revendications 1 à 9 dans des conditions appropriées à la formation d'un complexe entre ledit anticorps et l'antigène,
(b) on détecte la présence de tout complexe ainsi formé
(c) on met en relation la présence dudit complexe avec la présence d'un stroma de tumeur

**43.** Procédé selon la revendication 42, d'après lequel ledit anticorps est marqué par un marqueur détectable.

**44.** Protéine anticorps qui contient une séquence d'acides aminés telle qu'elle se trouve dans SEQ ID n° 2.

**45.** Protéine anticorps selon la revendication 44 qui contient en outre une séquence d'acides aminés telle qu'elle se trouve dans SEQ ID n° 12.

**46.** Protéine anticorps selon la revendication 44 ou 45 qui contient en outre une séquence d'acides aminés telle qu'elle se trouve dans SEQ ID n° 20 et une séquence d'acides aminés telle qu'elle se trouve dans SEQ ID n° 22.

**47.** Molécule d'ADN codant une protéine anticorps selon l'une des revendications 44 à 46.

**48.** Cellule hôte qui porte une molécule d'ADN selon la revendication 47.

**49.** Procédé pour préparer une protéine anticorps selon l'une des revendications 44 à 46, ledit procédé comprend les étapes de

(a) cultiver la cellule hôte selon la revendication 48 dans des conditions où ladite protéine anticorps est exprimée par ladite cellule hôte et
(b) isoler ladite protéine.

**50.** Protéine anticorps selon l'une des revendications 44 à 46 laquelle est conjuguée à un radio-isotope, de préférence $^{131}I$, $^{125}I$, $^{186}Re$,$^{188}Re$ ou $^{90}Y$.

**51.** Composition pharmaceutique qui comprend une protéine anticorps selon l'une des revendications 44 à 46 ou 50 et un porteur admissible en pharmacie.

**Fig. 1**

1 GACATTGTGA 11 TGACCCAATC 21 TCCAGACTCT 31 TTGGCTGTGT 41 CTCTAGGGGA
51 GAGGGCCACC 61 ATCAACTGCA 71 AGTCCAGTCA 81 GAGCCTTTTA 91 TATTCTAGAA
101 ATCAAAAGAA 111 CTACTTGGCC 121 TGGTATCAGC 131 AGAAACCAGG 141 ACAGCCACCC
151 AAACTCCTCA 161 TCTTTTGGGC 171 TAGCACTAGG 181 GAATCTGGGG 191 TACCTGATAG
201 GTTCAGTGGC 211 AGTGGGTTTG 221 GGACAGACTT 231 CACCCTCACC 241 ATTAGCAGCC
251 TGCAGGCTGA 261 AGATGTGGCA 271 GTTTATTACT 281 GTCAGCAATA 291 TTTTAGCTAT
301 CCGCTCACGT 311 TCGGACAAGG 321 GACCAAGGTG 331 GAAATAAAA 339

**Fig. 2**

1 DIVMTQSPDS 11 LAVSLGERAT 21 INCKSSQSLL 31 YSRNQKNYLA 41 WYQQKPGQPP
51 KLLIFWASTR 61 ESGVPDRFSG 71 SGFGTDFTLT 81 ISSLQAEDVA 91 VYYCQQYFSY
101 PLTFGQGTKV 111 EIK

**Fig. 3**

1 GACATTGTGA 11 TGACCCAATC 21 TCCAGACTCT 31 TTGGCTGTGT 41 CTCTAGGGGA
51 GAGGGCCACC 61 ATCAACTGCA 71 AGTCCAGTCA 81 GAGCCTTTTA 91 TATTCTAGAA
101 ATCAAAAGAA 111 CTACTTGGCC 121 TGGT**TC**CAGC 131 AGAAACCAGG 141 ACAGCCACCC
151 AAACTCCTCA 161 TCTTTTGGGC 171 TAGCACTAGG 181 GAATCTGGGG 191 TACCTGATAG
201 GTTCAGTGGC 211 AGTGGGTTTG 221 GGACAGACTT 231 CACCCTCACC 241 ATTAGCAGCC
251 TGCAGGCTGA 261 AGATGTGGCA 271 GTTTAT**G**ACT 281 GTCA**A**CAATA 291 TTTTAGCTAT
301 CCGCTCACGT 311 TCGGACAAGG 321 GACCAAGGTG 331 GAAATAAAA 339

**Fig. 4**

```
1          11         21         31         41
DIVMTQSPDS LAVSLGERAT INCKSSQSLL YSRNQKNYLA WFQQKPGQPP
51         61         71         81         91
KLLIFWASTR ESGVPDRFSG SGFGTDFTLT ISSLQAEDVA VYDCQQYFSY
101        111
PLTFGQGTKV EIK
```

**Fig. 5**

```
1          11         21         31         41
GACATTGTGA TGACCCAATC TCCAGACTCT TTGGCTGTGT CTCTAGGGGA
51         61         71         81         91
GAGGGCCACC ATCAACTGCA AGTCCAGTCA GAGCCTTTTA TATTCTAGAA
101        111        121        131        141
ATCAAAAGAA CTACTTGGCC TGGTATCAGC AGAAACCAGG ACAGCCACCC
151        161        171        181        191
AAACTCCTCA TCTATTGGGC TAGCACTAGG GAATCTGGGG TACCTGATAG
201        211        221        231        241
GTTCAGTGGC AGTGGGTTTG GGACAGACTT CACCCTCACC ATTAGCAGCC
251        261        271        281        291
TGCAGGCTGA AGATGTGGCA GTTTATTACT GTCAGCAATA TTTTAGCTAT
301        311        321        331   339
CCGCTCACGT TCGGACAAGG GACCAAGGTG GAAATAAAA
```

**Fig. 6**

```
1          11         21         31         41
DIVMTQSPDS LAVSLGERAT INCKSSQSLL YSRNQKNYLA WYQQKPGQPP
51         61         71         81         91
KLLIYWASTR ESGVPDRFSG SGFGTDFTLT ISSLQAEDVA VYYCQQYFSY
101        111
PLTFGQGTKV EIK
```

## Fig. 7

```
1
CAGGTGCAAC TAGTGCAGTC CGGCGCCGAA GTGAAGAAAC CCGGTGCTTC
51
CGTGAAAGTC AGCTGTAAAA CTAGTAGATA CACCTTCACT GAATACACCA
101
TACACTGGGT TAGACAGGCC CCTGGCCAAA GGCTGGAGTG GATAGGAGGT
151
ATTAATCCTA ACAATGGTAT TCCTAACTAC AACCAGAAGT TCAAGGGCCG
201
GGCCACCTTG ACCGTAGGCA AGTCTGCCAG CACCGCCTAC ATGGAACTGT
251
CCAGCCTGCG CTCCGAGGAC ACTGCAGTCT ACTACTGCGC CAGAAGAAGA
301
ATCGCCTATG GTTACGACGA GGGCCATGCT ATGGACTACT GGGGTCAAGG
351                   372
AACCCTTGTC ACCGTCTCCT CA
```

## Fig. 8

```
1          11         21         31         41
QVQLVQSGAE VKKPGASVKV SCKTSRYTFT EYTIHWVRQA PGQRLEWIGG
51         61         71         81         91
INPNNGIPNY NQKFKGRATL TVGKSASTAY MELSSLRSED TAVYYCARRR
101        111        121-124
IAYGYDEGHA MDYWGQGTLV TVSS
```

## Fig. 9

```
1
CAGGTGCAAC TAGTGCAGTC CGGCGCCGAA GTGAAGAAAC CCGGTGCTTC
51
CGTGAAAGTC AGCTGTAAAA CTAGTAGATA CACCTTCACT GAATACACCA
101
TACACTGGGT TAGACAGGCC CCTGGCCAAA GGCTGGAGTG GATAGGAGGT
151
ATTAATCCTA ACAATGGTAT TCCTAACTAC AACCAGAAGT TCAAGGGCCG
201
GGCCACCTTG ACCGTAGGCA AGTCTGCCAG CACCGCCTAC ATGGAACTGT
251
CCAGCCTGCG CTCCGAGGAC ACTGCAGTCT ACTTCTGCGC CAGAAGAAGA
301
ATCGCCTATG GTTACGACGA GGGCCATGCT ATGGACTACT GGGGTCAAGG
351                   372
AACCCTTGTC ACCGTCTCCT CA
```

**Fig. 10**

```
1          11         21         31         41
QVQLVQSGAE VKKPGASVKV SCKTSRYTFT EYTIHWVRQA PGQRLEWIGG
51         61         71         81         91
INPNNGIPNY NQKFKGRATL TVGKSASTAY MELSSLRSED TAVYFCARRR
101        111        121-124
IAYGYDEGHA MDYWGQGTLV TVSS
```

**Fig. 11**

```
1
CAGGTGCAAC TAGTGCAGTC CGGCGCCGAA GTGAAGAAAC CCGGTGCTTC
51
CGTGAAAGTC AGCTGTAAAA CTAGTAGATA CACCTTCACT GAATACACCA
101
TACACTGGGT TAGACAGGCC CCTGGCCAAA GGCTGGAGTG GATAGGAGGT
151
ATTAATCCTA ACAATGGTAT TCCTAACTAC AACCAGAAGT TCAAGGGCCG
201
GGTCACCATC ACCGTAGACA CCTCTGCCAG CACCGCCTAC ATGGAACTGT
251
CCAGCCTGCG CTCCGAGGAC ACTGCAGTCT ACTACTGCGC CAGAAGAAGA
301
ATCGCCTATG GTTACGACGA GGGCCATGCT ATGGACTACT GGGGTCAAGG
351                   372
AACCCTTGTC ACCGTCTCCT CA
```

**Fig. 12**

```
1          11         21         31         41
QVQLVQSGAE VKKPGASVKV SCKTSRYTFT EYTIHWVRQA PGQRLEWIGG
51         61         71         81         91
INPNNGIPNY NQKFKGRVTI TVDTSASTAY MELSSLRSED TAVYYCARRR
101        111        121-124
IAYGYDEGHA MDYWGQGTLV TVSS
```

**Fig. 13**

```
1
CAGGTGCAAC TAGTGCAGTC CGGCGCCGAA GTGAAGAAAC CCGGTGCTTC
51
CGTGAAAGTC AGCTGTAAAA CTAGTAGATA CACCTTCACT GAATACACCA
101
TACACTGGGT TAGACAGGCC CCTGGCCAAA GGCTGGAGTG GATAGGAGGT
151
ATTAATCCTA ACAATGGTAT TCCTAACTAC AACCAGAAGT TCAAGGGCCG
201
GGTCACCATC ACCGTAGACA CCTCTGCCAG CACCGCCTAC ATGGAACTGT
251
CCAGCCTGCG CTCCGAGGAC ACTGCAGTCT ACTTCTGCGC CAGAAGAAGA
301
ATCGCCTATG GTTACGACGA GGGCCATGCT ATGGACTACT GGGGTCAAGG
351                   372
AACCCTTGTC ACCGTCTCCT CA
```

**Fig. 14**

```
1          11         21         31         41
QVQLVQSGAE VKKPGASVKV SCKTSRYTFT EYTIHWVRQA PGQRLEWIGG
51         61         71         81         91
INPNNGIPNY NQKFKGRVTI TVDTSASTAY MELSSLRSED TAVYFCARRR
101        111        121-124
IAYGYDEGHA MDYWGQGTLV TVSS
```

**Fig. 15**

```
1
CAGGTGCAAC TAGTGCAGTC CGGCGCCGAA GTGAAGAAAC CCGGTGCTTC
51
CGTGAAAGTC AGCTGTAAAA CTAGTGGATA CACCTTCACT GAATACACCA
101
TACACTGGGT TAGACAGGCC CCTGGCCAAA GGCTGGAGTG GATAGGAGGT
151
ATTAATCCTA ACAATGGTAT TCCTAACTAC AACCAGAAGT TCAAGGGCCG
201
GGTCACCATC ACCGTAGACA CCTCTGCCAG CACCGCCTAC ATGGAACTGT
251
CCAGCCTGCG CTCCGAGGAC ACTGCAGTCT ACTACTGCGC CAGAAGAAGA
301
ATCGCCTATG GTTACGACGA GGGCCATGCT ATGGACTACT GGGGTCAAGG
351                   372
AACCCTTGTC ACCGTCTCCT CA
```

Fig. 16

```
1          11         21         31         41
QVQLVQSGAE VKKPGASVKV SCKTSGYTFT EYTIHWVRQA PGQRLEWIGG
51         61         71         81         91
INPNNGIPNY NQKFKGRVTI TVDTSASTAY MELSSLRSED TAVYYCARRR
101        111        121-124
IAYGYDEGHA MDYWGQGTLV TVSS
```

Fig. 17

```
1
DIVMSQSPSS LAVSVGEKVT MSCKSSQSLL YSRNQKNYLA WFQQKPGQSP
51
KLLIFWASTR ESGVPDRFTG SGFGTDFNLT ISSVQAEDLA VYDCQQYFSY
101
PLTFGAGTKL ELKRTVAAPS VFIFPPSDEQ LKSGTASVVC LLNNFYPREA
151
KVQWKVDNAL QSGNSQESVT EQDSKDSTYS LSSTLTLSKA DYEKHKVYAC
201
EVTHQGLSSP VTKSFNRGEC
```

Fig. 18

```
1
VQLQQSGPEL VKPGASVKMS CKTSRYTFTE YTIHWVRQSH GKSLEWIGGI
51
NPNNGIPNYN QKFKGRATLT VGKSSSTAYM ELRSLTSEDS AVYFCARRRI
101
AYGYDEGHAM DYWGQGTSVT VSSASTKGPS VFPLAPSSKS TSGGTAALGC
151
LVKDYFPEPV TVSWNSGALT SGVHTFPAVL QSSGLYSLSS VVTVPSSSLG
201
TQTYICNVNH KPSNTKVDKK VEPKSCDKTH TCPPCPAPEL LGGPSVFLFP
251
PKPKDTLMIS RTPEVTCVVV DVSHEDPEVK FNWYVDGVEV HNAKTKPREE
301
QYNSTYRVVS VLTVLHQDWL NGKEYKCKVS NKALPAPIEK TISKAKGQPR
351
EPQVYTLPPS REEMTKNQVS LTCLVKGFYP SDIAVEWESN GQPENNYKTT
401
PPVLDSDGSF FLYSKLTVDK SRWQQGNVFS CSVMHEALHN HYTQKSLSLS
451
PGK
```

## Fig. 19

```
340        350        360        370        380
CGTACTGTGG CTGCACCATC TGTCTTCATC TTCCCGCCAT CTGATGAGCA
390        400        410        420        430
GTTGAAATCT GGAACTGCCT CTGTTGTGTG CCTGCTGAAT AACTTCTATC
440        450        460        470        480
CCAGAGAGGC CAAAGTACAG TGGAAGGTGG ATAACGCCCT CCAATCGGGT
490        500        510        520        530
AACTCCCAGG AGAGTGTCAC AGAGCAGGAC AGCAAGGACA GCACCTACAG
540        550        560        570        580
CCTCAGCAGC ACCCTGACGC TGAGCAAAGC AGACTACGAG AAACACAAAG
590        600        610        620        630
TCTACGCCTG CGAAGTCACC CATCAGGGCC TGAGCTCGCC CGTCACAAAG
640        650        660
AGCTTCAACA GGGGAGAGTGT
```

## Fig. 20

```
114        124        134        144        154
RTVAAPSVFI FPPSDEQLKS GTASVVCLLN NFYPREAKVQ WKVDNALQSG
164        174        184        194        204
NSQESVTEQD SKDSTYSLSS TLTLSKADYE KHKVYACEVT HQGLSSPVTK
214-220
SFNRGEC
```

Fig. 21

373
GCCTCCACCA AGGGCCCATC GGTCTTCCCC CTGGCACCCT CCTCCAAGAG
423
CACCTCTGGG GGCACAGCGG CCCTGGGCTG CCTGGTCAAG GACTACTTCC
473
CCGAACCGGT GACGGTGTCG TGGAACTCAG GCGCCCTGAC CAGCGGCGTG
523
CACACCTTCC CGGCTGTCCT ACAGTCCTCA GGACTCTACT CCCTCAGCAG
573
CGTGGTGACC GTGCCCTCCA GCAGCTTGGG CACCCAGACC TACATCTGCA
623
ACGTGAATCA CAAGCCCAGC AACACCAAGG TGGACAAGAA AGTTGAGCCC
673
AAATCTTGTG ACAAAACTCA CACATGCCCA CCGTGCCCAG CACCTGAACT
723
CCTGGGGGGA CCGTCAGTCT TCCTCTTCCC CCCAAAACCC AAGGACACCC
773
TCATGATCTC CCGGACCCCT GAGGTCACAT GCGTGGTGGT GGACGTGAGC
823
CACGAAGACC CTGAGGTCAA GTTCAACTGG TACGTGGACG GCGTGGAGGT
873
GCATAATGCC AAGACAAAGC CGCGGGAGGA GCAGTACAAC AGCACGTACC
923
GGGTGGTCAG CGTCCTCACC GTCCTGCACC AGGACTGGCT GAATGGCAAG
973
GAGTACAAGT GCAAGGTCTC CAACAAAGCC CTCCCAGCCC CCATCGAGAA
1023
AACCATCTCC AAAGCCAAAG GGCAGCCCCG AGAACCACAG GTGTACACCC
1073
TGCCCCCATC CCGGGAGGAG ATGACCAAGA ACCAGGTCAG CCTGACCTGC
1123
CTGGTCAAAG GCTTCTATCC CAGCGACATC GCCGTGGAGT GGGAGAGCAA
1173
TGGGCAGCCG GAGAACAACT ACAAGACCAC GCCTCCCGTG CTGGACTCCG
1223
ACGGCTCCTT CTTCCTCTAC AGCAAGCTCA CCGTGGACAA GAGCAGGTGG
1273
CAGCAGGGGA ACGTCTTCTC ATGCTCCGTG ATGCATGAGG CTCTGCACAA
1323 1362
CCACTACACG CAGAAGAGCC TCTCCCTGTC TCCGGGTAAA

**Fig. 22**

125
ASTKGPSVFP LAPSSKSTSG GTAALGCLVK DYFPEPVTVS WNSGALTSGV
175
HTFPAVLQSS GLYSLSSVVT VPSSSLGTQT YICNVNHKPS NTKVDKKVEP
225
KSCDKTHTCP PCPAPELLGG PSVFLFPPKP KDTLMISRTP EVTCVVVDVS
275
HEDPEVKFNW YVDGVEVHNA KTKPREEQYN STYRVVSVLT VLHQDWLNGK
325
EYKCKVSNKA LPAPIEKTIS KAKGQPREPQ VYTLPPSREE MTKNQVSLTC
375
LVKGFYPSDI AVEWESNGQP ENNYKTTPPV LDSDGSFFLY SKLTVDKSRW
425 454
QQGNVFSCSV MHEALHNHYT QKSLSLSPGK

Fig. 23A

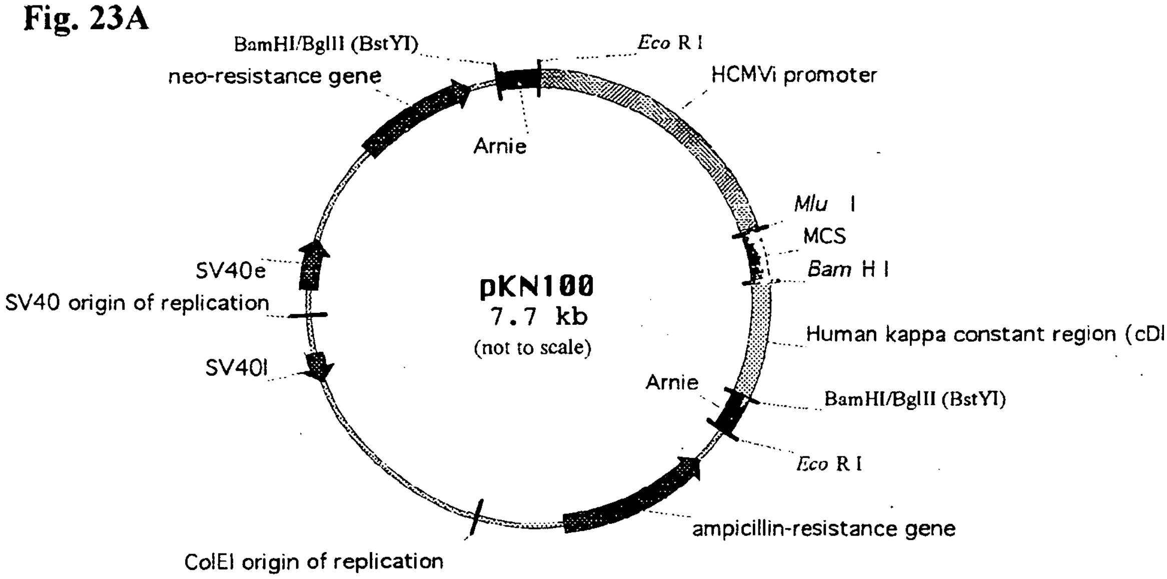


HCMVi enhancer/promoter/intron elements
Multiple cloning site: 5' MluI-HindIII-SalI-XbaI-BamHI 3'
spaC2 termination signal sequence (also known as Arnie)
pSV2neo vector fragment

Fig. 23B

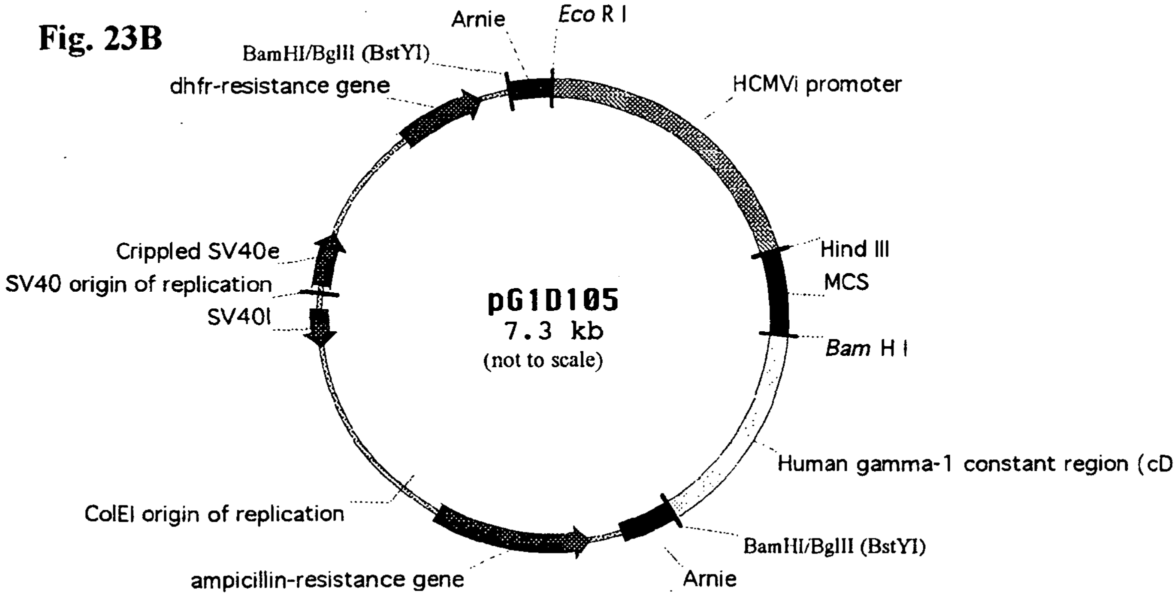


HCMVi enhancer/promoter/intron elements
Multiple cloning site: 5' MluI-HindIII-Sse8387I-PmeI-SalI-XbaI-PacI-BamHI 3'
spaC2 termination signal sequence (also known as Arnie)
pSV2dhfr vector fragment

Fig. 24

```
    HindIII
    aagcttGCCGCCACCatggattcacaggcccaggttcttatgttactgccgctatgggta
  1 ---------+---------+---------+---------+---------+---------+
    ttcgaaCGGCGGTGGtacctaagtgtccgggtccaagaatacaatgacggcgatacccat
       Kozak sequence
                              M  D  S  Q  A  Q  V  L  M  L  L  P  L  W  V

    tctggtacctgtggggacattgtgatgtcacagtctccatcctccctagctgtgtcagtt
 61 ---------+---------+---------+---------+---------+---------+
    agaccatggacacccctgtaacactacagtgtcagaggtaggagggatcgacacagtcaa

     S  G  T  C  G  D  I  V  M  S  Q  S  P  S  S  L  A  V  S  V

    ggagagaaggttactatgagctgcaagtccagtcagagccttttatatagtcgtaatcaa
121 ---------+---------+---------+---------+---------+---------+
    cctctcttccaatgatactcgacgttcaggtcagtctcggaaaatatatcagcattagtt

     G  E  K  V  T  M  S  C  K  S  S  Q  S  L  L  Y  S  R  N  Q
                                         CDR 1

    aagaactacttggcctggttccagcagaagccagggcagtctcctaaactgctgattttc
181 ---------+---------+---------+---------+---------+---------+
    ttcttgatgaaccggaccaaggtcgtcttcggtcccgtcagaggatttgacgactaaaag

     K  N  Y  L  A  W  F  Q  Q  K  P  G  Q  S  P  K  L  L  I  F

    tgggcatccactagggaatctggggtccctgatcgcttcacaggcagtggatttgggacg
241 ---------+---------+---------+---------+---------+---------+
    acccgtaggtgatcccttagaccccagggactagcgaagtgtccgtcacctaaaccctgc

     W  A  S  T  R  E  S  G  V  P  D  R  F  T  G  S  G  F  G  T
          CDR 2

    gatttcaatctcaccatcagcagtgtgcaggctgaggacctggcagtttatgactgtcag
301 ---------+---------+---------+---------+---------+---------+
    ctaaagttagagtggtagtcgtcacacgtccgactcctggaccgtcaaatactgacagtc

     D  F  N  L  T  I  S  S  V  Q  A  E  D  L  A  V  Y  D  C  Q

    caatattttagctatccgctcacgttcggtgctgggaccaagctggagctgaAACGTGAG
361 ---------+---------+---------+---------+---------+---------+
    gttataaaatcgataggcgagtgcaagccacgaccctggttcgacctcgactTTGCACTG
                                                   splice donor site

     Q  Y  F  S  Y  P  L  T  F  G  A  G  T  K  L  E  L  K
           CDR 3

     BamHI
    Tggatcc
421 ------- 427
    Acctagg
```

**Fig. 25**

```
    HindIII
    AAGCTTGCCGCCACCATGGGATGGAGCTGGGTCTTTCTCTTTCTCCTGTCAGGAACTGCA
  1 ---------+---------+---------+---------+---------+---------+
    TTCGAACGGCGGTGGTACCCTACCTCGACCCAGAAAGAGAAAGAGGACAGTCCTTGACGT
        Kozak sequence
                     M   G   W   S   W   V   F   L   F   L   L   S   G   T   A

    GGTGTCCTCTCTGAGGTCCAGCTGCAACAGTCTGGACCTGAGCTGGTGAAGCCTGGGGCT
 61 ---------+---------+---------+---------+---------+---------+
    CCACAGGAGAGACTCCAGGTCGACGTTGTCAGACCTGGACTCGACCACTTCGGACCCCGA

     G   V   L   S   E   V   Q   L   Q   Q   S   G   P   E   L   V   K   P   G   A

    TCAGTAAAGATGTCCTGCAAGACTTCTAGATACACATTCACTGAATACACCATACACTGG
121 ---------+---------+---------+---------+---------+---------+
    AGTCATTTCTACAGGACGTTCTGAAGATCTATGTGTAAGTGACTTATGTGGTATGTGACC

     S   V   K   M   S   C   K   T   S   R   Y   T   F   T   E   Y   T   I   H   W
                                                             CDR 1

    GTGAGACAGAGCCATGGAAAGAGCCTTGAGTGGATTGGAGGTATTAATCCTAACAATGGT
181 ---------+---------+---------+---------+---------+---------+
    CACTCTGTCTCGGTACCTTTCTCGGAACTCACCTAACCTCCATAATTAGGATTGTTACCA

     V   R   Q   S   H   G   K   S   L   E   W   I   G   G   I   N   P   N   N   G
                                                           CDR 2

    ATTCCTAACTACAACCAGAAGTTCAAGGGCAGGGCCACATTGACTGTAGGCAAGTCCTCC
241 ---------+---------+---------+---------+---------+---------+
    TAAGGATTGATGTTAGTCTTCAAGTTCCCGTCCCGGTGTAACTGACATCCGTTCAGGAGG

     I   P   N   Y   N   Q   K   F   K   G   R   A   T   L   T   V   G   K   S   S

    AGCACCGCCTACATGGAGCTCCGCAGCCTGACATCTGAGGATTCTGCGGTCTATTTCTGT
301 ---------+---------+---------+---------+---------+---------+
    TCGTGGCGGATGTACCTCGAGGCGTCGGACTGTAGACTCCTAAGACGCCAGATAAAGACA

     S   T   A   Y   M   E   L   R   S   L   T   S   E   D   S   A   V   Y   F   C

    GCAAGAAGAAGAATCGCCTATGGTTACGACGAGGGCCATGCTATGGACTACTGGGGTCAA
361 ---------+---------+---------+---------+---------+---------+
    CGTTCTTCTTCTTAGCGGATACCAATGCTGCTCCCGGTACGATACCTGATGACCCCAGTT

     A   R   R   R   I   A   Y   G   Y   D   E   G   H   A   M   D   Y   W   G   Q
                                 CDR 3

                                  BamHI
    GGAACCTCAGTCACCGTCTCCTCAGGTGAGTGGATCC
421 ---------+---------+---------+-------- 468
    CCTTGGAGTCAGTGGCAGAGGAGTCCACTCACCTAGG
                        splice donor site
     G   T   S   V   T   V   S   S
```

Fig. 26 /1

```
                                   Spe I
   1  gaattccagc acactggcgg ccgttACTAG TTATTAATAG TAATCAATTA
  51  CGGGGTCATT AGTTCATAGC CCATATATGG AGTTCCGCGT TACATAACTT
 101  ACGGTAAATG GCCCGCCTGG CTGACCGCCC AACGACCCCC GCCCATTGAC
 151  GTCAATAATG ACGTATGTTC CCATAGTAAC GCCAATAGGG ACTTTCCATT
 201  GACGTCAATG GGTGGAGTAT TTACGGTAAA CTGCCCACTT GGCAGTACAT
 251  CAAGTGTATC ATATGCCAAG TACGCCCCCT ATTGACGTCA ATGACGGTAA
 301  ATGGCCCGCC TGGCATTATG CCCAGTACAT GACCTTATGG GACTTTCCTA
                    SnaB I
 351  CTTGGCAGTA CATCTACGTA TTAGTCATCG CTATTACCAT GGTGATGCGG
 401  TTTTGGCAGT ACATCAATGG GCGTGGATAG CGGTTTGACT CACGGGGATT
 451  TCCAAGTCTC CACCCCATTG ACGTCAATGG GAGTTTGTTT TGGCACCAAA
 501  ATCAACGGGA CTTTCCAAAA TGTCGTAACA ACTCCGCCCC ATTGACGCAA
 551  ATGGGCGGTA GGCGTGTACG GTGGGAGGTC TATATAAGCA GAGCTCGTTT
 601  AGTGAACCGT CAGATCGCCT GGAGACGCCA TCCACGCTGT TTTGACCTCC
                                     Sac II
 651  ATAGAAGACA CCGGGACCGA TCCAGCCTCC GCGGCCGGGA ACGGTGCATT
 701  GGAACGCGGA TTCCCCGTGC CAAGAGTGAC GTAAGTACCG CCTATAGAGT
 751  CTATAGGCCC ACCCCCTTGG CTTCTTATGC ATGCTATACT GTTTTTGGCT
 801  TGGGGTCTAT ACACCCCCGC TTCCTCATGT TATAGGTGAT GGTATAGCTT
 851  AGCCTATAGG TGTGGGTTAT TGACCATTAT TGACCACTCC CCTATTGGTG
 901  ACGATACTTT CCATTACTAA TCCATAACAT GGCTCTTTGC CACAACTCTC
 951  TTTATTGGCT ATATGCCAAT ACACTGTCCT TCAGAGACTG ACACGGACTC
1001  TGTATTTTTA CAGGATGGGG TCTCATTTAT TATTTACAAA TTCACATATA
1051  CAACACCACC GTCCCCAGTG CCCGCAGTTT TTATTAAACA TAACGTGGGA
                                   BspE I
1101  TCTCCACGCG AATCTCGGGT ACGTGTTCCG GACATGGGCT CTTCTCCGGT
1151  AGCGGCGGAG CTTCTACATC CGAGCCCTGC TCCCATGCCT CCAGCGACTC
1201  ATGGTCGCTC GGCAGCTCCT TGCTCCTAAC AGTGGAGGCC AGACTTAGGC
1251  ACAGCACGAT GCCCACCACC ACCAGTGTGC CGCACAAGGC CGTGGCGGTA
```

**Fig. 26 /2**

```
1301  GGGTATGTGT CTGAAAATGA GCTCggggag cgggcttgca ccgctgacgc
                 Afl II
1351  atttggaaga cttaaggcag cggcagaaga agatgcaggc agctgagttg

1401  ttgtgttctg ataagagtca gaggtaactc ccgttgcggt gctgttaacg

1451  gtggagggca gtgtagtctg agcagtactc gttgctgccg cgcgcgccac

1501  cagacataat agctgacaga ctaacagact gttcctttcc atgggtcttt
                             Mlu I        Hind III
1551  tctgcagtca ccgtccttga cacgcgtctc gggaagcttG CCGCCACCAT
                                                       M
                                                     Kpn I
1601  GGATTCACAG GCCCAGGTTC TTATGTTACT GCCGCTATGG GTATCTGGTA
        D  S  Q   A  Q  V   L  M  L  L   P  L  W   V  S  G
1651  CCTGTGGGGA CATTGTGATG TCACAGTCTC CATCCTCCCT AGCTGTGTCA
      T  C  G  D   I  V  M   S  Q  S   P  S  S  L   A  V  S
1701  GTTGGAGAGA AGGTTACTAT GAGCTGCAAG TCCAGTCAGA GCCTTTTATA
       V  G  E   K  V  T  M   S  C  K   S  S  Q   S  L  L  Y
      XbaI                                CDR 1
1751  TTCTAGAAAT CAAAAGAACT ACTTGGCCTG GTTCCAGCAG AAGCCAGGGC
       S  R  N   Q  K  N   Y  L  A  W   F  Q  Q   K  P  G

1801  AGTCTCCTAA ACTGCTGATT TTCTGGGCAT CCACTAGGGA ATCTGGGGTC
      Q  S  P  K   L  L  I   F  W  A   S  T  R  E   S  G  V
                                          CDR 2
1851  CCTGATCGCT TCACAGGCAG TGGATTTGGG ACGGATTTCA ATCTCACCAT
       P  D  R   F  T  G  S   G  F  G   T  D  F   N  L  T  I

1901  CAGCAGTGTG CAGGCTGAGG ACCTGGCAGT TTATGACTGT CAGCAATATT
        S  S  V   Q  A  E   D  L  A  V   Y  D  C   Q  Q  Y

1951  TTAGCTATCC GCTCACGTTC GGTGCTGGGA CCAAGCTGGA GCTGAAACGT
      F  S  Y  P   L  T  F   G  A  G   T  K  L  E   L  K  R
         CDR 3
        BamH I
2001  GAGTggatcc ATCTGGGATA AGCATGCTGT TTTCTGTCTG TCCCTAACAT

2051  GCCCTGTGAT TATGCGCAAA CAACACACCC AAGGGCAGAA CTTTGTTACT

2101  TAAACACCAT CCTGTTTGCT TCTTTCCTCA GGAACTGTGG CTGCACCATC
                                        T  V   A  A  P  S
2151  TGTCTTCATC TTCCCGCCAT CTGATGAGCA GTTGAAATCT GGAACTGCCT
       V  F  I   F  P  P   S  D  E  Q   L  K  S   G  T  A
2201  CTGTTGTGTG CCTGCTGAAT AACTTCTATC CCAGAGAGGC CAAAGTACAG
      S  V  V  C   L  L  N   N  F  Y   P  R  E  A   K  V  Q
2251  TGGAAGGTGG ATAACGCCCT CCAATCGGGT AACTCCCAGG AGAGTGTCAC
       W  K  V   D  N  A  L   Q  S  G   N  S  Q   E  S  V  T
2301  AGAGCAGGAC AGCAAGGACA GCACCTACAG CCTCAGCAGC ACCCTGACGC
        E  Q  D   S  K  D   S  T  Y  S   L  S  S   T  L  T
```

Fig. 26 /3

```
2351  TGAGCAAAGC AGACTACGAG AAACACAAAG TCTACGCCTG CGAAGTCACC
       L  S  K  A   D  Y  E   K  H  K   V  Y  A  C   E  V  T
2401  CATCAGGGCC TGAGCTCGCC CGTCACAAAG AGCTTCAACA GGGGAGAGTG
        H  Q  G   L  S  S  P   V  T  K   S  F  N   R  G  E  C
2451  TTAGAGGGAG AAGTGCCCCC ACCTGCTCCT CAGTTCCAGC CTGACCCCCT
        *
2501  CCCATCCTTT GGCCTCTGAC CCTTTTTCCA CAGGGGACCT ACCCCTATTG

2551  CGGTCCTCCA GCTCATCTTT CACCTCACCC CCCTCCTCCT CCTTGGCTTT

2601  AATTATGCTA ATGTTGGAGG AGAATGAATA AATAAAGTGA ATCTTTGCAC

2651  CTGTGGTGGA TCTAATAAAA GATATTTATT TTCATTAGAT ATGTGTGTTG

2701  GTTTTTTGTG TGCAGTGCCT CTATCTGGAG GCCAGGTAGG GCTGGCCTTG

2751  GGGGAGGGGG AGGCCAGAAT GACTCCAAGA GCTACAGGAA GGCAGGTCAG

2801  AGACCCCACT GGACAAACAG TGGCTGGACT CTGCACCATA ACACACAATC

2851  AACAGGGGAG TGAGCTGGAA ATTTGCTAGC GAATTCTTGA AGACGAAAGG

2901  GCCTCGTGAT ACGCCTATTT TTATAGGTTA ATGTCATGAT AATAATGGTT

2951  TCTTAGACGT CAGGTGGCAC TTTTCGGGGA AATGTGCGCG GAACCCCTAT

3001  TTGTTTATTT TTCTAAATAC ATTCAAATAT GTATCCGCTC ATGAGACAAT

3051  AACCCTGATA AATGCTTCAA TAATATTGAA AAAGGAAGAG TATGAGTATT

3101  CAACATTTCC GTGTCGCCCT TATTCCCTTT TTTGCGGCAT TTTGCCTTCC

3151  TGTTTTTGCT CACCCAGAAA CGCTGGTGAA AGTAAAAGAT GCTGAAGATC

3201  AGTTGGGTGC ACGAGTGGGT TACATCGAAC TGGATCTCAA CAGCGGTAAG

3251  ATCCTTGAGA GTTTTCGCCC CGAAGAACGT TTTCCAATGA TGAGCACTTT

3301  TAAAGTTCTG CTATGTGGCG CGGTATTATC CCGTGTTGAC GCCGGGCAAG

3351  AGCAACTCGG TCGCCGCATA CACTATTCTC AGAATGACTT GGTTGAGTAC

3401  TCACCAGTCA CAGAAAAGCA TCTTACGGAT GGCATGACAG TAAGAGAATT

3451  ATGCAGTGCT GCCATAACCA TGAGTGATAA CACTGCGGCC AACTTACTTC
                Pvu I
3501  TGACAACGAT CGGAGGACCG AAGGAGCTAA CCGCTTTTTT GCACAACATG

3551  GGGGATCATG TAACTCGCCT TGATCGTTGG GAACCGGAGC TGAATGAAGC

3601  CATACCAAAC GACGAGCGTG ACACCACGAT GCCTGCAGCA ATGGCAACAA
```

**Fig. 26 /4**

3651 CGTTGCGCAA ACTATTAACT GGCGAACTAC TTACTCTAGC TTCCCGGCAA

3701 CAATTAATAG ACTGGATGGA GGCGGATAAA GTTGCAGGAC CACTTCTGCG

3751 CTCGGCCCTT CCGGCTGGCT GGTTTATTGC TGATAAATCT GGAGCCGGTG

3801 AGCGTGGGTC TCGCGGTATC ATTGCAGCAC TGGGGCCAGA TGGTAAGCCC

3851 TCCCGTATCG TAGTTATCTA CACGACGGGG AGTCAGGCAA CTATGGATGA

3901 ACGAAATAGA CAGATCGCTG AGATAGGTGC CTCACTGATT AAGCATTGGT

3951 AACTGTCAGA CCAAGTTTAC TCATATATAC TTTAGATTGA TTTAAAACTT

4001 CATTTTTAAT TTAAAAGGAT CTAGGTGAAG ATCCTTTTTG ATAATCTCAT

4051 GACCAAAATC CCTTAACGTG AGTTTTCGTT CCACTGAGCG TCAGACCCCG

4101 TAGAAAAGAT CAAAGGATCT TCTTGAGATC CTTTTTTTCT GCGCGTAATC

4151 TGCTGCTTGC AAACAAAAAA ACCACCGCTA CCAGCGGTGG TTTGTTTGCC

4201 GGATCAAGAG CTACCAACTC TTTTTCCGAA GGTAACTGGC TTCAGCAGAG

4251 CGCAGATACC AAATACTGTC CTTCTAGTGT AGCCGTAGTT AGGCCACCAC

4301 TTCAAGAACT CTGTAGCACC GCCTACATAC CTCGCTCTGC TAATCCTGTT

4351 ACCAGTGGCT GCTGCCAGTG GCGATAAGTC GTGTCTTACC GGGTTGGACT

4401 CAAGACGATA GTTACCGGAT AAGGCGCAGC GGTCGGGCTG AACGGGGGGT

4451 TCGTGCACAC AGCCCAGCTT GGAGCGAACG ACCTACACCG AACTGAGATA

4501 CCTACAGCGT GAGCTATGAG AAAGCGCCAC GCTTCCCGAA GGGAGAAAGG

4551 CGGACAGGTA TCCGGTAAGC GGCAGGGTCG GAACAGGAGA GCGCACGAGG

4601 GAGCTTCCAG GGGGAAACGC CTGGTATCTT TATAGTCCTG TCGGGTTTCG

4651 CCACCTCTGA CTTGAGCGTC GATTTTTGTG ATGCTCGTCA GGGGGGCGGA

4701 GCCTATGGAA AAACGCCAGC AACGCGGCCT TTTTACGGTT CCTGGCCTTT

BspLU11I

4751 TGCTGGCCTT TTGCTC**ACAT GT**TCTTTCCT GCGTTATCCC CTGATTCTGT

4801 GGATAACCGT ATTACCGCCT TTGAGTGAGC TGATACCGCT CGCCGCAGCC

4851 GAACGACCGA GCGCAGCGAG TCAGTGAGCG AGGAAGCGGA AGAGCGCCTG

4901 ATGCGGTATT TTCTCCTTAC GCATCTGTGC GGTATTTCAC ACCGCATATG

Fig. 26 /5

```
                                                                Bst1107I
4951  GTGCACTCTC AGTACAATCT GCTCTGATGC CGCATAGTTA AGCCAGTATA

5001  CACTCCGCTA TCGCTACGTG ACTGGGTCAT GGCTGCGCCC CGACACCCGC

5051  CAACACCCGC TGACGCGCCC TGACGGGCTT GTCTGCTCCC GGCATCCGCT

5101  TACAGACAAG CTGTGACCGT CTCCGGGAGC TGCATGTGTC AGAGGTTTTC

5151  ACCGTCATCA CCGAAACGCG CGAGGCAGCT GTGGAATGTG TGTCAGTTAG

5201  GGTGTGGAAA GTCCCCAGGC TCCCCAGCAG GCAGAAGTAT GCAAAGCATG

5251  CATCTCAATT AGTCAGCAAC CAGGCTCCCC AGCAGGCAGA AGTATGCAAA

5301  GCATGCATCT CAATTAGTCA GCAACCATAG TCCCGCCCCT AACTCCGCCC

5351  ATCCCGCCCC TAACTCCGCC CAGTTCCGCC CATTCTCCGC CCCATGGCTG
                                              Sfi I
5401  ACTAATTTTT TTTATTTATG CAGAGGCCGA GGCCGCCTCG GCCTCTGAGC
                                           Stu I/Avr II
5451  TATTCCAGAA GTAGTGAGGA GGCTTTTTTG GAGGCCTAGG CTTTTGCAAA

5501  AAGCTAGCTT CACGCTGCCG CAAGCACTCA GGGCGCAAGG GCTGCTAAAG

5551  GAAGCGGAAC ACGTAGAAAG CCAGTCCGCA GAAACGGTGC TGACCCCGGA

5601  TGAATGTCAG CTACTGGGCT ATCTGGACAA GGGAAAACGC AAGCGCAAAG

5651  AGAAAGCAGG TAGCTTGCAG TGGGCTTACA TGGCGATAGC TAGACTGGGC

5701  GGTTTTATGG ACAGCAAGCG AACCGGAATT GCCAGCTGGG GCGCCCTCTG

5751  GTAAGGTTGG GAAGCCCTGC AAAGTAAACT GGATGGCTTT CTTGCCGCCA
                                 Bgl II/Bcl I
5801  AGGATCTGAT GGCGCAGGGG ATCAAGATCT GATCAAGAGA CAGGATGAGG

5851  ATCGTTTCGC ATGATTGAAC AAGATGGATT GCACGCAGGT TCTCCGGCCG

5901  CTTGGGTGGA GAGGCTATTC GGCTATGACT GGGCACAACA GACAATCGGC

5951  TGCTCTGATG CCGCCGTGTT CCGGCTGTCA GCGCAGGGGC GCCCGGTTCT

6001  TTTTGTCAAG ACCGACCTGT CCGGTGCCCT GAATGAACTG CAGGACGAGG
                         Msc I
6051  CAGCGCGGCT ATCGTGGCTG GCCACGACGG GCGTTCCTTG CGCAGCTGTG

6101  CTCGACGTTG TCACTGAAGC GGGAAGGGAC TGGCTGCTAT TGGGCGAAGT

6151  GCCGGGGCAG GATCTCCTGT CATCTCACCT TGCTCCTGCC GAGAAAGTAT

6201  CCATCATGGC TGATGCAATG CGGCGGCTGC ATACGCTTGA TCCGGCTACC
```

Fig. 26 /6

6251 TGCCCATTCG ACCACCAAGC GAAACATCGC ATCGAGCGAG CACGTACTCG

6301 GATGGAAGCC GGTCTTGTCG ATCAGGATGA TCTGGACGAA GAGCATCAGG

6351 GGCTCGCGCC AGCCGAACTG TTCGCCAGGC TCAAGGCGCG CATGCCCGAC

6401 GGCGAGGATC TCGTCGTGAC CCATGGCGAT GCCTGCTTGC CGAATATCAT

6451 GGTGGAAAAT GGCCGCTTTT CTGGATTCAT CGACTGTGGC CGGCTGGGTG

Rsr II

6501 TGG**CGGACCG** CTATCAGGAC ATAGCGTTGG CTACCCGTGA TATTGCTGAA

6551 GAGCTTGGCG GCGAATGGGC TGACCGCTTC CTCGTGCTTT ACGGTATCGC

6601 CGCTCCCGAT TCGCAGCGCA TCGCCTTCTA TCGCCTTCTT GACGAGTTCT

Nsp V

6651 TCTGAGCGGG ACTCTGGGG**T TCGAA**ATGAC CGACCAAGCG ACGCCCAACC

6701 TGCCATCACG AGATTTCGAT TCCACCGCCG CCTTCTATGA AAGGTTGGGC

6751 TTCGGAATCG TTTTCCGGGA CGCCGGCTGG ATGATCCTCC AGCGCGGGGA

Sma I — Nru I

6801 TCTCATGCTG GAGTTCTTCG CCCAC**CCCGG G**CTCGATCCC C**TCGCGA**GTT

6851 GGTTCAGCTG CTGCCTGAGG CTGGACGACC TCGCGGAGTT CTACCGGCAG

6901 TGCAAATCCG TCGGCATCCA GGAAACCAGC AGCGGCTATC CGCGCATCCA

6951 TGCCCCCGAA CTGCAGGAGT GGGGAGGCAC GATGGCCGCT TTGGTCCCGG

7001 ATCTTTGTGA AGGAACCTTA CTTCTGTGGT GTGACATAAT TGGACAAACT

7051 ACCTACAGAG ATTTAAAGCT CTAAGGTAAA TATAAAATTT TTAAGTGTAT

7101 AATGTGTTAA ACTACTGATT CTAATTGTTT GTGTATTTTA GATTCCAACC

7151 TATGGAACTG ATGAATGGGA GCAGTGGTGG AATGCCTTTA ATGAGGAAAA

7201 CCTGTTTTGC TCAGAAGAAA TGCCATCTAG TGATGATGAG GCTACTGCTG

7251 ACTCTCAACA TTCTACTCCT CCAAAAAAGA AGAGAAAGGT AGAAGACCCC

7301 AAGGACTTTC CTTCAGAATT GCTAAGTTTT TTGAGTCATG CTGTGTTTAG

7351 TAATAGAACT CTTGCTTGCT TTGCTATTTA CACCACAAAG GAAAAAGCTG

7401 CACTGCTATA CAAGAAAATT ATGGAAAAAT ATTCTGTAAC CTTTATAAGT

7451 AGGCATAACA GTTATAATCA TAACATACTG TTTTTTCTTA CTCCACACAG

7501 GCATAGAGTG TCTGCTATTA ATAACTATGC TCAAAAATTG TGTACCTTTA

**Fig. 26 /7**

7551 GCTTTTTAAT TTGTAAAGGG GTTAATAAGG AATATTTGAT GTATAGTGCC

7601 TTGACTAGAG ATCATAATCA GCCATACCAC ATTTGTAGAG GTTTTACTTG

7651 CTTTAAAAAA CCTCCCACAC CTCCCCCTGA ACCTGAAACA TAAAATGAAT

Mun I

7701 G**CAATTG**TTG TTGTTAACTT GTTTATTGCA GCTTATAATG GTTACAAATA

7751 AAGCAATAGC ATCACAAATT TCACAAATAA AGCATTTTTT TCACTGCATT

7801 CTAGTTGTGG TTTGTCCAAA CTCATCAATG TATCTTATCA TGTCTGGATC

7851 TAATAAAAGA TATTTATTTT CATTAGATAT GTGTGTTGGT TTTTTGTGTG

7901 CAGTGCCTCT ATCTGGAGGC CAGGTAGGGC TGGCCTTGGG GGAGGGGGAG

7951 GCCAGAATGA CTCCAAGAGC TACAGGAAGG CAGGTCAGAG ACCCCACTGG

8001 ACAAACAGTG GCTGGACTCT GCACCATAAC ACACAATCAA CAGGGGAGTG

8051 AGCTGGAAAT TTGCTAGC

**Fig. 27/1**

1 TTGAAGACGAAAGGGCCTCGTGATACGCCTATTTTTATAGGTTAATGTCATGATAATAAT

61 GGTTTCTTAGACGTCAGGTGGCACTTTTCGGGGAAATGTGCGCGGAACCCCTATTTGTTT

121 ATTTTTCTAAATACATTCAAATATGTATCCGCTCATGAGACAATAACCCTGATAAATGCT

181 TCAATAATATTGAAAAAGGAAGAGTATGAGTATTCAACATTTCCGTGTCGCCCTTATTCC

241 CTTTTTTGCGGCATTTTGCCTTCCTGTTTTTGCTCACCCAGAAACGCTGGTGAAAGTAAA

301 AGATGCTGAAGATCAGTTGGGTGCACGAGTGGGTTACATCGAACTGGATCTCAACAGCGG

361 TAAGATCCTTGAGAGTTTTCGCCCCGAAGAACGTTTTCCAATGATGAGCACTTTTAAAGT

421 TCTGCTATGTGGCGCGGTATTATCCCGTGTTGACGCCGGGCAAGAGCAACTCGGTCGCCG

481 CATACACTATTCTCAGAATGACTTGGTTGAGTACTCACCAGTCACAGAAAAGCATCTTAC

541 GGATGGCATGACAGTAAGAGAATTATGCAGTGCTGCCATAACCATGAGTGATAACACTGC

Pvu I

601 GGCCAACTTACTTCTGACAA**CGATCG**GAGGACCGAAGGAGCTAACCGCTTTTTTGCACAA

661 CATGGGGGATCATGTAACTCGCCTTGATCGTTGGGAACCGGAGCTGAATGAAGCCATACC

Fsp I

721 AAACGACGAGCGTGACACCACGATGCCTGCAGCAATGGCAACAACGT**TGCGCA**AACTATT

781 AACTGGCGAACTACTTACTCTAGCTTCCCGGCAACAATTAATAGACTGGATGGAGGCGGA

Fig. 27 /2

841 TAAAGTTGCAGGACCACTTCTGCGCTCGGCCCTTCCGGCTGGCTGGTTTATTGCTGATAA

901 ATCTGGAGCCGGTGAGCGTGGGTCTCGCGGTATCATTGCAGCACTGGGGCCAGATGGTAA

961 GCCCTCCCGTATCGTAGTTATCTACACGACGGGGAGTCAGGCAACTATGGATGAACGAAA

1021 TAGACAGATCGCTGAGATAGGTGCCTCACTGATTAAGCATTGGTAACTGTCAGACCAAGT

1081 TTACTCATATATACTTTAGATTGATTTAAAACTTCATTTTTAATTTAAAAGGATCTAGGT

1141 GAAGATCCTTTTTGATAATCTCATGACCAAAATCCCTTAACGTGAGTTTTCGTTCCACTG

1201 AGCGTCAGACCCCGTAGAAAAGATCAAAGGATCTTCTTGAGATCCTTTTTTTCTGCGCGT

1261 AATCTGCTGCTTGCAAACAAAAAAACCACCGCTACCAGCGGTGGTTTGTTTGCCGGATCA

1321 AGAGCTACCAACTCTTTTTCCGAAGGTAACTGGCTTCAGCAGAGCGCAGATACCAAATAC

1381 TGTCCTTCTAGTGTAGCCGTAGTTAGGCCACCACTTCAAGAACTCTGTAGCACCGCCTAC

1441 ATACCTCGCTCTGCTAATCCTGTTACCAGTGGCTGCTGCCAGTGGCGATAAGTCGTGTCT

1501 TACCGGGTTGGACTCAAGACGATAGTTACCGGATAAGGCGCAGCGGTCGGGCTGAACGGG

1561 GGGTTCGTGCACACAGCCCAGCTTGGAGCGAACGACCTACACCGAACTGAGATACCTACA

1621 GCGTGAGCTATGAGAAAGCGCCACGCTTCCCGAAGGGAGAAAGGCGGACAGGTATCCGGT

1681 AAGCGGCAGGGTCGGAACAGGAGAGCGCACGAGGGAGCTTCCAGGGGGAAACGCCTGGTA

1741 TCTTTATAGTCCTGTCGGGTTTCGCCACCTCTGACTTGAGCGTCGATTTTTGTGATGCTC

1801 GTCAGGGGGGCGGAGCCTATGGAAAAACGCCAGCAACGCGGCCTTTTTACGGTTCCTGGC

BspLU11I

1861 CTTTTGCTGGCCTTTTGCTC**ACATGT**TCTTTCCTGCGTTATCCCCTGATTCTGTGGATAA

1921 CCGTATTACCGCCTTTGAGTGAGCTGATACCGCTCGCCGCAGCCGAACGACCGAGCGCAG

1981 CGAGTCAGTGAGCGAGGAAGCGGAAGAGCGCCTGATGCGGTATTTTCTCCTTACGCATCT

2041 GTGCGGTATTTCACACCGCATATGGTGCACTCTCAGTACAATCTGCTCTGATGCCGCATA

Bst1107 I

2101 GTTAAGCCA**GTATAC**ACTCCGCTATCGCTACGTGACTGGGTCATGGCTGCGCCCCGACAC

2161 CCGCCAACACCCGCTGACGCGCCCTGACGGGCTTGTCTGCTCCCGGCATCCGCTTACAGA

2221 CAAGCTGTGACCGTCTCCGGGAGCTGCATGTGTCAGAGGTTTTCACCGTCATCACCGAAA

2281 CGCGCGAGGCAGCATGCATCTCAATTAGTCAGCAACCATAGTCCCGCCCCTAACTCCGCC

2341 CATCCCGCCCCTAACTCCGCCCAGTTCCGCCCATTCTCCGCCCCATGGCTGACTAATTTT

Sfi I

2401 TTTTATTTATGCAGAGGCCGA**GGCCGCCTCGGCC**TCTGAGCTATTCCAGAAGTAGTGAGG

Stu I/Avr II

2461 AGGCTTTTTTGG**AGGCCTAGG**CTTTTGCAAAAAGCTAGCTTACAGCTCAGGGCTGCGATT

**Fig. 27 /3**

2521 TCGCGCCAAACTTGACGGCAATCCTAGCGTGAAGGCTGGTAGGATTTTATCCCCGCTGCC

2581 ATCATGGTTCGACCATTGAACTGCATCGTCGCCGTGTCCCAAAATATGGGGATTGGCAAG

2641 AACGGAGACCTACCCTGGCCTCCGCTCAGGAACGAGTTCAAGTACTTCCAAAGAATGACC

2701 ACAACCTCTTCAGTGGAAGGTAAACAGAATCTGGTGATTATGGGTAGGAAAACCTGGTTC

2761 TCCATTCCTGAGAAGAATCGACCTTTAAAGGACAGAATTAATATAGTTCTCAGTAGAGAA

2821 CTCAAAGAACCACCACGAGGAGCTCATTTTCTTGCCAAAAGTTTGGATGATGCCTTAAGA

2881 CTTATTGAACAACCGGAATTGGCAAGTAAAGTAGACATGGTTTGGATAGTCGGAGGCAGT

2941 TCTGTTTACCAGGAAGCCATGAATCAACCAGGCCACCTCAGACTCTTTGTGACAAGGATC

3001 ATGCAGGAATTTGAAAGTGACACGTTTTTCCCAGAAATTGATTTGGGGAAATATAAACTT

3061 CTCCCAGAATACCCAGGCGTCCTCTCTGAGGTCCAGGAGGAAAAAGGCATCAAGTATAAG

3121 TTTGAAGTCTACGAGAAGAAAGACTAACAGCAAGATGCTTTCAAGTTCTCTGCCCCTC

Bgl II

3181 CTAAAGCTATGCATTTTTATAAGACCATGGGACTTTTGCTGGCTTT**AGATCT**TTGTGAAG

3241 GAACCTTACTTCTGTGGTGTGACATAATTGGACAAACTACCTACAGAGATTTAAAGCTCT

3301 AAGGTAAATATAAAATTTTTAAGTGTATAATGTGTTAAACTACTGATTCTAATTGTTTGT

3361 GTATTTTAGATTCCAACCTATGGAACTGATGAATGGGAGCAGTGGTGGAATGCCTTTAAT

3421 GAGGAAAACCTGTTTTGCTCAGAAGAAATGCCATCTAGTGATGATGAGGCTACTGCTGAC

3481 TCTCAACATTCTACTCCTCCAAAAAAGAAGAGAAAGGTAGAAGACCCCAAGGACTTTCCT

3541 TCAGAATTGCTAAGTTTTTTGAGTCATGCTGTGTTTAGTAATAGAACTCTTGCTTGCTTT

3601 GCTATTTACACCACAAAGGAAAAAGCTGCACTGCTATACAAGAAAATTATGGAAAAATAT

3661 TCTGTAACCTTTATAAGTAGGCATAACAGTTATAATCATAACATACTGTTTTTTCTTACT

3721 CCACACAGGCATAGAGTGTCTGCTATTAATAACTATGCTCAAAAATTGTGTACCTTTAGC

3781 TTTTTAATTTGTAAAGGGGTTAATAAGGAATATTTGATGTATAGTGCCTTGACTAGA**GAT**

BsaB I

3841 **CATAATC**AGCCATACCACATTTGTAGAGGTTTTACTTGCTTTAAAAAACCTCCCACACCT

Mun I

3901 CCCCCTGAACCTGAAACATAAAATGAATG**CAATTG**TTGTTGTTAACTTGTTTATTGCAGC

3961 TTATAATGGTTACAAATAAAGCAATAGCATCACAAATTTCACAAATAAAGCATTTTTTTC

4021 ACTGCATTCTAGTTGTGGTTTGTCCAAACTCATCAATGTATCTTATCATGTCTGGATCTA

4081 ATAAAAGATATTTATTTTCATTAGATATGTGTGTTGGTTTTTTGTGTGCAGTGCCTCTAT

4141 CTGGAGGCCAGGTAGGGCTGGCCTTGGGGGAGGGGGAGGCCAGAATGACTCCAAGAGCTA

4201 CAGGAAGGCAGGTCAGAGACCCCACTGGACAAACAGTGGCTGGACTCTGCACCATAACAC

**Fig. 27 /4**

```
                                                     EcoR I
4261 ACAATCAACAGGGGAGTGAGCTGGAAATTTGCTAGCGAATTCcagcacactggcggccgt
       Spe I
4321 tACTAGTTATTAATAGTAATCAATTACGGGGTCATTAGTTCATAGCCCATATATGGAGTT

4381 CCGCGTTACATAACTTACGGTAAATGGCCCGCCTGGCTGACCGCCCAACGACCCCCGCCC

4441 ATTGACGTCAATAATGACGTATGTTCCCATAGTAACGCCAATAGGGACTTTCCATTGACG

4501 TCAATGGGTGGAGTATTTACGGTAAACTGCCCACTTGGCAGTACATCAAGTGTATCATAT

4561 GCCAAGTACGCCCCCTATTGACGTCAATGACGGTAAATGGCCCGCCTGGCATTATGCCCA
                                                    SnaB I
4621 GTACATGACCTTATGGGACTTTCCTACTTGGCAGTACATCTACGTATTAGTCATCGCTAT

4681 TACCATGGTGATGCGGTTTTGGCAGTACATCAATGGGCGTGGATAGCGGTTTGACTCACG

4741 GGGATTTCCAAGTCTCCACCCCATTGACGTCAATGGGAGTTTGTTTTGGCACCAAAATCA

4801 ACGGGACTTTCCAAAATGTCGTAACAACTCCGCCCCATTGACGCAAATGGGCGGTAGGCG

4861 TGTACGGTGGGAGGTCTATATAAGCAGAGCTCGTTTAGTGAACCGTCAGATCGCCTGGAG

4921 ACGCCATCCACGCTGTTTTGACCTCCATAGAAGACACCGGGACCGATCCAGCCTCCGCGG

4981 CCGGGAACGGTGCATTGGAACGCGGATTCCCCGTGCCAAGAGTGACGTAAGTACCGCCTA

5041 TAGAGTCTATAGGCCCACCCCCTTGGCTTCTTATGCATGCTATACTGTTTTTGGCTTGGG
                                                       Bpu1102I
5101 GTCTATACACCCCCGCTTCCTCATGTTATAGGTGATGGTATAGCTTAGCCTATAGGTGTG
                         Xcm I
5161 GGTTATTGACCATTATTGACCACTCCCCTATTGGTGACGATACTTTCCATTACTAATCCA

5221 TAACATGGCTCTTTGCCACAACTCTCTTTATTGGCTATATGCCAATACACTGTCCTTCAG

5281 AGACTGACACGGACTCTGTATTTTTACAGGATGGGGTCTCATTTATTATTTACAAATTCA

5341 CATATACAACACCACCGTCCCCAGTGCCCGCAGTTTTTATTAAACATAACGTGGGATCTC
                          BspE I
5401 CACGCGAATCTCGGGTACGTGTTCCGGACATGGGCTCTTCTCCGGTAGCGGCGGAGCTTC

5461 TACATCCGAGCCCTGCTCCCATGCCTCCAGCGACTCATGGTCGCTCGGCAGCTCCTTGCT

5521 CCTAACAGTGGAGGCCAGACTTAGGCACAGCACGATGCCCACCACCACCAGTGTGCCGCA

5581 CAAGGCCGTGGCGGTAGGGTATGTGTCTGAAAATGAGCTCggggagcgggcttgcaccgc
                                                      (Pvu II)
5641 tgacgcatttggaagacttaaggcagcggcagaagaagatgcaggcagctgagttgttgt

5701 gttctgataagagtcagaggtaactcccgttgcggtgctgttaacggtggagggcagtgt

5761 agtctgagcagtactcgttgctgccgcgcgcgccaccagacataatagctgacagactaa
                                                         Mlu I
5821 cagactgttcctttccatgggtcttttctgcagtcaccgtccttgacACGCGTCTCGGGA
  Hind III
5881 AGCTTGCCGCCACCATGGGATGGAGCTGGGTCTTTCTCTTTCTCCTGTCAGGAACTGCAG
                    M  G  W  S  W  V  F  L  F  L  L  S  G  T  A
```

**Fig. 27 /5**

```
                        (Pvu II)
5941 GTGTCCTCTCTGAGGTCCAGCTGCAACAGTCTGGACCTGAGCTGGTGAAGCCTGGGGCTT
     G  V  L  S  E  V  Q  L  Q  Q  S  G  P  E  L  V  K  P  G  A
                                Xba I                        Dra III
6001 CAGTAAAGATGTCCTGCAAGACTTCTAGATACACATTCACTGAATACACCATACACTGGG
     S  V  .K  M  S  C  K  T  S  R  Y  T  F  T  E  Y  T  I  H  W
                                                    CDR 1
6061 TGAGACAGAGCCATGGAAAGAGCCTTGAGTGGATTGGAGGTATTAATCCTAACAATGGTA

     V  R  Q  S  H  G  K  S  L  E  W  I  G  G  I  N  P  N  N  G
6121 TTCCTAACTACAACCAGAAGTTCAAGGGCAGGGCCACATTGACTGTAGGCAAGTCCTCCA
     I  P  N  Y  N  Q  K  F  K  G  R  A  T  L  T  V  G  K  S  S
           CDR 2
6181 GCACCGCCTACATGGAGCTCCGCAGCCTGACATCTGAGGATTCTGCGGTCTATTTCTGTG
     S  T  A  Y  M  E  L  R  S  L  T  S  E  D  S  A  V  Y  F  C

6241 CAAGAAGAAGAATCGCCTATGGTTACGACGAGGGCCATGCTATGGACTACTGGGGTCAAG
     A  R  R  R  I  A  Y  G  Y  D  E  G  H  A  M  D  Y  W  G  Q
                     CDR 3           BamH I
6301 GAACCTCAGTCACCGTCTCCTCAGGTGAGTGGATCCTCTGCGCCTGGGCCCAGCTCTGTC
     G  T  S  V  T  V  S  S

6361 CCACACCGCGGTCACATGGCACCACCTCTCTTGCAGCCTCCACCAAGGGCCCATCGGTCT
                                                 S  T  K  G  P  S  V

6421 TCCCCCTGGCACCCTCCTCCAAGAGCACCTCTGGGGGCACAGCGGCCCTGGGCTGCCTGG
     F  P  L  A  P  S  S  K  S  T  S  G  G  T  A  A  L  G  C  L
                                Age I
6481 TCAAGGACTACTTCCCCGAACCGGTGACGGTGTCGTGGAACTCAGGCGCCCTGACCAGCG
     V  K  D  Y  F  P  E  P  V  T  V  S  W  N  S  G  A  L  T  S

6541 GCGTGCACACCTTCCCGGCTGTCCTACAGTCCTCAGGACTCTACTCCCTCAGCAGCGTGG
     G  V  H  T  F  P  A  V  L  Q  S  S  G  L  Y  S  L  S  S  V
   BstE II
6601 TGACCGTGCCCTCCAGCAGCTTGGGCACCCAGACCTACATCTGCAACGTGAATCACAAGC
     V  T  V  P  S  S  S  L  G  T  Q  T  Y  I  C  N  V  N  H  K

6661 CCAGCAACACCAAGGTGGACAAGAAAGTTGAGCCCAAATCTTGTGACAAAACTCACACAT
     P  S  N  T  K  V  D  K  K  V  E  P  K  S  C  D  K  T  H  T

6721 GCCCACCGTGCCCAGCACCTGAACTCCTGGGGGGACCGTCAGTCTTCCTCTTCCCCCCAA
     C  P  P  C  P  A  P  E  L  L  G  G  P  S  V  F  L  F  P  P

6781 AACCCAAGGACACCCTCATGATCTCCCGGACCCCTGAGGTCACATGCGTGGTGGTGGACG
   ..... K  P  K  D  T  L  M  I  S  R  T  P  E  V  T  C  V  V  V  D

6841 TGAGCCACGAAGACCCTGAGGTCAAGTTCAACTGGTACGTGGACGGCGTGGAGGTGCATA
     V  S  H  E  D  P  E  V  K  F  N  W  Y  V  D  G  V  E  V  H

6901 ATGCCAAGACAAAGCCGCGGGAGGAGCAGTACAACAGCACGTACCGGGTGGTCAGCGTCC
     N  A  K  T  K  P  R  E  E  Q  Y  N  S  T  Y  R  V  V  S  V

6961 TCACCGTCCTGCACCAGGACTGGCTGAATGGCAAGGAGTACAAGTGCAAGGTCTCCAACA
     L  T  V  L  H  Q  D  W  L  N  G  K  E  Y  K  C  K  V  S  N
```

**Fig. 27 /6**

```
7021 AAGCCCTCCCAGCCCCCATCGAGAAAACCATCTCCAAAGCCAAAGGGCAGCCCCGAGAAC
      K  A  L  P  A  P  I  E  K  T  I  S  K  A  K  G  Q  P  R  E

7081 CACAGGTGTACACCCTGCCCCCATCCCGGGAGGAGATGACCAAGAACCAGGTCAGCCTGA
      P  Q  V  Y  T  L  P  P  S  R  E  E  M  T  K  N  Q  V  S  L

7141 CCTGCCTGGTCAAAGGCTTCTATCCCAGCGACATCGCCGTGGAGTGGGAGAGCAATGGGC
      T  C  L  V  K  G  F  Y  P  S  D  I  A  V  E  W  E  S  N  G

7201 AGCCGGAGAACAACTACAAGACCACGCCTCCCGTGCTGGACTCCGACGGCTCCTTCTTCC
      Q  P  E  N  N  Y  K  T  T  P  P  V  L  D  S  D  G  S  F  F

7261 TCTACAGCAAGCTCACCGTGGACAAGAGCAGGTGGCAGCAGGGGAACGTCTTCTCATGCT
      L  Y  S  K  L  T  V  D  K  S  R  W  Q  Q  G  N  V  F  S  C

7321 CCGTGATGCATGAGGCTCTGCACAACCACTACACGCAGAAGAGCCTCTCCCTGTCTCCGG
      S  V  M  H  E  A  L  H  N  H  Y  T  Q  K  S  L  S  L  S  P
                          NgoM I
7381 GTAAATGAGTGCGACGGCCGGCAAGCCCCGCTCCCCGGGCTCTCGCGGTCGCACGAGGAT
      G  K  *
7441 GCTTGGCACGTACCCCCTGTACATACTTCCCGGGCGCCCAGCATGGAAATAAAGCACCGG

7501 ATCTAATAAAAGATATTTATTTTCATTAGATATGTGTGTTGGTTTTTTGTGTGCAGTGCC

7561 TCTATCTGGAGGCCAGGTAGGGCTGGCCTTGGGGGAGGGGGAGGCCAGAATGACTCCAAG

7621 AGCTACAGGAAGGCAGGTCAGAGACCCCACTGGACAAACAGTGGCTGGACTCTGCACCAT

7681 AACACACAATCAACAGGGGAGTGAGCTGGaaatttgctagcgaattaattc 7731
```

Fig. 28:

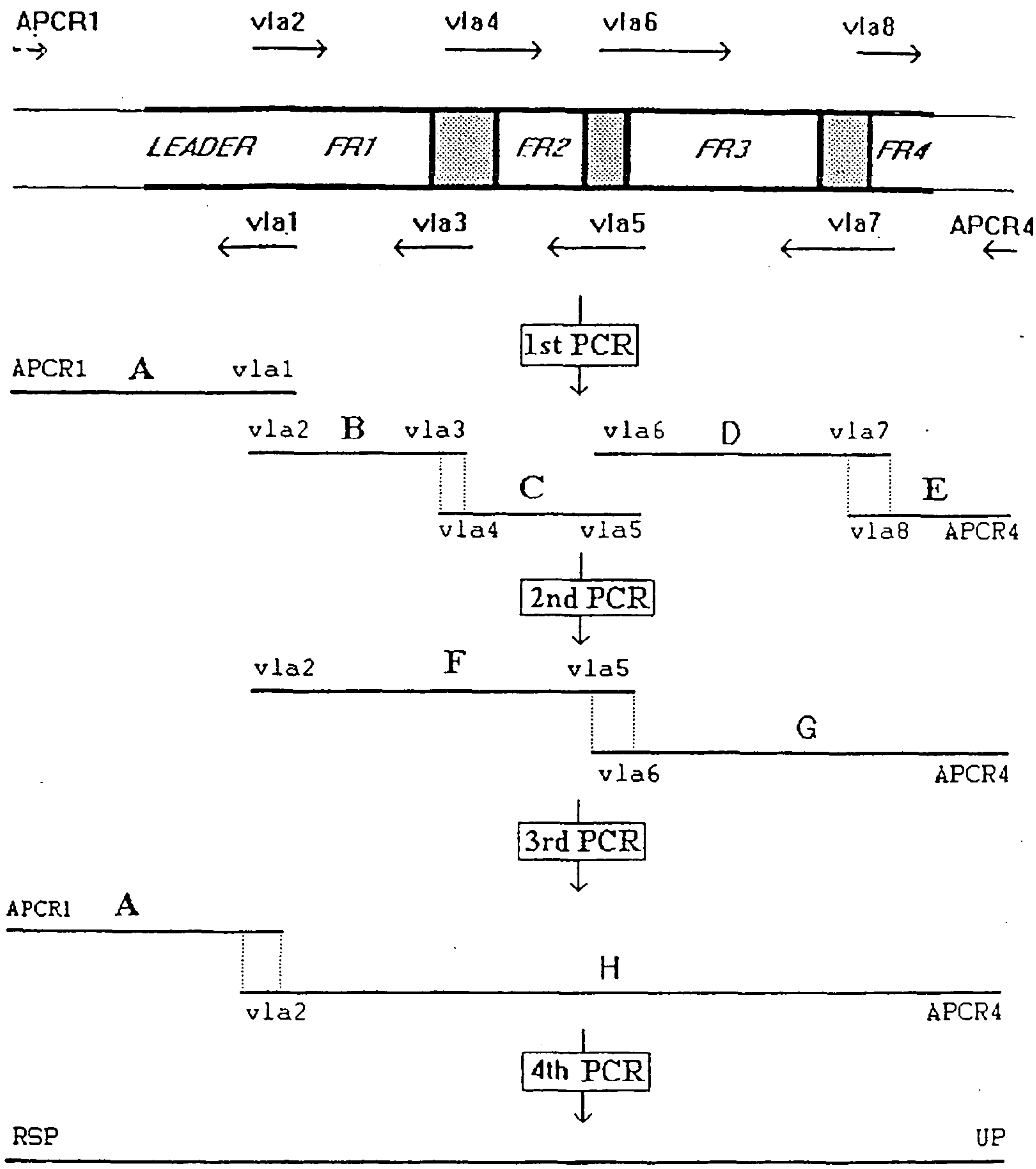
APCR1
vla2
vla4
vla6
vla8
LEADER
FR1
FR2
FR3
FR4
vla1
vla3
vla5
vla7
APCR4
1st PCR
APCR1 A vla1
vla2 B vla3
vla6 D vla7
C
E
vla4 vla5
vla8 APCR4
2nd PCR
vla2 F vla5
G
vla6 APCR4
3rd PCR
APCR1 A
H
vla2 APCR4
4th PCR
RSP
UP

## Fig. 29 /1

```
   1                                                                          19
   D   I   V   M   T   Q   S   P   D   S   L   A   V   S   L   G   E   R   A
A  GAC ATT GTG ATG ACC CAA TCT CCA GAC TCT TTG GCT GTG TCT CTA GGG GAG AGG GCC
    .   .   .   .   .   .   .   .   .   .   .   .   .   .   .   .   .   .   .
B  --- --- --- --- --- --- --- --- --- --- --- --- --- --- --- --- --- --- ---
    .   .   .   .   .   .   .   .   .   .   .   .   .   .   .   .   .   .   .
C  --- --- --- --- --- --- --- --- --- --- --- --- --- --- --- --- --- --- ---

   20                   CDR1    27  A   B   C   D   E   F   28              32
   T   I   N   C  | K   S   S   Q   S   L   L   Y   S   R   N   Q   K   N   Y
A  ACC ATC AAC TGC|AAG TCC AGT CAG AGC CTT TTA TAT TCT AGA AAT CAA AAG AAC TAC
    .   .   .   . | .   .   .   .   .   .   .   .   .   .   .   .   .   .   .
B  --- --- --- ---|--- --- --- --- --- --- --- --- --- --- --- --- --- --- ---
    .   .   .   . | .   .   .   .   .   .   .   .   .   .   .   .   .   .   .
C  --- --- --- ---|--- --- --- --- --- --- --- --- --- --- --- --- --- --- ---

   33                                                                       51
   L   A  | W   Y   Q   Q   K   P   G   Q   P   P   K   L   L   I   F  | W   A
A  TTG GCC|TGG TAT CAG CAG AAA CCA GGA CAG CCA CCC AAA CTC CTC ATC TTT|TGG GCT
    .   . | .   F   .   .   .   .   .   .   .   .   .   .   .   .   . | .   .
B  --- ---|--- -TC --- --- --- --- --- --- --- --- --- --- --- --- ---|--- ---
    .   . | .   .   .   .   .   .   .   .   .   .   .   .   .   .   Y | .   .
C  --- ---|--- --- --- --- --- --- --- --- --- --- --- --- --- --- -A-|--- ---

   52        CDR2                                                       70
   S   T   R   E   S  | G   V   P   D   R   F   S   G   S   G   F   G   T   D
A  AGC ACT AGG GAA TCT|GGG GTA CCT GAT AGG TTC AGT GGC AGT GGG TTT GGG ACA GAC
    .   .   .   .   . | .   .   .   .   .   .   .   .   .   .   .   .   .   .
B  --- --- --- --- ---|--- --- --- --- --- --- --- --- --- --- --- --- --- ---
    .   .   .   .   . | .   .   .   .   .   .   .   .   .   .   .   .   .   .
C  --- --- --- --- ---|--- --- --- --- --- --- --- --- --- --- --- --- --- ---

   71                                                               88
   F   T   L   T   I   S   S   L   Q   A   E   D   V   A   V   Y   Y   C
A  TTC ACC CTC ACC ATT AGC AGC CTG CAG GCT GAA GAT GTG GCA GTT TAT TAC TGT
    .   .   .   .   .   .   .   .   .   .   .   .   .   .   .   .   D   .
B  --- --- --- --- --- --- --- --- --- --- --- --- --- --- --- --- G-- ---
    .   .   .   .   .   .   .   .   .   .   .   .   .   .   .   .   .   .
C  --- --- --- --- --- --- --- --- --- --- --- --- --- --- --- --- --- ---

   89               CDR3                                            107
 | Q   Q   Y   F   S   Y   P   L   T  | F   G   Q   G   T   K   V   E   I   K
A| CAG CAA TAT TTT AGC TAT CCG CTC ACG|TTC GGA CAA GGG ACC AAG GTG GAA ATA AAA
 |  .   .   .   .   .   .   .   .   . | .   .   .   .   .   .   .   .   .   .
B| --A --- --- --- --- --- --- --- ---|--- --- --- --- --- --- --- --- --- ---
 |  .   .   .   .   .   .   .   .   . | .   .   .   .   .   .   .   .   .   .
C| --- --- --- --- --- --- --- --- ---|--- --- --- --- --- --- --- --- --- ---
```

**Fig. 30 /1**

```
                                    Spe I
   1  gaattccagc acactggcgg ccgttACTAG TTATTAATAG TAATCAATTA

  51  CGGGGTCATT AGTTCATAGC CCATATATGG AGTTCCGCGT TACATAACTT

 101  ACGGTAAATG GCCCGCCTGG CTGACCGCCC AACGACCCCC GCCCATTGAC

 151  GTCAATAATG ACGTATGTTC CCATAGTAAC GCCAATAGGG ACTTTCCATT

 201  GACGTCAATG GGTGGAGTAT TTACGGTAAA CTGCCCACTT GGCAGTACAT

 251  CAAGTGTATC ATATGCCAAG TACGCCCCCT ATTGACGTCA ATGACGGTAA

 301  ATGGCCCGCC TGGCATTATG CCCAGTACAT GACCTTATGG GACTTTCCTA
                    SnaB I
 351  CTTGGCAGTA CATCTACGTA TTAGTCATCG CTATTACCAT GGTGATGCGG

 401  TTTTGGCAGT ACATCAATGG GCGTGGATAG CGGTTTGACT CACGGGGATT

 451  TCCAAGTCTC CACCCCATTG ACGTCAATGG GAGTTTGTTT TGGCACCAAA

 501  ATCAACGGGA CTTTCCAAAA TGTCGTAACA ACTCCGCCCC ATTGACGCAA

 551  ATGGGCGGTA GGCGTGTACG GTGGGAGGTC TATATAAGCA GAGCTCGTTT

 601  AGTGAACCGT CAGATCGCCT GGAGACGCCA TCCACGCTGT TTTGACCTCC
                                        Sac II
 651  ATAGAAGACA CCGGGACCGA TCCAGCCTCC GCGGCCGGGA ACGGTGCATT

 701  GGAACGCGGA TTCCCCGTGC CAAGAGTGAC GTAAGTACCG CCTATAGAGT

 751  CTATAGGCCC ACCCCCTTGG CTTCTTATGC ATGCTATACT GTTTTTGGCT

 801  TGGGGTCTAT ACACCCCCGC TTCCTCATGT TATAGGTGAT GGTATAGCTT

 851  AGCCTATAGG TGTGGGTTAT TGACCATTAT TGACCACTCC CCTATTGGTG

 901  ACGATACTTT CCATTACTAA TCCATAACAT GGCTCTTTGC CACAACTCTC

 951  TTTATTGGCT ATATGCCAAT ACACTGTCCT TCAGAGACTG ACACGGACTC

1001  TGTATTTTTA CAGGATGGGG TCTCATTTAT TATTTACAAA TTCACATATA

1051  CAACACCACC GTCCCCAGTG CCCGCAGTTT TTATTAAACA TAACGTGGGA
                                   (BspE I)
1101  TCTCCACGCG AATCTCGGGT ACGTGTTCCG GACATGGGCT CTTCTCCGGT

1151  AGCGGCGGAG CTTCTACATC CGAGCCCTGC TCCCATGCCT CCAGCGACTC

1201  ATGGTCGCTC GGCAGCTCCT TGCTCCTAAC AGTGGAGGCC AGACTTAGGC
```

## Fig. 30 /2

```
1251 ACAGCACGAT GCCCACCACC ACCAGTGTGC CGCACAAGGC CGTGGCGGTA

1301 GGGTATGTGT CTGAAAATGA GCTCggggag cgggcttgca ccgctgacgc
                Afl II
1351 atttggaaga cttaaggcag cggcagaaga agatgcaggc agctgagttg

1401 ttgtgttctg ataagagtca gaggtaactc ccgttgcggt gctgttaacg

1451 gtggagggca gtgtagtctg agcagtactc gttgctgccg cgcgcgccac

1501 cagacataat agctgacaga ctaacagact gttcctttcc atgggtcttt
                                 Mlu I        Hind III
1551 tctgcagtca ccgtccttga cacgcgtctc gggaagcttG CCGCCACCAT
                                                        M
1601 GGAGACAGAC ACACTCCTGC TATGGGTGCT GCTGCTCTGG GTTCCAGGTT
       E  T  D   T  L  L   L  W  V  L   L  L  W   V  P  G
     (BspE I)
1651 CCTCCGGAGA CATTGTGATG ACCCAATCTC CAGACTCTTT GGCTGTGTCT
     S  S  G  D   I  V  M   T  Q  S   P  D  S  L   A  V  S

1701 CTAGGGGAGA GGGCCACCAT CAACTGCAAG TCCAGTCAGA GCCTTTTATA
      L  G  E   R  A  T  I   N  C  K   S  S  Q   S  L  L  Y
      XbaI                                        CDR 1
1751 TTCTAGAAAT CAAAAGAACT ACTTGGCCTG GTATCAGCAG AAACCAGGAC
       S  R  N   Q  K  N   Y  L  A  W   Y  Q  Q   K  P  G
                                                      KpnI
1801 AGCCACCCAA ACTCCTCATC TTTTGGGCTA GCACTAGGGA ATCTGGGGTA
     Q  P  P  K   L  L  I   F  W  A   S  T  R  E   S  G  V
                                          CDR 2
1851 CCTGATAGGT TCAGTGGCAG TGGGTTTGGG ACAGACTTCA CCCTCACCAT
     P  D  R   F  S  G  S   G  F  G   T  D  F   T  L  T  I

1901 TAGCAGCCTG CAGGCTGAAG ATGTGGCAGT TTATTACTGT CAGCAATATT
       S  S  L   Q  A  E   D  V  A  V   Y  Y  C   Q  Q  Y
1951 TTAGCTATCC GCTCACGTTC GGACAAGGGA CCAAGGTGGA AATAAAACGT
     F  S  Y  P   L  T  F   G  Q  G   T  K  V  E   I  K  R
          CDR 3
       BamH I
2001 GAGTggatcc ATCTGGGATA AGCATGCTGT TTTCTGTCTG TCCCTAACAT

2051 GCCCTGTGAT TATGCGCAAA CAACACACCC AAGGGCAGAA CTTTGTTACT

2101 TAAACACCAT CCTGTTTGCT TCTTTCCTCA GGAACTGTGG CTGCACCATC
                                          T  V   A  A  P  S
2151 TGTCTTCATC TTCCCGCCAT CTGATGAGCA GTTGAAATCT GGAACTGCCT
       V  F  I   F  P  P   S  D  E  Q   L  K  S   G  T  A
2201 CTGTTGTGTG CCTGCTGAAT AACTTCTATC CCAGAGAGGC CAAAGTACAG
     S  V  V  C   L  L  N   N  F  Y   P  R  E  A   K  V  Q
2251 TGGAAGGTGG ATAACGCCCT CCAATCGGGT AACTCCCAGG AGAGTGTCAC
      W  K  V   D  N  A  L   Q  S  G   N  S  Q   E  S  V  T
2301 AGAGCAGGAC AGCAAGGACA GCACCTACAG CCTCAGCAGC ACCCTGACGC
       E  Q  D   S  K  D   S  T  Y  S   L  S  S   T  L  T
```

## Fig. 30 /3

2351 TGAGCAAAGC AGACTACGAG AAACACAAAG TCTACGCCTG CGAAGTCACC
L S K A D Y E K H K V Y A C E V T
2401 CATCAGGGCC TGAGCTCGCC CGTCACAAAG AGCTTCAACA GGGGAGAGTG
H Q G L S S P V T K S F N R G E C
2451 TTAGAGGGAG AAGTGCCCCC ACCTGCTCCT CAGTTCCAGC CTGACCCCCT
*
Psp5 II
2501 CCCATCCTTT GGCCTCTGAC CCTTTTTCCA CAG**GGGACCT** ACCCCTATTG

2551 CGGTCCTCCA GCTCATCTTT CACCTCACCC CCCTCCTCCT CCTTGGCTTT

2601 AATTATGCTA ATGTTGGAGG AGAATGAATA AATAAAGTGA ATCTTTGCAC

2651 CTGTGGTGGA TCTAATAAAA GATATTTATT TTCATTAGAT ATGTGTGTTG

2701 GTTTTTTGTG TGCAGTGCCT CTATCTGGAG GCCAGGTAGG GCTGGCCTTG

2751 GGGGAGGGGG AGGCCAGAAT GACTCCAAGA GCTACAGGAA GGCAGGTCAG

2801 AGACCCCACT GGACAAACAG TGGCTGGACT CTGCACCATA ACACACAATC

2851 AACAGGGGAG TGAGCTGGAA ATTTGCTAGC GAATTCTTGA AGACGAAAGG

2901 GCCTCGTGAT ACGCCTATTT TTATAGGTTA ATGTCATGAT AATAATGGTT

2951 TCTTAGACGT CAGGTGGCAC TTTTCGGGGA AATGTGCGCG GAACCCCTAT

3001 TTGTTTATTT TTCTAAATAC ATTCAAATAT GTATCCGCTC ATGAGACAAT

3051 AACCCTGATA AATGCTTCAA TAATATTGAA AAAGGAAGAG TATGAGTATT

3101 CAACATTTCC GTGTCGCCCT TATTCCCTTT TTTGCGGCAT TTTGCCTTCC

3151 TGTTTTTGCT CACCCAGAAA CGCTGGTGAA AGTAAAAGAT GCTGAAGATC

3201 AGTTGGGTGC ACGAGTGGGT TACATCGAAC TGGATCTCAA CAGCGGTAAG

3251 ATCCTTGAGA GTTTTCGCCC CGAAGAACGT TTTCCAATGA TGAGCACTTT

3301 TAAAGTTCTG CTATGTGGCG CGGTATTATC CCGTGTTGAC GCCGGGCAAG

3351 AGCAACTCGG TCGCCGCATA CACTATTCTC AGAATGACTT GGTTGAGTAC

3401 TCACCAGTCA CAGAAAAGCA TCTTACGGAT GGCATGACAG TAAGAGAATT

3451 ATGCAGTGCT GCCATAACCA TGAGTGATAA CACTGCGGCC AACTTACTTC
Pvu I
3501 TGACAA**CGAT CG**GAGGACCG AAGGAGCTAA CCGCTTTTTT GCACAACATG

3551 GGGGATCATG TAACTCGCCT TGATCGTTGG GAACCGGAGC TGAATGAAGC

Fig. 30 /4

3601 CATACCAAAC GACGAGCGTG ACACCACGAT GCCTGCAGCA ATGGCAACAA

3651 CGTTGCGCAA ACTATTAACT GGCGAACTAC TTACTCTAGC TTCCCGGCAA

3701 CAATTAATAG ACTGGATGGA GGCGGATAAA GTTGCAGGAC CACTTCTGCG

3751 CTCGGCCCTT CCGGCTGGCT GGTTTATTGC TGATAAATCT GGAGCCGGTG

3801 AGCGTGGGTC TCGCGGTATC ATTGCAGCAC TGGGGCCAGA TGGTAAGCCC

3851 TCCCGTATCG TAGTTATCTA CACGACGGGG AGTCAGGCAA CTATGGATGA

3901 ACGAAATAGA CAGATCGCTG AGATAGGTGC CTCACTGATT AAGCATTGGT

3951 AACTGTCAGA CCAAGTTTAC TCATATATAC TTTAGATTGA TTTAAAACTT

4001 CATTTTTAAT TTAAAAGGAT CTAGGTGAAG ATCCTTTTTG ATAATCTCAT

4051 GACCAAAATC CCTTAACGTG AGTTTTCGTT CCACTGAGCG TCAGACCCCG

4101 TAGAAAAGAT CAAAGGATCT TCTTGAGATC CTTTTTTTCT GCGCGTAATC

4151 TGCTGCTTGC AAACAAAAAA ACCACCGCTA CCAGCGGTGG TTTGTTTGCC

4201 GGATCAAGAG CTACCAACTC TTTTTCCGAA GGTAACTGGC TTCAGCAGAG

4251 CGCAGATACC AAATACTGTC CTTCTAGTGT AGCCGTAGTT AGGCCACCAC

4301 TTCAAGAACT CTGTAGCACC GCCTACATAC CTCGCTCTGC TAATCCTGTT

4351 ACCAGTGGCT GCTGCCAGTG GCGATAAGTC GTGTCTTACC GGGTTGGACT

4401 CAAGACGATA GTTACCGGAT AAGGCGCAGC GGTCGGGCTG AACGGGGGGT

4451 TCGTGCACAC AGCCCAGCTT GGAGCGAACG ACCTACACCG AACTGAGATA

4501 CCTACAGCGT GAGCTATGAG AAAGCGCCAC GCTTCCCGAA GGGAGAAAGG

4551 CGGACAGGTA TCCGGTAAGC GGCAGGGTCG GAACAGGAGA GCGCACGAGG

4601 GAGCTTCCAG GGGGAAACGC CTGGTATCTT TATAGTCCTG TCGGGTTTCG

4651 CCACCTCTGA CTTGAGCGTC GATTTTTGTG ATGCTCGTCA GGGGGGCGGA

4701 GCCTATGGAA AAACGCCAGC AACGCGGCCT TTTTACGGTT CCTGGCCTTT

BspLU11I

4751 TGCTGGCCTT TTGCTC**ACAT GT**TCTTTCCT GCGTTATCCC CTGATTCTGT

4801 GGATAACCGT ATTACCGCCT TTGAGTGAGC TGATACCGCT CGCCGCAGCC

Fig. 30 /5

```
4851  GAACGACCGA GCGCAGCGAG TCAGTGAGCG AGGAAGCGGA AGAGCGCCTG

4901  ATGCGGTATT TTCTCCTTAC GCATCTGTGC GGTATTTCAC ACCGCATATG
                                                      Bst1107I
4951  GTGCACTCTC AGTACAATCT GCTCTGATGC CGCATAGTTA AGCCAGTATA

5001  CACTCCGCTA TCGCTACGTG ACTGGGTCAT GGCTGCGCCC CGACACCCGC

5051  CAACACCCGC TGACGCGCCC TGACGGGCTT GTCTGCTCCC GGCATCCGCT

5101  TACAGACAAG CTGTGACCGT CTCCGGGAGC TGCATGTGTC AGAGGTTTTC

5151  ACCGTCATCA CCGAAACGCG CGAGGCAGCT GTGGAATGTG TGTCAGTTAG

5201  GGTGTGGAAA GTCCCCAGGC TCCCCAGCAG GCAGAAGTAT GCAAAGCATG

5251  CATCTCAATT AGTCAGCAAC CAGGCTCCCC AGCAGGCAGA AGTATGCAAA

5301  GCATGCATCT CAATTAGTCA GCAACCATAG TCCCGCCCCT AACTCCGCCC

5351  ATCCCGCCCC TAACTCCGCC CAGTTCCGCC CATTCTCCGC CCCATGGCTG
                                      Sfi I
5401  ACTAATTTTT TTTATTTATG CAGAGGCCGA GGCCGCCTCG GCCTCTGAGC
                                       Stu I/Avr II
5451  TATTCCAGAA GTAGTGAGGA GGCTTTTTTG GAGGCCTAGG CTTTTGCAAA

5501  AAGCTAGCTT CACGCTGCCG CAAGCACTCA GGGCGCAAGG GCTGCTAAAG

5551  GAAGCGGAAC ACGTAGAAAG CCAGTCCGCA GAAACGGTGC TGACCCCGGA

5601  TGAATGTCAG CTACTGGGCT ATCTGGACAA GGGAAAACGC AAGCGCAAAG

5651  AGAAAGCAGG TAGCTTGCAG TGGGCTTACA TGGCGATAGC TAGACTGGGC

5701  GGTTTTATGG ACAGCAAGCG AACCGGAATT GCCAGCTGGG GCGCCCTCTG

5751  GTAAGGTTGG GAAGCCCTGC AAAGTAAACT GGATGGCTTT CTTGCCGCCA
                                 Bgl II/Bcl I
5801  AGGATCTGAT GGCGCAGGGG ATCAAGATCT GATCAAGAGA CAGGATGAGG

5851  ATCGTTTCGC ATGATTGAAC AAGATGGATT GCACGCAGGT TCTCCGGCCG

5901  CTTGGGTGGA GAGGCTATTC GGCTATGACT GGGCACAACA GACAATCGGC

5951  TGCTCTGATG CCGCCGTGTT CCGGCTGTCA GCGCAGGGGC GCCCGGTTCT

6001  TTTGTCAAGG ACCGACCTGT CCGGTGCCCT GAATGAACTG CAGGACGAGG
                          Msc I
6051  CAGCGCGGCT ATCGTGGCTG GCCACGACGG GCGTTCCTTG CGCAGCTGTG
```

Fig. 30 /6

6101 CTCGACGTTG TCACTGAAGC GGGAAGGGAC TGGCTGCTAT TGGGCGAAGT

6151 GCCGGGGCAG GATCTCCTGT CATCTCACCT TGCTCCTGCC GAGAAAGTAT

6201 CCATCATGGC TGATGCAATG CGGCGGCTGC ATACGCTTGA TCCGGCTACC

6251 TGCCCATTCG ACCACCAAGC GAAACATCGC ATCGAGCGAG CACGTACTCG

6301 GATGGAAGCC GGTCTTGTCG ATCAGGATGA TCTGGACGAA GAGCATCAGG

6351 GGCTCGCGCC AGCCGAACTG TTCGCCAGGC TCAAGGCGCG CATGCCCGAC

6401 GGCGAGGATC TCGTCGTGAC CCATGGCGAT GCCTGCTTGC CGAATATCAT

6451 GGTGGAAAAT GGCCGCTTTT CTGGATTCAT CGACTGTGGC CGGCTGGGTG

Rsr II

6501 TGG**CGGACCG** CTATCAGGAC ATAGCGTTGG CTACCCGTGA TATTGCTGAA

6551 GAGCTTGGCG GCGAATGGGC TGACCGCTTC CTCGTGCTTT ACGGTATCGC

6601 CGCTCCCGAT TCGCAGCGCA TCGCCTTCTA TCGCCTTCTT GACGAGTTCT

Nsp V

6651 TCTGAGCGGG ACTCTGGGG**T TCGAA**ATGAC CGACCAAGCG ACGCCCAACC

6701 TGCCATCACG AGATTTCGAT TCCACCGCCG CCTTCTATGA AAGGTTGGGC

6751 TTCGGAATCG TTTTCCGGGA CGCCGGCTGG ATGATCCTCC AGCGCGGGGA

Sma I Nru I

6801 TCTCATGCTG GAGTTCTTCG CCCAC**CCCGG G**CTCGATCCC C**TCGCGA**GTT

6851 GGTTCAGCTG CTGCCTGAGG CTGGACGACC TCGCGGAGTT CTACCGGCAG

6901 TGCAAATCCG TCGGCATCCA GGAAACCAGC AGCGGCTATC CGCGCATCCA

6951 TGCCCCCGAA CTGCAGGAGT GGGGAGGCAC GATGGCCGCT TTGGTCCCGG

7001 ATCTTTGTGA AGGAACCTTA CTTCTGTGGT GTGACATAAT TGGACAAACT

7051 ACCTACAGAG ATTTAAAGCT CTAAGGTAAA TATAAAATTT TTAAGTGTAT

7101 AATGTGTTAA ACTACTGATT CTAATTGTTT GTGTATTTTA GATTCCAACC

7151 TATGGAACTG ATGAATGGGA GCAGTGGTGG AATGCCTTTA ATGAGGAAAA

7201 CCTGTTTTGC TCAGAAGAAA TGCCATCTAG TGATGATGAG GCTACTGCTG

7251 ACTCTCAACA TTCTACTCCT CCAAAAAAGA AGAGAAAGGT AGAAGACCCC

7301 AAGGACTTTC CTTCAGAATT GCTAAGTTTT TTGAGTCATG CTGTGTTTAG

**Fig. 30 /7**

7351 TAATAGAACT CTTGCTTGCT TTGCTATTTA CACCACAAAG GAAAAAGCTG

7401 CACTGCTATA CAAGAAAATT ATGGAAAAAT ATTCTGTAAC CTTTATAAGT

7451 AGGCATAACA GTTATAATCA TAACATACTG TTTTTTCTTA CTCCACACAG

7501 GCATAGAGTG TCTGCTATTA ATAACTATGC TCAAAAATTG TGTACCTTTA

7551 GCTTTTTAAT TTGTAAAGGG GTTAATAAGG AATATTTGAT GTATAGTGCC

7601 TTGACTAGAG ATCATAATCA GCCATACCAC ATTTGTAGAG GTTTTACTTG

7651 CTTTAAAAAA CCTCCCACAC CTCCCCCTGA ACCTGAAACA TAAAATGAAT

Mun I

7701 G**CAATTG**TTG TTGTTAACTT GTTTATTGCA GCTTATAATG GTTACAAATA

7751 AAGCAATAGC ATCACAAATT TCACAAATAA AGCATTTTTT TCACTGCATT

7801 CTAGTTGTGG TTTGTCCAAA CTCATCAATG TATCTTATCA TGTCTGGATC

7851 TAATAAAAGA TATTTATTTT CATTAGATAT GTGTGTTGGT TTTTTGTGTG

7901 CAGTGCCTCT ATCTGGAGGC CAGGTAGGGC TGGCCTTGGG GGAGGGGGAG

7951 GCCAGAATGA CTCCAAGAGC TACAGGAAGG CAGGTCAGAG ACCCCACTGG

8001 ACAAACAGTG GCTGGACTCT GCACCATAAC ACACAATCAA CAGGGGAGTG

8051 AGCTGGAAAT TTGCTAGC

Fig. 31

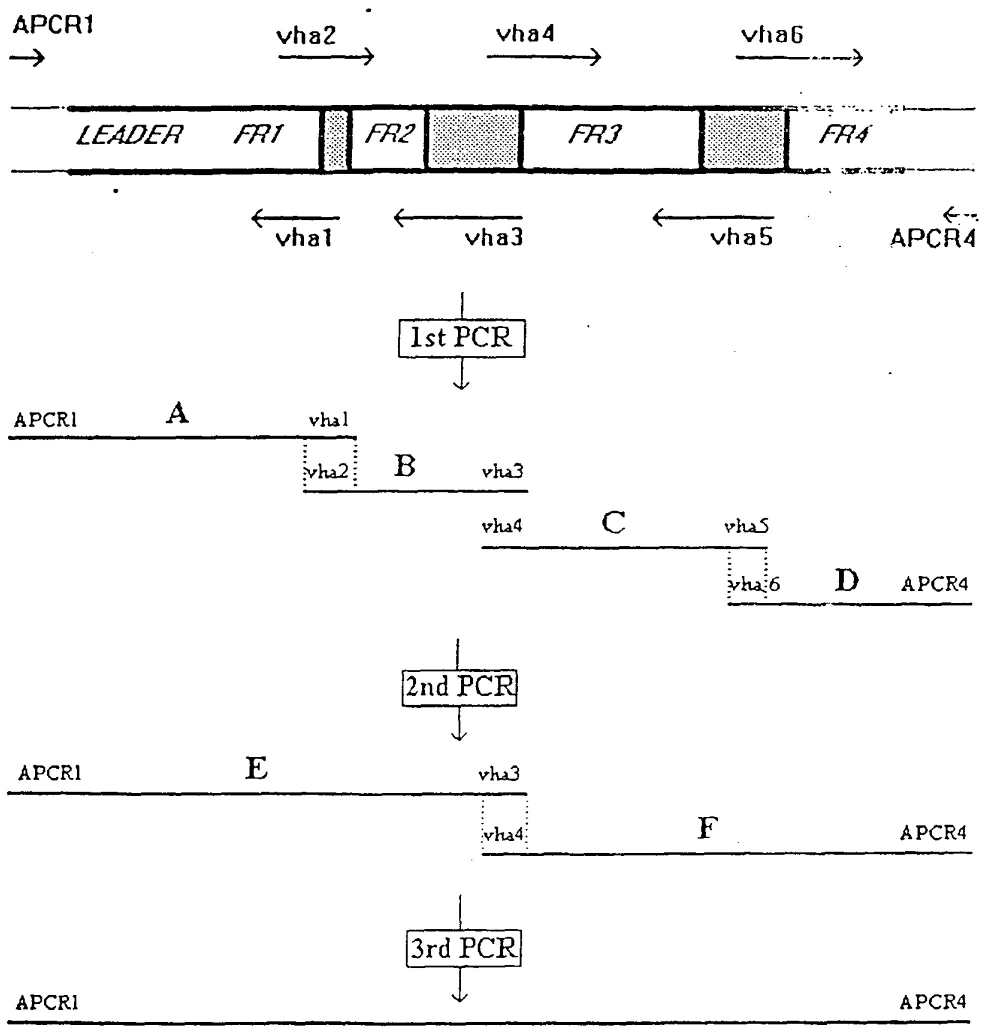
APCR1
vha2
vha4
vha6
LEADER
FR1
FR2
FR3
FR4
vha1
vha3
vha5
APCR4
1st PCR
APCR1
A
vha1
vha2
B
vha3
vha4
C
vha5
vha6
D
APCR4
2nd PCR
APCR1
E
vha3
vha4
F
APCR4
3rd PCR
APCR1
APCR4

## Fig. 32 /1

```
   1                                   10                                  19
   Q   V   Q   L   V   Q   S   G   A   E   V   K   K   P   G   A   S   V   K
A  CAG GTG CAA CTA GTG CAG TCC GGC GCC GAA GTG AAG AAA CCC GGT GCT TCC GTG AAA
   .   .   .   .   .   .   .   .   .   .   .   .   .   .   .   .   .   .   .
B  --- --- --- --- --- --- --- --- --- --- --- --- --- --- --- --- --- --- ---
   .   .   .   .   .   .   .   .   .   .   .   .   .   .   .   .   .   .   .
C  --- --- --- --- --- --- --- --- --- --- --- --- --- --- --- --- --- --- ---
   .   .   .   .   .   .   .   .   .   .   .   .   .   .   .   .   .   .   .
D  --- --- --- --- --- --- --- --- --- --- --- --- --- --- --- --- --- --- ---
   .   .   .   .   .   .   .   .   .   .   .   .   .   .   .   .   .   .   .
E  --- --- --- --- --- --- --- --- --- --- --- --- --- --- --- --- --- --- ---

   20                                      30      CDR1                    38
   V   S   C   K   T   S   R   Y   T   F   T  |E   Y   T   I   H  |W   V   R
A  GTC AGC TGT AAA ACT AGT AGA TAC ACC TTC ACT|GAA TAC ACC ATA CAC|TGG GTT AGA
   .   .   .   .   .   .   .   .   .   .   .  |.   .   .   .   .  |.   .   .
B  --- --- --- --- --- --- --- --- --- --- ---|--- --- --- --- ---|--- --- ---
   .   .   .   .   .   .   .   .   .   .   .  |.   .   .   .   .  |.   .   .
C  --- --- --- --- --- --- --- --- --- --- ---|--- --- --- --- ---|--- --- ---
   .   .   .   .   .   .   .   .   .   .   .  |.   .   .   .   .  |.   .   .
D  --- --- --- --- --- --- --- --- --- --- ---|--- --- --- --- ---|--- --- ---
   .   .   .   .   .   .   G   .   .   .   .  |.   .   .   .   .  |.   .   .
E  --- --- --- --- --- --- G-- --- --- --- ---|--- --- --- --- ---|--- --- ---

   39                                      49          52  A   53          56
   Q   A   P   G   Q   R   L   E   W   I   G  |G   I   N   P   N   N   G   I
A  CAG GCC CCT GGC CAA AGG CTG GAG TGG ATA GGA|GGT ATT AAT CCT AAC AAT GGT ATT
   .   .   .   .   .   .   .   .   .   .   .  |.   .   .   .   .   .   .   .
B  --- --- --- --- --- --- --- --- --- --- ---|--- --- --- --- --- --- --- ---
   .   .   .   .   .   .   .   .   .   .   .  |.   .   .   .   .   .   .   .
C  --- --- --- --- --- --- --- --- --- --- ---|--- --- --- --- --- --- --- ---
   .   .   .   .   .   .   .   .   .   .   .  |.   .   .   .   .   .   .   .
D  --- --- --- --- --- --- --- --- --- --- ---|--- --- --- --- --- --- --- ---
   .   .   .   .   .   .   .   .   .   .   .  |.   .   .   .   .   .   .   .
E  --- --- --- --- --- --- --- --- --- --- ---|--- --- --- --- --- --- --- ---

   57              CDR2                                70                  75
   P   N   Y   N   Q   K   F   K   G  |R   A   T   L   T   V   G   K   S   A
A  CCT AAC TAC AAC CAG AAG TTC AAG GGC|CGG GCC ACC TTG ACC GTA GGC AAG TCT GCC
   .   .   .   .   .   .   .   .   .  |.   .   .   .   .   .   .   .   .   .
B  --- --- --- --- --- --- --- --- ---|--- --- --- --- --- --- --- --- --- ---
   .   .   .   .   .   .   .   .   .  |.   V   .   I   .   .   D   T   .   .
C  --- --- --- --- --- --- --- --- ---|--- -T- --- A-C --- --- -A- -CC --- ---
   .   .   .   .   .   .   .   .   .  |.   V   .   I   .   .   D   T   .   .
D  --- --- --- --- --- --- --- --- ---|--- -T- --- A-C --- --- -A- -CC --- ---
   .   .   .   .   .   .   .   .   .  |.   V   .   I   .   .   D   T   .   .
E  --- --- --- --- --- --- --- --- ---|--- -T- --- A-C --- --- -A- -CC --- ---

   76                      82  A   B   C   83                              91
   S   T   A   Y   M   E   L   S   S   L   R   S   E   D   T   A   V   Y   Y
A  AGC ACC GCC TAC ATG GAA CTG TCC AGC CTG CGC TCC GAG GAC ACT GCA GTC TAC TAC
   .   .   .   .   .   .   .   .   .   .   .   .   .   .   .   .   .   .   F
B  --- --- --- --- --- --- --- --- --- --- --- --- --- --- --- --- --- --- -T-
   .   .   .   .   .   .   .   .   .   .   .   .   .   .   .   .   .   .   .
C  --- --- --- --- --- --- --- --- --- --- --- --- --- --- --- --- --- --- ---
   .   .   .   .   .   .   .   .   .   .   .   .   .   .   .   .   .   .   F.
D  --- --- --- --- --- --- --- --- --- --- --- --- --- --- --- --- --- --- -T-
   .   .   .   .   .   .   .   .   .   .   .   .   .   .   .   .   .   .   .
E  --- --- --- --- --- --- --- --- --- --- --- --- --- --- --- --- --- --- ---
```

## Fig. 32 /2

| | 92 | | | CDR3 | | | | | 100 | A | B | C | D | I | J | K | 101 | | 103 |
|---|---|---|---|---|---|---|---|---|---|---|---|---|---|---|---|---|---|---|---|
| | C | A | R | *R* | *R* | *I* | *A* | *Y* | *G* | *Y* | *D* | *E* | *G* | *H* | *A* | *M* | *D* | *Y* | W |
| A | TGC | GCC | AGA | ***AGA*** | ***AGA*** | ***ATC*** | ***GCC*** | ***TAT*** | ***GGT*** | ***TAC*** | ***GAC*** | ***GAG*** | ***GGC*** | ***CAT*** | ***GCT*** | ***ATG*** | ***GAC*** | ***TAC*** | TGG |
| | . | . | . | . | . | . | . | . | . | . | . | . | . | . | . | . | . | . | . |
| B | --- | --- | --- | --- | --- | --- | --- | --- | --- | --- | --- | --- | --- | --- | --- | --- | --- | --- | --- |
| | . | . | . | . | . | . | . | . | . | . | . | . | . | . | . | . | . | . | . |
| C | --- | --- | --- | --- | --- | --- | --- | --- | --- | --- | --- | --- | --- | --- | --- | --- | --- | --- | --- |
| | . | . | . | . | . | . | . | . | . | . | . | . | . | . | . | . | . | . | . |
| D | --- | --- | --- | --- | --- | --- | --- | --- | --- | --- | --- | --- | --- | --- | --- | --- | --- | --- | --- |
| | . | . | . | . | . | . | . | . | . | . | . | . | . | . | . | . | . | . | . |
| E | --- | --- | --- | --- | --- | --- | --- | --- | --- | --- | --- | --- | --- | --- | --- | --- | --- | --- | --- |

| | 104 | | | | | | | | | 113 |
|---|---|---|---|---|---|---|---|---|---|---|
| | G | Q | G | T | L | V | T | V | S | S |
| A | GGT | CAA | GGA | ACC | CTT | GTC | ACC | GTC | TCC | TCA |
| | . | . | . | . | . | . | . | . | . | . |
| B | --- | --- | --- | --- | --- | --- | --- | --- | --- | --- |
| | . | . | . | . | . | . | . | . | . | . |
| C | --- | --- | --- | --- | --- | --- | --- | --- | --- | --- |
| | . | . | . | . | . | . | . | . | . | . |
| D | --- | --- | --- | --- | --- | --- | --- | --- | --- | --- |
| | . | . | . | . | . | . | . | . | . | . |
| E | --- | --- | --- | --- | --- | --- | --- | --- | --- | --- |

## Fig. 33 /1

1 TTGAAGACGAAAGGGCCTCGTGATACGCCTATTTTTATAGGTTAATGTCATGATAATAAT

61 GGTTTCTTAGACGTCAGGTGGCACTTTTCGGGGAAATGTGCGCGGAACCCCTATTTGTTT

121 ATTTTTCTAAATACATTCAAATATGTATCCGCTCATGAGACAATAACCCTGATAAATGCT

181 TCAATAATATTGAAAAAGGAAGAGTATGAGTATTCAACATTTCCGTGTCGCCCTTATTCC

241 CTTTTTTGCGGCATTTTGCCTTCCTGTTTTTGCTCACCCAGAAACGCTGGTGAAAGTAAA

301 AGATGCTGAAGATCAGTTGGGTGCACGAGTGGGTTACATCGAACTGGATCTCAACAGCGG

361 TAAGATCCTTGAGAGTTTTCGCCCCGAAGAACGTTTTCCAATGATGAGCACTTTTAAAGT

421 TCTGCTATGTGGCGCGGTATTATCCCGTGTTGACGCCGGGCAAGAGCAACTCGGTCGCCG

481 CATACACTATTCTCAGAATGACTTGGTTGAGTACTCACCAGTCACAGAAAAGCATCTTAC

541 GGATGGCATGACAGTAAGAGAATTATGCAGTGCTGCCATAACCATGAGTGATAACACTGC

Pvu I

601 GGCCAACTTACTTCTGACAA**CGATCG**GAGGACCGAAGGAGCTAACCGCTTTTTTGCACAA

661 CATGGGGGATCATGTAACTCGCCTTGATCGTTGGGAACCGGAGCTGAATGAAGCCATACC

Fsp I

721 AAACGACGAGCGTGACACCACGATGCCTGCAGCAATGGCAACAACGT**TGCGCA**AACTATT

781 AACTGGCGAACTACTTACTCTAGCTTCCCGGCAACAATTAATAGACTGGATGGAGGCGGA

Fig. 33 /2

841 TAAAGTTGCAGGACCACTTCTGCGCTCGGCCCTTCCGGCTGGCTGGTTTATTGCTGATAA

901 ATCTGGAGCCGGTGAGCGTGGGTCTCGCGGTATCATTGCAGCACTGGGGCCAGATGGTAA

961 GCCCTCCCGTATCGTAGTTATCTACACGACGGGGAGTCAGGCAACTATGGATGAACGAAA

1021 TAGACAGATCGCTGAGATAGGTGCCTCACTGATTAAGCATTGGTAACTGTCAGACCAAGT

1081 TTACTCATATATACTTTAGATTGATTTAAAACTTCATTTTTAATTTAAAAGGATCTAGGT

1141 GAAGATCCTTTTTGATAATCTCATGACCAAAATCCCTTAACGTGAGTTTTCGTTCCACTG

1201 AGCGTCAGACCCCGTAGAAAAGATCAAAGGATCTTCTTGAGATCCTTTTTTTCTGCGCGT

1261 AATCTGCTGCTTGCAAACAAAAAAACCACCGCTACCAGCGGTGGTTTGTTTGCCGGATCA

1321 AGAGCTACCAACTCTTTTTCCGAAGGTAACTGGCTTCAGCAGAGCGCAGATACCAAATAC

1381 TGTCCTTCTAGTGTAGCCGTAGTTAGGCCACCACTTCAAGAACTCTGTAGCACCGCCTAC

1441 ATACCTCGCTCTGCTAATCCTGTTACCAGTGGCTGCTGCCAGTGGCGATAAGTCGTGTCT

1501 TACCGGGTTGGACTCAAGACGATAGTTACCGGATAAGGCGCAGCGGTCGGGCTGAACGGG

1561 GGGTTCGTGCACACAGCCCAGCTTGGAGCGAACGACCTACACCGAACTGAGATACCTACA

1621 GCGTGAGCTATGAGAAAGCGCCACGCTTCCCGAAGGGAGAAAGGCGGACAGGTATCCGGT

1681 AAGCGGCAGGGTCGGAACAGGAGAGCGCACGAGGGAGCTTCCAGGGGGAAACGCCTGGTA

1741 TCTTTATAGTCCTGTCGGGTTTCGCCACCTCTGACTTGAGCGTCGATTTTTGTGATGCTC

1801 GTCAGGGGGGCGGAGCCTATGGAAAAACGCCAGCAACGCGGCCTTTTTACGGTTCCTGGC

BspLU11I

1861 CTTTTGCTGGCCTTTTGCTC**ACATGT**TCTTTCCTGCGTTATCCCCTGATTCTGTGGATAA

1921 CCGTATTACCGCCTTTGAGTGAGCTGATACCGCTCGCCGCAGCCGAACGACCGAGCGCAG

1981 CGAGTCAGTGAGCGAGGAAGCGGAAGAGCGCCTGATGCGGTATTTTCTCCTTACGCATCT

2041 GTGCGGTATTTCACACCGCATATGGTGCACTCTCAGTACAATCTGCTCTGATGCCGCATA

Bst1107 I

2101 GTTAAGCCA**GTATAC**ACTCCGCTATCGCTACGTGACTGGGTCATGGCTGCGCCCCGACAC

2161 CCGCCAACACCCGCTGACGCGCCCTGACGGGCTTGTCTGCTCCCGGCATCCGCTTACAGA

2221 CAAGCTGTGACCGTCTCCGGGAGCTGCATGTGTCAGAGGTTTTCACCGTCATCACCGAAA

2281 CGCGCGAGGCAGCATGCATCTCAATTAGTCAGCAACCATAGTCCCGCCCCTAACTCCGCC

2341 CATCCCGCCCCTAACTCCGCCCAGTTCCGCCCATTCTCCGCCCCATGGCTGACTAATTTT

Sfi I

2401 TTTTATTTATGCAGAGGCCGA**GGCC**GCCTC**GGCC**TCTGAGCTATTCCAGAAGTAGTGAGG

Stu I/Avr II

2461 AGGCTTTTTTGG**AGGCCTAGG**CTTTTGCAAAAAGCTAGCTTACAGCTCAGGGCTGCGATT

Fig. 33 /3

2521 TCGCGCCAAACTTGACGGCAATCCTAGCGTGAAGGCTGGTAGGATTTTATCCCCGCTGCC

2581 ATCATGGTTCGACCATTGAACTGCATCGTCGCCGTGTCCCAAAATATGGGGATTGGCAAG

2641 AACGGAGACCTACCCTGGCCTCCGCTCAGGAACGAGTTCAAGTACTTCCAAAGAATGACC

2701 ACAACCTCTTCAGTGGAAGGTAAACAGAATCTGGTGATTATGGGTAGGAAAACCTGGTTC

2761 TCCATTCCTGAGAAGAATCGACCTTTAAAGGACAGAATTAATATAGTTCTCAGTAGAGAA

2821 CTCAAAGAACCACCACGAGGAGCTCATTTTCTTGCCAAAAGTTTGGATGATGCCTTAAGA

2881 CTTATTGAACAACCGGAATTGGCAAGTAAAGTAGACATGGTTTGGATAGTCGGAGGCAGT

2941 TCTGTTTACCAGGAAGCCATGAATCAACCAGGCCACCTCAGACTCTTTGTGACAAGGATC

3001 ATGCAGGAATTTGAAAGTGACACGTTTTTCCCAGAAATTGATTTGGGGAAATATAAACTT

3061 CTCCCAGAATACCCAGGCGTCCTCTCTGAGGTCCAGGAGGAAAAAGGCATCAAGTATAAG

3121 TTTGAAGTCTACGAGAAGAAAGACTAACAGGAAGATGCTTTCAAGTTCTCTGCTCCCCTC

Bgl II

3181 CTAAAGCTATGCATTTTTATAAGACCATGGGACTTTTGCTGGCTTT**AGATCT**TTGTGAAG

3241 GAACCTTACTTCTGTGGTGTGACATAATTGGACAAACTACCTACAGAGATTTAAAGCTCT

3301 AAGGTAAATATAAAATTTTTAAGTGTATAATGTGTTAAACTACTGATTCTAATTGTTTGT

3361 GTATTTTAGATTCCAACCTATGGAACTGATGAATGGGAGCAGTGGTGGAATGCCTTTAAT

3421 GAGGAAAACCTGTTTTGCTCAGAAGAAATGCCATCTAGTGATGATGAGGCTACTGCTGAC

3481 TCTCAACATTCTACTCCTCCAAAAAAGAAGAGAAAGGTAGAAGACCCCAAGGACTTTCCT

3541 TCAGAATTGCTAAGTTTTTTGAGTCATGCTGTGTTTAGTAATAGAACTCTTGCTTGCTTT

3601 GCTATTTACACCACAAAGGAAAAAGCTGCACTGCTATACAAGAAAATTATGGAAAAATAT

3661 TCTGTAACCTTTATAAGTAGGCATAACAGTTATAATCATAACATACTGTTTTTTCTTACT

3721 CCACACAGGCATAGAGTGTCTGCTATTAATAACTATGCTCAAAAATTGTGTACCTTTAGC

3781 TTTTTAATTTGTAAAGGGGTTAATAAGGAATATTTGATGTATAGTGCCTTGACTAGA**GAT**

BsaB I

3841 **CATAATC**AGCCATACCACATTTGTAGAGGTTTTACTTGCTTTAAAAAACCTCCCACACCT

Mun I

3901 CCCCCTGAACCTGAAACATAAAATGAATG**CAATTG**TTGTTGTTAACTTGTTTATTGCAGC

3961 TTATAATGGTTACAAATAAAGCAATAGCATCACAAATTTCACAAATAAAGCATTTTTTTC

4021 ACTGCATTCTAGTTGTGGTTTGTCCAAACTCATCAATGTATCTTATCATGTCTGGATCTA

4081 ATAAAAGATATTTATTTTCATTAGATATGTGTGTTGGTTTTTTGTGTGCAGTGCCTCTAT

4141 CTGGAGGCCAGGTAGGGCTGGCCTTGGGGGAGGGGGAGGCCAGAATGACTCCAAGAGCTA

**Fig. 33 /4**

4201 CAGGAAGGCAGGTCAGAGACCCCACTGGACAAACAGTGGCTGGACTCTGCACCATAACAC

EcoR I<br>
4261 ACAATCAACAGGGGAGTGAGCTGGAAATTTGCTAGC**GAATTC**cagcacactggcggccgt

(Spe I)<br>
4321 t**ACTAGT**TATTAATAGTAATCAATTACGGGGTCATTAGTTCATAGCCCATATATGGAGTT

4381 CCGCGTTACATAACTTACGGTAAATGGCCCGCCTGGCTGACCGCCCAACGACCCCCGCCC

4441 ATTGACGTCAATAATGACGTATGTTCCCATAGTAACGCCAATAGGGACTTTCCATTGACG

4501 TCAATGGGTGGAGTATTTACGGTAAACTGCCCACTTGGCAGTACATCAAGTGTATCATAT

4561 GCCAAGTACGCCCCCTATTGACGTCAATGACGGTAAATGGCCCGCCTGGCATTATGCCCA

SnaB I<br>
4621 GTACATGACCTTATGGGACTTTCCTACTTGGCAGTACATC**TACGTA**TTAGTCATCGCTAT

4681 TACCATGGTGATGCGGTTTTGGCAGTACATCAATGGGCGTGGATAGCGGTTTGACTCACG

4741 GGGATTTCCAAGTCTCCACCCCATTGACGTCAATGGGAGTTTGTTTTGGCACCAAAATCA

4801 ACGGGACTTTCCAAAATGTCGTAACAACTCCGCCCCATTGACGCAAATGGGCGGTAGGCG

4861 TGTACGGTGGGAGGTCTATATAAGCAGAGCTCGTTTAGTGAACCGTCAGATCGCCTGGAG

4921 ACGCCATCCACGCTGTTTTGACCTCCATAGAAGACACCGGGACCGATCCAGCCTCCGCGG

4981 CCGGGAACGGTGCATTGGAACGCGGATTCCCCGTGCCAAGAGTGACGTAAGTACCGCCTA

5041 TAGAGTCTATAGGCCCACCCCCTTGGCTTCTTATGCATGCTATACTGTTTTTGGCTTGGG

Bpu1102I<br>
5101 GTCTATACACCCCCGCTTCCTCATGTTATAGGTGATGGTATA**GCTTAGC**CTATAGGTGTG

Xcm I<br>
5161 GGTTATTGACCATTATTGA**CCACTCCCCTATTGG**TGACGATACTTTCCATTACTAATCCA

5221 TAACATGGCTCTTTGCCACAACTCTCTTTATTGGCTATATGCCAATACACTGTCCTTCAG

5281 AGACTGACACGGACTCTGTATTTTTACAGGATGGGGTCTCATTTATTATTTACAAATTCA

5341 CATATACAACACCACCGTCCCCAGTGCCCGCAGTTTTTATTAAACATAACGTGGGATCTC

BspE I<br>
5401 CACGCGAATCTCGGGTACGTGT**TCCGGA**CATGGGCTCTTCTCCGGTAGCGGCGGAGCTTC

5461 TACATCCGAGCCCTGCTCCCATGCCTCCAGCGACTCATGGTCGCTCGGCAGCTCCTTGCT

5521 CCTAACAGTGGAGGCCAGACTTAGGCACAGCACGATGCCCACCACCACCAGTGTGCCGCA

5581 CAAGGCCGTGGCGGTAGGGTATGTGTCTGAAAATGAGCTCggggagcgggcttgcaccgc

(Pvu II)<br>
5641 tgacgcatttggaagacttaaggcagcggcagaagaagatgcagg**cagctg**agttgttgt

5701 gttctgataagagtcagaggtaactcccgttgcggtgctgttaacggtggagggcagtgt

5761 agtctgagcagtactcgttgctgccgcgcgcgccaccagacataatagctgacagactaa

Mlu I<br>
5821 cagactgttcctttccatgggtcttttctgcagtcaccgtccttgac**ACGCGT**CTCGGG**A**

**Fig. 33 /5**

```
    Hind III
5881 AGCTTGCCGCCACCATGGACTGGACCTGGCGCGTGTTTTGCCTGCTCGCCGTGGCTCCTG
                    M   D   W   T   W   R   V   F   C   L   L   A   V   A   P

5941 GGGCCCACAGCCAGGTGCAACTGGTGCAGTCCGGCGCCGAAGTGAAGAAACCCGGTGCTT
     G   A   H   S   Q   V   Q   L   V   Q   S   G   A   E   V   K   K   P   G   A
                (Pvu II)   (Spe I)
6001 CCGTGAAAGTCAGCTGTAAAACTAGTAGATACACCTTCACTGAATACACCATACACTGGG
     S   V   K   V   S   C   K   T   S   R   Y   T   F   T   E   Y   T   I   H   W
                Msc I                                      CDR 1
6061 TTAGACAGGCCCCTGGCCAAAGGCTGGAGTGGATAGGAGGTATTAATCCTAACAATGGTA
     V   R   Q   A   P   G   Q   R   L   E   W   I   G   G   I   N   P   N   N   G

6121 TTCCTAACTACAACCAGAAGTTCAAGGGCCGGGCCACCTTGACCGTAGGCAAGTCTGCCA
     I   P   N   Y   N   Q   K   F   K   G   R   A   T   L   T   V   G   K   S   A
         CDR 2
6181 GCACCGCCTACATGGAACTGTCCAGCCTGCGCTCCGAGGACACTGCAGTCTACTACTGCG
     S   T   A   Y   M   E   L   S   S   L   R   S   E   D   T   A   V   Y   Y   C

6241 CCAGAAGAAGAATCGCCTATGGTTACGACGAGGGCCATGCTATGGACTACTGGGGTCAAG
     A   R   R   R   I   A   Y   G   Y   D   E   G   H   A   M   D   Y   W   G   Q
                     CDR 3        BamH I
6301 GAACCCTTGTCACCGTCTCCTCAGGTGAGTGGATCCTCTGCGCCTGGGCCCAGCTCTGTC
     G   T   L   V   T   V   S   S

6361 CCACACCGCGGTCACATGGCACCACCTCTCTTGCAGCCTCCACCAAGGGCCCATCGGTCT
                                                 S   T   K   G   P   S   V

6421 TCCCCCTGGCACCCTCCTCCAAGAGCACCTCTGGGGGCACAGCGGCCCTGGGCTGCCTGG
     F   P   L   A   P   S   S   K   S   T   S   G   G   T   A   A   L   G   C   L
                        Age I
6481 TCAAGGACTACTTCCCCGAACCGGTGACGGTGTCGTGGAACTCAGGCGCCCTGACCAGCG
     V   K   D   Y   F   P   E   P   V   T   V   S   W   N   S   G   A   L   T   S

6541 GCGTGCACACCTTCCCGGCTGTCCTACAGTCCTCAGGACTCTACTCCCTCAGCAGCGTGG
     G   V   H   T   F   P   A   V   L   Q   S   S   G   L   Y   S   L   S   S   V
    BstE II
6601 TGACCGTGCCCTCCAGCAGCTTGGGCACCCAGACCTACATCTGCAACGTGAATCACAAGC
     V   T   V   P   S   S   S   L   G   T   Q   T   Y   I   C   N   V   N   H   K

6661 CCAGCAACACCAAGGTGGACAAGAAAGTTGAGCCCAAATCTTGTGACAAAACTCACACAT
     P   S   N   T   K   V   D   K   K   V   E   P   K   S   C   D   K   T   H   T

6721 GCCCACCGTGCCCAGCACCTGAACTCCTGGGGGGACCGTCAGTCTTCCTCTTCCCCCCAA
     C   P   P   C   P   A   P   E   L   L   G   G   P   S   V   F   L   F   P   P

6781 AACCCAAGGACACCCTCATGATCTCCCGGACCCCTGAGGTCACATGCGTGGTGGTGGACG
     K   P   K   D   T   L   M   I   S   R   T   P   E   V   T   C   V   V   V   D

6841 TGAGCCACGAAGACCCTGAGGTCAAGTTCAACTGGTACGTGGACGGCGTGGAGGTGCATA
     V   S   H   E   D   P   E   V   K   F   N   W   Y   V   D   G   V   E   V   H

6901 ATGCCAAGACAAAGCCGCGGGAGGAGCAGTACAACAGCACGTACCGGGTGGTCAGCGTCC
     N   A   K   T   K   P   R   E   E   Q   Y   N   S   T   Y   R   V   V   S   V
```

**Fig. 33 /6**

```
6961 TCACCGTCCTGCACCAGGACTGGCTGAATGGCAAGGAGTACAAGTGCAAGGTCTCCAACA
      L  T  V  L  H  Q  D  W  L  N  G  K  E  Y  K  C  K  V  S  N

7021 AAGCCCTCCCAGCCCCCATCGAGAAAACCATCTCCAAAGCCAAAGGGCAGCCCCGAGAAC
      K  A  L  P  A  P  I  E  K  T  I  S  K  A  K  G  Q  P  R  E

7081 CACAGGTGTACACCCTGCCCCCATCCCGGGAGGAGATGACCAAGAACCAGGTCAGCCTGA
      P  Q  V  Y  T  L  P  P  S  R  E  E  M  T  K  N  Q  V  S  L

7141 CCTGCCTGGTCAAAGGCTTCTATCCCAGCGACATCGCCGTGGAGTGGGAGAGCAATGGGC
      T  C  L  V  K  G  F  Y  P  S  D  I  A  V  E  W  E  S  N  G

7201 AGCCGGAGAACAACTACAAGACCACGCCTCCCGTGCTGGACTCCGACGGCTCCTTCTTCC
      Q  P  E  N  N  Y  K  T  T  P  P  V  L  D  S  D  G  S  F  F

7261 TCTACAGCAAGCTCACCGTGGACAAGAGCAGGTGGCAGCAGGGGAACGTCTTCTCATGCT
      L  Y  S  K  L  T  V  D  K  S  R  W  Q  Q  G  N  V  F  S  C

7321 CCGTGATGCATGAGGCTCTGCACAACCACTACACGCAGAAGAGCCTCTCCCTGTCTCCGG
      S  V  M  H  E  A  L  H  N  H  Y  T  Q  K  S  L  S  L  S  P
                         NgoM I
7381 GTAAATGAGTGCGACGGCCGGCAAGCCCCGCTCCCCGGGCTCTCGCGGTCGCACGAGGAT
      G  K  *
7441 GCTTGGCACGTACCCCCTGTACATACTTCCCGGGCGCCCAGCATGGAAATAAAGCACCGG

7501 ATCTAATAAAAGATATTTATTTTCATTAGATATGTGTGTTGGTTTTTTGTGTGCAGTGCC

7561 TCTATCTGGAGGCCAGGTAGGGCTGGCCTTGGGGGAGGGGGAGGCCAGAATGACTCCAAG

7621 AGCTACAGGAAGGCAGGTCAGAGACCCCACTGGACAAACAGTGGCTGGACTCTGCACCAT

7681 AACACACAATCAACAGGGGAGTGAGCTGGaaatttgctagcgaattaattc 7731
```

**Fig. 34 A**

INTRON

3' end V gene ------------------------------------------------------------------ 5' end of CH1

ACC GTC TCC T*CA G::GTGAGT*<u>GGATCC</u>-(N)$_{48}$-*CCTCTCTTGCAG::C*C-

T V S S ***splice donor site*** BamHI ***splice acceptor site***

-TCC ACC AAG GGC

S T K G

⇓

ACC GTC TCC T*CA G::::C*C TCC ACC AAG GGC

T V S S S T K G

⇓

ACC GTC TCC TCA **GCC** TCC ACC AAG GGC

T V S S **A** S T K G

**Fig. 34 B**

INTRON

3' end V gene ---------------------------------------------------- 5' end Kappa constant

GAA ATA AA*A* ***C::GTGAGT***<u>GGATCC</u>-$(N)_{108}$-***CTTCTTTCCTCAG::G***A-

E I K ***splice donor site***<u>BamHI</u> ***splice acceptor site***

-ACT GTG GCT GCA

T V A A

⇓

GAA ATA AA*A* ***C::::G***A ACT GTG GCT GCA

E I K T V A A

⇓

GAA ATA AAA **CGA** ACT GTG GCT GCA

E I K **R** T V A A

**Fig. 35**

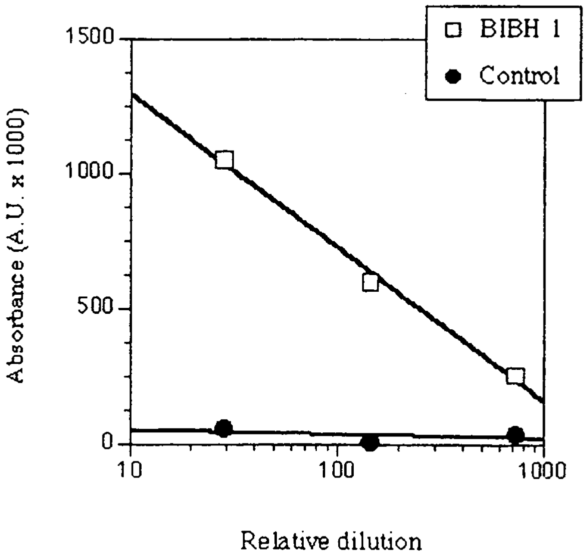

Fig. 36

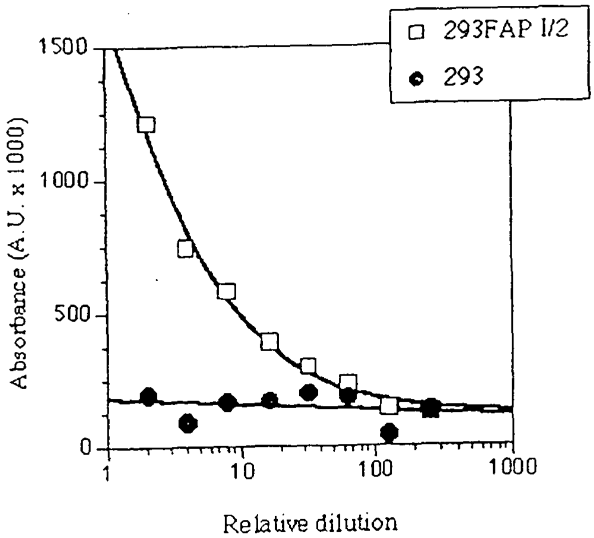
293FAP I/2
293
1500
1000
500
0
Absorbance (A.U. x 1000)
1
10
100
1000
Relative dilution

Fig. 37

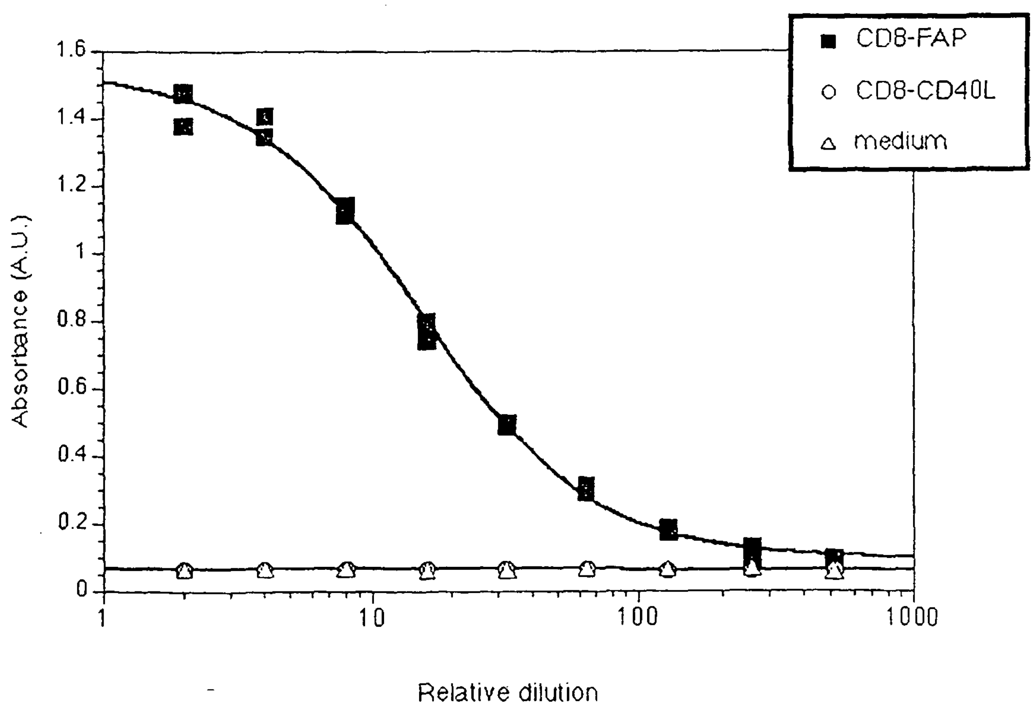
CD8-FAP
CD8-CD40L
medium
1.6
1.4
1.2
1
0.8
0.6
0.4
0.2
0
Absorbance (A.U.)
1
10
100
1000
Relative dilution